(19) Europäisches Patentamt
European Patent Office
Office européen des brevets

(11) EP 2 287 317 B1

(12) # EUROPEAN PATENT SPECIFICATION

(45) Date of publication and mention of the grant of the patent:
**22.10.2014 Bulletin 2014/43**

(21) Application number: **10178368.6**

(22) Date of filing: **15.01.2004**

(51) Int Cl.:
*C12N 15/54* (2006.01) *C12N 15/55* (2006.01)
*C12N 9/10* (2006.01) *C12N 9/18* (2006.01)
*C12N 11/00* (2006.01) *C12P 7/62* (2006.01)
*C12P 7/64* (2006.01)

(54) **Method of producing a protein ester or a protein subunit ester**

Verfahren zur Herstellung eines Proteinesters oder eines Proteinuntereinheitsesters

Méthode de production d'un ester de protéine ou d'un ester de sous-unité protéique

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority:
**17.01.2003 GB 0301117**
**17.01.2003 GB 0301118**
**17.01.2003 GB 0301119**
**17.01.2003 GB 0301120**
**17.01.2003 GB 0301122**
**17.01.2003 GB 0301121**
**23.07.2003 US 489441 P**
**24.12.2003 GB 0330016**

(43) Date of publication of application:
**23.02.2011 Bulletin 2011/08**

(83) **Declaration under Rule 32(1) EPC (expert solution)**

(62) Document number(s) of the earlier application(s) in accordance with Art. 76 EPC:
**04702392.4 / 1 599 278**

(73) Proprietor: **DuPont Nutrition Biosciences ApS
1001 Copenhagen K. (DK)**

(72) Inventors:
- **de Kreij, Arno
1206 Genève (CH)**
- **Madrid, Susan Mampusti
Millbrae, CA 94030 (US)**
- **Mikkelsen, Jørn Dalgaard
2650 Hvidovre (DK)**
- **Søe, Jørn Borch
8381 Tilst (DK)**

(74) Representative: **Williams, Aylsa
D Young & Co LLP
120 Holborn
London EC1N 2DY (GB)**

(56) References cited:
**EP-A- 1 275 711 WO-A1-00/05396**
**WO-A1-91/06661 US-A- 6 066 482**

- **UPTON C ET AL: "A new family of lipolytic enzymes?", TIBS TRENDS IN BIOCHEMICAL SCIENCES, ELSEVIER PUBLICATION, CAMBRIDGE, EN, vol. 20, no. 5, May 1995 (1995-05), pages 178-179, XP004222260, ISSN: 0968-0004**
- **BRUMLIK MICHAEL J ET AL: "Identification of the catalytic triad of the lipase/acyltransferase from Aeromonas hydrophila", JOURNAL OF BACTERIOLOGY, vol. 178, no. 7, 1996, pages 2060-2064, XP002315734, ISSN: 0021-9193**
- **HILTON S ET AL: "PURIFICATION AND SPECTRAL STUDY OF A MICROBIAL FATTY ACYLTRANSFERASE ACTIVATION BY LIMITED PROTEOLYSIS", BIOCHEMISTRY, vol. 29, no. 38, 1990, pages 9072-9078, XP002337706, ISSN: 0006-2960 -& DATABASE UniProt [Online] 1 July 1989 (1989-07-01), "Phosphatidylcholine-sterol acyltransferase precursor (EC 2.3.1.43) (Glycerophospholipid-cholesterol acyltransferase) (GCAT).", XP002337710, retrieved from EBI accession no. UNIPROT: GCAT_AERHY Database accession no. P10480**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 287 317 B1

- "USE OF LIPOLYTIC ENZYME FROM AEROMONAS IN DETERGENTS", RESEARCH DISCLOSURE, KENNETH MASON PUBLICATIONS, WESTBOURNE, GB, no. 390, October 1996 (1996-10), pages 661-662, XP000639927, ISSN: 0374-4353

## Description

### FIELD OF INVENTION

The present invention relates to a method for the bioconversion of lipids to produce a protein ester and/or a protein subunit ester by use of a lipid acyltransferase.

The present invention further relates to the use of a lipid acyltransferase to bioconvert a lipid into a protein and/or a protein subunit ester.

The present invention further relates to the use of an immobilised lipid acyltransferase- as defined herein in methods and uses of the present invention.

### TECHNICAL BACKGROUND

Lipases have been extensively used in bioconversion of lipids to make high value products, for example sugar esters, for use in a wide range of industries, including the food and/or feed industries, the cosmetics and/or skin care industries, the oleochemical industry and the pharmaceutical industry.

When bioconversion processes require hydrolysis of lipid substrates, lipolytic enzymes can be used in high water environments. However, when bioconversion processes require interesterification or transesterification reactions such as by alcoholysis the use of lipases in high water environments can be detrimental due to unwanted hydrolysis reactions, which result in unwanted bioproducts and/or lower yields of the bioconversion product.

Typically, bioconversion processes requiring interesterification and/or transesterification have utilised lipases in non-water environments such as in oil systems and/or in organic solvent systems such as in butanol, methanol or hexane. Such systems provide an environment in which both the polar acceptor molecule and the lipid donor molecule can be at least partially solubilised, and the lipase has sufficient enzyme activity. Although a small amount of water is required for any enzymatic activity, the amount of water is strictly maintained at a low level to avoid hydrolytic activity of the enzyme.

Conventionally sugar esters, protein esthers or hydroxyacid esters have been produced by chemical synthesis using inorganic catalysts. Convention bioconversion processes for the production of sugar esters or hydroxyacid esters utilise lipases in organic solvent environments or supercritical fluids where there is only a low amount of (if any) water present.

Lecointe et al Biotechnology Letters, Vol 18., No. 8 (August), pp869-874 disclose a study of a number of lipase enzymes and their activity in an aqueous media on the production of methyl ester or butyl ester from methanol and butanol, respectively. Lecointe *et al* teach a lipase/acyltransferase from *Candida parapsilosis* which as methanol or butanol concentrations increased showed a reduced hydrolysis activity and an enhanced capability of the enzyme to produce methyl ester and butyl ester. The use of a lipase/acyltransferase from *C. parapsilosis* in the production of fatty hydroxamic acid is taught in Vaysse et al J. of Biotechnology 53 (1997) 41-46.

WO 00/05396 discloses the use of a conversion agent to prepare from a food material a food stuff comprising at least one functional ingredient, wherein the at least one functional ingredient has been generated from at least one constituent of the food material by the conversion agent.

Lipase:cholesterol acyltransferases have been known for some time (see for example Buckley - Biochemistry 1983, 22, 5490-5493). In particular, glycerophospholipid:cholesterol acyl transferases (often referred to as GCATs) have been found, which like the plant and/or mammalian lecithin:cholesterol acyltransferases (LCATs), will catalyse fatty acid transfer between phosphatidylcholine and cholesterol.

Upton and Buckley (TIBS 20, May 1995 p 178-179) and Brumlik and Buckley (J. of Bacteriology Apr. 1996 p 2060-2064) teach a lipase/acyltransferase from *Aeromonas hydrophila* which has the ability to carry out acyl transfer to alcohol acceptors in an aqueous media.

### SUMMARY ASPECTS OF THE PRESENT INVENTION

According to a first aspect of the present invention there is provided a method of producing a protein ester and/or a protein subunit ester, which method comprises admixing an acyl donor, an acyl acceptor and water to produce a high water environment comprising 5-98% water, wherein said acyl donor is a lipid substrate selected from one or more of the group consisting of a phospholipid, a lysophospholipid, a triacylglyceride, a diglyceride, a glycolipid or a lysoglycolipid and said acyl acceptor is a protein and/or a protein subunit; and contacting the admixture with a lipid acyltransferase, such that said lipid acyltransferase catalyses one or both of the following reactions: alcoholysis or transesterification wherein the lipid acyltransferase is one which when tested using the Transferase Assay in Buffered Substrate has at least 2% acyltransferase activity; said transferase assay comprising the steps of:

i) dissolving 450mg phosphatidylcholine and 50mg cholesterol in chloroform, evaporating to dryness under vacuum;
ii) transferring 300mg cholesterol/phosphatidylcholine mixture to a Wheaton glass, adding 15ml 50mM HEPES buffer pH 7 and dispersing the lipid in the buffer during agitation;
iii) heating the substrate to 35° C during mixing with a magnetic stirrer and adding 0.25ml enzyme solution;
iv) taking samples of 2 ml at 0, 5, 10, 15, 25, 40 and 60 minutes reaction time and immediately stopping the enzyme reaction by the addition of 25 μl 4M HCl to acidify the free fatty acid;
v) adding 3ml chloroform and shaking vigorously for 30 seconds; centrifuging and isolating 2ml of the chloroform phase, filtering through a 0.45 μm filter into a 10ml tared Dram glass;
vi) evaporating the chloroform under a stream of nitrogen at 60°C, and scaling the samples;
vii) analysing the extracted lipid by GLC.

In a further aspect the present invention provides use of a lipid acyltransferase to produce a protein ester and/or a protein subunit ester by catalysis of one or both of alcoholysis or transesterification in an admixture of an acyl donor, an acyl acceptor and water, which admixture comprises 5-98% water, wherein said acyl donor is a lipid substrate selected from one or more of the group consisting of a phospholipid, a lysophospholipid, a triacylglyceride, a diglyceride, a glycolipid or a lysoglycolipid and said acyl acceptor is a protein and/or a protein subunit, wherein the lipid acyltransferase is one which when tested using the Transferase Assay in Buffered Substrate has at least 2% acyltransferase activity; said transferase assay comprising the steps of:

i) dissolving 450mg phosphatidylcholine and 50mg cholesterol in chloroform, evaporating to dryness under vacuum;
ii) transferring 300mg cholesterol/phosphatidylcholine mixture to a Wheaton glass, adding 15ml 50mM HEPES buffer pH 7 and dispersing the lipid in the buffer during agitation;
iii) heating the substrate to 35°C during mixing with a magnetic stirrer and adding 0.25ml enzyme solution;
iv) taking samples of 2 ml at 0, 5, 10, 15, 25, 40 and 60 minutes reaction time and immediately stopping the enzyme reaction by the addition of 25μl 4M HCl to acidify the free fatty acid;
v) adding 3ml chloroform and shaking vigorously for 30 seconds, centrifuging and isolating 2ml of the chloroform phase, filtering through a 0.45μm filter into a 10ml tared Dram glass;
vi) evaporating the chloroform under a stream of nitrogen at 60°C, and scaling the samples;
vii) analysing the extracted lipid by GLC.

A protein ester and/or a protein subunit ester may be produced by a method according to the present invention.
A pharmaceutical, a cosmetic, a foodstuff, a feedstuff, a paint comprising a protein ester and/or a protein subunit ester may be produced by a method according to the present invention.
An immobilised lipid acyltransferase enzyme as defined herein may be used in methods and uses of the present invention.

DETAILED ASPECTS OF THE PRESENT INVENTION

The term "lipid acyltransferase" as used herein means an enzyme which as well as having lipase activity (generally classified as E.C. 3.1.1.x in accordance with the Enzyme Nomenclature Recommendations (1992) of the Nomenclature Committee of the International Union of Biochemistry and Molecular Biology) also has acyltransferase activity (generally classified as E.C. 2.3.1.x), whereby the enzyme is capable of transferring an acyl group from a lipid to one or more of the following acceptor substrates: a carbohydrate; a protein; a protein subunit or a hydroxy acid.
Preferably, the "acyl acceptor" according to the present invention is not water.
The enzyme is capable of transferring an acyl group from a lipid substrate to a protein and/or a protein subunit.
Preferably the protein sub-unit is one or more of the following: an amino acid, a protein hydrolysate, a peptide, a dipeptide, an oligopeptide, a polypeptide.
Suitable proteins may be one or more of the following: proteins found in a food product, for example in a dairy product and/or a meat product. By way of example only, suitable proteins may be those found in curd or whey, such as lactoglobulin. Other suitable proteins include ovalbumin (from egg), gliadin, glutenin, puroindoline, wheat protein, lipid transfer proteins from grains, myosin from meat, or the following milk proteins: caseins, lactalbumins and lactoferrins.
Suitably in the protein or protein subunit the acyl acceptor may be one or more of the following constituents of the protein or protein subunit: a serine, a threonine, a tyrosine or a cysteine.
When the protein subunit is an amino acid, suitably the amino acid may be any amino acid. Preferably the amino acid is one or more of a serine, a threonine, a tyrosine or a cysteine for example.
In one aspect, the lipid acyltransferase may, as well as being able to transfer an acyl group from a lipid substrate to a protein and/or a protein subunit, the lipid acyltranferase is additionally able to transfer the acyl group from a lipid to one or more of the following: a sterol and/or a stanol, in particular a phytosterol and/or a phytostanol, a carbohydrate or a hydroxy acid.

Suitably, when the lipid substrate is a phospholipid it may be a lecithin, e.g. phosphatidylcholine. The term lecithin as used herein encompasses phosphatidylcholine, phosphatidylethanolamine, phosphatidylinositol, phosphatidylserine and phosphatidylglycerol.

Suitably, when the lipid substrate is a lysophospholipid it may be a lysolecithin, e.g. lysophosphatidylcholine. The term lysopbosphatidylcholine as used herein is synonymous with the term lysolecithin and these terms may be used herein interchangeably.

Suitably, when the lipid substrate is a glycolipid it may be digalactosyldiglyceride (DGDG) for example.

The lipid substrate may be referred to herein as the "lipid acyl donor" or "acyl donor". These terms are used interchangeably herein.

For some aspects, preferably the lipid substrate upon which the lipid acyltransferase acts is a phospholipid, such as lecithin, for example phosphatidylcholine.

For some aspects, preferably the lipid substrate is a glycolipid, such as DGDG for example.

For some aspects the lipid substrate may be a food lipid, that is to say a lipid component of a foodstuff.

For some aspects, the lipid acyltransferase according to the present invention may be incapable, or substantially incapable, of acting on a triglyceride and/or a 1-monoglyceride and/or 2-monoglyceride.

Suitably, the lipid substrate or lipid acyl donor may be one or more lipids present in one or more of the following substrates: fats, including lard, tallow and butter fat; oils including oils extracted from or derived from palm oil, sunflower oil, soya bean oil, safflower oil, cotton seed oil, ground nut oil, corn oil, olive oil, peanut oil, coconut oil, and rape seed oil. Lecithin from soya, rape seed or egg yolk is also a suitable lipid substrate. The lipid substrate may be an oat lipid or other plant based material containing galactolipids.

For some aspects of the present invention, the lipid may be selected from lipids having a fatty acid chain length of from 8 to 22 carbons.

For some aspects of the present invention, the lipid may be selected from lipids having a fatty acid chain length of from 16 to 22 carbons, more preferably of from 16 to 20 carbons.

For some aspects of the present invention, the lipid may be selected from lipids having a fatty acid chain length of no greater than 14 carbons, suitably from lipids having a fatty acid chain length of from 4 to 14 carbons, suitably 4 to 10 carbons, suitably 4 to 8 carbons.

Preferably the acyl donor is not a free fatty acid.

Preferably, the acyl donor is not a carbohydrate (sugar) ester.

Suitably, the lipid acyltransferase according to the present invention may exhibit one or more of the following lipase activities: glycolipase activity (E.C. 3.1.1.26), triacylglycerol lipase activity (E.C. 3.1.1.3), phospholipase A2 activity (B.C. 3.1.1.4) or phospholipase A1 activity (E.C. 3.1.1.32). The term "glycolipase activity" as used herein encompasses "galactolipase activity".

Suitably, the lipid acyltransferase according to the present invention may have at least one or more of the following activities: glycolipase activity (E.C. 3.1.1.26) and/or phospholipase A1 activity (E.C. 3.1.1.32) and/or phospholipase A2 activity (E.C. 3.1.1.4).

For some aspects, the lipid acyltransferase according to the present invention may have at least glycolipase activity (E.C. 3.1.1.26).

Suitably, for some aspects the lipid acyltransferase according to the present invention may be capable of transferring an acyl group from a glycolipid and/or a phospholipid to one or more of the following acceptor substrates: a carbohydrate, a protein, a protein subunit, a hydroxy acid.

For some aspects, preferably the lipid acyltransferase according to the present invention is capable of transferring an acyl group from a glycolipid and/or a phospholipid to a carbohydrate to form at least a carbohydrate ester.

For some aspects, preferably the lipid acyltransferase according to the present invention is capable of transferring an acyl group from a glycolipid and/or a phospholipid to a protein or a protein subunit to form at least a protein ester (or a protein fatty acid condensate) or a protein subunit ester.

The term "protein subunit ester" as used herein means the ester formed from any protein subunit, such as a dipeptide ester, an oligopeptide ester, a polypeptide ester or a protein hydrolysate ester for example.

For some aspects, preferably the lipid acyltransferase according to the present invention does not exhibit triacylglycerol lipase activity (E.C. 3.1.1.3).

Preferably, the lipid acyltransferase enzyme according to the present invention may be characterised using the following criteria:

(i) the enzyme possesses acyl transferase activity which may be defined as ester transfer activity whereby the acyl part of an original ester bond of a lipid acyl donor is transferred to one or more of a carbohydrate, protein, protein subunit or hydroxy acid acyl acceptor to form a new ester, i.e. a carbohydrate ester and/or a protein ester and/or a protein subunit ester and/or a hydroxy acid ester; and

(ii) the enzyme comprises the amino acid sequence motif GDSX, wherein X is one or more of the following amino acid residues L, A, V, I, F, Y, H, Q, T, N, M or S.

Preferably, X of the GDSX motif is L. Thus, preferably the enzyme according to the present invention comprises the amino acid sequence motif GSDL.

The GDSX motif is comprised of four conserved amino acids. Preferably, the serine within the motif is a catalytic serine of the lipid acyltransferase enzyme. Suitably, the serine of the GDSX motif may be in a position corresponding to Ser-16 in *Aeromonas hydrophila* lipolytic enzyme taught in Brumlik & Buckley (Journal of Bacteriology Apr. 1996, Vol. 178, No. 7, p 2060-2064).

To determine if a protein has the GDSX motif according to the present invention, the sequence is preferably compared with the hidden markov model profiles (HMM profiles) of the pfam database.

Pfam is a database of protein domain families. Pfam contains curated multiple sequence alignments for each family as well as profile hidden Markov models (profile HMMs) for identifying these domains in new sequences. An introduction to Pfam can be found in Bateman A et al. (2002) Nucleic Acids Res. 30; 276-280. Hidden Markov models are used in a number of databases that aim at classifying proteins, for review see Bateman A and Haft DH (2002) Brief Bioinform 3; 236-245.

http://www.ncbi.nlm.nih.gov/entrez/query.fcgi?cmd=Retrieve&db=PubMed&list uids =12230032&dopt=Abstract
http://www.ncbi.nlm.nih.gov/entrez/query.fcgi?cmd=Retrieve&db=PubMed&list uids =11752314&dopt=Abstract

For a detailed explanation of hidden Markov models and how they are applied in the Pfam database see Durbin R, Eddy S, and Krogh A (1998) Biological sequence analysis; probabilistic models of proteins and nucleic acids. Cambridge University Press, ISBN 0-521-62041-4. The Hammer software package can be obtained from Washington University, St Louis, USA.

Alternatively, the GDSX motif can be identified using the Hammer software package, the instructions are provided in Durbin R, Eddy S, and Krogh A (1998) Biological sequence analysis; probabilistic models of proteins and nucleic acids. Cambridge University Press, ISBN 0-521-62041-4 and the references therein, and the HMMER2 profile provided within this specification.

The PFAM database can be accessed, for example, through several servers which are currently located at the following websites.

http://www.sanger.ac.uk/Software/Pfam/index.shtml
http://pfam.wustl.edu/
http://pfam.jouy.inra.fr/
http://pfam.cgb.ki.se/

The database offers a search facility where one can enter a protein sequence. Using the default parameters of the database the protein sequence will then be analysed for the presence of Pfam domains. The GDSX domain is an established domain in the database and as such its presence in any query sequence will be recognised . The database will return the alignment of the Pfam00657 consensus sequence to the query sequence.

A multiple alignment, including *Aeromonas salmonicida or Aeromonas hydrophila* can be obtained by:

a) manual
obtain an alignment of the protein of interest with the Pfam00657 consensus sequence and obtain an alignment of P10480 with the Pfam00657 consensus sequence following the procedure described above;
Or

b) through the database
After identification of the Pfam00657 consensus sequence the database offers the option to show an alignment of the query sequence to the seed alignment of the Pfam00657 consensus sequence. P10480 is part of this seed alignment and is indicated by GCAT_AERHY. Both the query sequence and P10480 will be displayed in the same window.

The *Aeromonas hydrophila* reference sequence:

The residues of *Aeromonas hydrophila* GDSX lipase are numbered in the NCBI file P10480, the numbers in this text refer to the numbers given in that file which in the present invention is used to determine specific amino acids residues which, in a preferred embodiment are present in the lipid acyltransferase enzymes of the invention.

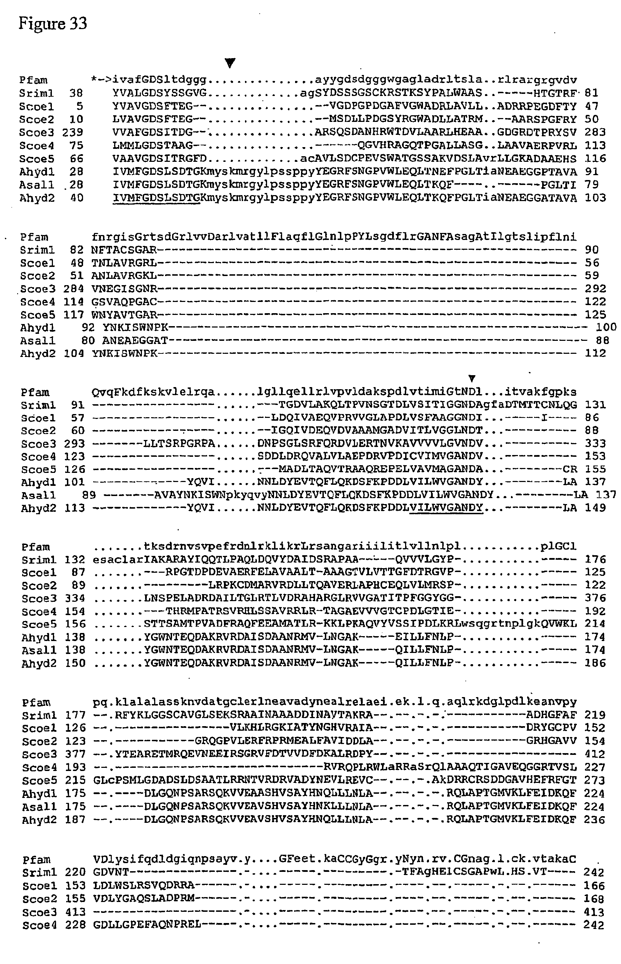

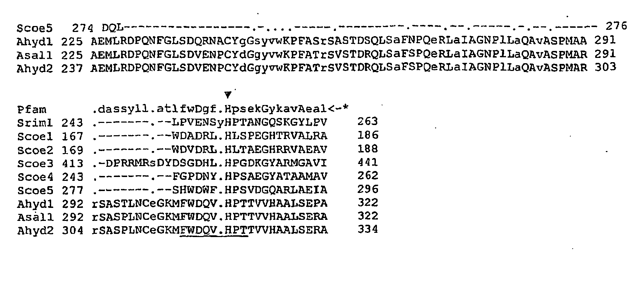

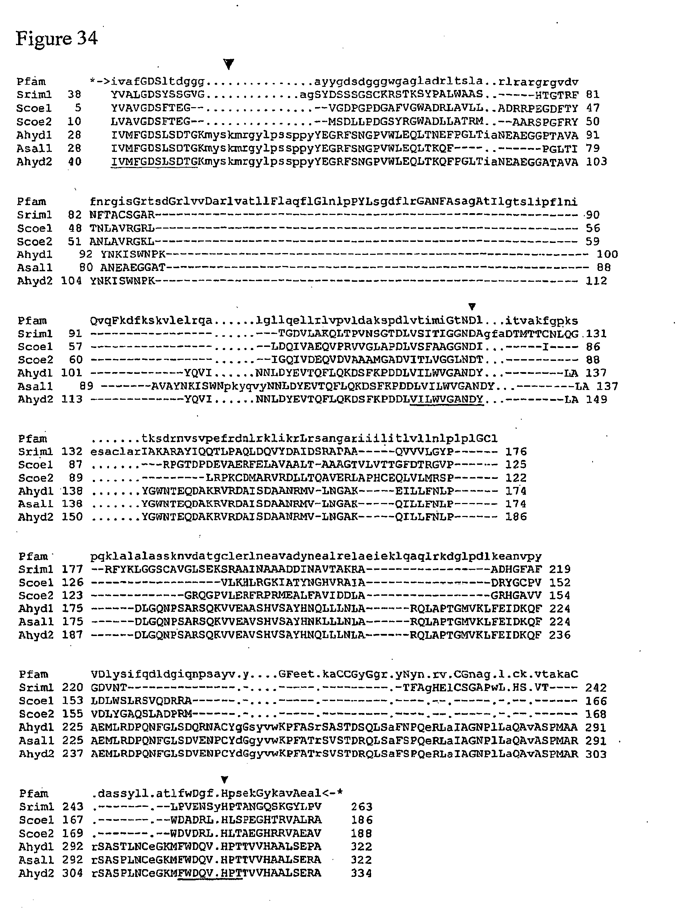

The Pfam alignment was performed (Figure 33 and 34):
The following conserved residues can be recognised and in a preferable embodiment may be present in the enzymes for use in the compositions and methods of the invention;

Block 1 - GDSX block

| hid | hid | hid | hid | Gly | Asp | Ser | hid |
|---|---|---|---|---|---|---|---|
| 28 | 29 | 30 | 31 | 32 | 33 | 34 | 35 |

Block 2 - GANDY block

| hid | Gly | hid | Asn | Asp | hid |
|---|---|---|---|---|---|
| 130 | 131 | 132 | 133 | 134 | 135 |

Block 3 - HPT block

| His |
|---|
| 309 |

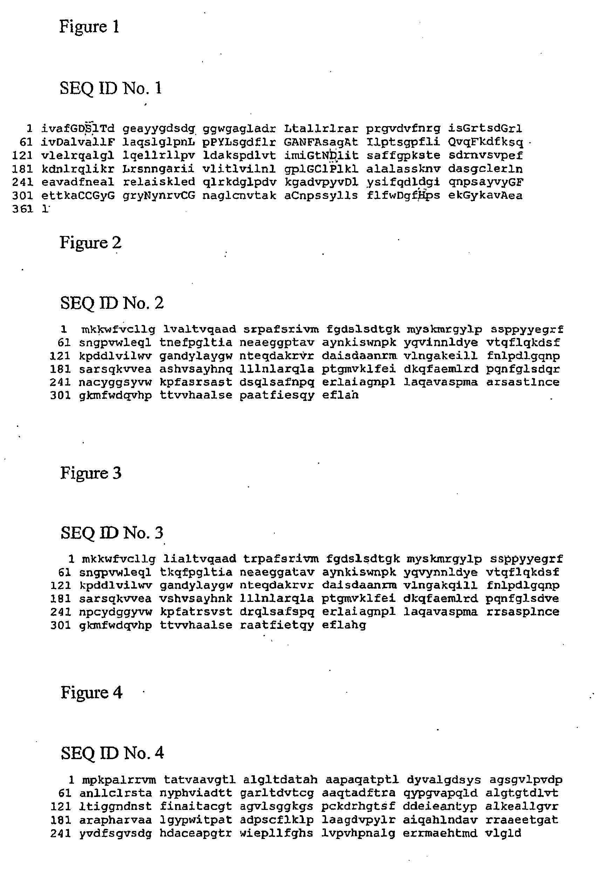

Where 'hid' means a hydrophobic residue selected from Met, Ile, Leu, Val, Ala, Gly, Cys, His, Lys, Trp, Tyr, Phe.
Preferably the lipid acyltransferase enzyme for use in the compositions/methods of the invention can be aligned using the Pfam00657 consensus sequence.
Preferably, a positive match with the hidden markov model profile (HMM profile) of the pfam00657 domain family indicates the presence of the GDSL or GDSX domain according to the present invention.
Preferably when aligned with the Pfam00657 consensus sequence the lipid acyltransferase for use in the compositions/methods of the invention have at least one, preferably more than one, preferably more than two, of the following, a GDSx block, a GANDY block, a HPT block. Suitably, the lipid acyltransferase may have a GDSx block and a GANDY block. Alternatively, the enzyme may have a GDSx block and a HPT block. Preferably the enzyme comprises at least a GDSx block.
Preferably, when aligned with the Pfam00657 consensus sequence the enzyme for use in the compositions/methods of the invention have at least one, preferably more than one, preferably more than two, preferably more than three, preferably more than four, preferably more than five, preferably more than six, preferably more than seven, preferably more than eight, preferably more than nine, preferably more than ten, preferably more than eleven, preferably more than twelve, preferably more than thirteen, preferably more than fourteen, of the following amino acid residues when compared to the reference *A.hydrophilia* polypeptide sequence, namely SEQ ID No. 32: 28hid, 29hid, 30hid, 31hid, 32gly, 33Asp, 34Ser, 35hid, 130hid, 131Gly, 132Hid, 133Asn, 134Asp, 135hid, 309His
The pfam00657 GDSX domain is a unique identifier which distinguishes proteins possessing this domain from other enzymes.
The pfam00657 consensus sequence is presented in Figure 1 as SEQ ID No. 1. This is derived from the identification of the pfam family 00657, database version 6, which may also be referred to as pfam00657.6 herein.
The consensus sequence may be updated by using further releases of the pfam database.
For example, Figures 33 and 34 show the pfam alignment of family 00657, from database version 11, which may also be referred to as pfam00657.11 herein.
The presence of the GDSx, GANDY and HPT blocks are found in the pfam family 00657 from both releases of the database. Future releases of the pfam database can be used to identify the pfam family 00657.
Preferably, the lipid acyltransferase enzyme according to the present invention may be characterised using the following criteria:

(i) the enzyme possesses acyl transferase activity which may be defined as ester transfer activity whereby the acyl part of an original ester bond of a lipid acyl donor is transferred to one or more of a carbohydrate, protein, protein subunit or hydroxy acid acyl acceptor to form a new ester, i.e. a carbohydrate ester and/or a protein ester and/or a protein subunit ester and/or a hydroxy acid ester;
(ii) the enzyme comprises the amino acid sequence motif GDSX, wherein X is one or more of the following amino acid residues L, A, V, I, F, Y, H, Q, T, N, M or S.;
(iii) the enzyme comprises His-309 or comprises a histidine residue at a position corresponding to His-309 in the *Aeromonas hydrophila* lipolytic enzyme shown in Figure 2 (SEQ ID No. 2 or SEQ ID No. 32).

Preferably, the amino acid residue of the GDSX motif is L.
In SEQ ID No. 2 or SEQ ID No. 32 the first 18 amino acid residues form a signal sequence. His-309 of the full length sequence, that is the protein including the signal sequence, equates to His-291 of the mature part of the protein, i.e. the sequence without the signal sequence.

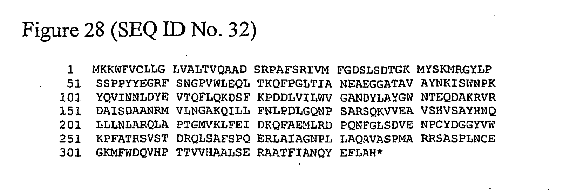

Preferably, the lipid acyltransferase enzyme according to the present invention comprises the following catalytic triad: Ser-34, Asp-134 and His-309 or comprises a serine residue, an aspartic acid residue and a histidine residue, respectively, at positions corresponding to Ser-34, Asp-134 and His-309 in the *Aeromonas hydrophila* lipolytic enzyme shown in Figure 2 (SEQ ID No. 2) or Figure 28 (SEQ ID No. 32). As stated above, in the sequence shown in SEQ ID No. 2 or SEQ ID No. 32 the first 18 amino acid residues form a signal sequence. Ser-34, Asp-134 and His-309 of the full length sequence, that is the protein including the signal sequence, equate to Ser-16, Asp-116 and His-291 of the mature part of the protein, i.e. the sequence without the signal sequence. In the pfam00657 consensus sequence, as given in Figure 1 (SEQ ID No. 1) the active site residues correspond to Ser-7, Asp-157 and His-348.

Preferably, the lipid acyltransferase enzyme according to the present invention may be characterised using the following criteria:

(i) the enzyme possesses acyl transferase activity which may be defined as ester transfer activity whereby the acyl part of an original ester bond of a first lipid acyl donor is transferred to one or more of a carbohydrate, protein, protein subunit or hydroxy acid acyl acceptor to form a new ester, i.e. a carbohydrate ester and/or a protein ester and/or a protein subunit ester and/or a hydroxy acid ester; and
(ii) the enzyme comprises at least Gly-32, Asp-33, Ser-34, Asp-134 and His-309 or comprises glycine, aspartic acid; serine, aspartic acid and histidine residues at positions corresponding to Gly-32, Asp-33, Ser-34, Asp-134 and His-309, respectively, in the *Aeromonas hydrophila* lipolytic enzyme shown in Figure 2 (SEQ ID No. 2) or Figure 28 (SEQ ID No. 32).

Suitably, the lipid acyltransferase enzyme according to the present invention may be obtainable, preferably obtained, from organisms from one or more of the following genera: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* and *Candida.*

Suitably, the lipid acyltransferase enzyme according to the present invention may be obtainable, preferably obtained, from one or more of the following organisms: *Aeromonas hydrophila, Aeromonas salmonicida, Streptomyces coelicolor, Streptomyces rimosus, Mycobacterium, Streptococcus pyogenes, Lactococcus lactis, Streptococcus pyogenes, Streptococcus thermophilus, Lactobacillus helveticus, Desuolfitobacterium dehalogenans, Bacillus sp, Campylobacter jejuni, Vibrionaceae, Xylella fastidiosa, Sulfolobus solfataricus, Saccharomyces cerevisiae, Aspergillus terreus, Schizosaccharomyces pombe, Listeria innocua, Listeria monocytogenes, Neisseria meningitidis, Mesorhizobium loti, Ralstonia solanacearum, Xanthomonas campestris, Xanthomonas axonopodis* and *Candida parapsilosis.*

In one aspect, preferably the lipid acyltransferase enzyme according to the present invention is obtainable, preferably obtained, from one or more of *Aeromonas hydrophila* or *Aeromonas salmonicida.*

Suitably, the lipid acyltransferase enzyme according to the present invention comprises one or more of the following amino acid sequences:

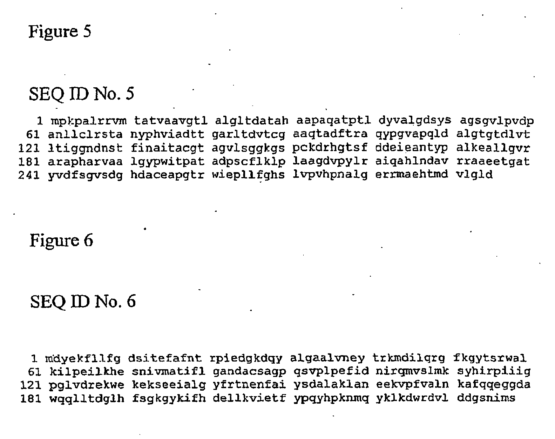

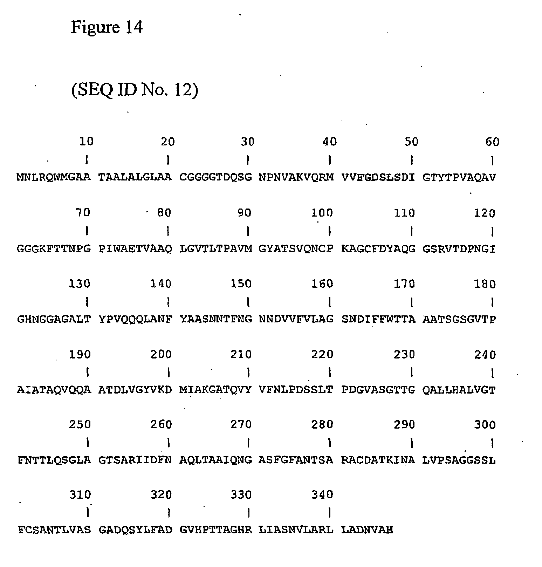

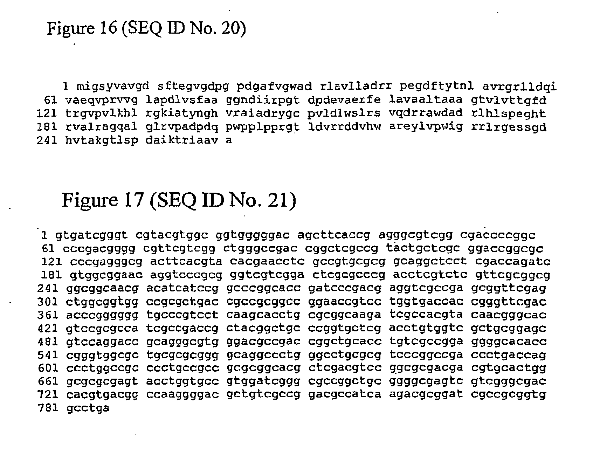

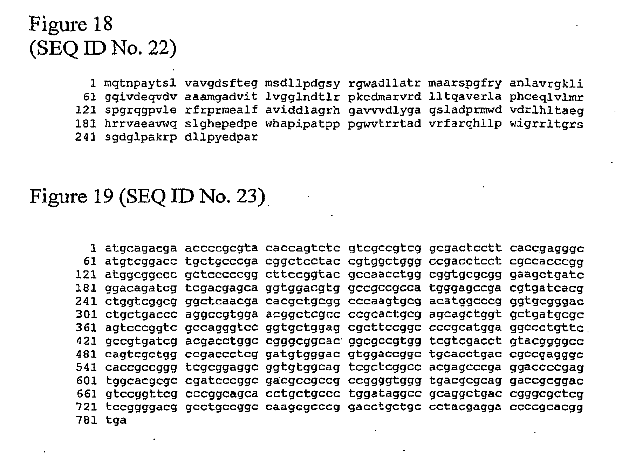

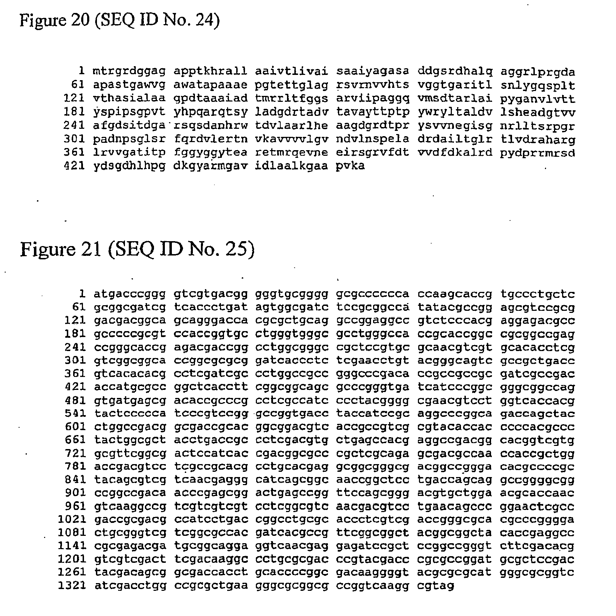

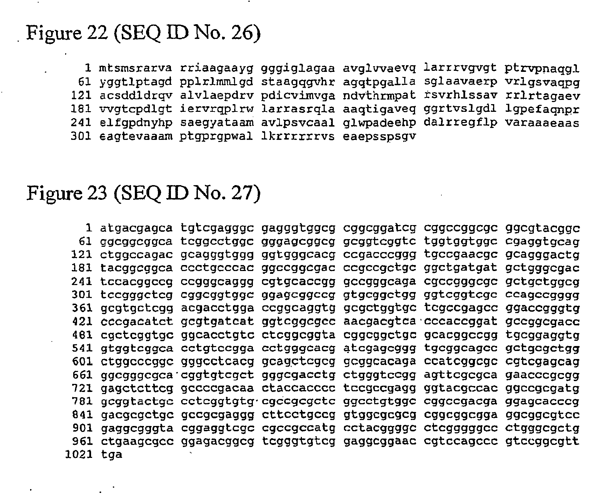

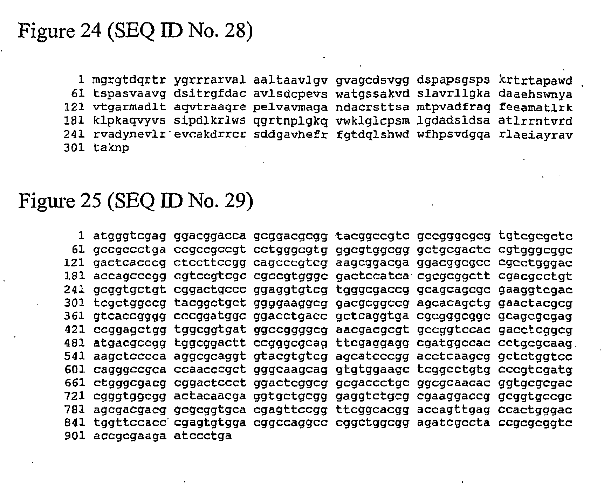

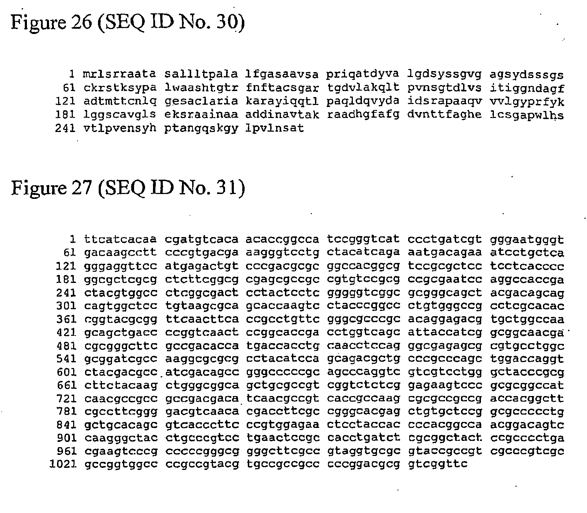

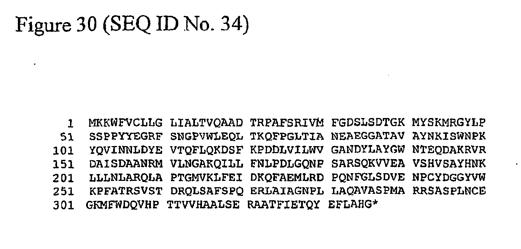

(i) the amino acid sequence shown as SEQ ID No. 2 (see Figure 2)
(ii) the amino acid sequence shown as SEQ ID No. 3 (see Figure 3)
(iii) the amino acid sequence shown as SEQ ID No. 4 (see Figure 4)
(iv) the amino acid sequence shown as SEQ ID No. 5 (see Figure 5)
(v) the amino acid sequence shown as SEQ ID No. 6 (see Figure 6)
(vi) the amino acid sequence shown as SEQ ID No. 12 (see Figure 14)
(vii) the amino acid sequence shown as SEQ ID No. 20 (Figure 16)
(viii) the amino acid sequence shown as SEQ ID No. 22 (Figure 18)
(ix) the amino acid sequence shown as SEQ ID No. 24 (Figure 20)
(x) the amino acid sequence shown as SEQ ID No. 26 (Figure 22)
(xi) the amino acid sequence shown as SEQ ID No. 28 (Figure 24)
(xii) the amino acid sequence shown as SEQ ID No. 30 (Figure 26)
(xiii) the amino acid sequence shown as SEQ ID No. 32 (Figure 28)
(xiv) the amino acid sequence shown as SEQ ID No. 34 (Figure 30) or

an amino acid sequence which has 75% or more identity with any one of the sequences shown as SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32, or SEQ ID No. 34.

Suitably, the lipid acyltransferase enzyme according to the present invention comprises either the amino acid sequence shown as SEQ ID No. 2 or as SEQ ID No. 3 or SEQ ID No. 32 or SEQ ID No. 34 or comprises an amino acid sequence which has 75% or more, preferably 80% or more, preferably 85% or more, preferably 90% or more, preferably 95% or more, identity with the amino acid sequence shown as SEQ ID No. 2 or the amino acid sequence shown as SEQ ID No. 3 or the amino acid sequence shown as SEQ ID No. 32 or the amino acid sequence shown as SEQ ID No. 34.

For the purposes of the present invention, the degree of identity is based on the number of sequence elements which are the same. The degree of identity in accordance with the present invention may be suitably determined by means of computer programs known in the art, such as GAP provided in the GCG program package (Program Manual for the Wisconsin Package, Version 8, August 1994, Genetics Computer Group, 575 Science Drive, Madison, Wisconsin, US53711) (Needleman & Wunsch (1970), J. of Molecular Biology. 48, 443-45) using the following settings for polypeptide sequence comparison: GAP creation penalty of 3.0 and GAP extension penalty of 0.1.

Suitably the lipid acyltransferase enzyme according to the present invention comprises an amino acid sequence which has 80% or more, preferably 85% or more, more preferably 90% or more and even more preferably 95% or more identity with any one of the sequences shown as SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32, or SEQ ID No. 34.

Suitably, the lipid acyltransferase enzyme according to the present invention comprises one or more of the following amino acid sequences:

(a) an amino acid sequence shown as amino acid residues 1-100 of SEQ ID No. 2 or SEQ ID No. 32;
(b) an amino acid sequence shown as amino acids residues 101-200 of SEQ ID No. 2 or SEQ ID No. 32;
(c) an amino acid sequence shown as amino acid residues 201-300 of SEQ ID No. 2 or SEQ ID No. 32; or
(d) an amino acid sequence which has 75% or more, preferably 85% or more, more preferably 90% or more, even more preferably 95% or more identity to any one of the amino acid sequences defined in (a)-(c) above.

Suitably, the lipid acyltransferase enzyme according to the present invention comprises one or more of the following amino acid sequences:

(a) an amino acid sequence shown as amino acid residues 28-39 of SEQ ID No. 2 or SEQ ID No. 32;
(b) an amino acid sequence shown as amino acids residues 77-88 of SEQ ID No. 2 or SEQ ID No. 32;
(c) an amino acid sequence shown as amino acid residues 126-136 of SEQ ID No. 2 or SEQ ID No. 32;
(d) an amino acid sequence shown as amino acid residues 163-175 of SEQ ID No. 2 or SEQ ID No. 32;
(e) an amino acid sequence shown as amino acid residues 304-311 of SEQ ID No. 2 or SEQ ID No. 32; or
(f) an amino acid sequence which has 75% or more, preferably 85% or more, more preferably 90% or more, even more preferably 95% or more identity to any one of the amino acid sequences defined in (a)-(e) above.

Suitably, the lipid acyltransferase enzyme according to the present invention may comprise an amino acid sequence produced by the expression or one or more of the following nucleotide sequences:

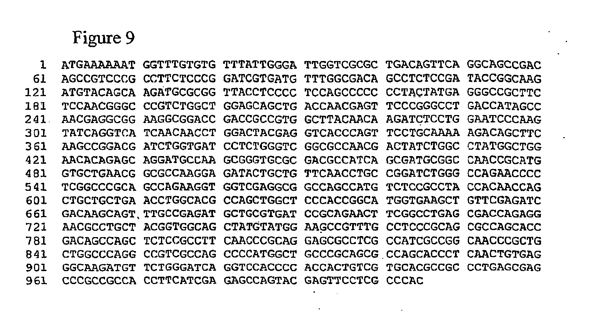

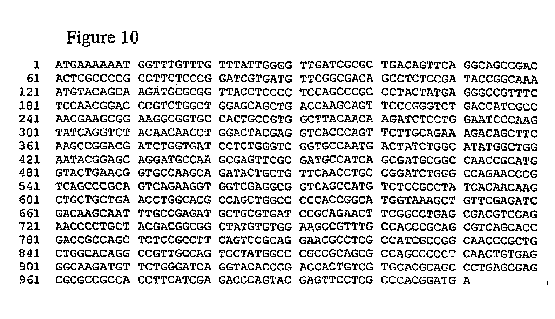

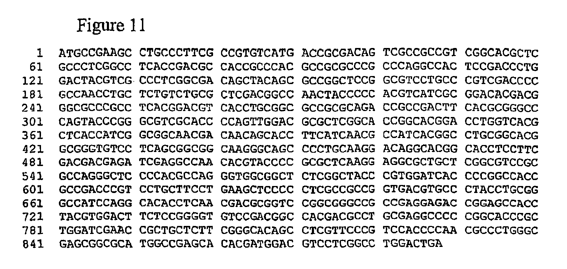

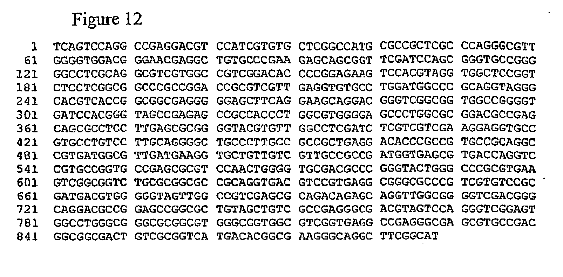

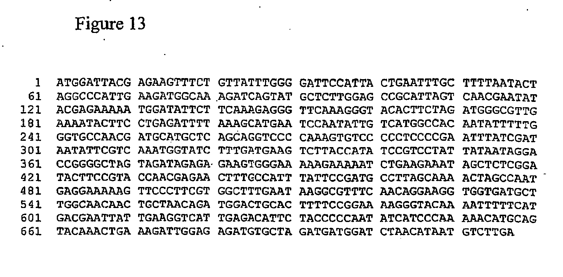

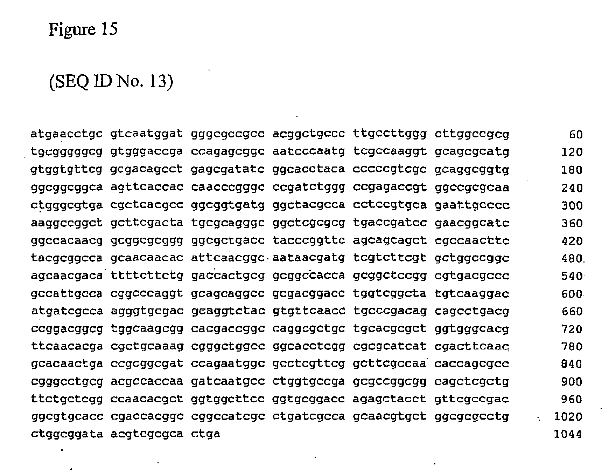

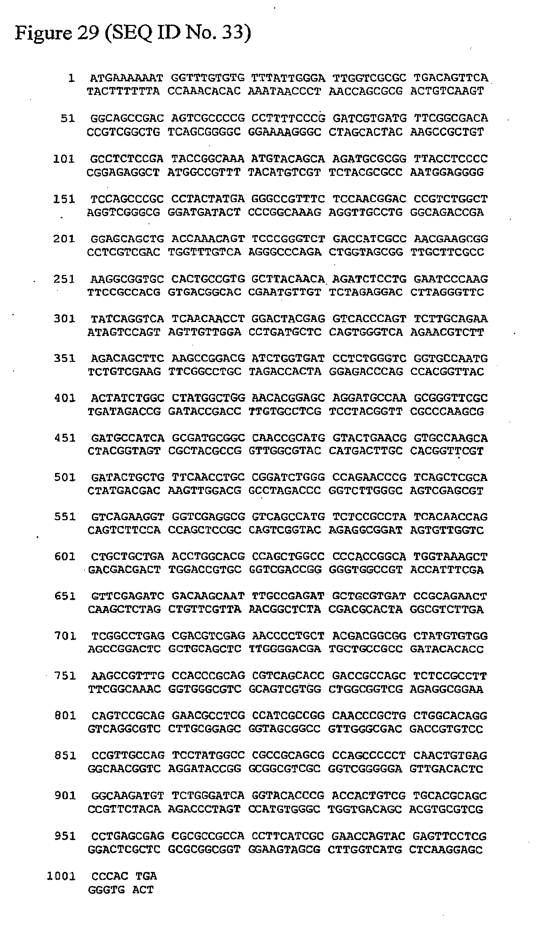

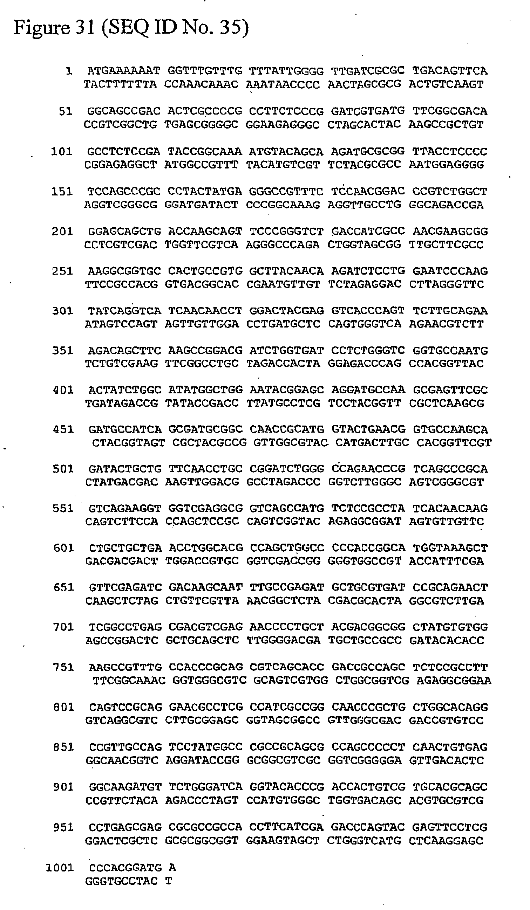

(a) the nucleotide sequence shown as SEQ ID No. 7 (see Figure 9);
(b) the nucleotide sequence shown as SEQ ID No. 8 (see Figure 10);
(c) the nucleotide sequence shown as SEQ ID No. 9 (see Figure 11);
(d) the nucleotide sequence shown as SEQ ID No. 10 (see Figure 12);
(e) the nucleotide sequence shown as SEQ ID No. 11 (see Figure 13);
(f) the nucleotide sequence shown as SEQ ID No. 13 (see Figure 15);
(g) the nucleotide sequence shown as SEQ ID No. 21 (see Figure 17);
(h) the nucleotide sequence shown as SEQ ID No. 23 (see Figure 19);
(i) the nucleotide sequence shown as SEQ ID No. 25 (see Figure 21);
(j) the nucleotide sequence shown as SEQ ID No. 27 (see Figure 23);
(k) the nucleotide sequence shown as SEQ ID No. 29 (see Figure 25);
(l) the nucleotide sequence shown as SEQ ID No. 31 (see Figure 27);
(m)the nucleotide sequence shown as SEQ ID No. 33 (see Figure 29);
(n) the nucleotide sequence shown as SEQ ID No. 35 (see Figure 31);
(o) or

a nucleotide sequence which has 75% or more identity with any one of the sequences shown as SEQ ID No. 7, SEQ ID No. 8, SEQ ID No. 9, SEQ ID No. 10, SEQ No. 11, SEQ ID No. 13, SEQ ID No. 21, SEQ ID No. 23, SEQ ID No. 25, SEQ ID No. 27, SEQ ID No. 29, SEQ ID No. 31, SEQ ID No. 33 or SEQ ID No. 35.

Suitably the nucleotide sequence may have 80% or more, preferably 85% or more, more preferably 90% or more and even more preferably 95% or more identity with any one of the sequences shown as SEQ ID No. 7, SEQ ID No. 8, SEQ ID No. 9, SEQ ID No. 10, SEQ ID No. 11, SEQ ID No. 13, SEQ ID No. 21, SEQ ID No. 23, SEQ ID No. 25, SEQ ID No. 27, SEQ ID No. 29, SEQ ID No. 31, SEQ ID No. 33 or SEQ ID No. 35.

In one aspect, the lipid acyltransferase according to the present invention may be a lecithin:cholesterol acyltransferases (LCAT) or variant thereof (for example a variant made by molecular evolution)

Suitable LCATs are known in the art and may be obtainable from one or more of the following organisms for example: mammals, rat, mice, chickens, *Drosophila melanogaster,* plants, including *Arabidopsis* and *Oryza sativa,* nematodes, fungi and yeast.

In one embodiment the lipid acyltransferase enzyme according to the present invention may be the lipid acyltransferase obtainable, preferably obtained, from the *E. coli* strains TOP 10 harbouring pPet12aAhydro and pPet12aASalmo deposited by Danisco A/S of Langebrogade 1, DK-1001 Copenhagen K, Denmark under the Budapest Treaty on the International Recognition of the Deposit of Microorganisms for the purposes of Patent Procedure at the National Collection of Industrial, Marine and Food Bacteria (NCIMB) 23 St. Machar Street, Aberdeen Scotland, GB on 22 December 2003 under accession numbers NICMB 41204 and NCIMB 41205, respectively.

The term "transferase" as used herein is interchangeable with the term "lipid acyltransferase".

Suitably, the lipid acyltransferase as defined herein catalyses one or both of the following reactions: transesterification, alcoholysis.

Thus in accordance with the present invention, one or more of the following advantageous properties can be achieved: the bioconversion of lipids to form one or more of a carbohydrate ester, a protein ester, a protein subunit ester or a hydroxy acid ester can take place in a high water environment which comprises no organic solvent or a reduced amount of organic solvent compared with conventional bioconversion processes.

The term "bioconversion" as used herein means the modification of one organic compound to produce another organic compound and/or synthesis of organic compounds from other organic compounds by enzyme catalysis.

The term "transesterification" as used herein means the enzymatic catalysed transfer of an acyl group from a lipid donor (other than a free fatty acid) to an acyl acceptor (other than water). For the avoidance of doubt, the use of the term "transesterification" as used herein includes transfer of an acyl group from a lipid donor to an acyl acceptor (other than water) where the acyl acceptor comprises a suitable chemical group, which may for example be either an -OH or -SH group.

As used herein, the term "alcoholysis" refers to the enzymatic cleavage of a covalent bond of an acid derivative by reaction with an alcohol group ROH so that one of the products combines with the H of the alcohol group and the other product combines with the OR group of the alcohol group.

As used herein, the term "hydrolysis" refers to the enzymatic catalysed transfer of an acyl group from a lipid to the OH group of a water molecule. Acyl transfer which results from hydrolysis requires the separation of the water molecule.

The term "interesterification" refers to the enzymatic catalysed transfer of acyl groups between a lipid donor and lipid acceptor, wherein the lipid donor is not a free acyl group. In other words "interesterification" refers to the interchange of a fatty acid between two lipid molecules.

In one aspect, the lipid acyl transferase as defined herein catalyses interesterification.

Suitably, the method or use according to the present invention may further comprise one or more of the following steps: dissolving the acyl acceptor in water; adding a lipid acyl donor to a dissolved acyl acceptor to form a two-phase system or an emulsion; stirring or sonicating the reaction mixture; heating the reaction mixture, for example to denature the enzyme; separating the water phase from the fat/emulsifier phase by standard separation techniques, such as solvent extraction or water evaporation for example; fractionating the fat phase by hydrophobic interaction chromatography, crystallisation or high vacuum distillation. Suitably, one or more of the heating, separating or fractionating steps may be carried out after the reaction has reached equilibrium.

In one embodiment the lipase acyl transferase for use in the methods of the present invention may be immobilised. When it is the case that the enzyme is immobilised the admixture comprising an acyl donor, an acyl acceptor and water passed through a column for example comprising the immobilised enzyme. By immobilising the enzyme it is possible to easily reuse it.

Suitably the immobilised enzyme may be used in a flow reactor or in a batch reactor containing a reaction mixture which comprises an acyl acceptor dissolved in water and a lipid acyl donor as a two-phase system or as an emulsion. The reaction mixture may be optionally stirred or sonicated. Once the reaction has reached equilibrium for example, the reaction mixture and the immobilised enzyme may be separated. Suitably, the reaction product may be fractionated for example by hydrophobic interaction chromatography, crystallisation or high vacuum distillation.

Immobilized lipid acyl transferase can be prepared using immobilisation techniques known in the art. There are numerous methods of preparing immobilised enzymes, which will be apparent to a person skilled in the art (for example the techniques referred to in EP 0 746 608; or Balcao V.M., Paiva A.L., Malcata F.X., Enzyme Microb Technol. 1996 May 1;18(6):392-416; or Retz M.T., Jaeger K.E. Chem Phys Lipids. 1998 Jun;93(1-2):3-14; Bomscheuer U.T.,

Bessler C, Srinivas R, Krishna S.H. Trends Biotechnol. 2002 Oct; 20(10):433-7; Plou et al, J. Biotechnology 92 (2002) 55-66; Warmuth et al., 1992. Bio Forum 9, 282-283; Ferrer et al., 2000. J. Chem. Technol. Biotechnol. 75, 1-8; or Christensen et al., 1998. Nachwachsende Rohstoff 10, 98-105; Petersen and Christenen, 2000, Applied Biocatalysis. Harwood Academic Publishers, Amsterdam. Techniques which may be used herein include covalent coupling to Eupergit C, adsorption on polypropylene and silica-granulation for example.

The term "high water environment" as used herein preferably means an environment which is low in or absent an organic solvent, preferably low in or absent a polar organic solvent. The term organic solvent as used herein preferably does not encompass food oils when used as lipid substrate, and preferably does not encompass food oils that are high in non-polar lipids for example. Suitably, the high water environment according to the present invention may comprise less than 50% by volume organic solvents, less than 30% by volume organic solvents, more preferably less than 15% by volume organic solvents, more preferably less than 5%, more preferably less than 1%, more preferably less than 0.5% by volume organic solvent, more preferably 0% by volume organic solvents.

When it is the case that a carbohydrate ester is produced in accordance with the present invention, the carbohydrate ester is preferably an oligosaccharide ester, a monosaccharide ester or a disaccharide ester.

Suitably, the carbohydrate ester when produced in accordance with the present invention may be one or more of the following: glucose ester, fructose ester, anhydrofructose ester, maltose ester, lactose ester, galactose ester, xylose ester, xylooligosaccharide ester, arabinose ester, maltooligosaccharide ester, tagatose ester, sucrose ester, microthecin ester, ascopyrone P ester, ascopyrone T ester or cortalcerone ester.

Preferably, the carbohydrate ester when produced in accordance with the present invention is one or more of the following: a carbohydrate mono-ester, a sugar mono-ester, an oligosaccharide mono-ester, a trisaccharide mono-ester, a disaccharide mono-ester, a monosaccharide mono-ester, a glucose mono-ester, a fructose mono-ester, anhydrofructose mono-ester, maltose mono-ester, lactose mono-ester, galactose mono--ester, xylose mono-ester, xylooligosacchride mono-ester, arabinose mono-ester, maltooligosaccharide mono-ester, tagatose mono-ester, sucrose mono-ester, microthecin ester, ascopyrone P ester, ascopyrone T ester or cortalcerone ester.

In one embodiment, the microthecin ester, ascopyrone P ester, ascopyrone T ester and/or cortalcerone ester may function as an antimicrobial agent. Alternatively or in addition thereto, the microthecin ester, ascopyrone P ester, ascopyrone T ester and/or cortalcerone ester may function as one or both of an antioxidant and/or emulsifier.

Preferably, the formation of the carbohydrate ester (if any) in accordance with the present invention is independent of UDP-glucose. Preferably, the foodstuff according to the present invention does not comprise UDP- ’ glucose, or only comprises UDP-glucose in insignificant amounts.

The lipid acyl transferases used in the methods of the invention have been found to have unique properties when compared to lipolytic enzymes in that they have a marked preference for transfer of acyl groups from lipids to acceptors other than water, even in the presence of significant water. In a comparison with prior art enzymes, the lipid acyl transferase used in the invention were found to have a high relative transferase activity in the presence of 6% water, 54% water, 73% water, 89% water and approximately 95%. Lipolytic enzymes tested had virtually no significant relative transferase activity at these water concentrations.

The % transferase activity (i.e. the transferase activity as a percentage of the total enzymatic activity) may be determined by the following protocol:

*Protocol for the determination of % acyltransferase activity:*

A substrate to which a lipid acyltransferase according to the present invention has been added may be extracted following the enzymatic reaction with $CHCl_3$:$CH_3OH$ 2:1 and the organic phase containing the lipid material is isolated and analysed by GLC and HPLC according to the procedure detailed hereinbelow. From the GLC and HPLC analyses the amount of free fatty acids and one or more of carbohydrate esters, protein esters; protein subunit esters; hydroxy acid esters are determined. A control substrate to which no enzyme according to the present invention has been added, is analysed in the same way.

*Calculation:*

From the results of the GLC and HPLC analyses the increase in free fatty acids and carbohydrate esters and/or protein esters and/or protein subunit esters and/or hydroxy acid can be calculated:

A % fatty acid = % Fatty acid(enzyme) - % fatty acid(control); Mv fatty acid = average molecular weight of the fatty acids; A = A % protein ester/Mv protein ester (where A % protein ester = % protein ester(enzyme) - % protein ester(control) and Mv protein ester = average molecular weight of the protein esters) - applicable where the acyl acceptor is a protein; B = A % carbohydrate ester/Mv carbohydrate ester (where Δ % carbohydrate ester = % carbohydrate ester(enzyme) - % carbohydrate ester(control) and Mv carbohydrate ester = average molecular weight of the carbohydrate ester) - applicable whore the acyl acceptor is a carbohydrate;

C = Δ % protein subunit ester/Mv protein subunit ester (where Δ % protein subunit ester = % protein subunit ester(enzyme) - % protein subunit ester(control) and Mv protein subunit ester = average molecular weight of the protein subunit ester) - applicable where the acyl acceptor is a protein subunit and

D = A % hydroxy acid ester/Mv hydroxy acid ester (where A % hydroxy acid ester = % hydroxy acid ester(enzyme) - % hydroxy acid ester(control) and Mv hydroxy acid ester = average molecular weight of the hydroxy acid ester) - applicable where the acyl acceptor is a hydroxy acid.

The transferase activity is calculated as a percentage of the total enzymatic activity:

$$\%\ \text{transferase activity} = \frac{A^* + B^* + C^* + D^* \times 100}{A^* + B^* + C^* + D^* + \Delta\ \%\ \text{fatty acid}/(\text{Mv fatty acid})}$$

* - delete as appropriate.

The lipase and acyltransferase activity of an enzyme may be evaluated using the following assays. In this way, a lipid acyltransferase having the enzyme characteristics defined herein may be obtained/identified.

**<u>Transferase Assay in Buffered Substrate (see Example 6)</u>**

Enzymes which function as lipid acyltransferases for use in the methods of the invention can be routinely identified using the assay taught herein in Example 6. This assay will be hereinafter referred to as the 'Transferase Assay in Buffered Substrate'. In Example 6 the lipid acyltransferase enzyme from *Aeromonas salmonicida* in accordance with the present invention was analysed and compared with a range of lipolytic enzymes not encompassed by the present invention. As can be seen, of the lipolytic enzymes only LIPOPAN® F (Novozymes, Denmark was found to have any transferase activity and then only a very low level (1.3%).

Enzymes suitable for use in the methods of the invention can be routinely identified using the Transferase Assay in Buffered Substrate. Using this assay, in which there is a very high water content - approximately 95%, lipid acyltransferases in accordance with the present invention are those which have at least 2% acyltransferase activity (relative transferase activity), preferably at least 5% relative transferase activity, preferably at least 10% relative transferase activity, preferably at least 15%, 20%, 25% 26%, 28%, 30%, 40% 50%, 60% or 75% relative transferase activity. Suitably, the lipid acyltransferase in accordance with the present invention may have less than 28%, less than 30%, preferably less than 40%, 50%, 60%, 70%, 80%, 90% or 100% acyltransferase activity.

**Tansferase Assay in a Low Water Environment**

As an alternative to (or in addition to) using the "Transferase Assay in Buffered Substrate", lipid acyltransferases for use in accordance with the present invention may be identified using the "Transferase Assay in a Low Water Environment".

In order to determine if an enzyme is a lipid acyltransferase according to the present invention, one may carry out a "Transferase Assay in a Low Water Environment", namely in an oily environment with 6% water as taught in Example 9. This example illustrates that in an oily environment with 6% water content the lipid acyltransferase of the invention has a high relative transferase activity, where the prior art lipolytic enzymes have hydrolytic activity.

In one embodiment, the lipid acyltransferase suitable for use in the methods and/or uses according to the present invention is one which when tested using the "Transferase Assay in a Low Water Environment", measured after a time period selected from 30, 20 or 120 minutes, has a relative transferase activity of at least 1%, preferably at least 2%, preferably at least 5%, preferably at least 10%, preferably at least 20%, preferably at least 30%, preferably at least 40%, preferably at least 50%, preferably at least 60%, preferably at least 70%, preferably at least 75%. Suitably, the lipid acyl transferase in accordance with the present invention may have less than 30%, 40%, 50%, 60%, 70%, or 80% activity when measured after a time period of 10, 20, 30 or 120 minutes using the "Transferase Assay in a Low Water Environment".

As described above, the lipase acyltransferase of the invention can be identified using either the "Transferase Assay in Buffered Substrate" or in the "Transferase Assay in Low Water Environment" using cholesterol as the acyl acceptors. Of course, the skilled person would be readily aware that, with obvious amendments to the analytical methods the 'Transferase Assay in Buffered Substrate' or the 'Transferase Assay in Low Water Environment may be used to determine the lipid acyltransferase activity for any lipid acyl donor or any acyl acceptor combination. The skilled person would, if necessary, simply replace the acyl donor substrate (e.g. phospholipid) with an alternative acyl donor substrate (e.g. glycolipid, triacylglyceride) and/or replace the acyl acceptor (e.g. cholesterol) with an alternative acyl acceptor substrate (e.g. a carbohydrate, a protein, a protein subunit or a hydroxy acid) (for example see Examples 10-13).

The term "high water environment'' as used herein means any environment comprising 5-98% water. Preferably the environment comprises more than 6% water content, preferably more than 7%, 8%, 9%, 10%, 20%, 30%, 40%, 50%, 60%, 70%, 80% or 90%. Suitably, the high water environment may be comprised of 20-98%, suitably 50-98%, suitably of 70-98%, suitably 75-98% water.

In one embodiment, in the admixture the ratio of the amount of lipid acyltransferase added compared with water is at least 1:700, preferably 1:10,000, as measured on a by weight basis.

The term "low water" as used herein means any substrate or foodstuff with less than 5% water content, preferably less than 4%, 3%, 2%, 1% or 0.5%.

Preferably the method and/or use according to the present invention may be carried out at a temperature of 15-60°C, preferably at a temperature of 20-60°C, preferably 20-50°C, preferably 20-45°C, preferably 20-40°C.

Suitably, the method or use according to the present invention comprises a further step or purifying and/or isolating the reaction product, namely one or more of a carbohydrate ester a protein ester, a protein subunit ester, or a hydroxy acid ester. Thus, preferably the reaction product is in a purified and/or isolated form.

Numerous methods for purification of esters are known to the skilled person. By way of example only the esters produced by the methods/uses taught herein may be purified using chromatography, such as hydrophobic interaction, filtration, centrifugation, solvent extraction/distillation or crystallisation. Suitable methodologies are taught in Ulmann's Encyclopedia of Industrial Chemistry (2002) by Wiley-VCH Verlag GmbH & Co. KgaA.

The lipid acyl-transferase of the invention may be expressed in any suitable expression host. For example the lipid acyltransferase of the invention may be expressed in *Bacillus subtilis* and may be purified by ultrafiltration and/or by precipitation in ethanol and/or centrifugation, and may be subsequently spray dried using starch (maltodextrin) as carrier for the enzyme. The spray-dried enzyme may be standardized to specified PLU activity by adding further carrier in powder form. The techniques involved are well established and routine in the art.

In one embodiment, the method according to the present invention is an *in vitro* process. The method may suitably be a continuous or batch process.

The enzyme according to the present invention may be used in combination with one or more other further enzymes. Thus, it is within the scope of the present invention that, in addition to the enzyme of the invention, the admixture is contacted with at least one further enzyme. Such further enzymes include starch degrading enzymes such as endo- or exoamylases, pullulanases, debranching enzymes, hemicellulases including xylanases, cellulases, oxidoreductases, e.g: glucose oxidase or a carbohydrate oxidase such as one which oxidises maltose, for example hexose oxidase (HOX), lipases, phospholipases and hexose oxidase, and proteases. The admixure may be contacted with the enzyme of the invention and the at least one further enzyme at the same time or sequentially.

In one embodiment for example the lipid acyltransferase may be used in combination with a lipase having one or more of the following lipase activities: glycolipase activity (E.C. 3.1.1.26, triacylglycerol lipase activity (E.C. 3.1.1.3), phospholipase A2 activity (E.C. 3.1.1.4) or phospholipase A1 activity (E.C. 3.1.1.32). Suitable lipase enzymes are well know within the art and include by way of example the following lipases: LIPOPAN® F and/or LECITASE® ULTRA (Novozymes A/S, Denmark), phospholipase A2 (e.g. phospholipase A2 from LIPOMOD™ 22L from Biocatalysts, LIPOMAX™ from Genecor), LIPOLASE® (Novozymes A/S, Denmark), the lipases taught in WO03/97835, EP 0 977 869 or EP 1 193 314.

USES

Thus, the methods according to the present invention produce one or more of a carbohydrate ester, a protein ester, a protein subunit ester, a hydroxyacid ester. Many of these esters are useful emulsifiers. By way of example only amino acid esters, peptide esters, protein esters, carbohydrate esters and hydroxy acid esters (such as tartaric acid esters) for example are functionally important emulsifiers. Emulsifiers are useful in a wide range of industries, such as the food industry, the feed industry, the cosmetics industry (for example in cosmetic bases), the pharmaceutical industry (in both pharmaceutical synthesis and formulation for example) and the paint industry for example. Emulsifiers can function as wetting agents, food ingredients and active ingredients.

In addition protein fatty acid condensates owing to their excellent physiological properties, are suited for use in cosmetics and personal hygiene products for example. For example, protein esters may be used in shower and bath preparations as well as in shampoos and body cleansers. The protein fatty acid condensates may also be useful in pharmaceutical compositions, for example as a base.

Protein fatty acid condensates are well known for their application in the cosmetic industry. Conventionally, these products are produced by reacting protein hydrolyzate with fatty acid chloride under Schotten-Baumann conditions, using water as solvent. (http://www.scf-online.com/english/26 e/rawmaterials26 e .htm#5).

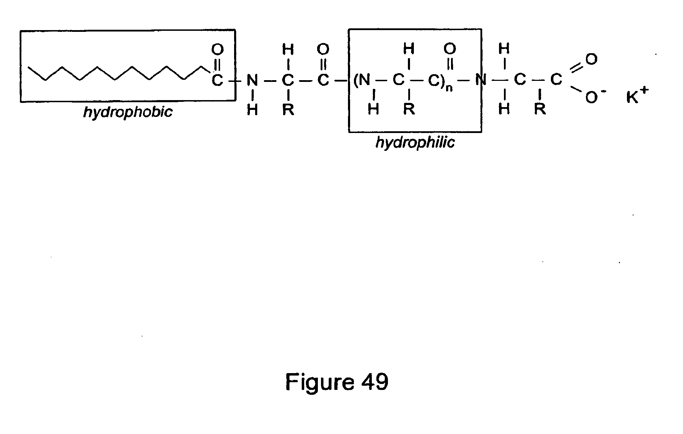

In the development of the protein-fatty acid condensates it is possible to combine the renewable resources fatty acids (from vegetable oil) and protein, which can be obtained from both animal waste (leather) as well as from many plants, to construct a surfactant structure with a hydrophobic (fatty acid) and a hydrophilic (protein) part. In this process the fatty acid chloride reacts with the amine group of the amino acid and forms the protein fatty acid condensate (See Figure 49). Products are obtained which have an excellent skin compatibility and additionally have a good cleaning effect.

The fact that even small additions of the acylated protein hydrolysate have a synergistic effect on the skin compatibility of other surfactants is highly important from a technical formulation point of view. An explanation for this protective effect could lie in the amphoteric behaviour of the product. There is an interaction between the protein-fatty acid condensate and skin collagen. This leads to the formation of a protective layer, which reduces the excessive attack of surfactants on the upper layers of the skin, their strong degreasing effect and the direct interaction of anionic surfactants with the skin.

In the cosmetic branch, protein-based surfactants are mainly used in mild shower and bath products, mild shampoos, surfactant-based face cleansers, cold-wave preparations and fixatives or surfactant preparations for babies.

Protein hydrolysate fatty acid condensates are also useful as bases for pharmaceutical preparations, for example for creams and ointments which contain active ingredients for topical application to the skin.

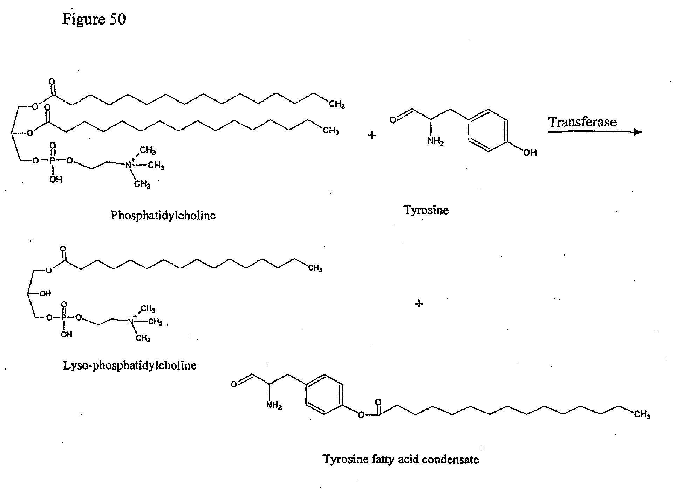

The present invention provides a new way to produce protein fatty acid condensate without using fatty acid chloride. The reaction according to the present invention is depicted in Figure 50. This reaction can be conducted in water or buffer system at low temperature without formation of waste products.

The term "protein fatty acid condensate" as used herein encompasses all of the following protein esters, polypeptide esters, dipeptide esters, oligopeptide esters, peptide esters, and amino acid esters.

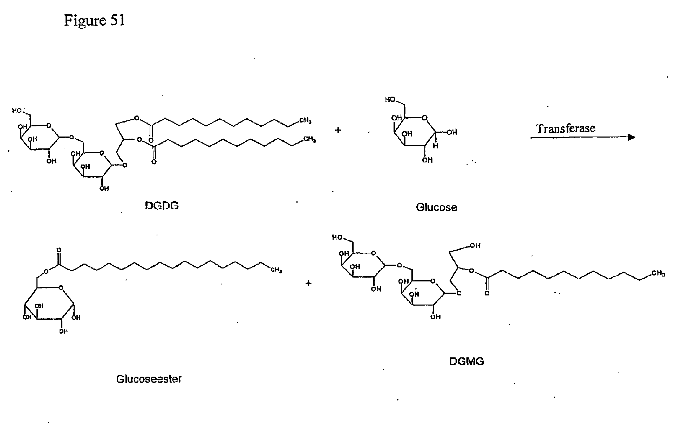

As a skilled person would be readily aware, carbohydrate esters (particularly sugar esters) have a broad application in the food industry. Other fields of application include cosmetics, oral-care products and medical supplies. In addition, these compounds can be used as antibiotics, antitumorals, fungicides and insecticides. The lipid acyltransferase according to the present invention is able to catalyse the formation of glucose ester in a high water environment (Figure 51).

The esters produced in accordance with the present invention find application in the following fields:

Cosmetics: including essential oil emulsions (o/w, HLB 16-18) Paraffin oil emulsions, o/w, HLB 10 - 14; Stearic acid emulsions; Wax emulsions, o/w, HLB 14 - 16; Lanolin emulsions, o/w, HLB 12 -14; Silicone emulsions; Toothpastes; o/w; Foam baths, o/w, HLB 14 - 18; Hair Lotion.

Pharmaceutical Preparations: including in drug emulsions; ointment bases; suppository compound, w/o; encapsulation; injection preparation.

Agriculture: including in soil improvement; as a fertilizer additive; as all-purpose cleaners; cleaners for fruit and vegetables; cleaners for milk churns.

Crop Protection: including in naturally occurring insecticides; chlorinated hydrocarbons, and 140; phosphoric acid esters o/w, HLB 10-14; fungicides, o/w; herbicides, o/w.

Food Industry: including in bread and cakes; margarine; chocolate; fat bloom prevention, w/o, HLB 5 - 10; sugar frosting, o/w, HLB 14 -16; softeners for caramels and chewing gum, w/o, HLB. 2 - 4; prevention of sticking, w/o, HLB 2 - 4; ice cream additives w/o, HLB 4 - 6; wetting of milk and baking powders, w/o, HLB 9-11; custard powder, w/o, HLB 2 - 4; in the drinks industry; in fruit and vegetables; in flavourings, w/o and o/w, HLB 10 -12; in meat, salad, or other flavouring sauces, o/w; in food dyes, w/o, HLB 2 - 4; o/w, HLB 8 - 18; in foam inhibitors.

The benefit of using protein fatty acid esters, hydroxy acid esters and carbohydrate esters produced in accordance with the present invention as emulsifiers in food applications is that these are harmless food compatible components which are more easily biodegradable compared to other conventionally used emulsifier like ethoxylated fatty acid esters for example. These emulsifiers are thus more environmentally friendly to use in both the food industry and the non-food industry.

In one embodiment, the microthecin ester, ascopyrone P ester, ascopyrone T ester and/or cortalcerone ester may function as an antimicrobial agent. Alternatively or in addition thereto, the microthecin ester, ascopyrone P ester, ascopyrone T ester and/or cortalcerone ester may function as one or both of an antioxidant and/or emulsifier

In one embodiment, the methods or uses of the present invention can be used to produce emulsifiers for use in drug formulations, particularly in the production of controlled release formulations of active ingredients, wherein the active ingredient is acylated using the lipid acyl-transferase. Such slow release formulations are particularly useful for pharmaceutical compositions administered orally, where the gradual hydrolysis of the ester in the digestive tract provides gradual delivery of the active ingredient. Such acylated compositions could further be used for a subcutaneous or an intravenous formulation.

In another embodiment, the methods or uses of the present invention can be used to produce phase transfer catalysts for transfer of salts into a solution of organic solvents for instance in an organic reaction. For example, the transfer of an acyl group to an appropriate cationic acceptor, such as a hydroxy acid (citric acid), or alternatively with an anionic acceptor group, such as hydroxy-amines can produce phase transfer catalysts for transfer of salts into a solution of organic solvents.

In another embodiment, the methods of the present invention may be used to produce ester prodrugs of pharmaceutical compounds with low biological availability and/or low solubility, for instance antiviral agents like aciclovir and gangaciaovir. The method could further be used for other medicinal compounds with a free hydroxy-group, for instance a primary, secondary or tertiary hydroxy-group.

Preferably, the ester produced in accordance with the present invention is used in a pharmaceutical formulation.

Preferably, the ester produced in accordance with the present invention is used in a cosmetic and/or a personal hygiene product.

Preferably, the ester produced in accordance with the present invention is used in a foodstuff and/or a feedstuff.

The method in accordance with the present invention may be one step in the manufacturing process of one or more of a pharmaceutical, a cosmetic, a personal hygiene product a foodstuff or a feedstuff.

## ADVANTAGES

One advantage of the method according to the present invention is that it results in the manufacture of a protein ester and/or a protein subunit ester without the need to use organic solvents. Thus, the present invention allows the use of the organic solvents to be reduced or eliminated. This has many advantages, for example in reduced production costs, reduced human and/or environmental exposure to organic solvents, simplification of the production process.

In the production of esters for food applications it is particularly advantageous to use lipids rather than fatty acids because it is not necessary to remove surplus lipids because these can from part of the food item where the reaction product is used. On the other hand, surplus free fatty acids would have to be removed because these are deleterious for most food products.

## ISOLATED

In one aspect, preferably the polypeptide or protein for use in the present invention is in an isolated form. The term "isolated" means that the sequence is at least substantially free from at least one other component with which the sequence is naturally associated in nature and as found in nature.

In one aspect, preferably the bioconversion product according to the present invention for example the carbohydrate ester and/or the protein ester and/or the protein subunit ester and/or the hydroxy acid ester is isolated from the reaction mixture. The term "isolated" means that the bioconversion product is at least substantially free from at least one other component with which the bioconversion product is associated during the bioconversion reaction.

## PURIFIED

In one aspect, preferably the polypeptide or protein for use in the present invention is in a purifed form. The term "purified" means that the sequence is in a relatively pure state - e.g. at least about 51% pure, or at least about 75%, or at least about 80%, or at least about 90% pure, or at least about 95% pure or at least about 98% pure.

In one aspect, preferably the bioconversion product produced in accordance with the present invention, for example the carbohydrate ester and/or the protein ester and/or the protein subunit ester and/or the hydroxy acid ester is purified from the reaction mixture and is therefore in a purified form. The term "purified" means that the bioconversion product is in a relatively pure state - e.g. at least about 51 % pure, or at least about 75%, or at least about 80%, or at least about 90% pure, or at least about 95% pure or at least about 98% pure.

## PHARMACEUTICAL COMPOSITIONS

The present invention also provides a pharmaceutical composition comprising the product of the present invention and a pharmaceutically acceptable carrier, diluent or excipient (including combinations thereof).

The pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition of the present invention may be formulated to be administered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be administered by a number of routes.

Where the agent is to be administered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

## CLONING A NUCLEOTIDE SEQUENCE ENCODING A POLYPEPTIDE ACCORDING TO THE PRESENT INVENTION

A nucleotide sequence encoding either a polypeptide which has the specific properties as defined herein or a polypeptide which is suitable for modification may be isolated from any cell or organism producing said polypeptide. Various methods are well known within the art for the isolation of nucleotide sequences.

For example, a genomic DNA and/or cDNA library may be constructed using chromosomal DNA or messenger RNA from the organism producing the polypeptide. If the amino acid sequence of the polypeptide is known, labelled oligonucleotide probes may be synthesised and used to identify polypeptide-encoding clones from the genomic library prepared from the organism. Alternatively, a labelled oligonucleotide probe containing sequences homologous to another known polypeptide gene could be used to identify polypeptide-encoding clones. In the latter case, hybridisation and washing conditions of lower stringency are used.

Alternatively, polypeptide-encoding clones could be identified by inserting fragments of genomic DNA into an expression vector, such as a plasmid, transforming enzyme-negative bacteria with the resulting genomic DNA library, and then plating the transformed bacteria onto agar containing an enzyme inhibited by the polypeptide, thereby allowing clones expressing the polypeptide to be identified.

In a yet further alternative, the nucleotide sequence encoding the polypeptide may be prepared synthetically by established standard methods, e.g. the phosphoroamidite method described by Beucage S.L. et al (1981) Tetrahedron Letters 22, p 1859-1869, or the method described by Matthes et al (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al (Science (1988) 239, pp 487-491).

## NUCLEOTIDE SEQUENCES

The present invention also encompasses nucleotide sequences encoding polypeptides having the specific properties as defined herein. The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be doublestranded or single-stranded whether representing the sense or antisense strand.

The term "nucleotide sequence" in relation to the present invention includes genomic DNA, cDNA, synthetic DNA, and RNA. Preferably it means DNA, more preferably cDNA for the coding sequence.

In a preferred embodiment, the nucleotide sequence *per se* encoding a polypeptide having the specific properties as defined herein does not cover the native nucleotide sequence in its natural environment when it is linked to its naturally associated sequence(s) that is/are also in its/their natural environment. For ease of reference, we shall call this preferred embodiment the "non-native nucleotide sequence". In this regard, the term "native nucleotide sequence" means an entire nucleotide sequence that is in its native environment and when operatively linked to an entire promoter with which it is naturally associated, which promoter is also in its native environment. Thus, the polypeptide of the present invention can be expressed by a nucleotide sequence in its native organism but wherein the nucleotide sequence is not under the control of the promoter with which it is naturally associated within that organism.

Preferably the polypeptide is not a native polypeptide. In this regard, the term "native polypeptide" means an entire polypeptide that is in its native environment and when it has been expressed by its native nucleotide sequence.

Typically, the nucleotide sequence encoding polypeptides having the specific properties as defined herein is prepared using recombinant DNA techniques (i.e. recombinant DNA). However, in an alternative embodiment of the invention, the nucleotide sequence could be synthesised, in whole or in part, using chemical methods well known in the art (see Caruthers MH et al (1980) Nuc Acids Res Symp Ser 215-23 and Horn T et al (1980) Nuc Acids Res Symp Ser 225-232).

MOLECULAR EVOLUTION

Once an enzyme-encoding nucleotide sequence has been isolated, or a putative enzyme-encoding nucleotide sequence has been identified, it may be desirable to modify the selected nucleotide sequence, for example it may be desirable to mutate the sequence in order to prepare an enzyme in accordance with the present invention.

Mutations may be introduced using synthetic oligonucleotides. These oligonucleotides contain nucleotide sequences flanking the desired mutation sites.

A suitable method is disclosed in Morinaga et al (Biotechnology (1984) 2, p646-649). Another method of introducing mutations into enzyme-encoding nucleotide sequences is described in Nelson and Long (Analytical Biochemistry (1989), 180, p 147-151).

Instead of site directed mutagenesis, such as described above, one can introduce mutations randomly for instance using a commercial kit such as the GeneMorph PCR mutagenesis kit from Stratagene, or the Diversify PCR random mutagenesis kit from Clontech. EP 0 583 265 refers to methods of optimising PCR based mutagenesis, which can also be combined with the use of mutagenic DNA analogues such as those described in EP 0 866 796. Error prone PCR technologies are suitable for the production of variants of lipid acyl transferases with preferred characteristics. WO0206457 refers to molecular evolution of lipases.

A third method to obtain novel sequences is to fragment non-identical nucleotide sequences, either by using any number of restriction enzymes or an enzyme such as Dnase I, and reassembling full nucleotide sequences coding for functional proteins. Alternatively one can use one or multiple non-identical nucleotide sequences and introduce mutations during the reassembly of the full nucleotide sequence. DNA shuffling and family shuffling technologies are suitable for the production of variants of lipid acyl transferases with preferred characteristics. Suitable methods for performing 'shuffling' can be found in EP0 752 008, EP1 138 763, EP1 103 606. Shuffling can also be combined with other forms of DNA mutagenesis as described in US 6,180,406 and WO 01/34835.

Thus, it is possible to produce numerous site directed or random mutations into a nucleotide sequence, either *in vivo* or *in vitro,* and to subsequently screen for improved functionality of the encoded polypeptide by various means. Using in silico and exo mediated recombination methods (see WO 00/58517, US 6,344,328, US 6,361,974), for example, molecular evolution can be performed where the variant produced retains very low homology to known enzymes or proteins. Such variants thereby obtained may have significant structural analogy to known transferase enzymes, but have very low amino acid sequence homology.

As a non-limiting example, In addition, mutations or natural variants of a polynucleotide sequence can be recombined with either the wild type or other mutations or natural variants to produce new variants. Such new variants can also be screened for improved functionality of the encoded polypeptide.

The application of the above-mentioned and similar molecular evolution methods allows the identification and selection of variants of the enzymes of the present invention which have preferred characteristics without any prior knowledge of protein structure or function, and allows the production of non-predictable but beneficial mutations or variants. There are numerous examples of the application of molecular evolution in the art for the optimisation or alteration of enzyme activity, such examples include, but are not limited to one or more of the following: optimised expression and/or activity in a host cell or in vitro, increased enzymatic activity, altered substrate and/or product specificity, increased or decreased enzymatic or structural stability, altered enzymatic activity/specificity in preferred environmental conditions, e.g. temperature, pH, substrate

As will be apparent to a person skilled in the art, using molecular evolution tools an enzyme may be altered to improve the functionality of the enzyme.

Suitably, the lipid acyltransferase used in the invention may be a variant, i.e. may contain at least one amino acid substitution, deletion or addition, when compared to a parental enzyme. Variant enzymes retain at least 1%, 2%, 3%, 5%, 10%, 15%, 20%, 30%, 40%, 50 %, 60%, 70%, 80%, 90%, 95%, 97%, 99% homology with the parent enzyme. Suitable parent enzymes may include any enzyme with esterase or lipase activity. Preferably, the parent enzyme aligns to the pfam00657 consensus sequence.

In a preferable embodiment a variant lipid acyltransferase enzyme retains or incorporates at least one or more of the pfam00657 consensus sequence amino acid residues found in the GDSx, GANDY and HPT blocks.

Enzymes, such as lipases with no or low lipid acyltransferase activity in an aqueous environment may be mutated using molecular evolution tools to introduce or enhance the transferase activity, thereby producing a lipid acyltransferase enzyme with significant transferase activity suitable for use in the compositions and methods of the present invention. Suitably, the lipid acyltransferase for use in the invention may be a variant with enhanced enzyme activity on polar lipids, preferably phospholipids and/or glycolipids when compared to the parent enzyme. Preferably, such variants also have low or no activity on lyso polar lipids. The enhanced activity on polar lipids, phospholipids and/or glycolipids may be the result of hydrolysis and/or transferase activity or a combination of both.

Variant lipid acyltransferases for use in the invention may have decreased activity on triglycerides, and/or monoglycerides and/or diglycerides compared with the parent enzyme.

Suitably the variant enzyme may have no activity on triglycerides and/or monoglycerides and/or diglycerides.

Alternatively, the variant enzyme for use in the invention may have increased activity on triglycerides, and/or may also have increased activity on one or more of the following, polar lipids, phospholipids, lecithin, phosphatidylcholine, glycolipids, digalactosyl monoglyceride, monogalactosyl monoglyceride.

Variants of lipid acyltransferases are known, one or more of such variants may be suitable for use in the methods and uses of the invention. For example, variants of lipid acyl transferases are described in the following references:

Hilton S, Buckley JT. Studies on the reaction mechanism of a microbial lipase/acyltransferase using chemical modification and site-directed mutagenesis.J Biol Chem. 1991 Jan 15;266(2):997-1000.

Robertson DL, Hilton S, Wong KR, Koepke A, Buckley JT. Influence of active site and tyrosine modification on the secretion and activity of the Aeromonas hydrophila lipase/acyltransferase.J Biol Chem. 1994 Jan 21;269(3):2145-50.

Brumlik MJ, Buckley JT.Identification of the catalytic triad of the lipase/acyltransferase from Aeromonas hydrophila. J Bacteriol. 1996 Apr;178(7):2060-4.

Peelman F, Vinaimont N, Verhee A, Vanloo B, Verschelde JL, Labeur C, Seguret-Mace S, Duverger N, Hutchinson G, Vandekerckhove J, Tavernier J, Rosseneu M. A proposed architecture for lecithin cholesterol acyl transferase (LCAT): identification of the catalytic triad and molecular modeling. Protein Sci. 1998 Mar;7(3):587-99.

AMINO ACID SEQUENCES

The present invention also encompasses amino acid sequences of polypeptides having the specific properties as defined herein.

As used herein, the term "amino acid sequence" is synonymous with the term "polypeptide" and/or the term "protein". In some instances, the term "amino acid sequence" is synonymous with the term "peptide".

The amino acid sequence may be prepared/isolated from a suitable source, or it may be made synthetically or it may be prepared by use of recombinant DNA techniques.

Suitably, the amino acid sequences may be obtained from the isolated polypeptides taught herein by standard techniques.

One suitable method for determining amino acid sequences from isolated polypeptides is as follows:

Purified polypeptide may be freeze-dried and 100 μg of the freeze-dried material may be dissolved in 50 μl of a mixture of 8 M urea and 0.4 M ammonium hydrogen carbonate, pH 8.4. The dissolved protein may be denatured and reduced for 15 minutes at 50°C following overlay with nitrogen and addition of 5 μl of 45 mM dithiothreitol. After cooling to room temperature, 5 μl of 100 mM iodoacetamide may be added for the cysteine residues to be derivatized for 15 minutes at room temperature in the dark under nitrogen.

135 μl of water and 5 μg of endoproteinase Lys-C in 5 μl of water may be added to the above reaction mixture and the digestion may be carried out at 37°C under nitrogen for 24 hours.

The resulting peptides may be separated by reverse phase HPLC on a VYDAC C18 column (0.46x15cm;10μm; The Separation Group, California, USA) using solvent A: 0.1 % TFA in water and solvent B: 0.1% TFA in acetonitrile. Selected peptides may be re-chromatographed on a Develosil C18 column using the same solvent system, prior to N-terminal sequencing. Sequencing may be done using an Applied Biosystems 476A sequencer using pulsed liquid fast cycles according to the manufacturer's instructions (Applied Biosystems, California, USA).

SEQUENCE IDENTITY OR SEQUENCE HOMOLOGY

The present invention also encompasses the use of sequences having a degree of sequence identity or sequence homology with amino acid sequence(s) of a polypeptide having the specific properties defined herein or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

The homologous amino acid sequence and/or nucleotide sequence should provide and/or encode a polypeptide which retains the functional activity and/or enhances the activity of the enzyme.

In the present context, a homologous sequence is taken to include an amino acid sequence which may be at least 75, 85 or 90% identical, preferably at least 95 or 98% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 75, 85 or 90% identical, preferably at least 95 or 98% identical to a nucleotide sequence encoding a polypeptide of the present invention (the subject sequence). Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 foi a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (Devereux et al 1984 Nuc. Acids Research 12 p387). Examples of other software that can perform sequence comparisons include, but are not limited to, the BLAST package (see Ausubel et al 1999 Short Protocols in Molecular Biology, 4th Ed - Chapter 18), FASTA (Altschul et al 1990 J. Mol. Biol. 403-410) and the GENEWORKS suite of comparison tools. Both BLAST and FASTA are available for offline and online searching (see Ausubel *et al* 1999, pages 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8 and tatiana@ncbi.nlm.nih.gov).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins DG & Sharp PM (1988), Gene 73(1), 237-244).

Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The present invention also encompasses homologous substitution (substitution and replacement are both used herein to mean the interchange of an existing amino acid residue, with an alternative residue) that may occur i.e, like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

Nucleotide sequences for use in the present invention or encoding a polypeptide having the specific properties defined herein may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vivo* activity or life span of nucleotide sequences.

The present invention also encompasses the use of nucleotide sequences that are complementary to the sequences discussed herein, or any derivative, fragment or derivative thereof. If the sequence is complementary to a fragment thereof then that sequence can be used as a probe to identify similar coding sequences in other organisms etc.

Polynucleotides which are not 100% homologous to the sequences of the present invention but fall within the scope of the invention can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences of the invention.

Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction polypeptide recognition sites, or to alter the property or function of the polypeptides encoded by the polynucleotides.

Polynucleotides (nucleotide sequences) of the invention may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the invention as used herein.

Polynucleotides such as DNA polynucleotides and probes according to the invention may be produced recombinantly, synthetically, or by any means available to those of skill in the art. They may also be cloned by standard techniques.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

## HYBRIDISATION

The present invention also encompasses sequences that are complementary to the sequences of the present invention or sequences that are capable of hybridising either to the sequences of the present invention or to sequences that are complementary thereto.

The term "hybridisation" as used herein shall include "the process by which a strand of nucleic acid joins with a complementary strand through base pairing" as well as the process of amplification as carried out in polymerase chain reaction (PCR) technologies.

The present invention also encompasses the use of nucleotide sequences that are capable of hybridising to the sequences that are complementary to the subject sequences discussed herein, or any derivative, fragment or derivative thereof.

The present invention also encompasses sequences that are complementary to sequences that are capable of hybridising to the nucleotide sequences discussed herein.

Hybridisation conditions are based on the melting temperature (Tm) of the nucleotide binding complex, as taught in Berger and Kimmel (1987, Guide to Molecular Cloning Techniques, Methods in Enzymology, Vol. 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridisation can be used to identify or detect identical nucleotide sequences while an intermediate (or low) stringency hybridisation can be used to identify or detect similar or related polynucleotide sequences.

Preferably, the present invention encompasses sequences that are complementary to sequences that are capable of hybridising under high stringency conditions or intermediate stringency conditions to nucleotide sequences encoding polypeptides having the specific properties as defined herein.

More preferably, the present invention encompasses sequences that are complementary to sequences that are capable of hybridising under high stringent conditions (e.g. 65°C and 0.1xSSC {1xSSC = 0.15 M NaCl, 0.015 M Na-citrate pH 7.0}) to nucleotide sequences encoding polypeptides having the specific properties as defined herein.

The present invention also relates to nucleotide sequences that can hybridise to the nucleotide sequences discussed herein (including complementary sequences of those discussed herein).

The present invention also relates to nucleotide sequences that are complementary to sequences that can hybridise to the nucleotide sequences discussed herein (including complementary sequences of those discussed herein).

Also included within the scope of the present invention are polynucleotide sequences that are capable of hybridising to the nucleotide sequences discussed herein under conditions of intermediate to maximal stringency.

In a preferred aspect, the present invention covers nucleotide sequences that can hybridise to the nucleotide sequences discussed herein, or the complement thereof, under stringent conditions (e.g. 50°C and 0.2xSSC).

In a more preferred aspect, the present invention covers nucleotide sequences that can hybridise to the nucleotide sequences discussed herein, or the complement thereof, under high stringent conditions (e.g. 65°C and 0.1xSSC).

## EXPRESSION OF POLYPEPTIDES

A nucleotide sequence for use in the present invention or for encoding a polypeptide having the specific properties as defined herein can be incorporated into a recombinant replicable vector. The vector may be used to replicate and express the nucleotide sequence, in polypeptide form, in and/or from a compatible host cell. Expression may be controlled using control sequences which include promoters/enhancers and other expression regulation signals. Prokaryotic promoters and promoters functional in eukaryotic cells may be used. Tissue specific or stimuli specific promoters may be used. Chimeric promoters may also be used comprising sequence elements from two or more different promoters described above.

The polypeptide produced by a host recombinant cell by expression of the nucleotide sequence may be secreted or may be contained intracellularly depending on the sequence and/or the vector used. The coding sequences can be designed with signal sequences which direct secretion of the substance coding sequences through a particular prokaryotic or eukaryotic cell membrane.

## EXPRESSION VECTOR

The term "expression vector" means a construct capable of *in vivo* or *in vitro* expression.

Preferably, the expression vector is incorporated in the genome of the organism. The term "incorporated" preferably covers stable incorporation into the genome.

The nucleotide sequence of the present invention or coding for a polypeptide having the specific properties as defined herein may be present in a vector, in which the nucleotide sequence is operably linked to regulatory sequences such that the regulatory sequences are capable of providing the expression of the nucleotide sequence by a suitable host organism, i.e. the vector is an expression vector.

The vectors of the present invention may be transformed into a suitable host cell as described below to provide for expression of a polypeptide having the specific properties as defined herein.

The choice of vector, e.g. plasmid, cosmid, virus or phage vector, will often depend on the host cell into which it is to be introduced.

The vectors may contain one or more selectable marker genes - such as a gene which confers antibiotic resistance e.g. ampicillin, kanamycin, chloramphenicol or tetracyclin resistance. Alternatively, the selection may be accomplished by co-transformation (as described in WO91/17243).

Vectors may be used *in vitro,* for example for the production of RNA or used to transfect or transform a host cell.

Thus, in a further embodiment, the invention provides a method of making nucleotide sequences of the present invention or nucleotide sequences encoding polypeptides having the specific properties as defined herein by introducing a nucleotide sequence into a replicable vector, introducing the vector into a compatible host cell, and growing the host cell under conditions which bring about replication of the vector.

The vector may further comprise a nucleotide sequence enabling the vector to replicate in the host cell in question. Examples of such sequences are the origins of replication of plasmids pUC19, pACYC177, pUB110, pE194, pAMB1 and pIJ702.

REGULATORY SEQUENCES

In some applications, a nucleotide sequence for use in the present invention or a nucleotide sequence encoding a polypeptide having the specific properties as defined herein may be operably linked to a regulatory sequence which is capable of providing for the expression of the nucleotide sequence, such as by the chosen host cell. By way of example, the present invention covers a vector comprising the nucleotide sequence of fhe present invention operably linked to such a regulatory sequence, i.e. the vector is an expression vector.

The term "operably linked" refers to a juxtaposition wherein the components described are in a relationship permitting them to function in their intended manner. A regulatory sequence "operably linked" to a coding sequence is ligated in such a way that expression of the coding sequence is achieved under conditions compatible with the control sequences.

The term "regulatory sequences" includes promoters and enhancers and other expression regulation signals.

The term "promoter" is used in the normal sense of the art, e.g. an RNA polymerase binding site. Enhanced expression of the nucleotide sequence encoding the enzyme having the specific properties as defined herein may also be achieved by the selection of heterologous regulatory regions, e.g. promoter, secretion leader and terminator regions.

Preferably, the nucleotide sequence of the present invention may be operably linked to at least a promoter.

Examples of suitable promoters for directing the transcription of the nucleotide sequence in a bacterial, fungal or yeast host are well known in the art.

CONSTRUCTS

The term "construct" - which is synonymous with terms such as "conjugate", "cassette" and "hybrid" - includes a nucleotide sequence encoding a polypeptide having the specific properties as defined herein for use according to the present invention directly or indirectly attached to a promoter. An example of an indirect attachment is the provision of a suitable spacer group such as an intron sequence, such as the Sh1-intron or the ADH intron, intermediate the promoter and the nucleotide sequence of the present invention. The same is true for the term "fused" in relation to the present invention which includes direct or indirect attachment. In some cases, the terms do not cover the natural combination of the nucleotide sequence coding for the protein ordinarily associated with the wild type gene promoter and when they are both in their natural environment.

The construct may even contain or express a marker which allows for the selection of the genetic construct.

For some applications, preferably the construct comprises at least a nucleotide sequence of the present invention or a nucleotide sequence encoding a polypeptide having the specific properties as defined herein operably linked to a promoter.

HOST CELLS

The term "host cell" - in relation to the present invention includes any cell that comprises either a nucleotide sequence encoding a polypeptide having the specific properties as defined herein or an expression vector as described above and which is used in the recombinant production of a polypeptide having the specific properties as defined herein.

Thus, a further embodiment of the present invention provides host cells transformed or transfected with a nucleotide sequence of the present invention or a nucleotide sequence that expresses a polypeptide having the specific properties as defined herein. The cells will be chosen to be compatible with the said vector and may for example be prokaryotic (for example bacterial), fungal, yeast or plant cells. Preferably, the host cells are not human cells.

Examples of suitable bacterial host organisms are gram negative bacterium or gram positive bacteria.

Depending on the nature of the nucleotide sequence encoding a polypeptide having the specific properties as defined herein, and/or the desirability for further processing of the expressed protein, eukaryotic hosts such as yeasts or other fungi may be preferred. In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.

The use of suitable host cells, such as yeast, fungal and plant host cells - may provide for post-translational modifications (e.g. myristoylation, glycosylation, truncation, lapidation and tyrosine, serine or threonine phosphorylation) as may be needed to confer optimal biological activity on recombinant expression products of the present invention.

The host cell may be a protease deficient or protease minus strain.

ORGANISM

The term "organism" in relation to the present invention includes any organism that could comprise a nucleotide sequence according to the present invention or a nucleotide sequence encoding for a polypeptide having the specific properties as defined herein and/or products obtained therefrom.

Suitable organisms may include a prokaryote, fungus, yeast or a plant.

The term "transgenic organism" in relation to the present invention includes any organism that comprises a nucleotide sequence coding for a polypeptide having the specific properties as defined herein and/or the products obtained therefrom, and/or wherein a promoter can allow expression of the nucleotide sequence coding for a polypeptide having the specific properties as defined herein within the organism. Preferably the nucleotide sequence is incorporated in the genome of the organism.

The term "transgenic organism" does not cover native nucleotide coding sequences in their natural environment when they are under the control of their native promoter which is also in its natural environment.

Therefore, the transgenic organism of the present invention includes an organism comprising any one of, or combinations of, a nucleotide sequence coding for a polypeptide having the specific properties as defined herein, constructs as defined herein, vectors as defined herein, plasmids as defined herein, cells as defined herein, or the products thereof. For example the transgenic organism can also comprise a nucleotide sequence coding for a polypeptide having the specific properties as defined herein under the control of a heterologous promoter.

TRANSFORMATION OF HOST CELLS/ORGANISM

As indicated earlier, the host organism can be a prokaryotic or a eukaryotic organism. Examples of suitable prokaryotic hosts include *E. coli* and *Bacillus subtilis.*

Teachings on the transformation of prokaryotic hosts is well documented in the art, for example see Sambrook et al (Molecular Cloning: A Laboratory Manual, 2nd edition, 1989, Cold Spring Harbor Laboratory Press). If a prokaryotic host is used then the nucleotide sequence may need to be suitably modified before transformation - such as by removal of introns.

In another embodiment the transgenic organism can be a yeast.

Filamentous fungi cells may be transformed using various methods known in the art - such as a process involving protoplast formation and transformation of the protoplasts followed by regeneration of the cell wall in a manner known. The use of *Aspergillus* as a host microorganism is described in EP 0 238 023.

Another host organism can be a plant. A review of the general techniques used for transforming plants may be found in articles by Potrykus (Annu Rev Plant Physiol Plant Mol Biol [1991] 42:205-225) and Christou (Agro-Food-Industry Hi-Tech. March/April 1994 17-27). Further teachings on plant transformation may be found in EP-A-0449375.

General teachings on the transformation of fungi, yeasts and plants are presented in following sections.

TRANSFORMED FUNGUS

A host organism may be a fungus - such as a filamentous fungus. Examples of suitable such hosts include any member belonging to the genera Thermomyces, Acremonium, Aspergillus, Penicillium, Mucor, Neurospora, Trichoderma and the like.

Teachings on transforming filamentous fungi are reviewed in US-A-5741665 which states that standard techniques for transformation of filamentous fungi and culturing the fungi are well known in the art. An extensive review of techniques as applied to *N. crassa* is found, for example in Davis and de Serres, Methods Enzymol (1971) 17A: 79-143.

Further teachings on transforming filamentous fungi are reviewed in US-A-5674707.

In one aspect, the host organism can be of the *genus Aspergillus,* such as *Aspergillus niger.*

A transgenic *Aspergillus* according to the present invention can also be prepared by following, for example, the teachings of Turner G. 1994 (Vectors for genetic manipulation. In: Martinelli S.D., Kinghorn J.R.( Editors) Aspergillus: 50 years on. Progress in industrial microbiology vol 29. Elsevier Amsterdam 1994. pp. 641-666).

Gene expression in filamentous fungi has been reviewed in Punt et al. (2002) Trends Biotechnol 2002 May;20(5):200-6, Archer & Peberdy Crit Rev Biotechnol (1997) 17(4):273-306.

TRANSFORMED YEAST

In another embodiment, the transgenic organism can be a yeast.

A review of the principles of heterologous gene expression in yeast are provided in, for example, Methods Mol Biol (1995), 49:341-54, and Curr Opin Biotechnol (1997) Oct;8(5):554-60 .. In this regard, yeast - such as the species *Saccharomyces cerevisi or Pichia pastoris* (see FEMS Microbiol Rev (2000 24(1):45-66), may be used as a vehicle for heterologous gene expression.

A review of the principles of heterologous gene expression in *Saccharomyces cerevisiae* and secretion of gene products is given by E Hinchcliffe E Kenny (1993, "Yeast as a vehicle for the expression of heterologous genes", Yeasts, Vol 5, Anthony H Rose and J Stuart Harrison, eds, 2nd edition, Academic Press Ltd.).

For the transformation of yeast, several transformation protocols have been developed. For example, a transgenic Saccharomyces according to the present invention can be prepared by following the teachings of Hinnen et al., (1978, Proceedings of the National Academy of Sciences of the USA 75, 1929); Beggs, J D (1978, Nature, London, 275, 104); and Ito, H et al (1983, J Bacteriology 153, 163-168).
A suitable yeast host organism can be selected from the biotechnologically relevant yeasts species such as but not limited to yeast species such as *Pichia* sp., *Hansenula* sp or *Kluyveromyces, Yarrowinia* species or a species of *Saccharomyces* including *Saccharomyces cerevisiae* or a species belonging to *Schizosaccharomyce* such as, for example, *S. pombe*. species.
A strain of the methylotrophic yeast species *Pichia pastoris* can be used used as the host organism.
In one embodiment the host organism is a *Hansenula* species, such as *Hansenula polymorpha* (as described in WO01/38544).
The transformed yeast cells may be selected using various selective markers - such as auxotrophic markers dominant antibiotic resistance markers.

TRANSFORMED PLANTS/PLANT CELLS

A host organism suitable for the present invention may be a plant. A review of the general techniques may be found in articles by Potrykus (Annu Rev Plant Physiol Plant Mol Biol [1991] 42:205-225) and Christou (Agro-Food-Industry Hi-Tech March/April 1994 17-27), or in WO01/16308. The transgenic plant may produce enhanced levels of phytosterol esters and phytostanol esters, for example.
Therefore the present invention also relates to a method for the production of a transgenic plant with enhanced levels of phytosterol esters and phytostanol esters, comprising the steps of transforming a plant cell with a lipid acyltransferase as defined herein (in particular with an expression vector or construct comprising a lipid acyltransferase as defined herein), and growing a plant from the transformed plant cell.

SECRETION

Often, it is desirable for the polypeptide to be secreted from the expression host into the culture medium from where the enzyme may be more easily recovered. According to the present invention, the secretion leader sequence may be selected on the basis of the desired expression host. Hybrid signal sequences may also be used with the context of the present invention.
Typical examples of heterologous secretion leader sequences are those originating from the fungal amyloglucosidase (AG) gene (*glaA* - both 18 and 24 amino acid versions e.g. from *Aspergillus*), the a-factor gene (yeasts e.g. *Saccharomyces. Kluyveromyces* and *Hansenula*) or the $\alpha$-amylase gene (*Bacillus*).

DETECTION

A variety of protocols for detecting and measuring the expression of the amino acid sequence are known in the art. Examples include enzyme-linked immunosorbent assay (ELISA), radioimmunoassay (RIA) and fluorescent activated cell sorting (FACS).
A wide variety of labels and conjugation techniques are known by those skilled in the art and can be used in various nucleic and amino acid assays.
A number of companies such as Pharmacia Biotech (Piscataway, NJ), Promega (Madison, WI), and US Biochemical Corp (Cleveland, OH) supply commercial kits and protocols for these procedures.
Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US-A-3,817,837; US-A-3,850,752; US-A-3,939,350; US-A-3,996,345; US-A-4,277,437; US-A-4,275,149 and US-A-4,366,241.
Also, recombinant immunoglobulins may be produced as shown in US-A-4,816,567.

FUSION PROTEINS

A polypeptide having the specific properties as defined herein may be produced as a fusion protein, for example to aid in extraction and purification thereof. Examples of fusion protein partners include glutathione-S-transferase (GST), 6xHis, GAL4 (DNA binding and/or transcriptional activation domains) and $\beta$-galactosidase. It may also be convenient to include a proteolytic cleavage site between the fusion protein partner and the protein sequence of interest to allow removal of fusion protein sequences. Preferably the fusion protein will not hinder the activity of the protein sequence.
Gene fusion expression systems in *E. coli* have been reviewed in Curr. Opin. Biotechnol. (1995) 6(5):501-6.

In another embodiment of the invention, the amino acid sequence of a polypeptide having the specific properties as defined herein may be ligated to a heterologous sequence to encode a fusion protein. For example, for screening of peptide libraries for agents capable of affecting the substance activity, it may be useful to encode a chimeric substance expressing a heterologous epitope that is recognised by a commercially available antibody.

The invention will now be described, by way of example only, with reference to the following Figures and Examples.

Figure 1 shows a pfam00657 consensus sequence from database version 6 (SEQ ID No. 1);

Figure 2 shows an amino acid sequence (SEQ ID No. 2) obtained from the organism *Aeromonas hydrophila* (P10480; GI:121051);

Figure 3 shows an amino acid sequence (SEQ ID No. 3) obtained from the organism *Aeromonas salmonicida* (AAG098404; GI:9964017);

Figure 4 shows an amino acid sequence (SEQ ID No. 4) obtained from the organism *Streptomyces coelicolor* A3(2) (Genbank accession number NP_631558);

Figure 5 shows an amino acid sequence (SEQ ID No. 5) obtained from the organism *Streptomyces coelicolor* A3(2) (Genbank accession number: CAC42140);

Figure 6 shows an amino acid sequence (SEQ ID No. 6) obtained from the organism *Saccharomyces cerevisiae* (Genbank accession number P41734);

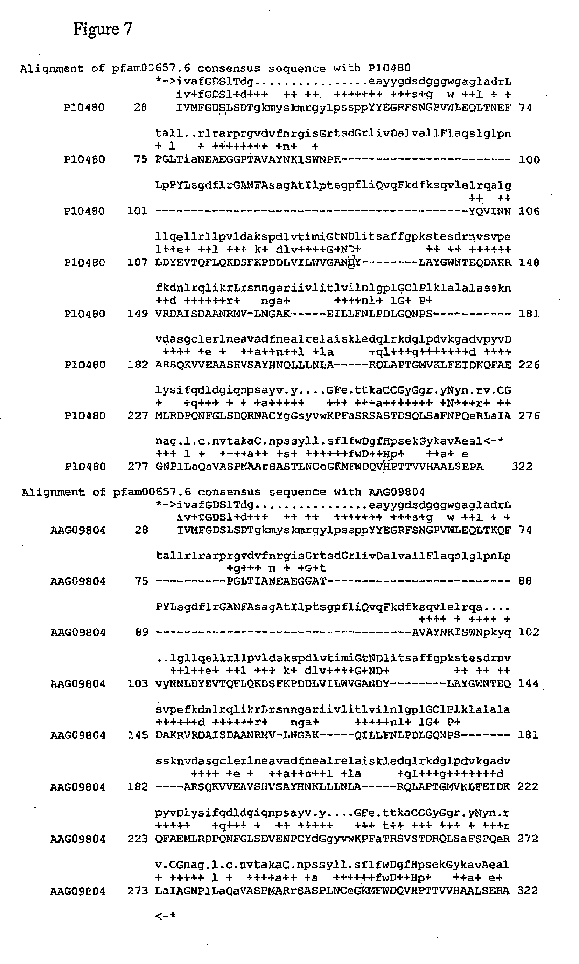

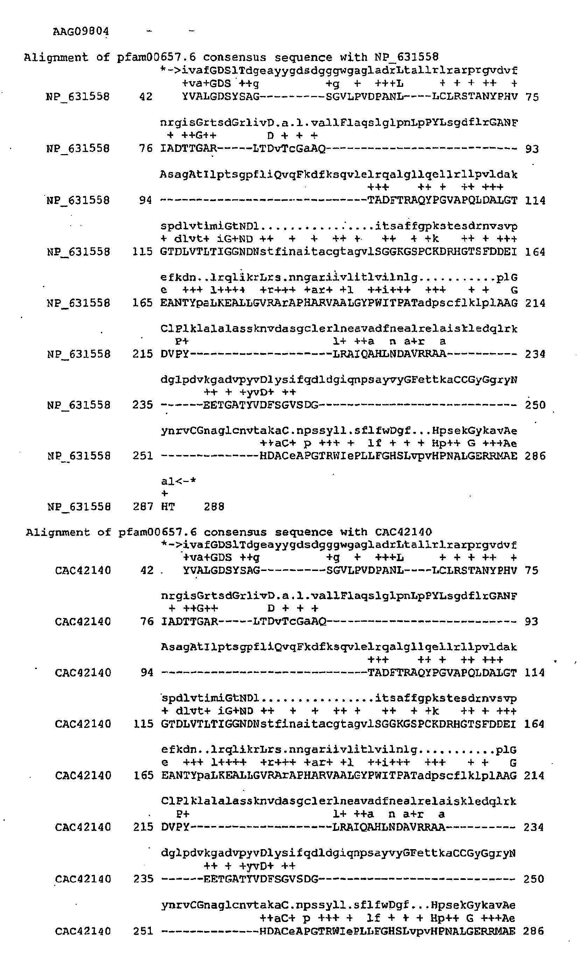

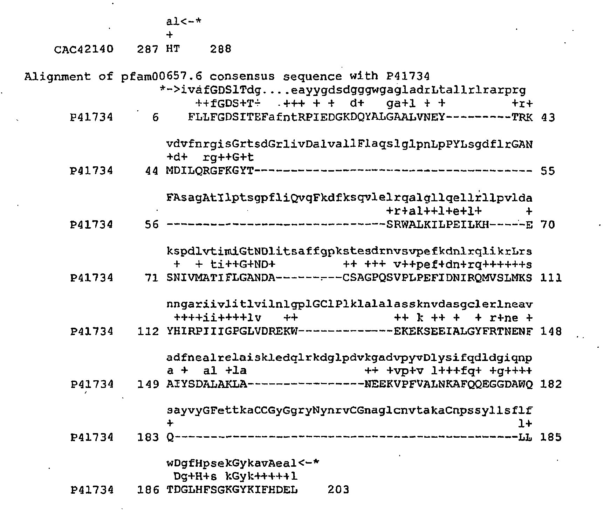

Figure 7 shows an alignment of selected sequences to pfam00657 consensus sequence;

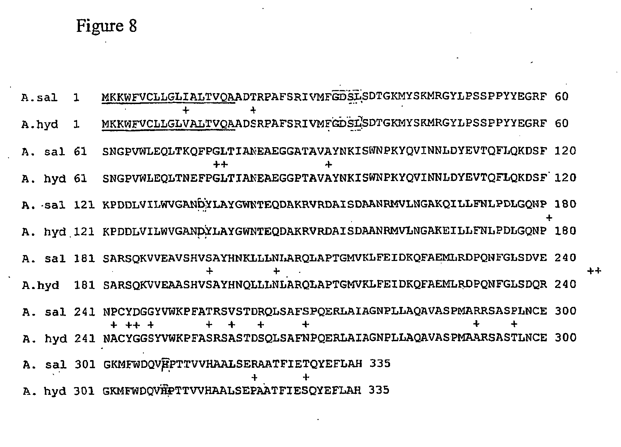

Figure 8 shows a pairwise alignment of SEQ ID No. 3 with SEQ No. 2 showing 93% amino acid sequence identity. 'The signal sequence is underlined. + denotes differences. The GDSX motif containing the active site serine 16, and the active sites aspartic acid 116 and histidine 291 are highlighted (see shaded regions). Numbers after the amino acid is minus the signal sequence;

Figure 9 shows a nucleotide sequence (SEQ ID No. 7) encoding a lipid acyl transferase according to the present invention obtained from the organism *Aeromonas hydrophila;*

Figure 10 shows a nucleotide sequence (SEQ ID No.8) encoding a lipid acyl transferase according to the present invention obtained from the organism *Aeromonas salmonicida;*

Figure 11 shows a nucleotide sequence (SEQ ID No. 9) encoding a lipid acyl transferase according to the present invention obtained from the organism *Streptomyces coelicolor* A3(2) (Genbank accession' number NC_003 888.1:8327480..8328367);

Figure 12 shows a nucleotide sequence (SEQ ID No. 10) encoding a lipid acyl transferase according to the present invention obtained from the organism *Streptomyces coelicolor* A3(2) (Genbank accession number AL939131.1:265480..265367);

Figure 13 shows a nucleotide sequence (SEQ ID No. 11) encoding a lipid acyl transferase according to the present invention obtained from the organism *Saccharomyces cerevisiae* (Genbank accession number 275034);

Figure 14 shows an amino acid sequence (SEQ ID No. 12) obtained from the organism *Ralstonia* (Genbank accession number: AL646052);

Figure 15 shows a nucleotide sequence (SEQ ID No. 13) encoding a lipid acyl transferase according to the present invention obtained from the organism *Ralstonia;*

Figure 16 shows SEQ ID No. 20. Scoe1 NCBI protein accession code CAB39707.1 GI:4539178 conserved hypothetical protein [Streptomyces coelicolor A3(2)];

Figure 17 shows a nucleotide sequence shown as SEQ ID No. 21 encoding NCBI protein accession code CAB39707.1 GI:4539178 conserved hypothetical protein [Streptomyces coelicolor A3(2)];

Figure 18 shows an amino acid shown as SEQ ID No.22. Scoe2 NCBI protein accession code CAC01477.1 GI:9716139 conserved hypothetical protein [Streptomyces coelicolor A3(2)];

Figure 19 shows a nucleotide sequence shown as SEQ ID No. 23 encoding Scoe2 NCBI protein accession code CAC01477.1 GI:9716139 conserved hypothetical protein [Streptomyces coelicolor A3(2)];

Figure 20 shows an amino acid sequence (SEQ ID No.24) Scoe3 NCBI protein accession code CAB88833.1 GI:7635996 putative secreted protein. [Streptomyces coelicolor A3(2)];

Figure 21 shows a nucleotide sequence shown as SEQ ID No. 25 encoding Scoe3 NCBI protein accession code CAB88833.1 GI:7635996 putative secreted protein. [Streptomyces coelicolor A3(2)];

Figure 22 shows an amino acid sequence (SEQ ID No.26) Scoe4 NCBI protein accession code CAB89450.1 GI:7672261 putative secreted protein. [Streptomyces coelicolor A3(2)];

Figure 23 shows an nucleotide sequence shown as SEQ ID No. 27 encoding Scoe4 NCBI protein accession code CAB89450.1 GI:7672261 putative secreted protein. [Streptomyces coelicolor A3(2)];

Figure 24 shows an amino acid sequence (SEQ ID No.28) Scoe5 NCBI protein accession code CAB62724.1 GI:6562793 putative lipoprotein [Streptomyces coelicolor A3(2)];

Figure 25 shows a nucleotide sequence shown as SEQ ID No. 29, encoding Scoe5 NCBI protein accession code CAB62724.1 GI:6562793 putative lipoprotein [Streptomyces coelicolor A3(2)];

Figure 26 shows an amino acid sequence (SEQ ID No.30) Srim1 NCBI protein accession code AAK84028.1 GI:15082088 GDSL-lipase [Streptomyces rimosus];

Figure 27 shows a nucleotide sequence shown as SEQ ID No. 31 encoding Srim1 NCBI protein accession code AAK84028.1 GI:15082088 GDSL-lipase [Streptomyces rimosus];

Figure 28 shows an amino acid sequence (SEQ ID No.32)A lipid acyl transferase from *Aeromonas hydrophila* (ATCC #7965);

Figure 29 shows a nucleotide sequence (SEQ ID No. 33) encoding a lipid acyltransferase from *Aeromonas hydrophila* (ATCC #7965);

Figure 30 shows an amino acid sequence (SEQ ID No.34) of a lipid acyltransferase from *Aeromonas salmonicida* subsp. *Salmonicida* (ATCC#14174);

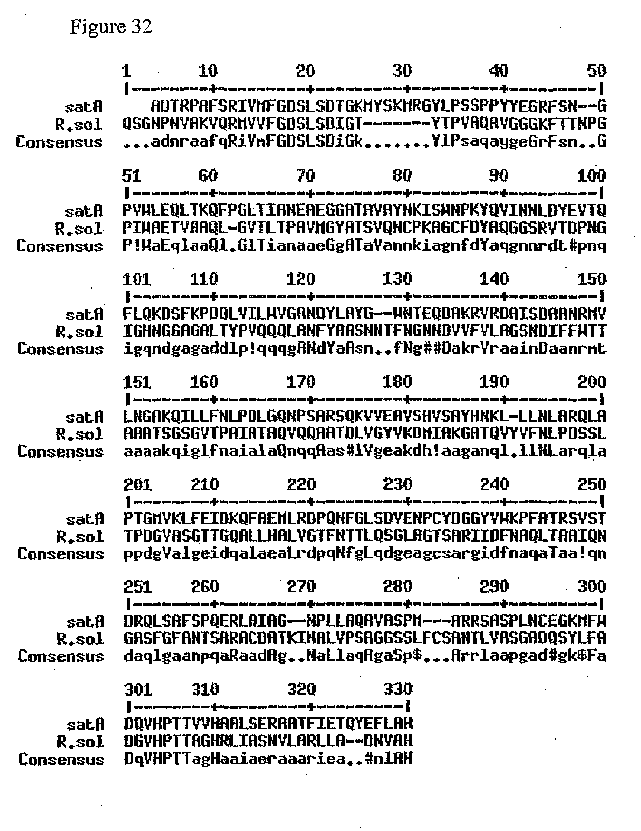

Figure 31 shows a nucleotide sequence (SEQ ID No 35) encoding a lipid acyltransferase from *Aeromonas salmonicida* subsp. *Salmonicida* (ATCC#14174); Figure 32 shows that homologues of the *Aeromonas* genes can be identified using the basic local alignment search tool service at the National Center for Biotechnology Information, NIH, MD, USA and the completed genome databases. The GDSX motif was used in the database search and a number of sequences/genes potentially encoding enzymes with lipolytic activity were identified. Genes were identified from the genus Streptomyces, Xanthomonas and Ralstonia. As an example below, the Ralstonia solanacearum was aligned to the Aeromonas salmonicida (satA) gene. Pairwise alignment showed 23% identity. The active site serine is present at the amino terminus and the catalytic residues histidine and aspartic acid can be identified;

Figure 33 shows the Pfam00657.11 [family 00657, database version 11] consensus sequence (hereafter called Pfam consensus) and the alignment of various sequences to the Pfam consensus sequence. The arrows indicate the active site residues, the underlined boxes indicate three of the homology boxes indicated by [Upton C and Buckley JT (1995) Trends Biochem Sci 20; 179-179]. Capital letters in the Pfam consensus indicate conserved residues in many family members. The - symbol indicates a position where the hidden Markov model of the Pfam consensus expected to find a residue but did not, so a gap is inserted. The symbol indicates a residue without a corresponding residue in the Pfam consensus. The sequences are the amino acid sequences listed in Figures 16, 18, 20, 22, 24, 26, 28 and 30.

Figure 34 shows the Pfam00657.11 [family 00657, database version 11] consensus sequence (hereafter called Pfam consensus) and the alignment of various sequences to the Pfam consensus sequence. The arrows indicate the active site residues, the underlined boxes indicate three of the homology boxes indicated by [Upton C and Buckley JT (1995) Trends Biochem Sci 20; 179-179]. Capital letters in the Pfam consensus indicate conserved residues in many family members. The - symbol indicates a position where the hidden Markov model of the Pfam consensus expected to find a residue but did not, so a gap is inserted. The symbol indicates a residue without a corresponding residue in the Pfam consensus. The sequences are the amino acid sequences listed in Figures 2, 16, 18, 20, 26, 28 and 30. All these proteins were found to be active against lipid substrates.

Figure 35 shows a expression vector pet12-AsalGCAT= pSM containing the C-terminal His-tagged *Aeromonas salmonicida* lipid acyltransferase gene;

Figure 36 shows the results of testing cell extracts in a NEFA Kit Assay, which depicts the activity of a recombinant, *A. salmonicida* lipid acyltransferase, towards lecithin. The wells from left to right indicate: a positive control, a negative control (i.e. extracts from empty plasmid) and samples collected after 0, 1, 2 and 3 hours cultivation after IPTG induction;

Figure 37 shows growth optimisation of BL21(DE3)pLysS harboring the expression vector pet12-AsalGCAT= pSM showing cultivation at 30 °C resulted in the production of enzyme with high activity towards lecithin. Cell extracts were tested for phospholipase activity using the NEFA kit assay. Wells from left to right: positive control; negative control; 20°C; 30°C;

Figure 38 shows crude cell extracts from BL21(DE3)pLysS expressing active lipid acyltransferase incubated with the substrate lecithin and reaction mixture was analyzed using thin layer chromatography showing the presence of degradation products. Lanes: 1. No enzyme; 2. + A.sal -10ul 37°C; 3. + A. sal -20ul 37°C; 4. + A.sal - 10ul 24°C; 5. + A. sal -20u 24°C;

Figure 39 shows partial purification of the *Aeromonas salmonicida* Acyl Transferase showing the phospholipase activity associated with purified His-tag protein. SE = Sonicated extracts, His = Purified with Ni-NTA spin-kit from Qiagen;

Figure 40 shows the expression vector pet12-A.h. GCAT=pSMa containing the C-terminal His-tagged *Aeromonas hydrophila* Glycerolipid Acyl Transferase (GCAT) gene was used to transform *E.coli* strain BL21(DE3)pLysS;

Figure 41 shows the activity of the crude extracts (5 & 10ul) containing the recombinant *Aeromonas hydrophila* GCAT enzyme was tested towards lecithin using Non-Esterified Fatty Acid (NEFA) kit (Roche, Switzerland), showing the presence of active enzyme towards the phospholipid, lecithin;

Figure 42 shows growth optimisation of BL21(DE3)pLysS harboring the expression vector pet12-AsalGCAT= pSM showing cultivation at 30 °C resulted in the production of enzyme with high activity towards lecithin. Cell extracts were tested for phospholipase activity using the NEFA kit assay;

Figure 43 shows the partial purification of the *Aeromonas hydrophila & A. salmonicida* Acyl Transferases showing the phospholipase activity associated with purified His-tag protein. SE = Sonicated extracts, His = Purified with Ni-NTA spin-kit from Qiagen);

Figure 44 shows the expression of the *Aeromonas* genes in *Bacillus subtilis* 163 showing the production of secreted enzyme with activity towards both lecithin and DGDG. pUB-AH= construct containing the *A. hydrophila* gene and pUB-AS, construct with the *A. salmonicida* gene, Culture filtrate was incubated with the substrates for 60 minutes.

Figure 45 and Figure 46 show graphs depicting fatty acid and cholesterol ester as a function of time. The graphs depict results obtained for GLC analysis in the assay for measurement of acyltransferase activity in a foodstuff using lecithin and cholesterol in buffer as substrate;

Figure 47 shows an amino acid sequence (SEQ ID No. 36) of the fusion construct used for mutagenesis of the *Aeromonas hydrophila* lipid acyltransferase gene in Example 17. The underlined amino acids is a xylanase signal peptide;

Figure 48 shows a nucleotide sequence (SEQ ID No. 54) encoding an enzyme from *Aeromonas hydrophila* including a xylanase signal peptide;

Figure 49 shows the structure of protein-fatty acid condensates of amino acids;

Figure 50 shows a schematic representing the reaction between a fatty acid from phosphatidylcholine when transferred to the free hydroxyl group of amino acids having a free hydroxyl group available for esterification, e.g. tyrosine or serine; and

Figure 51 shows a schematic of the reaction between DGDG and glucose when catalysed by a lipid.acyltransferase.

EXAMPLES

**<u>EXAMPLE 1: The cloning, sequencing and heterologous expression of a</u>**

**<u>transferase from *Aeromonas salmonicida* subsp. *Salmonicida*</u>**

**Strains used:**

*Aeromonas salmonicida subsp. Salmanicida* (ATCC 14174) was obtained from ATCC and grown overnight at 30°C in Luria-Bertani medium (LB). The cells were centrifuged and genomic DNA was isolated using the procedures for genomic DNA isolation from Qiagen Ltd. Genomic DNA buffer set (cat.19060), protease K (cat. 19131) and RNAse A (cat. 19101) were all obtained from Qiagen Ltd. (Boundary court Gatwick Court, West Sussex, RH10 2AX).

Host bacterial strain BL21(DE3)pLysS (Novagen) was used for production of the recombinant *Aeromonas* enzymes. Competent cells of BL21(DE3)pLysS were used as host for transformation with the expression vector **pet12-AsalGCAT=pSM.** Transformants containing the appropriate plasmid were grown at 37 °C in LB agar medium containing 100-ug ampicillin/ml.

**Construction of expression vector pet12-AsalGCAT- pSM:**

For all DNA amplifications of the transferase genes from *Aeromonas,* genomic DNA (0.2-1 ul) was used as template and *pfu* DNA polymerase (2.5 units) was used with 10ul of 10x pfu buffer, 1ul each primer (50pmol/ul), 200 uMdNTP in a total reaction volume of 100ul. PCR reactions were performed in a programmable thermal cycler using the following conditions: 95 °C for 30 seconds, 30 cycles of 95 °C for 30 seconds, 60 °C for 1 minute and 68 °C for 2 minutes. An additional extension of 5 minutes at 72 °C was applied.

The PCR amplification of the transferase gene from *A. salmonicida* was carried in 2 separate PCR reactions. PCR reaction 1 was performed using primer pairs, asIUSNEW(5'AGCATATGAAAA AATGGTTTGT TTGTTTATTG GGG 3' [SEQ ID No. 68]) and asls950new (5' GTG ATG GTG GGC GAG GAA CTC GTA CTG3' [SEQ ID No. 37]). A second PCR reaction was performed to incorporate a C-terminal Histidine tag using the PCR product from the first reaction and the primers: as1USNEW(5'AGCATATGAAAA AATGGTTTGT TTGTTTATTG GGG 3' [SEQ ID No. 38]) and AHLS1001(5'TTGGATCC GAATTCAT CAATG GTG ATG GTG ATG GTG GGC3' [SEQ ID No. 39]). The PCR product from the second reaction was purified and digested with restriction enzymes Ndel and BamHI. 2 ug of pET 12a vector DNA was also digested with restriction enzymes Ndel and BamHI and treated with phosphatase. The restriction enzyme-treated pet12a and PCR product from reaction 2 were purified and ligated using the Rapid Ligation Kit (Roche, Switzerland). The ligation mix was used to transform *E. coli* TOP10 cells. Transformants were plated on LB agar medium containing 100ug/ml ampicillin.

The T7 promoter primer (5'TAATACGACTCACTATAG3' [SEQ ID No. 40]) and the T7 terminator primer (5'CTAGTTATTGCTCAGCGG3' [SEQ ID No. 41]) were used to verify the sequences and the orientation of the cloned transferase genes in pET12a vector. DNA sequencing was performed using ABI Prism® BigDye™ Terminators Cycle sequencing kit with 500ng plasmid DNA as template and 3.2pmol T7 promoter and terminator primers.

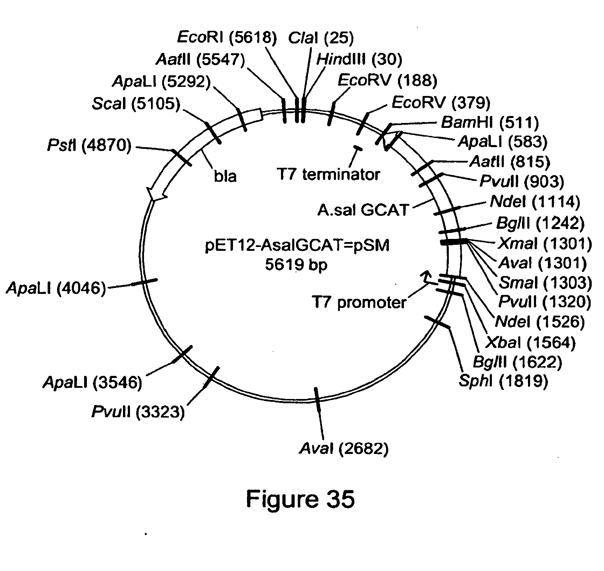

The construct shown in Figure 35 was used to transform competent bacterial host strain BL21(DE3)pLysS (Novagen) and ampicillin resistant transformants were picked and used for expression analysis

**Expression of the recombinant *Aeromonas salmonicida* lipid acyltransferase,**

Quantification of enzyme activity towards lecithin was determined on cell extracts using Non-Esterified Fatty Acid (NEFA) kit (Roche, Switzerland).

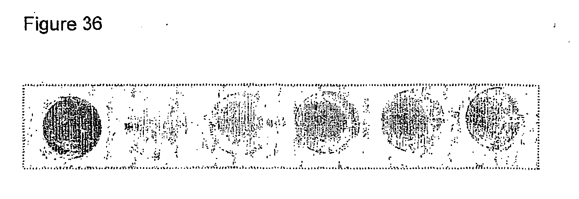

In Figure 36, BL21(DE3)pLysS harboring the expression vector pet12-AsalGCAT= pSM was grown in LB medium + 100ug/ml ampicillin and incubated with shaking at 37°C until $OD_{600}$ = 0.6 to1.0 is reached. The cultures are then induced using IPTG (0.4mM) and incubation was continued for the next 3 hours. Samples where taken at 0 hour, 1, 2, and 3 hours after IPTG induction. Enzyme Activity was tested using the NEFA. kit and lecithin as substrate.

Growth Optimisation for the production of more active enzymes

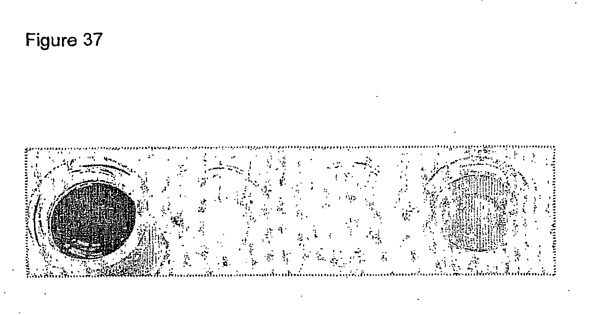

BL21(DE3)pLysS harboring the expression vector pet12-AsalGCAT= pSM was grown in LB medium + 100ug/ml ampicillin and incubated with shaking at different growth temperatures (37°C, 30 °C, & 20 °C). The optimal condition for the production of active lipid acyltransferase enzyme was when cultures are grown at 30°C as shown in Figure 37.

Partial purification of recombinant *Aeromonas salmonicida* transferase

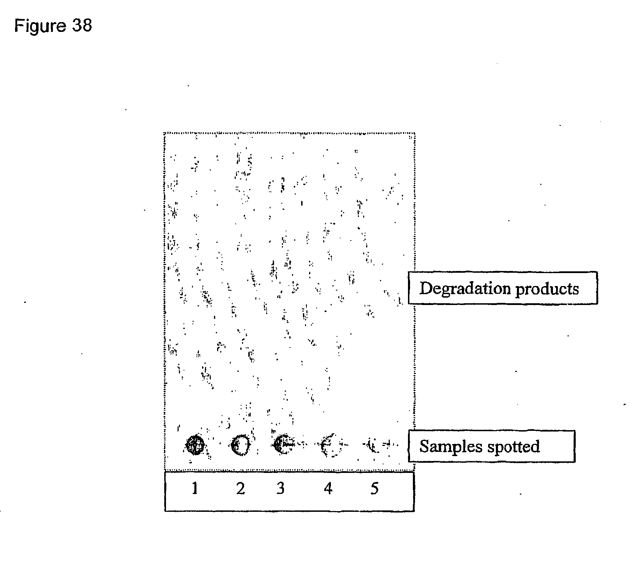

Strain BL21(DE3)pLysS harboring the expression vector pet12-ASalGCAT=pSM was grown at 37°C & crude cell extracts were prepared by sonication. The recombinant enzyme was further purified from the sonicated crude cell extracts using the Ni-NTA spin kit from Qiagen. Phospholipase activity using the NEFA kit & Lecithin as substrate. Crude cell extracts from BL21(DE3)pLysS expressing active transferase incubated with the substrate lecithin and reaction mixture was analysed using thin layer chromatography showing the presence of degradation products (see Figure 38).

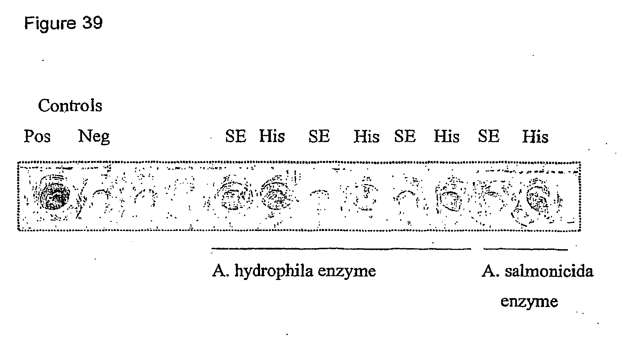

Partial Purification of recombinant *Aeromonas salmonicidae* transferase. Strain BL21(DE3)pLysS harbouring the expression vector pet12-AsalGCAT=pSM was grown at 37°C and crude cell extracts were prepared by sonication. The recombinant enzyme ware further purified from the sonicated crude cell extract using the Ni-NTA spin kit from Qiagen. Phospholipase activity using the NEFA kit and lecithin as substrate was tested (see Figure 39).

**<u>EXAMPLE 2 Cloning and Expression of *Aeromonas hydrophila* transferase in *E. coli*</u>**

*Aeromonas hydrophila* (ATCC # 7965) was obtained from ATCC and grown overnight at 30°C in Luria-Bertani medium (LB). The cells were centrifuged and genomic DNA was isolated using the procedures for genomic DNA isolation from Qiagen Ltd. Genomic DNA buffer set (cat.19060), protease K (cat. 19131) and RNAse A (cat. 19101) were all obtained from Qiagen Ltd. (Boundary court Gatwick Court, West Sussex, RH102AX).

Host bacterial strain BL21(DE3)pLysS (Novagen) was used for production of the recombinant *Aeromonas* enzymes. Competent cells of BL21(DE3)pLysS were used as host for transformation with the expression vector pet12a-A.h.GCAT=pSMa. Transformants containing the appropriate plasmid were grown at 37 °C in LB agar medium containing 100-ug ampicillin/ml.

**Construction of expression vector pet12a-A.h.GCAT- pSMa:**

For all DNA amplifications of the transferase gene from *Aeromonas,* genomic DNA (0.2-1 ul) was used as template and *pfu* DNA polymerase (2.5 units) was used with 10ul of 10x pfu buffer, 1ul each primer (50pmol/ul), 200 uMdNTP in a total reaction volume of 100ul. PCR reactions were performed in a programmable thermal cycler using the following conditions: 95 °C for 30 seconds, 30 cycles of 95 °C for 30 seconds, 60 °C for 1 minute and 68 °C for 2 minutes. An additional extension of 5 minutes at 72 °C was applied.

The PCR amplification of the transferase gene from *A. hydrophila* (ATCC # 7965) was carried out in 2 separate PCR reactions.

PCR reaction 1 was performed using primer pairs, AHUS1 (5'GTCATATGAAAAAATGGTTTGTGTGTTTATTGGGATTGGTC3', SEQ ID No. 42) and ahls950 (5'ATGGTGATGGTGGGCGAGGAACTCGTACTG3', SEQ ID No. 43).

A second PCR reaction was performed to incorporate a C-terminal Histidine tag using the PCR product from the first reaction and the primer pairs:

AHUS1(5'GTCATATGAAAAAATGGTTTGTGTGTTTATTGGGATTGGTC3' SEQ ID No.. 44, ) and
AHLS1001(5'TTGGATCCGAATTCATCAATGGTGATGGTGATGGTGGGC3' SEQ ID No. 45).

The PCR product from the second reaction was purified and digested with restriction enzymes Ndel and BamHI. 2 ug of pET 12a vector DNA was also digested with restriction enzymes Ndel and BamHI and treated with phosphatase. The restriction enzyme-treated pet12a and PCR product from reaction 2 were purified and ligated using the Rapid Ligation Kit (Roche, Switzerland). The ligation mix was used to transform *E. coli* TOP10 cells. Transformants were plated on LB agar medium containing 100ug/ml ampicillin.

The T7 promoter primer (5'TAATACGACTCACTATAG3') and the T7 terminator primer (5'CTAGTTATTGCTCAGCGG3') were used to verify the sequences and the orientation of the cloned GCAT genes in pET12a vector. DNA sequencing was performed using ABI Prism® BigDye™ Terminators Cycle sequencing kit with 500ng plasmid DNA as template and 3.2pmol T7 promoter and terminator primers.

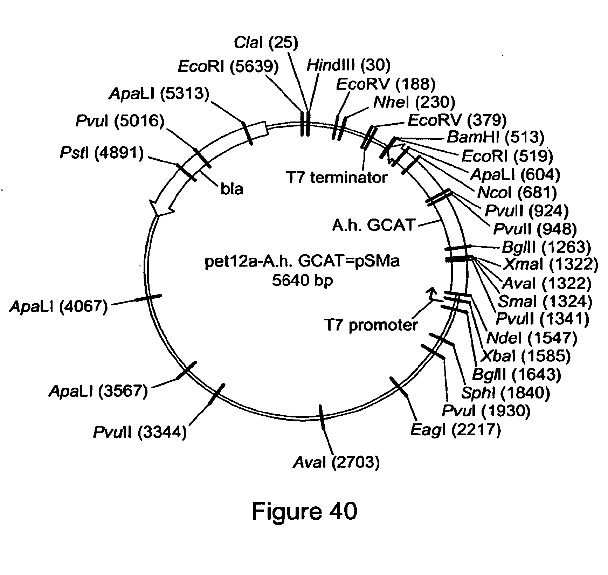

The construct shown in Figure 40 was used to transform competent bacterial host strain BL21 (DE3)pLysS (Novagen) and ampicillin resistant transformants were picked and used for expression analysis.

**Expression of the *Aeromonas hydrophila* transferases in BL21(DE3)pLysS**

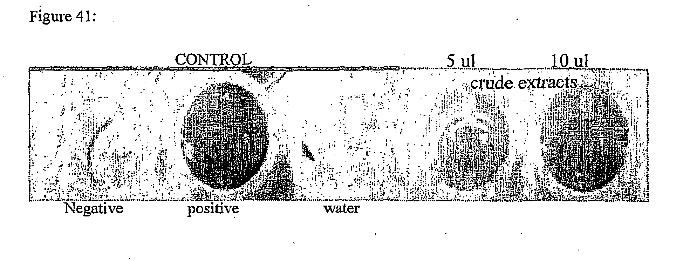

The *E. coli* strain BL21(DE3)pLysS harboring the expression vector pet12a-A.h.GCAT= pSMa was grown in LB medium + 100ug/ml ampicillin and incubated with shaking at 37°C until $OD_{600}$= 0.6 to 1.0 is reached. The cultures are then induced using IPTG (0.4mM) and incubation was continued for the next 3 hours. Samples where taken at 0hour, 1, 2, and 3 hours after IPTG induction. Enzyme Activity was tested using the NEFA kit and lecithin as substrate (Figure 41).

**Growth Optimisation for the production of more active enzymes**

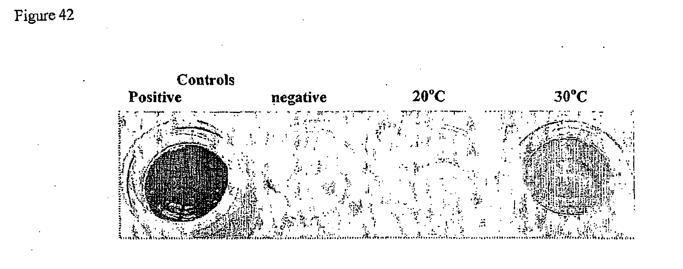

BL21(DE3)pLysS harboring the expression vector pet12a-A.h.GCAT= pSMa was grown in LB medium + 100ug/ml ampicillin and incubated with shaking at different growth temperatures (37°C, 30 °C, & 20 °C). The optimal condition for the production of active GCAT enzyme was when cultures are grown at 30°C as shown in Figure 42.

**Partial purification of recombinant *A.hydrophila* transferase (GCAT)**

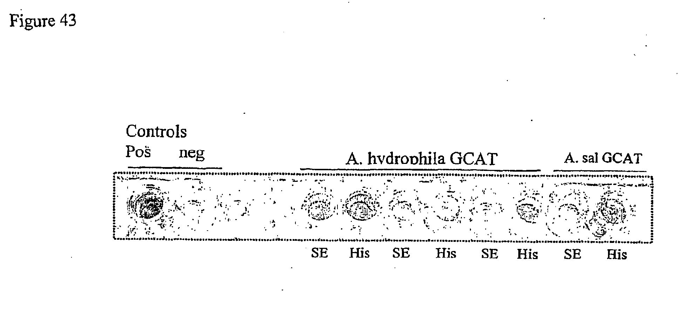

Strain BL21(DE3)pLysS harboring the expression vector pet12a-A.h.GCAT=pSMa was grown at 37°C & crude cell extracts were prepared by sonication. The recombinant enzyme was further purified from the sonicated crude cell extracts using the Ni-NTA spin kit from Qiagen. Phospholipase activity assay using the NEFA kit & Lecithin as substrate. (Figure 43).

**<u>EXAMPLE 3: Expression of *Aeromonas* transferases in *Bacillus subtilis 163*</u>**

**Plasmid Construction**

Two different *Bacillus subtilis* expression vectors (pUB 110 & pBE5) were used for the heterologous expression of the *Aeromonas* genes in *Bacillus subtilis.* The pUB110 vector contains the alpha amylase promoter while the pBE vector has the P32 promoter as the regulatory region for the expression of the fused *Aeromonas* genes. In pUB110, the first amino acid of the mature GCAT genes of *Aeromonas* were fused in frame with the last amino acid of the xylanase signal peptide sequence from *Bacillus subtilis* via the restriction site Nhe1, creating an additional 2 amino acids in front of the mature proteins. pBE5 contains the cgtase signal sequence fusion at the Nco1 site for secretion of the recombinant proteins into the culture filtrate.

PCR reactions were carried out to obtain the *Aeromonas* genes fuse in frame to the signal sequences of the pUB 110 and the pBE5 vectors. PCRs were performed using the following primer pairs for *A. hydrophila* gene:

PCR reaction 1: usAHncol (5'ATGCCATGGCCGACAGCCGTCCCGCC3', SEQ ID No. 46) and IsAH (5'TTGGATCCGAATTCATCAATGGTGATG3', SEQ ID No. 47)

PCR reaction 2: US-Ahnhel (5'TTGCTAGCGCCGACAGCCGTCCCGCC3', SEQ ID No. 48.) and IsAH (5'TTGGATCCGAATTCATCAATGGTGATG3, SEQ ID No. 49)

PCRs were performed using the following primer pairs for *A. salmonicida* gene:

PCR reaction 3: US-Asncol (5'TTGCCATGGCCGACACTCGCCCCGCC3', SEQ ID No. 50) and IsAH (5'TTGGATCCGAATTCATCAATGGTGATG3'; SEQ ID No. 51)

PCR reaction 4: US-ASnbel (5'TTGCTAGCGCCGACACTCGCCCCGCC3', SEQ ID No. 52) and IsAH (5'TTGGATCCGAATTCATCAATGGTGATG3', SEQ ID No. 53)

All the PCR products were cloned into PCR blunt II (TOPO vector) and sequenced with reverse & forward sequencing primers.

Clones from PCR reactions 1 & 3 were cut with Ncol & Bam HI and used as inserts for ligation to the pBE5 vector cut with Nco1/BamH1/phosphatase. Clones from PCR reactions 2 & 4 were cut with Nhe1 & Bam H1 and used as inserts for ligation to the pUB vector that was cut with Nhe1/BamH1/phosphatase..

**Expression of the *Aeromonas* transferase genes in *Bacillus subtilis* and characterization of the enzyme activity.**

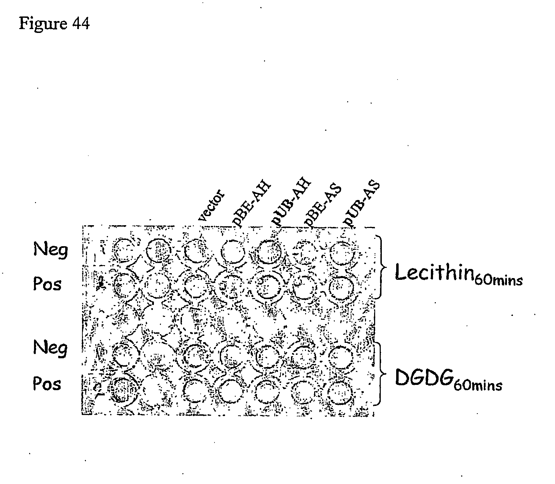

The acyl transferases from the two *Aeromonas* species have been successfully expressed in *E. coli* (results above). The *Bacillus* pUB110 & pBE5 gene fusion constructs were used to transform *Bacillus subtilis* and transfonnants were selected by plating on kanamycin plates. The kanamycin resistant transformants isolated and grown in 2xYT are capable of heterologous expression of the *Aeromonas* genes in *Bacillus.* The culture filtrates have digalactosyldiacylglycerol (DGDG) galactolipase activity, in addition to having both acyl transferase and phospholipase activities. The activity towards digalactosyldiacylglycerol (DGDG) was measured after 60 minutes of incubation of culture supernatant with the substrate, DGDG from wheat flour (obtainable form Sigma) as well as the activity towards lecithin as shown in Figure 44. *Bacillus* produced the enzyme after overnight (20-24 hours) to 48 hours of cultivation in the culture medium as a secreted protein. In some instances, the expression of the *Aeromonas* genes has been shown to interfere with cell viability and growth in *Bacillus & E. coli,* it is therefore necessary to carefully select expression strains and optimise the growth conditions to ensure expression. For example, several *Bacillus* host strains (B.s 163, DB104 and OS 21) were transformed with the expression vectors for growth comparison. B.s163 is transformable with the 2 *Aeromonas* genes and is capable of expressing active protein. DB104 is transformable with all the constructs but is only able to express *A. salmonicida* transferase.

**<u>EXAMPLE 4: Fermentation and Purification of *Aeromonas* lipid acyltransferases produced in *E.coli*</u>**

***E. coli* Fermentations:**

**Microorganisms**

Two strains of *Eschericia coli, one* containing an *Aeromonas hydrophila* (Example 2) lipid acyltransferase and two containing *Aeromonas salmonicida* lipid acyltransferases, (Example 1) were used in this study.

The *E. coli* strain containing the *A. hydrophila* gene was named DIDK0124, and the *E. coli* strain containing the *A. salmonicida* gene was named DIDK0125. The fermentation with DIDK0124 was named HYDRO0303 and the fermentation with DIDK0125 was named SAL0302. The purified protein from HYDRO025 was named REF#138. The purified protein from HYDRO0303 was named REF#135.

**Growth media and culture conditions**

**LB-agar**

The LB agar plates used for maintaining the strains contained: 10 g/L tryptone, 5 g/L yeast extract, 5 g/L NaCl, 15 g/L agar, 100 mg/L ampicillin and 35 mg/L chloramphenicol. The agar plates were incubated at 30°C.

**LB shake flask**

The LB medium (50 mL pr shake flask) used for production of inoculum material for the bioreactor cultivations contained: 10 g/L tryptone, 5 g/L yeast extract, 5 g/L NaCl, 100 mg/L ampicillin and 35 mg/L chloramphenicol. The shake flasks were inoculated from the LB agar plates, and incubated at 30°C and 200 rpm.

**Bioreactor cultivation**

The bioreactor cultivations were carried out in 6 L in-house built bioreactors filled with 4 L medium containing: 10 g/L tryptone, 5 g/L yeast extract, 5 g/L NaCl, 8 g/L $KH_2PO_4$, 0.9 g/L $MgSO_4$,$7H_2O$, 40 g/L glucose monohydrate, 0.4 mL/ ADD APT® Foamstop Sin 260 (ADD APT Chemicals AG, Helmond, The Netherlands), 10 mg/L $(NH_4)_2Fe(SO_4)_2{\cdot}6H_2O$, 0.7 mg/L $CuSO_4{\cdot}5H_2O$, 3 mg/L $ZnSO_4{\cdot}7H_2O$, 3 mg/L $MnSO_4{\cdot}H_2O$, 10 mg/L EDTA, 0.1 mg/L $NiSO_4{\cdot}6H_2O$, 0.1 mg/L $CoCl_2$, 0.1 mg/L $H_3BO_4$, 0.1 mg/L KI, 0.1 mg/L $Na_2MoO_4{\cdot}2H_2O$, 1 g/L ampicillin and 35 mg/L chloramphenicol.

The bioreactors were inoculated with an amount of LB culture ensuring end of growth after approximately 20 hours of cultivation (calculated from the maximum specific growth rate of 0.6 $h^{-1}$, the $OD_{600}$ of the LB shake flask and the final $OD_{600}$ in the bioreactor of approximately 20).

SAL0302 was inoculated with 10 mL of LB culture, and HYDRO0303 was inoculated with 4 mL of LB culture.

The bioreactors were operated at the following conditions: temperature 30°C, stirring 800-1000 rpm (depending on experiment), aeration 5 L/min, pH 6.9, pH control 8.75% (w/v) $NH_3$-water and 2 M $H_2SO_4$. Induction was achieved by addition of isopropyl β-D-thiogalactoside to a final concentration of 0.6 mM, when 0.4 moles (HYDRO0303) and 0.7

moles $CO_2$ was produced respectively.

### Harvest

The following procedure was used for harvest and homogenisation of the biomass:

1) The fermentation broth from the fermentations was centrifuged at 5000 × g and 4°C for 10 minutes, and the supernatant was discharged. The biomass was stored at -20°C until use. The biomass was thawed and resuspended in 500 mL of 20 mM $NaH_2PO_4$, pH 7.4, 500 mM NaCl, 10 mM Imidazole and Complete (EDTA-free) protease inhibitor (Roche, Germany).
2) The suspended biomass was homogenized at 2 kbar and 4°C in a cell disrupter from Constant Systems Ltd (Warwick, UK).
3) The cell debris was removed by centrifugation at 10.000 × g and 4°C for 30 minutes followed by collection of the supernatant.
4) The supernatant was clarified further by centrifugation at 13.700x g and 4°C for 60 minutes, followed by collection of the supernatant.
5) The supernatant was filtered through 0.2 μm Vacu Cap filters (Pall Life Sciences, UK) and the filtrate was collected for immediate chromatographic purification.

### Chromatographic purification of the Transferases

A column (2.5 x 10 cm) was packed with 50 ml of Chelating Sepharose ff. gel and charged with Ni-sulphate (according to the method described by manufacturer, Amersham Biosciences). The column was equilibrated with 200 ml of 20 mM $NaH_2PO_4$, pH 7.4, 500 mM NaCl, 10 mM Imidazole. 400 ml of crude was applied to the column at a flow rate of 5 ml/min. The column was then washed with 20 mM $NaH_2PO_4$, pH 7.4, 500 mM NaCl, 10 mM Imidazole until the $UV_{280}$ reached the base line. The GCAT was then eluted with 40 ml of 20 mM $NaH_2PO_4$, pH 7.4, 500 mM NaCl and 500 mM Imidazole.

## EXAMPLE 5: Fermentation and Purification of *Aeromonas* lipid acyltransferases produced in *Bacillus subtilis*.

### Fermentations

BAC0318-19, BAC0323-24

### Microorganism

The microorganisms used in this study originate from transformation of a *Bacillus subtilis* host strain, #163 with a plasmid containing the gene encoding the *Aeromonas salmonicida* transferase inserted in the vector pUB110OIS. The expression of the gene is controlled by an alpha-amylase promoter, and the secretion of the transferase is mediated by the *B. subtilis* xylanase signal sequence (Example 3). The strains were named DIDK0138 (fermentation BAC0318-19) and DIDK0153 (fermentation BAC0323-24).

### Growth media and culture conditions

### Pre culture medium

A shake flask (500 mL total volume, with baffles) was added 100 mL of a medium containing:

| | |
|---|---|
| NaCl | 5 g/L |
| $K_2HPO_4$ | 10 g/L |
| Soy flour | 20 g/L |
| Yeast extract, BioSpringer 106 | 20 g/L |
| Antifoam, SIN260 | 5 mL/L |

pH was adjusted to 7.0 before autoclaving

After autoclaving 6 mL 50% (w/w) Nutriose were added pr flask. Kanamycin was added at a concentration of 50 mg/L after autoclaving.

**Inoculation**

A pre culture shake flask was inoculated with frozen culture directly from a 25% (w/v) glycerol stock. The shake flask was incubated at 33°C and 175 rpm for approximately 16 hours, whereupon 50 mL was used to inoculate the fermentor.

*Fermentations*

The fermentations were carried out in 6 L in house built fermentors.
The batch medium (3 L) contained:

| | |
|---|---|
| Corn steep liquor (50% dw) | 40 g/L |
| Yeast extract BioSpringer 153 (50% dw) | 10 g/L |
| NaCl | 5 g/L |
| $CaCl_2$, $2H_2O$ | 0.25 g/L |
| $Mn(NO_3)_2$, $H_2O$ | 0.2 g/L |
| Antifoam SIN260 | 1 mL/L |
| Kanamycin (filter sterilised to the fermentor after autoclaving | 50 mg/L |

The feed contained:

| | |
|---|---|
| Glucose monohydrate | 540 g/kg |
| $MgSO_4$, $7H_2O$ | 4.8 g/kg |
| Antofoam SIN260 | 4 mL/kg |
| Yeast extract, BioSpringer 153 (50% dw) (autoclaved separately) | 150 g/kg |

The feed in fermentation BAC0318 and BAC0323 was started based on the accumulated $CO_2$, according to the equations below:

$$\text{Feed} - \text{flow}[g/h] = 0, AcCO_2 < 0.15$$

$$\text{Feed} - \text{flow}[g/h] = 2.85 + t \cdot 1.54, AcCO_2 \geq 0.15 \text{ and } t < 12$$

$$\text{Feed} - \text{flow}[g/h] = 21.3, t > 12$$

t: time (hours) from the point when the accumulated $CO_2$ ($AcCO_2$) reached 0.15 moles.
The feed in fermentation BAC0319 and BAC0324 was started based on the accumulated $CO_2$ according to the equations below:

$$\text{Feed} - \text{flow}[g/h] = 0, AcCO_2 < 0.15$$

$$\text{Feed} - \text{flow}[g/h] = 2.0 + t \cdot 1.08, AcCO_2 \geq 0.15 \text{ and } t < 12$$

$$\text{Feed} - \text{flow}[g/h] = 15, t > 12$$

t: time (hours) from the point when the accumulated $CO_2$ ($AcCO_2$) reached 0.15 moles.

The pH was controlled at 7.0 by adding 12.5%. (w/v) $NH_3$-water or 2M phosphoric acid.
The aeration was 3 L/min corresponding to 1 vvm.
The temperature was 33°C.
The fermentor was equipped with two 8 cm ø Rushton impellers placed with a distance of 10 cm.

**Harvest**

The biomass was removed by centrifugation at 16,000× g for 10 minutes at room temperature. The supernatant was filter sterilized, and the filtrate was used for purification and application tests.

**EXAMPLE 6 : The "Transferase Assay in Buffered Substrate" for measurement of acyltransferase activity of an enzyme.**

The lipid acyltransferase was isolated from *Aeromonas salmonicida* and expressed in *Bacillus subtilis.* This enzyme is very efficient in transferring fatty acid from lecithin to cholesterol during formation of cholesterol esters. It has also been shown that the enzyme has some hydrolytic activity, which is observed by the formation of free fatty acid. Traditional phospholipases (EC3.1.1.4 and EC3.1.1.32) have the ability to hydrolyse lecithin during formation of free fatty acids and lysolecithin, and no transferase reactions has been reported for these enzymes.
We detail herein an assay that is able to measure both transferase and hydrolytic activity of enzymes and thus to identify lipid acyltransferases in accordance with the present invention, the assay uses a substrate which contains lecithin and cholesterol. In this work a substrate based on phosphatidylcholine and cholesterol dispersed in a buffer was used. Quantification of reaction products was made by extraction of lipids from the substrate followed by GLC analysis of the lipid components.

Procedure

Materials

L-alpha-Phosphatidylcholine 95% (Plant) Avanti no. 441601
Cholesterol: Sigma cat C 8503
Cholesteryl Palmitate, Sigma C 6072
Cholesteryl Stearate, Sigma C 3549
HEPES buffer Sigma cat. No. H 3375
Chloroform, Analytical grade.

Enzymes

Purified GCAT from *A. salmonicida* #1.78-9

TLC analysis.

TLC-plate was activated in a heat cupboard (110°C) for ½ h.
100 ml running buffer was poured into a chromatography chamber with lid. The walls of the chamber were covered with filter paper (Whatman 2) in order to saturate the chamber with the solvent vapour.
The TLC-plate was placed in a frame and the sample was applied onto the TLC plate 2 cm from the bottom. The TLC plate was then placed in the TLC chamber with the running buffer. When the running buffer reached 14 cm from the bottom of the plate, the TLC plate was taken out and dried in fume board, and then placed in the heat cupboard at 110 °C for 10 minutes.
The TLC-plate was then immersed in the developing reagent, and dried in the heat cupboard at 110 °C for 15 minutes

Running-buffer:

Nr. IV: Chloroform : Methanol : $H_2O$ (65:25:4)
Nr. I: P-ether : MTBE : Acetic acid (60:40:1)

Developing buffer (Vanadate-buffer):

32 g $Na_2CO_3$ ad 300 ml $H_2O$ (1M)
18.2 g vanadate pentoxide ($V_2O_5$) is added and dissolved during gentle heating.
The solution is cooled to ambient.
Carefully 460 ml 2.5 M $H_2SO_4$. (460 ml $H_2O$ +61 ml $H_2SO_4$) is added
Water is added to 1000 ml.

GLC analysis

Perkin Elmer Autosystem 9000 Capillary Gas Chromatograph equipped with WCOT fused silica column 12.5 m x 0.25 mm ID x 0.1 μ film thickness 5% phenyl-methylsilicone (CP Sil 8 CB from Chrompack).

Carrier gas: Helium.

Injector. PSSI cold split injection (initial temp 50°C heated to 385°C), volume 1.0μl Detector FID: 395°C

| | | | |
|---|---|---|---|
| Oven program: | 1 | 2 | 3 |
| Oven temperature, °C. | 90 | 280 | 350 |
| Isothermal, time, min. | 1 | 0 | 10 |
| Temperature rate, °C/min. | 15 | 4 | |

Sample preparation: 30 mg of sample was dissolved in 9 ml Heptane:Pyridin, 2:1 containing internal standard heptadecane, 0.5 mg/ml. 300μl sample solution was transferred to a crimp vial, 300 μl MSTFA (N-Methyl-N-trimethylsilyl-trifluoraceamid) was added and reacted for 20 minutes at 60°C.

Calculation: Response factors for mono-di-triglycerides and free fatty acid are determined from Standard 2 (mono-di-triglyceride). The response factors for Cholesterol, Cholesteryl Palmitate and Cholesteryl Stearate were determined from pure reference materials.

Results: Transferase assay based on phosphatidylcholine and cholesterol as substrate.

In the following the transferase activity of the transferase was tested in a substrate based on phosphatidylcholine and cholesterol according to the following procedure.

450 mg phosphatidylcholine (>95% PC Avanti item no. 441601) and 50 mg cholesterol was dissolved in chloroform and evaporated to dryness under vacuum. 300 mg cholesterol/phosphatidylcholine mixture was transferred to a Wheaton glass and 15 ml 50mM HEPES buffer pH 7 was added. The lipid was dispersed in the buffer during agitation. The substrate was heated to 35 °C during mixing with a magnetic stirrer and 0.25 ml enzyme solution was added. This is a very high water environment of approximately 95% water.

Samples of 2 ml were taken out after 0, 5, 10, 15, 25, 40 and 60 minutes reaction time. Immediately 25 μl 4M HCl was added to acidify the free fatty acid and stop the enzyme reaction. 3.00 ml chloroform was added, and the sample was shaken vigorously on a Whirley for 30 seconds. The sample was centrifuged and 2 ml of the chloroform phase was isolated and filtered through 0.45-μm filters into a 10 ml tared Dram glass. The chloroform was evaporated under a stream of nitrogen at 60°C, and the samples were scaled again. The extracted lipid was analysed by GLC.

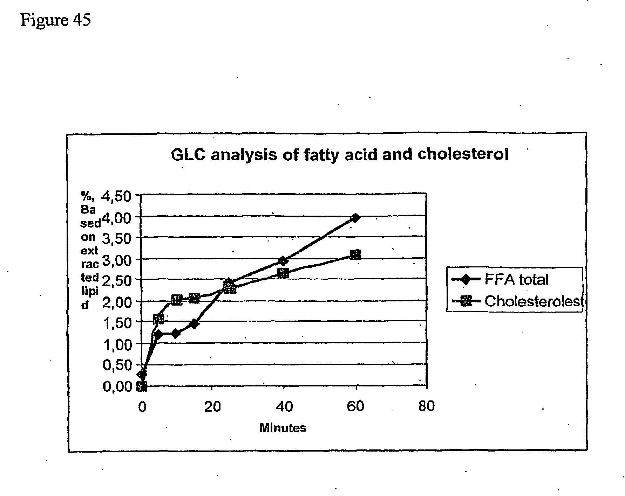

The results from the GLC analysis are shown in Table 1. The results are expressed in % calculated on extracted lipid. The amount of fatty acid and cholesterol ester formed as a function of time is illustrated in. Figure 45. It can be concluded from Figure 45 that the enzyme reaction is not linear as a function of time, because an initially strong both hydrolytic and transferase activity is observed. After approximately 10 minutes and until approximately 60 minutes the reaction shows an almost linear response of fatty acid and cholesterol ester formation as a function of time. It was therefore decided to look at the enzymatic reaction in this time interval.

**Table 1**

| Minutes | 0 | 5 | 10 | 15 | 25 | 40 | 60 |
|---|---|---|---|---|---|---|---|
| | | | | | | | |
| Cholesterol, % | 10.064 | 8.943 | 8.577 | 8.656 | 8.102 | 7.856 | 7.809 |
| Cholesterol ester, % | 0.000 | 1.571 | 2.030 | 2.058 | 2.282 | 2.659 | 3.081 |
| FFA total, % | 0.260 | 1.197 | 1.239 | 1.466 | 2.445 | 2.943 | 3.940 |

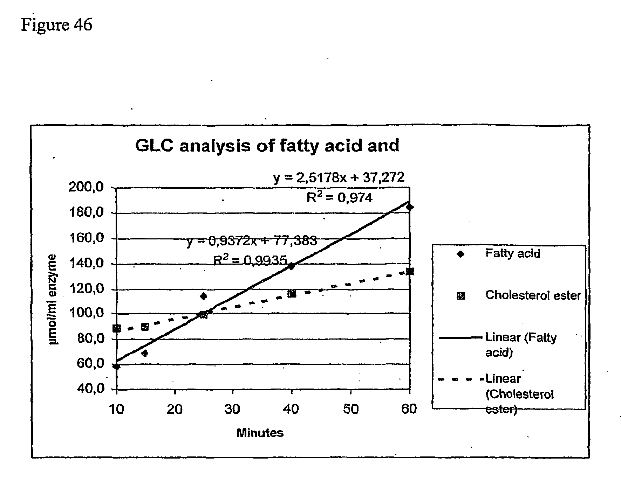

From the knowledge about the amount of lipid in the reaction mixture and the amount of enzyme added it was possible to calculate the formation of fatty acid and cholesterol ester expressed in μmol/ml enzyme (Table 2 and Figure 46).

**Table 2**

| Minutes | 10 | 15 | 25 | 40 | 60 |
|---|---|---|---|---|---|
| | μmol/ml | μmol/ml | μmol/ml | μmol/ml | μmol/ml |
| FFA total | 58.1 | 68.7 | 114.6 | 138.0 | 184.7 |
| Cholesterol ester | 88.8 | 90.0 | 99.3 | 115.6 | 133.8 |

From the results in Table 2 and the slope of the curves in Figure 46 it was possible to calculate the amount of fatty acid and cholesterol ester as a function of time expressed in μmol/min per ml enzyme.

The calculation of the hydrolytic activity and the transferase activity is shown in Table 3. The relative transferase activity was determined using the protocol for the determination of % acyltransferase activity as described hereinbefore.

**Table 3**

| | | |
|---|---|---|
| Hydrolytic activity (fatty acid) | 2.52 | μmol/min per ml enzyme |
| Transferase activity(cholesterol ester) | 0.94 | μmol/min per ml enzyme |
| Total activity | 3.45 | μmol/min per ml enzyme |
| | | |
| Relative Transferase activity | **27.1** | % |
| Relative hydrolytic activity | **72.9** | % |

**Screening of other enzymes for transferase activity.**

The method mentioned above was used to screen different lipolytic enzymes for transferase and hydrolytic activity. The enzymes were tested as shown in Table 4.

**Table 4**

| | | 1 | 2 | 3 | 4 | 5 |
|---|---|---|---|---|---|---|
| Substrate | ml | 15 | 15 | 15 | 15 | 15 |
| #178-9Transferase A. salmonicida 32 PLU-7/ml | ml | 0.25 | | | | |
| 5% #3016, LIPOPAN® F (F. oxysporum) | ml | | 0.25 | | | |
| 5%, Thermomyces lanuginosus | ml | ml | | 0.25 | | |
| 5% Candida rugosa #2983 | ml | | | | 0.25 | |
| 5% Candida cylindracea #3076 | ml | | | | | 0.25 |

The substrate containing 300mg phosphatidylcholine/cholesterol dispersed in 50 mM HEPES buffer pH 7.0 was heated to 35 °C with agitation. Enzyme solution was added and the sample was kept at 35 °C with agitation. Samples were taken out with regular interval and extracted with Chloroform. The isolated lipids were analysed by GLC with results shown in Table 5.

**Table 5**

| Sample | | | | | | | | |
|---|---|---|---|---|---|---|---|---|
| **1** | **Transferase 178-9** | | | | | | | |
| | Minutes | 0 | 5 | 10 | 15 | 25 | 40 | 6C |
| | FFA | 1.216 | 2.516 | 2.983 | 2.62 | 2.894 | 3.448 | 3.911 |

(continued)

| Sample | | | | | | | | |
|---|---|---|---|---|---|---|---|---|
| **1** | **Transferase 178-9** | | | | | | | |
| | Cholesterol | 7.547 | 6.438 | 6.365 | 6.15, | 6.136 | 5.936 | 5.662 |
| | Chl. Ester | 0 | 1.835 | 2.177 | 2.44 | 2.58 | 2.851 | 3.331 |
| | | | | | | | | |
| **2** | **Fusarium oxysporum (LIPOPAN® F)** | 0 | 5 | 10 | 15 | 25 | 40 | 60 |
| | FFA | 1.216 | 1.345 | 1.796 | 1.95 | 2.487 | 2.424 | 2.977 |
| | Cholesterol | 7.547 | 7.309 | 7.366 | 7.33 | 7.429 | 7.341 | 7.326 |
| | Chl. Ester | 0 | 0.26 | 0.386 | 0.35 | 0.267 | 0.36 | 0.394 |
| | | | | | | | | |
| **3** | **Thermomyces lanuginosus** | 0 | 5 | 10 | 15 | 25 | 40 | 60 |
| | FFA | 1.216 | 0.853 | 0.875 | 1 | 0.896 | 1.105 | 1.009 |
| | Cholesterol | 7.547 | 7.384 | 7.639. | 7.63 | 7.675 | 7.603 | 7.529 |
| | Chl. Ester | 0 | 0 | 0 | 0 | 0 | 0 | 0 |
| | | | | | | | | |
| **4** | **Candida rugosa (#2938)** | 0 | 5 | 10 | 15 | 25 | 40 | 60 |
| | FFA | 1.216 | 0.982 | 0.987 | 1.02 | 1,135 | 1.131 | 1.15 |
| | Cholesterol | 7.547 | 7.438 | 7.656 | 7.66 | 7.638 | 7.575 | 7.585 |
| | Chl. Ester | 0 | 0 | 0 | 0 | 0 | 0 | 0 |
| | | | | | | | | |
| **5** | **Candida cylandracea (#3076)** | 0 | 5 | 10 | 15 | 25 | 40 | 60 |
| | FFA | 1.216 | 1.032 | 1.097 | 1.07 | 1.203 | 1.131 | 1.43 |
| | Cholesterol | 7.547 | 7.502 | 7.425 | 7.65 | 7.619 | 7.502 | 7.411 |
| | Chl. Ester | 0 | 0 | 0 | 0 | 0 | 0 | 0 |

From the GLC analysis it was observed that only the lipid acyltransferase (178-9) produced significant amount of cholesterol ester and fatty acids. Phospholipase from *Fusarium oxysporum* also gave a steady increase in free fatty acid but only an initial small amount formation of cholesterol ester was formed but no increase in cholesterol ester as a function of time was observed.

Based on the knowledge about the amount of lipid substrate and the GLC analyses it was possible to calculate the relative transferase activity and the relative hydrolytic activity based on the results from 10 to 60 minutes reaction time. The results from Transferase 178-9 and *Fusarium oxysporum* lipase are shown in Table 6. The other enzymes tested showed no activity.

**Table 6**

| | Transferase 178-9 | Fusarium oxysporum |
|---|---|---|
| Hydrolytic activity, micromole/min per ml enzyme | 1.03 | 0.96 |
| Transferase activity, micromole/min per ml enzyme | 0.40 | 0.01 |
| Total activity, micromole/min per ml enzyme | 1.43 | 0.98 |
| | | |
| Relative hydrolytic activity | 71.8 | 98.7 |
| Relative transferase activity | 28.2 | 1.3 |

The result shown in Table 6 confirm a significant transferase activity from the lipid acyltransferase (sample 178-9). It is also observed that the relative transferase activity is in good agreement with the experiment mentioned in Table 3.

A very low transferase activity form Fusarium oxysporum phospholipase is however observed. This transferase level is so low that it falls within the uncertainty of the analysis. As expected *Fusarium oxysporum* phospholipase has a significant hydrolytic activity.

Conclusion.

An artificial substrate based on purified phosphatidylcholine and cholesterol was used as a substrate to measure the activity of transferase from *Aeromonas salmonicida.* Between 10 minutes and 60 minutes reaction time the assay gave an almost linear formation of free fatty acids and cholesterol ester as a function of time. Based on the activity between 10 and 60 minutes reaction time the hydrolytic activity and the transferase activity was calculated.

Based on the results from the assay of the lipid acyltransferase (in this instance a GCAT) from *Aeromonas salmonicida* in a artificial substrate of phosphatidylcholine/cholesterol in buffer it is concluded that this enzyme has very good transferase activity also in a system with a very high water content.

The phosphatidylcholine/cholesterol in buffer assay, can be used to measure the transferase and hydrolytic activity of an enzyme. The phosphatidylcholine/cholesterol in buffer is only linear within a certain time limit.

## EXAMPLE 7: Immobilisation of a lipid acyltransferase from *Aeromonas salmonicida*

A lipid acyltransferase (in this instance a GCAT) from *A. salmonicida* was immobilised on Celite 535 535 (from Fluka) by acetone precipitation. 10 ml enzyme solution in 20 mM TEA buffer pH 7 was agitated slowly with 0,1 gram Celite 535 535 (from Fluka) for 2 hours at room temperature.
50ml cool acetone was added during continued agitation.
The precipitate was isolated by centrifugation 5000 g for 1 minute.
The precipitate was washed 2 times with 20 ml cold acetone.
The Celite was tried at ambient temperature for about 1 hour

The enzyme has also been shown to have a high activity in environments with high water content (6- 89 % )water environments, the use of the transferase, and other transferases for use in the invention can therefore also be used in immobilised enzyme applications with a significant water content. This allows the replacement of the solvents used by the current immobilised lipases in the bioconvertion of lipids using transferases.

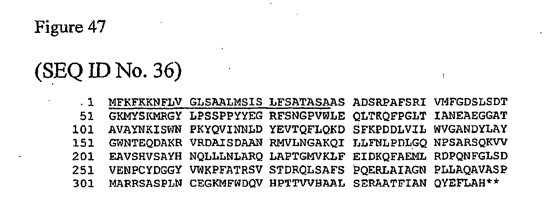

## EXAMPLE 8: Variants of a lipid acyltransferase from *Aeromonas hydrophila* (Ahyd2) (SEQ ID No. 36 (see Figure 47))

Mutations were introduced using the QuikChange® Multi-Site Directed Mutagenesis kit from Stratagene, La Jolla, CA 92037, USA following the instructions provided by Stratagene.

Variants at Tyr256 showed an increased activity towards phospholipids.

Variants at Tyr256 and Tyr260 showed an increased activity towards galactolipids.

Variants at Tyr265 show an increased transferase activity with galactolipids as the acyl donor.

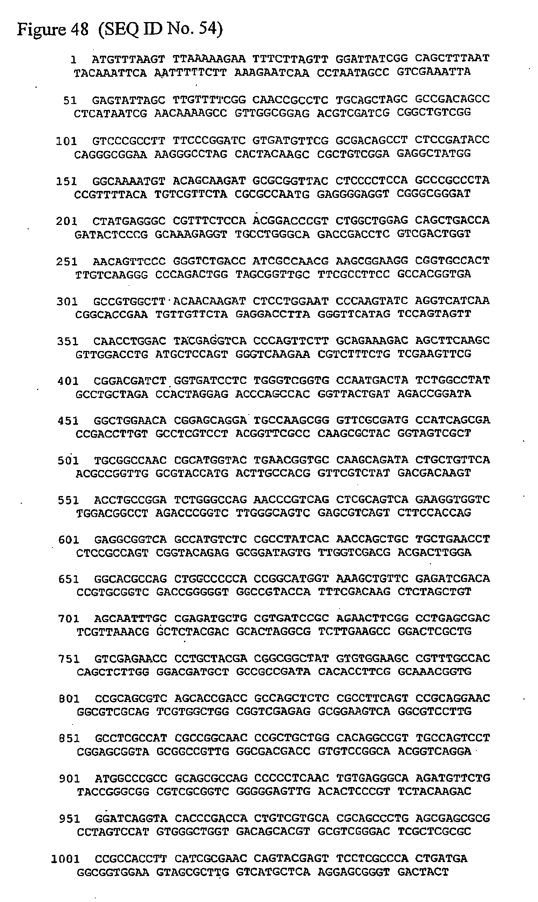

The numbers indicate positions on the following sequence: An enzyme from *Aeromonas hydrophila* the amino acid sequence of which is shown as SEQ ID No. 36 in Figure 47 (the underlined amino acids show a xylanase signal peptide). The nucleotide sequence is as shown as SEQ ID No. 54 in Figure 48.

## EXAMPLE 9 "Assay in Low Water Environment"

Transferase reactions of lipolytic enzymes in low water environment.

Procedure

Materials.

Cholesterol Sigma cat. C 8503
L-alpha-Phosphatidylcholine 95% (Plant) Avanti #441601
Soybean oil, Aarhus United, DK.
Chloroform, Analytical grade

Enzymes.

#179, GCAT from *A. salmonicida*
#2427, Phospholipase A1 from *Fusarium oxysporum*. LIPOPAN® F from Novozymes, Denmark
#1991, Phospholipase A2 from Pancreas, LIPOMOD 22L from Biocatalysts, UK
#2373, *Candida Antarctica* lipase, Novozyme 525 L from Novozymes Denmark.

Enzyme assay

13.1 % Lecithin and 6.6% cholesterol was dissolved in soybean oil by heating to 60 °C during agitation
The substrate was scaled in a 20ml Wheaton glass and heated to 46 °C
Water and enzyme solution was added and a stopwatch is started.
At regular intervals 50 mg samples ware transferred to a 10ml Dram glass and frozen.
The isolated lipids were analysed by GLC

GLC analysis

For GLC analysis protocols - see example 6

**Results**

The experiment was set up as shown in Table 8.
The substrate based on soybean oil containing 13.1 % lecithin and 6.6% cholesterol was heated to 46°C. The enzyme solution was added and a stopwatch started. After 30, 60 and 120 minutes reaction time samples were taken out for GLC analysis.

Table 8

| | | 1 | 2 | 3 | | 5 |
|---|---|---|---|---|---|---|
| Substrate | Gram | 5 | 5 | 5 | 5 | 5 |
| Transferase #179-C72, 56 PLU-7/ml | MI | | 0.3 | | | |
| #2427, 200 PLU-7/ml | MI | | | 0.3 | | |
| Pancreas PLA 2 #1991 6300 PLU/ml | MI | | | | 0.3 | |
| Novozyme 525 L, #2373, 200 LIPU/ml MI | | | | | | 0.3 |
| Water | MI | 0.3 | | | | |
| % water | | 6 | 6 | 6 | 6 | 6 |

The results from the GLC analysis is shown in Table 9. The results are expressed in percent based total sample composition. Based on the GLC results it was possible to calculate the amount of fatty acid and cholesterol ester produced by enzymatic reaction relative to the control sample without enzyme added. Under these experimental conditions the total enzymatic activity was estimated as the hydrolytic activity measured as free fatty acid formation and the transferase activity estimated as cholesterol ester formation. From these results and the information about molecular weight of fatty acid and cholesterol ester it was possible to calculate to relative molar hydrolytic activity and the relative molar transferase activity as shown in Table 10.

Table 9

| Enzyme | Reaction time minutes | Fatty acid % | Cholesterol % | Cholesterol ester % |
|---|---|---|---|---|
| Control | 120 | 0.533 | 7.094 | 0.000 |
| #179 | 30 | 0.770 | 5.761 | 2.229 |
| #179 | 60 | 0.852 | 5.369 | 2.883 |
| #179 | 120 | 0.876 | 4.900 | 3.667 |
| #2427 | 30 | 3.269 | 7.094 | 0.000 |
| #2427 | 60 | 3.420 | 7.094 | 0.000 |
| #2427 | 120 | 3.710 | 7.094 | 0.000 |

(continued)

| Enzyme | Reaction time minutes | Fatty acid % | Cholesterol % | Cholesterol ester % |
|---|---|---|---|---|
| #1991 | 30 | 2.871 | 7.094 | 0.000 |
| #1991 | 60 | 3.578 | 7.094 | 0.000 |
| #1991 | 120 | 3.928 | 7.094 | 0.000 |
| #2373 | 30 | 1.418 | 7.094 | 0.000 |
| #2373 | 60 | 1.421 | 7.094 | 0.000 |
| #2373 | 120 | 1.915 | 7.094 | 0.000 |

Table 10

| Enzyme | Reaction time minutes | Fatty acid produced | Cholesterol Used | Cholesterol ester produced | Hydrolytic activity % | Transferas Activity % |
|---|---|---|---|---|---|---|
| #179 | 30 | 0.238 | 1.334 | 2.229 | 20 | 80 |
| #179 | 60 | 0.319 | 1.725 | 2.883 | 21 | 79 |
| #179 | 120 | 0.343 | 2.195 | 3.667 | 18 | 82 |
| #2427 | 30 | 2.737 | 0.000 | 0.000 | 100 | 0 |
| #2427 | 60 | 2.887 | 0.000 | 0.000 | 100 | 0 |
| #2427 | 120 | 3.177 | 0.000 | 0.000 | 100 | 0 |
| #1991 | 30 | 2.338 | 0.000 | 0.000 | 100 | 0 |
| #1991 | 60 | 3.046 | 0.000 | 0.000 | 100 | 0 |
| #1991 | 120 | 3.395 | 0.000 | 0.000 | 100 | 0 |
| #2373 | 30 | 0.885 | 0.000 | 0.000 | 100 | 0 |
| #2373 | 60 | 0.888 | 0.000 | 0.000 | 100 | 0 |
| #2373 | 120 | 1.383 | 0.000 | 0.000 | 100 | 0 |

Conclusion

In these experiments it was observed that all the tested enzymes showed hydrolytic activity because the amount of fatty acid increased. However the only enzyme which showed transferase activity was GCAT from *A. salmonicida.* It is therefore concluded that in an oily system with lecithin and cholesterol containing 6% water phospholipase A1 from *Fusarium oxysporum*, phospholipase A2 from *pancreas* and a lipase from *Candida antarctica* only showed hydrolytic activity.

**Example 10: Carbohydrate ester production with immobilised lipid acytransferase is described herein by way of reference.**

Carbohydrate esters of fatty acids like sucrose esters and glucose esters, are traditionally produced by the reaction of a fatty acid or a fatty acid soap and the carbohydrate at high temperature (Journal of the Americal Oil Chemists' Society (1978) 55; 4; 398-401) This procedure however has the disadvantage of forming side reactions and coloured by-products.

Carbohydrate esters of fatty acids may be produced by a transferase reaction using lecithin as fatty acid donor and a carbohydrate like glucose as acceptor molecule.

The reaction is conducted in a flow reactor with a lipid acyl transferase immobilised on a solid support.

Procedure.

100 gram glucose is dissolved in 1000 ml water during agitation then 200 gram phosphatidylcholine is dispersed in the water phase during agitation and heated to 40°C.
pH is adjusted to pH 6.5.
A flow reactor is packed with 100 g of a lipid acyltransferase from *A. salmonicida* immobilised on a solid support.
The flow reactor is placed in a heating cabinet at 40 °C.
The reaction mixture is pumped into the column with 2 ml / min.
The reaction product is collected.

The water in the reaction product is removed by thin film vacuum evaporation and the lipids isolated.
The glucose ester is separated from the other lipids by solvent fractionation.

Carbohydrate esters can be used for many applications, such as efficient emulsifiers within the food and non-food industry

**Example 11 - Protein ester production with a lipid acytransferase according to the present invention.**

In the present invention fatty-acid condensates of amino acids, peptides or proteins are produced by a transferase reaction. In this reaction phosphatidylcholine is used as donor for the transfer of fatty acid to the free hydroxyl group of amino acids (such as tyrosine, serine or threonine) having a free hydroxyl group available for esterification.

Procedure 1.

50 gram 1-tyrosine (or serine or threonine) is dissolved in 1000 ml water during agitation then 200 gram phosphatidylcholine is dispersed in the water phase during agitation and heating to 40°C.
pH is adjusted to pH 7 and kept at this pH with NaOH or HCl.
50 ml of the lipid acyltransferase enzyme from *A. salmonicida* is added and the reaction is continued at 40 °C with agitation.
Samples are taken out at regular intervals and analysed by TLC and HPLC.
After 20 h reaction time the reaction has reached equilibrium and the reaction is stopped.
Tyrosine fatty acid condensate, lecithin and lysolecithin are isolated from the reaction media by centrifugation according to standard methods (see "Centrifiges, Filtering" in Ullmann's Encyclopedia of Industrial Chemistry for example (2002) by Wiley-VCH Verlag GmbH & Co. KgaA).

Tyrosine fatty acid condensate is further purified by hydrophobic interaction column chromatography and the fraction containing tyrosine fatty acid condensate is isolated and the solvent removed by evaporation. (see 'Basic Principles of Chromatography' in Ullmann's Encyclopedia of Industrial Chemistry (2002) by Wiley-VCH Verlag GmbH & Co. KGaA.)

Procedure 2.

In the following the transferase activity of a lipid acyltransferse is tested in a substrate based on phosphatidylcholin and 1-tyrosine according to the following procedure.

450 mg phophatidylcholine (>95% PC Avanti item no. 441601) and 50 mg 1-tyrosine is scaled in a Wheaton glass and 15 ml 50mM HEPES buffer pH 7 is added. The lipid is dispersed in the buffer during agitation.

The substrate is heated to 35 °C whilst mixing with a magnetic stirrer and 0.25 ml Transferase 10 PLU/ml is added.

Samples of 2 ml are taken out after 0, 5, 10, 15, 25, 40 and 60 minutes reaction time.

Immediately 25 μl 4M HCl is added to acidify the free fatty acid and stop the enzyme reaction. 3.00 ml chloroform is added, and the sample is shaken vigorously on a Whirley for 30 seconds. The sample is centrifuged and 2 ml of the chloroform phase is isolated and filtered through 0,45-μm filters into a 10 ml tared Dram glass.

The chloroform is evaporated under a steam of nitrogen at 60°C, and the sample is scaled again. The extracted lipid is analysed by TLC.

**Example 12 - Hydroxy acid ester (in particular lactic acid ester) production with a lipid acytransferase is disclosed by way of reference.**

Hydroxy esters of fatty acids are traditionally produced by the reaction between a fatty acid and a hydroxy acid at high temperature using an inorganic salts or metal ions as catalysts (see for example Bailey's Industrial Oil and Fat Products, Fifth edition, Volume 3. Edible Oil and Fat Products: Products and Application Technology, page 502-511.) This procedure however has the disadvantage of forming side reactions and coloured by-products.

Hydroxy acid esters of fatty acids may be produced by a transferase reaction using lecithin as fatty acid donor and a hydroxy acid (in particular lactic acid) as acceptor molecule.

Procedure.

50 gram lactic is dissolved in 1000 ml water whilst agitating, then 200 gram phosphatidylcholine is dispersed in the water phase during agitation and heated to 40°C.

pH is adjusted to pH 6.5 and kept at this pH with NaOH or HCl.

50 ml of lipid acyltransferase enzyme from *A. salmonicida* is added and the reaction is continued at 40 °C whilst agitating.

Samples are taken out at regular intervals and analysed by TLC and GLC.

After 20 h reaction time the reaction has reached equilibrium and the reaction is stopped.

Lactic acid ester, lecithin and lysolecithin are isolated from the reaction media by centrifugation according to standard methods (see "Centrifiges, Filtering" in Ullmann's Encyclopedia of Industrial Chemistry for example (2002) by Wiley-VCH Verlag GmbH & Co. KgaA).

Lactic acid ester is further purified by molecular distillation and a lactic acid ester of fatty acid with high purity is obtained.

**Example 13 -Citric acid ester production with a lipid acytransferase is disclosed by way of reference.**

Transferase assay based on phosphatidylcholin and citric acid as substrate.

In the following the transferase activity of lipid acyl transferase from *A. salmonicida* is tested in a substrate based on phosphatidylcholin and citric acid according to the following procedure.

450 mg phophatidylcholine (>95% PC Avanti item no. 441601) and 50 mg citric acid is scaled in a Wheaton glass and 15 ml 50mM HEPES buffer pH 7 is added. The lipid is dispersed in the buffer during agitation.

The substrate is heated to 35 °C during mixing with a magnetic stirrer and 0.25 ml lipid acyltransferase from *A. salmonicida* 10 PLU/ml is added.

Samples of 2 ml are taken out after 0, 5, 10, 15, 25, 40 and 60 minutes reaction time.

Immediately 25 μl 4M HCl is added to acidify the free fatty acid and stop the enzyme reaction. 3.00 ml chloroform is added, and the sample is shaken vigorously on a Whirley for 30 seconds. The sample is centrifuged and 2 ml of the chloroform phase is isolated and filtered through 0.45-μm filters into a 10 ml tared Dram glass.

The chloroform is evaporated under a steam of nitrogen at 60°C, and the sample is scaled again. The extracted lipid is analysed by TLC .

Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and biotechnology or related fields are intended to be within the scope of the following claims.

## Claims

1. A method of producing a protein ester and/or protein subunit ester, which method comprises admixing an acyl donor, an acyl acceptor and water to produce a high water environment comprising 5-98% water, wherein said acyl donor is a lipid substrate selected from one or more of the group consisting of a phospholipid, a lysophospholipid, a triacylglyceride, a diglyceride, a glycolipid or a lysoglycolipid and said acyl acceptor is a protein and/or protein subunit; and contacting the admixture with a lipid acyltransferase, such that said lipid acyltransferase catalyses one or both of the following reactions: alcoholysis or transesterification wherein the lipid acyltransferase is one which when tested using the Transferase Assay in Buffered Substrate has at least 2% acyltransferase activity; said transferase assay comprising the steps of:

   i) dissolving 450mg phosphatidylcholine and 50mg cholesterol in chloroform, evaporating to dryness under vacuum;
   ii) transferring 300mg cholesterol/phosphatidylcholine mixture to a Wheaton glass, adding 15ml 50mM HEPES buffer pH 7 and dispersing the lipid in the buffer during agitation;
   iii) heating the substrate to 35°C during mixing with a magnetic stirrer and adding 0.25ml enzyme solution;
   iv) taking samples of 2 ml at 0, 5, 10, 15, 25, 40 and 60 minutes reaction time and immediately stopping the enzyme reaction by the addition of 25μl 4M HCl to acidify the free fatty acid;
   v) adding 3ml chloroform and shaking vigorously for 30 seconds, centrifuging and isolating 2ml of the chloroform phase, filtering through a 0.45μm filter into a 10ml tared Dram glass;
   vi) evaporating the chloroform under a stream of nitrogen at 60°C, and scaling the samples;
   vii) analysing the extracted lipid by GLC.

2. A method according to claim 1 wherein the lipid acyltransferase is immobilised.

**3.** A method according to claim 1 or claim 2 wherein the method comprises purifying the protein ester and/or protein subunit ester.

**4.** A method according to any one of claims 1-3 wherein the lipid acyltransferase is characterised as an enzyme which possesses acyl transferase activity and which comprises the amino acid sequence motif GDSX, wherein X is one or more of the following amino acid residues L, A, V, I, F, Y, H, Q, T, N, M or S.

**5.** A method according to claim 1 wherein the lipid acyltransferase enzyme comprises an amino acid sequence produced by the expression of one or more of the following nucleotide sequences:

(a) the nucleotide sequence shown as SEQ ID No. 7 (see Figure 9);
(b) the nucleotide sequence shown as SEQ ID No. 8 (see Figure 10);
(c) the nucleotide sequence shown as SEQ ID No. 9 (see Figure 11);
(d) the nucleotide sequence shown as SEQ ID No. 10 (see Figure 12);
(e) the nucleotide sequence shown as SEQ ID No. 11 (see Figure 13);
(f) the nucleotide sequence shown as SEQ ID No. 13 (see Figure 15);
(g) the nucleotide sequence shown as SEQ ID No. 21 (see Figure 17);
(h) the nucleotide sequence shown as SEQ ID No. 23 (see Figure 19);
(i) the nucleotide sequence shown as SEQ ID No. 25 (see Figure 21);
(j) the nucleotide sequence shown as SEQ ID No. 27 (see Figure 23);
(k) the nucleotide sequence shown as SEQ ID No. 29 (see Figure 25);
(l) the nucleotide sequence shown as SEQ ID No. 31 (see Figure 27);
(m) the nucleotide sequence shown as SEQ ID No. 33 (see Figure 29);
(n) the nucleotide sequence shown as SEQ ID No. 35 (see Figure 31);
(o) or a nucleotide sequence which has 75% or more identity with any one of the sequences shown as SEQ ID No. 7, SEQ ID No. 8, SEQ ID No. 9, SEQ ID No. 10, SEQ ID No. 11, SEQ ID No. 13, SEQ ID No. 21, SEQ ID No. 23, SEQ ID No. 25, SEQ ID No. 27, SEQ ID No. 29, SEQ ID No. 31, SEQ ID No. 33 or SEQ ID No. 35.

**6.** A method according to any one of the preceding claims wherein the lipid acyltransferase is classified as E.C. 2.3.1.x.

**7.** A method according to any one of the preceding claims wherein the lipid acyltransferase is obtainable from an organism from one or more of the following genera: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* and *Candida.*

**8.** A method according to any one of the preceding claims wherein the lipid acyltransferase comprises one or more of the following amino acid sequences: (i) the amino acid sequence shown as SEQ ID No. 2; (ii) the amino acid sequence shown as SEQ ID No. 3; (iii) the amino acid sequence shown as SEQ ID No. 4; (iv) the amino acid sequence shown as SED ID No. 5; (v) the amino acid sequence shown as SEQ ID No. 6; (vi) the amino acid sequence shown as SEQ ID No. 12, (vii) the amino acid sequence shown as SEQ ID No. 20, (viii) the amino acid sequence shown as SEQ ID No. 22, (ix) the amino acid sequence shown as SEQ ID No. 24, (x) the amino acid sequence shown as SEQ ID No. 26, (xi) the amino acid sequence shown as SEQ ID No. 28, (xii) the amino acid sequence shown as SEQ ID No. 30, (xiii) the amino acid sequence shown as SEQ ID No. 32, (xiv) the amino acid sequence shown as SEQ ID No. 34, or an amino acid sequence which has 75% or more identity with any one of the sequences shown as SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32 or SEQ ID No. 34.

**9.** Use of a lipid acyltransferase to produce a protein ester and/or a protein subunit ester by catalysis of one or both of alcoholysis or transesterification in an admixture of an acyl donor, an acyl acceptor and water, which admixture comprises 5-98% water, wherein said acyl donor is a lipid substrate selected from one or more of the group consisting of a phospholipid, a lysophospholipid, a triacylglyceride, a diglyceride, a glycolipid or a lysoglycolipid and said acyl acceptor is a protein and/or a protein subunit, wherein the lipid acyltransferase is one which when tested using the Transferase Assay in Buffered Substrate has at least 2% acyltransferase activity; said transferase assay comprising the steps of:

i) dissolving 450mg phosphatidylcholine and 50mg cholesterol in chloroform, evaporating to dryness under vacuum;

ii) transferring 300mg cholesterol/phosphatidylcholine mixture to a Wheaton glass, adding 15ml 50mM HEPES buffer pH 7 and dispersing the lipid in the buffer during agitation;
iii) heating the substrate to 35°C during mixing with a magnetic stirrer and adding 0.25ml enzyme solution;
iv) taking samples of 2 ml at 0, 5, 10, 15, 25, 40 and 60 minutes reaction time and immediately stopping the enzyme reaction by the addition of 25 μl 4M HCl to acidify the free fatty acid;
v) adding 3ml chloroform and shaking vigorously for 30 seconds, centrifuging and isolating 2ml of the chloroform phase, filtering through a 0.45 μm filter into a 10ml tared Dram glass;
vi) evaporating the chloroform under a stream of nitrogen at 60°C, and scaling the samples;
vii) analysing the extracted lipid by GLC.

**10.** Use according to claim 9 wherein the lipid acyltransferase is immobilised.

**11.** Use according to claim 9 wherein the protein ester and/or protein subunit ester is purified.

**12.** Use according to any one of claims 9-11 wherein the lipid acyltransferase is characterised as an enzyme which possesses acyl transferase activity and which comprises the amino acid sequence motif GDSX, wherein X is one or more of the following amino acid residues L, A, V, I, F, Y, H, Q, T, N, M or S.

**13.** Use according to claim 9 wherein the lipid acyltransferase enzyme comprises an amino acid sequence produced by the expression of one or more of the following nucleotide sequences:

(a) the nucleotide sequence shown as SEQ ID No. 7 (see Figure 9);
(b) the nucleotide sequence shown as SEQ ID No. 8 (see Figure 10);
(c) the nucleotide sequence shown as SEQ ID No. 9 (see Figure 11);
(d) the nucleotide sequence shown as SEQ ID No. 10 (see Figure 12);
(e) the nucleotide sequence shown as SEQ ID No. 11 (see Figure 13);
(f) the nucleotide sequence shown as SEQ ID No. 13 (see Figure 15);
(g) the nucleotide sequence shown as SEQ ID No. 21 (see Figure 17);
(h) the nucleotide sequence shown as SEQ ID No. 23 (see Figure 19);
(i) the nucleotide sequence shown as SEQ ID No. 25 (see Figure 21);
(j) the nucleotide sequence shown as SEQ ID No. 27 (see Figure 23);
(k) the nucleotide sequence shown as SEQ ID No. 29 (see Figure 25);
(l) the nucleotide sequence shown as SEQ ID No. 31 (see Figure 27);
(m) the nucleotide sequence shown as SEQ ID No. 33 (see Figure 29);
(n) the nucleotide sequence shown as SEQ ID No. 35 (see Figure 31);
(o) or a nucleotide sequence which has 75% or more identity with any one of the sequences shown as SEQ ID No. 7, SEQ ID No. 8, SEQ ID No. 9, SEQ ID No. 10, SEQ ID No. 11, SEQ ID No. 13, SEQ ID No. 21, SEQ ID No. 23, SEQ ID No. 25, SEQ ID No. 27, SEQ ID No. 29, SEQ ID No. 31, SEQ ID No. 33 or SEQ ID No. 35.

**14.** Use according to any one of claims 9 to 13 wherein the lipid acyltransferase is classified as E.C. 2.3.1.x.

**15.** Use according to any one of claims 9-14 wherein the lipid acyltransferase is obtainable from an organism from one or more of the following genera: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* and *Candida.*

**16.** Use according to any one of claims 9-15 wherein the lipid acyltransferase comprises one or more of the following amino acid sequences: (i) the amino acid sequence shown as SEQ ID No. 2; (ii) the amino acid sequence shown as SEQ ID No. 3; (iii) the amino acid sequence shown as SEQ ID No. 4; (iv) the amino acid sequence shown as SED ID No. 5; (v) the amino acid sequence shown as SEQ ID No. 6; (vi) the amino acid sequence shown as SEQ ID No. 12, (vii) the amino acid sequence shown as SEQ ID No. 20, (viii) the amino acid sequence shown as SEQ ID No. 22, (ix) the amino acid sequence shown as SEQ ID No. 24, (x) the amino acid sequence shown as SEQ ID No. 26, (xi) the amino acid sequence shown as SEQ ID No. 28, (xii) the amino acid sequence shown as SEQ ID No. 30, (xiii) the amino acid sequence shown as SEQ ID No. 32, (xiv) the amino acid sequence shown as SEQ ID No. 34, or an amino acid sequence which has 75% or more identity with any one of the sequences shown as SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32 or SEQ ID No. 34.

**Patentansprüche**

1. Verfahren zur Herstellung eines Proteinesters und/oder Proteinuntereinheitsesters, wobei man bei dem Verfahren einen Acyl-Donor, einen Acyl-Akzeptor und Wasser mischt, so dass eine 5-98% Wasser enthaltende Umgebung mit hohem Wasseranteil entsteht, wobei es sich bei dem Acyl-Donor um ein aus einem oder mehreren Mitgliedern der aus einem Phospholipid, einem Lysophospholipid, einem Tri-acylglycerid, einem Diglycerid, einem Glykolipid oder einem Lysoglykolipid bestehenden Gruppe ausgewähltes Lipidsubstrat und bei dem Acyl-Akzeptor um ein Protein und/oder eine Proteinuntereinheit handelt; und das Gemisch mit einer Lipid-Acyltransferase so in Kontakt bringt, dass die Lipid-Acyltransferase eine oder beide der folgenden Reaktionen katalysiert: Alkoholyse oder Umesterung, wobei es sich um eine Lipid-Acyltransferase handelt, die beim Testen unter Verwendung des "Transferase Assay in Buffered Substrate" wenigstens 2% Acyltransferase-Aktivität aufweist; wobei man in den Schritten des Transferase-Tests:

   i) 450 mg Phosphatidylcholin und 50 mg Cholesterin in Chloroform löst, unter Vakuum zur Trockne einengt;
   ii) 300 mg Cholesterin/Phosphatidylcholin-Gemisch in ein Wheaton-Glas überführt, 15 ml 50 mM HEPES-Puffer pH 7 zugibt und das Lipid in dem Puffer unter Schütteln dispergiert;
   iii) das Substrat unter Mischen mit einem Magnetrührer auf 35°C erwärmt und 0,25 ml Enzymlösung zugibt;
   iv) nach einer Reaktionszeit von 0, 5, 10, 15, 25, 40 und 60 Minuten Proben von jeweils 2 ml entnimmt und die Enzymreaktion sofort durch Zugabe von 25 μl 4 M HCl zur Ansäuerung der freien Fettsäure stoppt;
   v) 3 ml Chloroform zugibt und 30 Sekunden kräftig schüttelt, zentrifugiert und 2 ml der Chloroformphase isoliert, durch ein 0,45 μm-Filter in ein austariertes 10-ml-Dram-Glas filtriert;
   vi) das Chloroform unter einem Stickstoffstrom bei 60°C abzieht und die Proben auswiegt;
   vii) das extrahierte Lipid mittels GLC analysiert.

2. Verfahren nach Anspruch 1, wobei die Lipid-Acyltransferase immobilisiert ist.

3. Verfahren nach Anspruch 1 oder Anspruch 2, wobei man bei dem Verfahren den Proteinester und/oder Proteinuntereinheitsester aufreinigt.

4. Verfahren nach einem der Ansprüche 1-3, wobei die Lipid-Acyltransferase als ein Enzym gekennzeichnet ist, das Acyltransferase-Aktivität besitzt und das das Aminosäuresequenzmotiv GDSX umfasst, wobei X für einen oder mehrere der folgenden Aminosäurereste L, A, V, I, F, Y, H, Q, T, N, M oder S steht.

5. Verfahren nach Anspruch 1, wobei das Lipid-Acyltransferase-Enzym eine durch die Expression einer oder mehrerer der folgenden Nukleotidsequenzen:

   (a) der als SEQ ID Nr. 7 dargestellten Nukleotidsequenz (siehe Figur 9);
   (b) der als SEQ ID Nr. 8 dargestellten Nukleotidsequenz (siehe Figur 10);
   (c) der als SEQ ID Nr. 9 dargestellten Nukleotidsequenz (siehe Figur 11);
   (d) der als SEQ ID Nr. 10 dargestellten Nukleotidsequenz (siehe Figur 12);
   (e) der als SEQ ID Nr. 11 dargestellten Nukleotidsequenz (siehe Figur 13);
   (f) der als SEQ ID Nr. 13 dargestellten Nukleotidsequenz (siehe Figur 15);
   (g) der als SEQ ID Nr. 21 dargestellten Nukleotidsequenz (siehe Figur 17);
   (h) der als SEQ ID Nr. 23 dargestellten Nukleotidsequenz (siehe Figur 19);
   (i) der als SEQ ID Nr. 25 dargestellten Nukleotidsequenz (siehe Figur 21);
   (j) der als SEQ ID Nr. 27 dargestellten Nukleotidsequenz (siehe Figur 23);
   (k) der als SEQ ID Nr. 29 dargestellten Nukleotidsequenz (siehe Figur 25);
   (l) der als SEQ ID Nr. 31 dargestellten Nukleotidsequenz (siehe Figur 27);
   (m) der als SEQ ID Nr. 33 dargestellten Nukleotidsequenz (siehe Figur 29);
   (n) der als SEQ ID Nr. 35 dargestellten Nukleotidsequenz (siehe Figur 31);
   (o) oder einer Nukleotidsequenz, die 75% oder mehr Identität mit einer der als SEQ ID Nr. 7, SEQ ID Nr. 8, SEQ ID Nr. 9, SEQ ID Nr. 10, SEQ ID Nr. 11, SEQ ID Nr. 13, SEQ ID Nr. 21, SEQ ID Nr. 23, SEQ ID Nr. 25, SEQ ID Nr. 27, SEQ ID Nr. 29, SEQ ID Nr. 31, SEQ ID Nr. 33 oder SEQ ID Nr. 35 dargestellten Sequenzen aufweist, produzierte Aminosäuresequenz umfasst.

6. Verfahren nach einem der vorhergehenden Ansprüche, wobei die Lipid-Acyltransferase als E.C. 2.3.1.x klassifiziert ist.

**7.** Verfahren nach einem der vorhergehenden Ansprüche, wobei die Lipid-Acyltransferase aus einem Organismus aus einer oder mehreren der folgenden Gattungen erhältlich ist: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* und *Candida.*

**8.** Verfahren nach einem der vorhergehenden Ansprüche, wobei die Lipid-Acyltransferase eine oder mehrere der folgenden Aminosäuresequenzen umfasst: (i) die als SEQ ID Nr. 2 dargestellte Aminosäuresequenz; (ii) die als SEQ ID Nr. 3 dargestellte Aminosäuresequenz; (iii) die als SEQ ID Nr. 4 dargestellte Aminosäuresequenz; (iv) die als SEQ ID Nr. 5 dargestellte Aminosäuresequenz; (v) die als SEQ ID Nr. 6 dargestellte Aminosäuresequenz; (vi) die als SEQ ID Nr. 12 dargestellte Aminosäuresequenz, (vii) die als SEQ ID Nr. 20 dargestellte Aminosäuresequenz, (viii) die als SEQ ID Nr. 22 dargestellte Aminosäuresequenz, (ix) die als SEQ ID Nr. 24 dargestellte Aminosäuresequenz, (x) die als SEQ ID Nr. 26 dargestellte Aminosäuresequenz, (xi) die als SEQ ID Nr. 28 dargestellte Aminosäuresequenz, (xii) die als SEQ ID Nr. 30 dargestellte Aminosäuresequenz, (xiii) die als SEQ ID Nr. 32 dargestellte Aminosäuresequenz, (xiv) die als SEQ ID Nr. 34 dargestellte Aminosäuresequenz oder eine Aminosäuresequenz, die 75% oder mehr Identität mit einer der als SEQ ID Nr. 2, SEQ ID Nr. 3, SEQ ID Nr. 4, SEQ ID Nr. 5, SEQ ID Nr. 6, SEQ ID Nr. 12, SEQ ID Nr. 20, SEQ ID Nr. 22, SEQ ID Nr. 24, SEQ ID Nr. 26, SEQ ID Nr. 28, SEQ ID Nr. 30, SEQ ID Nr. 32 oder SEQ ID Nr. 34 dargestellten Sequenzen aufweist.

**9.** Verwendung einer Lipid-Acyltransferase zur Herstellung eines Proteinesters und/oder eines Proteinuntereinheitsesters durch Katalyse von Alkoholyse oder/und Umesterung in einem Gemisch aus einem Acyl-Donor, einem Acyl-Akzeptor und Wasser, das 5-98% Wasser enthält, wobei es sich bei dem Acyl-Donor um ein aus einem oder mehreren Mitgliedern der aus einem Phospholipid, einem Lysophospholipid, einem Triacylglycerid, einem Diglycerid, einem Glykolipid oder einem Lysoglykolipid bestehenden Gruppe ausgewähltes Lipidsubstrat und bei dem Acyl-Akzeptor um ein Protein und/oder eine Proteinuntereinheit handelt, wobei es sich um eine Lipid-Acyltransferase handelt, die beim Testen unter Verwendung des "Transferase Assay in Buffered Substrate" wenigstens 2% Acyltransferase-Aktivität aufweist; wobei man in den Schritten des Transferase-Tests:

i) 450 mg Phosphatidylcholin und 50 mg Cholesterin in Chloroform löst, unter Vakuum zur Trockne einengt;
ii) 300 mg Cholesterin/Phosphatidylcholin-Gemisch in ein Wheaton-Glas überführt, 15 ml 50 mM HEPES-Puffer pH 7 zugibt und das Lipid in dem Puffer unter Schütteln dispergiert;
iii) das Substrat unter Mischen mit einem Magnetrührer auf 35°C erwärmt und 0,25 ml Enzymlösung zugibt;
iv) nach einer Reaktionszeit von 0, 5, 10, 15, 25, 40 und 60 Minuten Proben von jeweils 2 ml entnimmt und die Enzymreaktion sofort durch Zugabe von 25 $\mu$l 4 M HCl zur Ansäuerung der freien Fettsäure stoppt;
v) 3 ml Chloroform zugibt und 30 Sekunden kräftig schüttelt, zentrifugiert und 2 ml der Chloroformphase isoliert, durch ein 0,45 $\mu$m-Filter in ein austariertes 10-ml-Dram-Glas filtriert;
vi) das Chloroform unter einem Stickstoffstrom bei 60°C abzieht und die Proben auswiegt;
vii) das extrahierte Lipid mittels GLC analysiert.

**10.** Verwendung nach Anspruch 9, wobei die Lipid-Acyltransferase immobilisiert ist.

**11.** Verwendung nach Anspruch 9, wobei der Proteinester und/oder Proteinuntereinheitsester aufgereinigt wird.

**12.** Verwendung nach einem der Ansprüche 9-11, wobei die Lipid-Acyltransferase als ein Enzym gekennzeichnet ist, das Acyltransferase-Aktivität besitzt und das das Aminosäuresequenzmotiv GDSX umfasst, wobei X für einen oder mehrere der folgenden Aminosäurereste L, A, V, I, F, Y, H, Q, T, N, M oder S steht.

**13.** Verwendung nach Anspruch 9, wobei das Lipid-Acyltransferase-Enzym eine durch die Expression einer oder mehrerer der folgenden Nukleotidsequenzen:

(a) der als SEQ ID Nr. 7 dargestellten Nukleotidsequenz (siehe Figur 9);
(b) der als SEQ ID Nr. 8 dargestellten Nukleotidsequenz (siehe Figur 10);
(c) der als SEQ ID Nr. 9 dargestellten Nukleotidsequenz (siehe Figur 11);
(d) der als SEQ ID Nr. 10 dargestellten Nukleotidsequenz (siehe Figur 12);
(e) der als SEQ ID Nr. 11 dargestellten Nukleotidsequenz (siehe Figur 13);
(f) der als SEQ ID Nr. 13 dargestellten Nukleotidsequenz (siehe Figur 15);
(g) der als SEQ ID Nr. 21 dargestellten Nukleotidsequenz (siehe Figur 17);
(h) der als SEQ ID Nr. 23 dargestellten Nukleotidsequenz (siehe Figur 19);

(i) der als SEQ ID Nr. 25 dargestellten Nukleotidsequenz (siehe Figur 21);
(j) der als SEQ ID Nr. 27 dargestellten Nukleotidsequenz (siehe Figur 23);
(k) der als SEQ ID Nr. 29 dargestellten Nukleotidsequenz (siehe Figur 25);
(l) der als SEQ ID Nr. 31 dargestellten Nukleotidsequenz (siehe Figur 27);
(m) der als SEQ ID Nr. 33 dargestellten Nukleotidsequenz (siehe Figur 29);
(n) der als SEQ ID Nr. 35 dargestellten Nukleotidsequenz (siehe Figur 31);
(o) oder einer Nukleotidsequenz, die 75% oder mehr Identität mit einer der als SEQ ID Nr. 7, SEQ ID Nr. 8, SEQ ID Nr. 9, SEQ ID Nr. 10, SEQ ID Nr. 11, SEQ ID Nr. 13, SEQ ID Nr. 21, SEQ ID Nr. 23, SEQ ID Nr. 25, SEQ ID Nr. 27, SEQ ID Nr. 29, SEQ ID Nr. 31, SEQ ID Nr. 33 oder SEQ ID Nr. 35 dargestellten Sequenzen aufweist, produzierte Aminosäuresequenz umfasst.

**14.** Verwendung nach einem der Ansprüche 9 bis 13, wobei die Lipid-Acyltransferase als E.C. 2.3.1.x klassifiziert ist.

**15.** Verwendung nach einem der Ansprüche 9-14, wobei die Lipid-Acyltransferase aus einem Organismus aus einer oder mehreren der folgenden Gattungen erhältlich ist: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Meisseria, Mesorhizobium, Ralstonia, Xanthomonas* und *Candida.*

**16.** Verwendung nach einem der Ansprüche 9-15, wobei die Lipid-Acyltransferase eine oder mehrere der folgenden Aminosäuresequenzen umfasst: (i) die als SEQ ID Nr. 2 dargestellte Aminosäuresequenz; (ii) die als SEQ ID Nr. 3 dargestellte Aminosäuresequenz; (iii) die als SEQ ID Nr. 4 dargestellte Aminosäuresequenz; (iv) die als SEQ ID Nr. 5 dargestellte Aminosäuresequenz; (v) die als SEQ ID Nr. 6 dargestellte Aminosäuresequenz; (vi) die als SEQ ID Nr. 12 dargestellte Aminosäuresequenz, (vii) die als SEQ ID Nr. 20 dargestellte Aminosäuresequenz, (viii) die als SEQ ID Nr. 22 dargestellte Aminosäuresequenz, (ix) die als SEQ ID Nr. 24 dargestellte Aminosäuresequenz, (x) die als SEQ ID Nr. 26 dargestellte Aminosäuresequenz, (xi) die als SEQ ID Nr. 28 dargestellte Aminosäuresequenz, (xii) die als SEQ ID Nr. 30 dargestellte Aminosäuresequenz, (xiii) die als SEQ ID Nr. 32 dargestellte Aminosäuresequenz, (xiv) die als SEQ ID Nr. 34 dargestellte Aminosäuresequenz oder eine Aminosäuresequenz, die 75% oder mehr Identität mit einer der als SEQ ID Nr. 2, SEQ ID Nr. 3, SEQ ID Nr. 4, SEQ ID Nr. 5, SEQ ID Nr. 6, SEQ ID Nr. 12, SEQ ID Nr. 20, SEQ ID Nr. 22, SEQ ID Nr. 24, SEQ ID Nr. 26, SEQ ID Nr. 28, SEQ ID Nr. 30, SEQ ID Nr. 32 oder SEQ ID Nr. 34 dargestellten Sequenzen aufweist.

## Revendications

**1.** Procédé pour produire un ester de protéine et/ou un ester d'une sous-unité protéinique, le procédé comprenant le mélange d'un donneur d'acyle, d'un accepteur d'acyle et d'eau pour produire un environnement à haute teneur en eau, comprenant 5-98% d'eau, ledit donneur d'acyle étant un substrat lipidique choisi parmi un ou plusieurs substrats du groupe constitué par un phospholipide, un lysophospholipide, un triacylglycéride, un diglycéride, un glycolipide ou un lysoglycolipide et ledit accepteur d'acyle étant une protéine et/ou une sous-unité protéinique ; et la mise en contact du mélange avec une lipide acyltransférase, de manière telle que ladite lipide acyltransférase catalyse une des réactions suivantes ou les deux : alcoolyse ou transestérification dans laquelle la lipide acyltransférase est une transférase qui, lorsqu'elle est analysée au moyen d'un dosage de transférase dans un substrat tamponné, présente une activité d'acyltransférase d'au moins 2% ; ledit dosage de transférase comprenant les étapes de :

i) dissolution de 450 mg de phosphatidylcholine et de 50 mg de cholestérol dans du chloroforme, évaporation à sec sous vide ;
ii) transfert de 300 mg de mélange de cholestérol/phosphatidylcholine dans un verre Wheaton, addition de 15 ml de tampon HEPES à 50 mM pH 7 et dispersion du lipide dans le tampon sous agitation ;
iii) chauffage du substrat à 35°C pendant le mélange à l'aide d'un agitateur magnétique et addition de 0,25 ml de solution d'enzyme ;
iv) prélèvement d'échantillons de 2 ml au temps de réaction de 0, 5, 10, 15, 25, 40 et 60 minutes et arrêt immédiat de la réaction enzymatique par l'addition de 25 $\mu$l de HCL à 4 M pour acidifier l'acide gras libre ;
v) addition de 3 ml de chloroforme et agitation vigoureuse pendant 30 secondes, centrifugation et isolement de 2 ml de la phase de chloroforme, filtration au travers d'un filtre de 0,45 $\mu$m dans un verre Dram taré de 10 ml ;
vi) évaporation du chloroforme sous un flux d'azote à 60°C et pesée des échantillons ;
vii) analyse du lipide extrait par CGL.

2. Procédé selon la revendication 1, la lipide acyltransférase étant immobilisée.

3. Procédé selon la revendication 1 ou la revendication 2, le procédé comprenant la purification de l'ester de protéine et/ou de l'ester de sous-unité protéinique.

4. Procédé l'une quelconque des revendications 1-3, la lipide acyltransférase étant caractérisée en tant qu'enzyme qui possède une activité d'acyltransférase et qui comprend le motif de séquence d'acides aminés GDSX, X représentant un ou plusieurs parmi les résidus d'acides aminés suivants L, A, V, I, F, Y, H, Q, T, N, M ou S.

5. Procédé selon la revendication 1, l'enzyme lipide acyltransférase comprenant une séquence d'acides aminés produite par l'expression d'une ou de plusieurs des séquences nucléotidiques suivantes :

(a) la séquence nucléotidique représentée par la SEQ ID No. 7 (cf. Figure 9) ;
(b) la séquence nucléotidique représentée par la SEQ ID No. 8 (cf. Figure 10) ;
(c) la séquence nucléotidique représentée par la SEQ ID No. 9 (cf. Figure 11) ;
(d) la séquence nucléotidique représentée par la SEQ ID No. 10 (cf. Figure 12) ;
(e) la séquence nucléotidique représentée par la SEQ ID No. 11 (cf. Figure 13) ;
(f) la séquence nucléotidique représentée par la SEQ ID No. 13 (cf. Figure 15) ;
(g) la séquence nucléotidique représentée par la SEQ ID No. 21 (cf. Figure 17) ;
(h) la séquence nucléotidique représentée par la SEQ ID No. 23 (cf. Figure 19) ;
(i) la séquence nucléotidique représentée par la SEQ ID No. 25 (cf. Figure 21) ;
(j) la séquence nucléotidique représentée par la SEQ ID No. 27 (cf. Figure 23) ;
(k) la séquence nucléotidique représentée par la SEQ ID No. 29 (cf. Figure 25) ;
(l) la séquence nucléotidique représentée par la SEQ ID No. 31 (cf. Figure 27) ;
(m) la séquence nucléotidique représentée par la SEQ ID No. 33 (cf. Figure 29) ;
(n) la séquence nucléotidique représentée par la SEQ ID No. 35 (cf. Figure 31) ;
(o) ou une séquence nucléotidique qui présente 75% d'identité ou plus avec l'une quelconque des séquences représentées par les séquences SEQ ID No. 7, SEQ ID No. 8, SEQ ID No. 9, SEQ ID No. 10, SEQ ID No. 11, SEQ ID No. 13, SEQ ID No. 21, SEQ ID No. 23, SEQ ID No. 25, SEQ ID No. 27, SEQ ID No. 29, SEQ ID No. 31, SEQ ID No. 33 ou SEQ ID No. 35.

6. Procédé selon l'une quelconque des revendications précédentes, la lipide acyltransférase étant classée comme E.C. 2. 3. 1. x.

7. Procédé selon l'une quelconque des revendications précédentes, la lipide acyltransférase pouvant être obtenue à partir d'un organisme parmi un ou plusieurs des genres suivants : Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas et Candida.

8. Procédé selon l'une quelconque des revendications précédentes, la lipide acyltransférase comprenant une ou plusieurs des séquences d'acides aminés suivantes :

(i) la séquence d'acides aminés représentée par SEQ ID No. 2 ; (ii) la séquence d'acides aminés représentée par SEQ ID No. 3 ; (iii) la séquence d'acides aminés représentée par SEQ ID No. 4 ; (iv) la séquence d'acides aminés représentée par SEQ ID No. 5 ; (v) la séquence d'acides aminés représentée par SEQ ID No. 6 ; (vi) la séquence d'acides aminés représentée par SEQ ID No. 12, (vii) la séquence d'acides aminés représentée par SEQ ID No. 20, (viii) la séquence d'acides aminés représentée par SEQ ID No. 22, (ix) la séquence d'acides aminés représentée par SEQ ID No. 24, (x) la séquence d'acides aminés représentée par SEQ ID No. 26, (xi) la séquence d'acides aminés représentée par SEQ ID No. 28, (xii) la séquence d'acides aminés représentée par SEQ ID No. 30, (xiii) la séquence d'acides aminés représentée par SEQ ID No. 32, (xiv) la séquence d'acides aminés représentée par SEQ ID No. 34 ou une séquence d'acides aminés qui présente 75% d'identité ou plus avec l'une quelconque des séquences représentées par SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32 ou SEQ ID No. 34.

9. Utilisation d'une lipide acyltransférase pour produire un ester de protéine et/ou un ester d'une sous-unité protéinique par catalyse de l'une ou des deux parmi une alcoolyse ou une transestérification dans un mélange d'un donneur d'acyle, d'un accepteur d'acyle et d'eau, le mélange comprenant 5-98% d'eau, ledit donneur d'acyle étant un substrat lipidique choisi parmi un ou plusieurs substrats du groupe constitué par un phospholipide, un lysophospholipide, un triacylglycéride, un diglycéride, un glycolipide ou un lysoglycolipide et ledit accepteur d'acyle étant une protéine et/ou une sous-unité protéinique, la lipide acyltransférase étant une transférase qui, lorsqu'elle est analysée au moyen d'un dosage de transférase dans un substrat tamponné, présente une activité d'acyltransférase d'au moins 2% ; ledit dosage de transférase comprenant les étapes de :

i) dissolution de 450 mg de phosphatidylcholine et de 50 mg de cholestérol dans du chloroforme, évaporation à sec sous vide ;
ii) transfert de 300 mg de mélange de cholestérol/phosphatidylcholine dans un verre Wheaton, addition de 15 ml de tampon HEPES à 50 mM pH 7 et dispersion du lipide dans le tampon sous agitation ;
iii) chauffage du substrat à 35°C pendant le mélange à l'aide d'un agitateur magnétique et addition de 0,25 ml de solution d'enzyme ;
iv) prélèvement d'échantillons de 2 ml au temps de réaction de 0, 5, 10, 15, 25, 40 et 60 minutes et arrêt immédiat de la réaction enzymatique par l'addition de 25 μl de HCL à 4 M pour acidifier l'acide gras libre ;
v) addition de 3 ml de chloroforme et agitation vigoureuse pendant 30 secondes, centrifugation et isolement de 2 ml de la phase de chloroforme, filtration au travers d'un filtre de 0,45 μm dans un verre Dram taré de 10 ml ;
vi) évaporation du chloroforme sous un flux d'azote à 60°C et pesée des échantillons ;
vii) analyse du lipide extrait par CGL.

**10.** Utilisation selon la revendication 9, la lipide acyltransférase étant immobilisée.

**11.** Utilisation selon la revendication 9, l'ester de protéine et/ou l'ester de sous-unité protéinique étant purifié.

**12.** Utilisation l'une quelconque des revendications 9-11, la lipide acyltransférase étant caractérisée en tant qu'enzyme qui possède une activité d'acyltransférase et qui comprend le motif de séquence d'acides aminés GDSX, X représentant un ou plusieurs des résidus d'acides aminés suivants L, A, V, I, F, Y, H, Q, T, N, M ou S.

**13.** Utilisation selon la revendication 9, l'enzyme lipide acyltransférase comprenant une séquence d'acides aminés produite par l'expression d'une ou de plusieurs des séquences nucléotidiques suivantes :

(a) la séquence nucléotidique représentée par la SEQ ID No. 7 (cf. Figure 9) ;
(b) la séquence nucléotidique représentée par la SEQ ID No. 8 (cf. Figure 10) ;
(c) la séquence nucléotidique représentée par la SEQ ID No. 9 (cf. Figure 11) ;
(d) la séquence nucléotidique représentée par la SEQ ID No. 10 (cf. Figure 12) ;
(e) la séquence nucléotidique représentée par la SEQ ID No. 11 (cf. Figure 13) ;
(f) la séquence nucléotidique représentée par la SEQ ID No. 13 (cf. Figure 15) ;
(g) la séquence nucléotidique représentée par la SEQ ID No. 21 (cf. Figure 17) ;
(h) la séquence nucléotidique représentée par la SEQ ID No. 23 (cf. Figure 19) ;
(i) la séquence nucléotidique représentée par la SEQ ID No. 25 (cf. Figure 21) ;
(j) la séquence nucléotidique représentée par la SEQ ID No. 27 (cf. Figure 23) ;
(k) la séquence nucléotidique représentée par la SEQ ID No. 29 (cf. Figure 25) ;
(l) la séquence nucléotidique représentée par la SEQ ID No. 31 (cf. Figure 27) ;
(m) la séquence nucléotidique représentée par la SEQ ID No. 33 (cf. Figure 29) ;
(n) la séquence nucléotidique représentée par la SEQ ID No. 35 (cf. Figure 31) ;
(o) ou une séquence nucléotidique qui présente 75% d'identité ou plus avec l'une quelconque des séquences représentées par les séquences SEQ ID No. 7, SEQ ID No. 8, SEQ ID No. 9, SEQ ID No. 10, SEQ ID No. 11, SEQ ID No. 13, SEQ ID No. 21, SEQ ID No. 23, SEQ ID No. 25, SEQ ID No. 27, SEQ ID No. 29, SEQ ID No. 31, SEQ ID No. 33 ou SEQ ID No. 35.

**14.** Utilisation selon l'une quelconque des revendications 9 à 13, la lipide acyltransférase étant classée comme E.C. 2. 3. 1. x.

**15.** Utilisation selon l'une quelconque des revendications 9-14, la lipide acyltransférase pouvant être obtenue à partir d'un organisme parmi un ou plusieurs des genres suivants : Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas et Candida.

**16.** Utilisation selon l'une quelconque des revendications 9-15, la lipide acyltransférase comprenant une ou plusieurs des séquences d'acides aminés suivantes :

(i) la séquence d'acides aminés représentée par SEQ ID No. 2 ; (ii) la séquence d'acides aminés représentée par SEQ ID No. 3 ; (iii) la séquence d'acides aminés représentée par SEQ ID No. 4 ; (iv) la séquence d'acides aminés représentée par SEQ ID No. 5 ; (v) la séquence d'acides aminés représentée par SEQ ID No. 6 ; (vi) la séquence d'acides aminés représentée par SEQ ID No. 12, (vii) la séquence d'acides aminés représentée par SEQ ID No. 20, (viii) la séquence d'acides aminés représentée par SEQ ID No. 22, (ix) la séquence d'acides aminés représentée par SEQ ID No. 24, (x) la séquence d'acides aminés représentée par SEQ ID No. 26, (xi) la séquence d'acides aminés représentée par SEQ ID No. 28, (xii) la séquence d'acides aminés représentée par SEQ ID No. 30, (xiii) la séquence d'acides aminés représentée par SEQ ID No. 32, (xiv) la séquence d'acides aminés représentée par SEQ ID No. 34 ou une séquence d'acides aminés qui présente 75% d'identité ou plus avec l'une quelconque des séquences représentées par SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32 ou SEQ ID No. 34.

Figure 1

SEQ ID No. 1

```
  1 ivafGDSlTd geayygdsdg ggwgagladr Ltallrlrar prgvdvfnrg isGrtsdGrl
 61 ivDalvallF laqslglpnL pPYLsgdflr GANFAsagAt Ilptsgpfli QvqFkdfksq
121 vlelrqalgl lqellrllpv ldakspdlvt imiGtNDlit saffgpkste sdrnvsvpef
181 kdnlrqlikr Lrsnngarii vlitlvilnl gplGClPlkl alalassknv dasgclerln
241 eavadfneal relaiskled qlrkdglpdv kgadvpyvDl ysifqdldgi qnpsayvyGF
301 ettkaCCGyG gryNynrvCG naglcnvtak aCnpssylls flfwDgfHps ekGykavAea
361 l
```

Figure 2

SEQ ID No. 2

```
  1 mkkwfvcllg lvaltvqaad srpafsrivm fgdslsdtgk myskmrgylp ssppyyegrf
 61 sngpvwleql tnefpgltia neaeggptav aynkiswnpk yqvinnldye vtqflqkdsf
121 kpddlvilwv gandylaygw nteqdakrvr daisdaanrm vlngakeill fnlpdlgqnp
181 sarsqkvvea ashvsayhnq lllnlarqla ptgmvklfei dkqfaemlrd pqnfglsdqr
241 nacyggsyvw kpfasrsast dsqlsafnpq erlaiagnpl laqavaspma arsastlnce
301 gkmfwdqvhp ttvvhaalse paatfiesqy eflah
```

Figure 3

SEQ ID No. 3

```
  1 mkkwfvcllg lialtvqaad trpafsrivm fgdslsdtgk myskmrgylp ssppyyegrf
 61 sngpvwleql tkqfpgltia neaeggatav aynkiswnpk yqvynnldye vtqflqkdsf
121 kpddlvilwv gandylaygw nteqdakrvr daisdaanrm vlngakqill fnlpdlgqnp
181 sarsqkvvea vshvsayhnk lllnlarqla ptgmvklfei dkqfaemlrd pqnfglsdve
241 npcydggyvw kpfatrsvst drqlsafspq erlaiagnpl laqavaspma rrsasplnce
301 gkmfwdqvhp ttvvhaalse raatfietqy eflahg
```

Figure 4

SEQ ID No. 4

```
  1 mpkpalrrvm tatvaavgtl algltdatah aapaqatptl dyvalgdsys agsgvlpvdp
 61 anllclrsta nyphviadtt garltdvtcg aaqtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

Figure 5

SEQ ID No. 5

```
  1 mpkpalrrvm tatvaavgtl algltdatah aapaqatptl dyvalgdsys agsgvlpvdp
 61 anllclrsta nyphviadtt garltdvtcg aaqtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

Figure 6

SEQ ID No. 6

```
  1 mdyekfllfg dsitefafnt rpiedgkdqy algaalvney trkmdilqrg fkgytsrwal
 61 kilpeilkhe snivmatifl gandacsagp qsvplpefid nirqmvslmk syhirpiiig
121 pglvdrekwe kekseeialg yfrtnenfai ysdalaklan eekvpfvaln kafqqeggda
181 wqqlltdglh fsgkgykifh dellkvietf ypqyhpknmq yklkdwrdvl ddgsnims
```

## Figure 7

```
Alignment of pfam00657.6 consensus sequence with P10480
                   *->ivafGDSlTdg................eayygdsdgggwgagladrL
                      iv+fGDSl+d+++  ++ ++  +++++++ +++s+g  w ++l + +
    P10480      28    IVMFGDSLSDTgkmyskmrgylpssppYYEGRFSNGPVWLEQLTNEF 74

                   tall..rlrarprgvdvfnrgisGrtsdGrlivDalvallFlaqslglpn
                   + l    + ++++++++ +n+   +
    P10480      75 PGLTiaNEAEGGPTAVAYNKISWNPK------------------------ 100

                   LpPYLsgdflrGANFAsagAtIlptsgpfliQvqFkdfksqvlelrqalg
                                                               ++  ++
    P10480     101 --------------------------------------------YQVINN 106

                   llqellrllpvldakspdlvtimiGtNDlitsaffgpkstesdrnvsvpe
                   l++e+ ++l +++ k+ dlv++++G+ND+          ++ ++ ++++++
    P10480     107 LDYEVTQFLQKDSFKPDDLVILWVGANDY--------LAYGWNTEQDAKR 148

                   fkdnlrqlikrLrsnngariivlitlvilnlgplGClPlklalalasskn
                   ++d ++++++r+   nga+      ++++nl+ lG+ P+
    P10480     149 VRDAISDAANRMV-LNGAK-----EILLFNLPDLGQNPS----------- 181

                   vdasgclerlneavadfnealrelaiskledqlrkdglpdvkgadvpyvD
                    ++++ +e +  ++a++n++l +la     +ql+++g+++++++d ++++
    P10480     182 ARSQKVVEAASHVSAYHNQLLLNLA-----RQLAPTGMVKLFEIDKQFAE 226

                   lysifqdldgiqnpsayv.y....GFe.ttkaCCGyGgr.yNyn.rv.CG
                   +   +q+++ + + +a+++++   +++ +++a++++++++ +N+++r+ ++
    P10480     227 MLRDPQNFGLSDQRNACYgGsyvwKPFaSRSASTDSQLSaFNPQeRLaIA 276

                   nag.l.c.nvtakaC.npssyll.sflfwDgfHpsekGykavAeal<-*
                   +++ l +  ++++a++ +s+ ++++++fwD++Hp+   ++a+ e
    P10480     277 GNPlLaQaVASPMAArSASTLNCeGKMFWDQVHPTTVVHAALSEPA    322

Alignment of pfam00657.6 consensus sequence with AAG09804
                   *->ivafGDSlTdg................eayygdsdgggwgagladrL
                      iv+fGDSl+d+++  ++ ++  +++++++ +++s+g  w ++l + +
   AAG09804     28    IVMFGDSLSDTgkmyskmrgylpssppYYEGRFSNGPVWLEQLTKQF 74

                   tallrlrarprgvdvfnrgisGrtsdGrlivDalvallFlaqslglpnLp
                             +g+++ n + +G+t
   AAG09804     75 ----------PGLTIANEAEGGAT-------------------------- 88

                   PYLsgdflrGANFAsagAtIlptsgpfliQvqFkdfksqvlelrqa....
                                                        ++++ + ++++ +
   AAG09804     89 ------------------------------------AVAYNKISWNpkyq 102

                   ..lgllqellrllpvldakspdlvtimiGtNDlitsaffgpkstesdrnv
                     ++l++e+ ++l +++ k+ dlv++++G+ND+         ++ ++ ++
   AAG09804    103 vyNNLDYEVTQFLQKDSFKPDDLVILWVGANDY--------LAYGWNTEQ 144

                   svpefkdnlrqlikrLrsnngariivlitlvilnlgplGClPlklalala
                   ++++++d ++++++r+   nga+     +++++nl+ lG+ P+
   AAG09804    145 DAKRVRDAISDAANRMV-LNGAK-----QILLFNLPDLGQNPS------- 181

                   ssknvdasgclerlneavadfnealrelaiskledqlrkdglpdvkgadv
                       ++++ +e +  ++a++n++l +la     +ql+++g+++++++d
   AAG09804    182 ----ARSQKVVEAVSHVSAYHNKLLLNLA-----RQLAPTGMVKLFEIDK 222

                   pyvDlysifqdldgiqnpsayv.y....GFe.ttkaCCGyGgr.yNyn.r
                   +++++   +q+++ +  ++ +++++   +++ t++ +++ +++ + +++r
   AAG09804    223 QFAEMLRDPQNFGLSDVENPCYdGgyvwKPFaTRSVSTDRQLSaFSPQeR 272

                   v.CGnag.l.c.nvtakaC.npssyll.sflfwDgfHpsekGykavAeal
                   + +++++ l +  ++++a++ +s  ++++++fwD++Hp+   ++a+ e+
   AAG09804    273 LaIAGNPlLaQaVASPMARrSASPLNCeGKMFWDQVHPTTVVHAALSERA 322

                   <-*
```

```
AAG09804      -     -

Alignment of pfam00657.6 consensus sequence with NP_631558
                    *->ivafGDSlTdgeayygdsdgggwgagladrLtallrlrarprgvdvf
                       +va+GDS ++g          +g  +  +++L      + + + ++  +
   NP_631558     42    YVALGDSYSAG---------SGVLPVDPANL----LCLRSTANYPHV 75

                    nrgisGrtsdGrlivD.a.l.vallFlaqslglpnLpPYLsgdflrGANF
                     + ++G++       D + + +
   NP_631558     76 IADTTGAR-----LTDvTcGaAQ-------------------------- 93

                    AsagAtIlptsgpfliQvqFkdfksqvlelrqalgllqellrllpvldak
                                                  +++     ++ +  ++ +++
   NP_631558     94 ----------------------------TADFTRAQYPGVAPQLDALGT 114

                    spdlvtimiGtNDl...................itsaffgpkstesdrnvsvp
                    + dlvt+ iG+ND ++  +  +  ++ +   ++  + +k    ++ + +++
   NP_631558    115 GTDLVTLTIGGNDNstfinaitacgtagvlSGGKGSPCKDRHGTSFDDEI 164

                    efkdn..lrqlikrLrs.nngariivlitlvilnlg...........plG
                    e  +++ l++++  +r+++ +ar+ +l  ++i+++  +++    + +   G
   NP_631558    165 EANTYpaLKEALLGVRArAPHARVAALGYPWITPATadpscflklplAAG 214

                    ClPlklalalassknvdasgclerlneavadfnealrelaiskledqlrk
                      P+                     l+ ++a  n a+r  a
   NP_631558    215 DVPY-------------------LRAIQAHLNDAVRRAA---------- 234

                    dglpdvkgadvpyvDlysifqdldgiqnpsayvyGFettkaCCGyGgryN
                          ++ + +yvD+ ++
   NP_631558    235 ------EETGATYVDFSGVSDG--------------------------- 250

                    ynrvCGnaglcnvtakaC.npssyll.sflfwDgf...HpsekGykavAe
                                 ++aC+ p +++ +   lf + + + Hp++ G +++Ae
   NP_631558    251 --------------HDACeAPGTRWIePLLFGHSLvpvHPNALGERRMAE 286

                    al<-*
                    +
   NP_631558    287 HT     288

Alignment of pfam00657.6 consensus sequence with CAC42140
                    *->ivafGDSlTdgeayygdsdgggwgagladrLtallrlrarprgvdvf
                       +va+GDS ++g          +g  +  +++L      + + + ++  +
    CAC42140     42    YVALGDSYSAG---------SGVLPVDPANL----LCLRSTANYPHV 75

                    nrgisGrtsdGrlivD.a.l.vallFlaqslglpnLpPYLsgdflrGANF
                     + ++G++       D + + +
    CAC42140     76 IADTTGAR-----LTDvTcGaAQ-------------------------- 93

                    AsagAtIlptsgpfliQvqFkdfksqvlelrqalgllqellrllpvldak
                                                  +++     ++ +  ++ +++
    CAC42140     94 ----------------------------TADFTRAQYPGVAPQLDALGT 114

                    spdlvtimiGtNDl...................itsaffgpkstesdrnvsvp
                    + dlvt+ iG+ND ++  +  +  ++ +   ++  + +k    ++ + +++
    CAC42140    115 GTDLVTLTIGGNDNstfinaitacgtagvlSGGKGSPCKDRHGTSFDDEI 164

                    efkdn..lrqlikrLrs.nngariivlitlvilnlg...........plG
                    e  +++ l++++  +r+++ +ar+ +l  ++i+++  +++    + +   G
    CAC42140    165 EANTYpaLKEALLGVRArAPHARVAALGYPWITPATadpscflklplAAG 214

                    ClPlklalalassknvdasgclerlneavadfnealrelaiskledqlrk
                      P+                     l+ ++a  n a+r  a
    CAC42140    215 DVPY-------------------LRAIQAHLNDAVRRAA---------- 234

                    dglpdvkgadvpyvDlysifqdldgiqnpsayvyGFettkaCCGyGgryN
                          ++ + +yvD+ ++
    CAC42140    235 ------EETGATYVDFSGVSDG--------------------------- 250

                    ynrvCGnaglcnvtakaC.npssyll.sflfwDgf...HpsekGykavAe
                                 ++aC+ p +++ +   lf + + + Hp++ G +++Ae
    CAC42140    251 --------------HDACeAPGTRWIePLLFGHSLvpvHPNALGERRMAE 286
```

```
                     al<-*
                     +
    CAC42140     287 HT    288

Alignment of pfam00657.6 consensus sequence with P41734
                  *->ivafGDSlTdg....eayygdsdgggwgagladrLtallrlrarprg
                     ++fGDS+T+   .+++ + +  d+   ga+l + +         +r+
      P41734       6 FLLFGDSITEFafntRPIEDGKDQYALGAALVNEY---------TRK 43

                     vdvfnrgisGrtsdGrlivDalvallFlaqslglpnLpPYLsgdflrGAN
                     +d+  rg++G+t
      P41734      44 MDILQRGFKGYT-------------------------------------- 55

                     FAsagAtIlptsgpfliQvqFkdfksqvlelrqalgllqellrllpvlda
                                                   +r+al++l+e+l+      +
      P41734      56 ------------------------------SRWALKILPEILKH-----E 70

                     kspdlvtimiGtNDlitsaffgpkstesdrnvsvpefkdnlrqlikrLrs
                      +  + ti++G+ND+         ++ +++ v++pef+dn+rq++++++s
      P41734      71 SNIVMATIFLGANDA---------CSAGPQSVPLPEFIDNIRQMVSLMKS 111

                     nngariivlitlvilnlgplGClPlklalalassknvdasgclerlneav
                      ++++ii++++lv   ++             ++ k ++ +  + r+ne +
      P41734     112 YHIRPIIIGPGLVDREKW-------------EKEKSEEIALGYFRTNENF 148

                     adfnealrelaiskledqlrkdglpdvkgadvpyvDlysifqdldgiqnp
                     a +  al +la                ++ +vp+v l+++fq+ +g++++
      P41734     149 AIYSDALAKLA----------------NEEKVPFVALNKAFQQEGGDAWQ 182

                     sayvyGFettkaCCGyGgryNynrvCGnaglcnvtakaCnpssyllsflf
                     +                                               l+
      P41734     183 Q-----------------------------------------------LL 185

                     wDgfHpsekGykavAeal<-*
                      Dg+H+s kGyk+++++l
      P41734     186 TDGLHFSGKGYKIFHDEL    203
```

Figure 8

```
A.sal   1   MKKWFVCLLGLIALTVQAADTRPAFSRIVMFGDSLSDTGKMYSKMRGYLPSSPPYYEGRF 60
                       +        +
A.hyd   1   MKKWFVCLLGLVALTVQAADSRPAFSRIVMFGDSLSDTGKMYSKMRGYLPSSPPYYEGRF 60

A. sal 61   SNGPVWLEQLTKQFPGLTIANEAEGGATAVAYNKISWNPKYQVINNLDYEVTQFLQKDSF 120
                           ++             +
A. hyd 61   SNGPVWLEQLTNEFPGLTIANEAEGGPTAVAYNKISWNPKYQVINNLDYEVTQFLQKDSF 120

A. sal 121  KPDDLVILWVGANDYLAYGWNTEQDAKRVRDAISDAANRMVLNGAKQILLFNLPDLGQNP 180
                                                                        +
A. hyd 121  KPDDLVILWVGANDYLAYGWNTEQDAKRVRDAISDAANRMVLNGAKEILLFNLPDLGQNP 180

A. sal 181  SARSQKVVEAVSHVSAYHNKLLLNLARQLAPTGMVKLFEIDKQFAEMLRDPQNFGLSDVE 240
                          +        +                                         ++
A.hyd  181  SARSQKVVEAASHVSAYHNQLLLNLARQLAPTGMVKLFEIDKQFAEMLRDPQNFGLSDQR 240

A. sal 241  NPCYDGGYVWKPFATRSVSTDRQLSAFSPQERLAIAGNPLLAQAVASPMARRSASPLNCE 300
             + ++ +       +  +   +     +                      +    +
A. hyd 241  NACYGGSYVWKPFASRSASTDSQLSAFNPQERLAIAGNPLLAQAVASPMAARSASTLNCE 300

A. sal 301  GKMFWDQVHPTTVVHAALSERAATFIETQYEFLAH 335
                                +      +
A. hyd 301  GKMFWDQVHPTTVVHAALSEPAATFIESQYEFLAH 335
```

## Figure 9

```
  1 ATGAAAAAAT GGTTTGTGTG TTTATTGGGA TTGGTCGCGC TGACAGTTCA GGCAGCCGAC
 61 AGCCGTCCCG CCTTCTCCCG GATCGTGATG TTTGGCGACA GCCTCTCCGA TACCGGCAAG
121 ATGTACAGCA AGATGCGCGG TTACCTCCCC TCCAGCCCCC CCTACTATGA GGGCCGCTTC
181 TCCAACGGGC CCGTCTGGCT GGAGCAGCTG ACCAACGAGT TCCCGGGCCT GACCATAGCC
241 AACGAGGCGG AAGGCGGACC GACCGCCGTG GCTTACAACA AGATCTCCTG GAATCCCAAG
301 TATCAGGTCA TCAACAACCT GGACTACGAG GTCACCCAGT TCCTGCAAAA AGACAGCTTC
361 AAGCCGGACG ATCTGGTGAT CCTCTGGGTC GGCGCCAACG ACTATCTGGC CTATGGCTGG
421 AACACAGAGC AGGATGCCAA GCGGGTGCGC GACGCCATCA GCGATGCGGC CAACCGCATG
481 GTGCTGAACG GCGCCAAGGA GATACTGCTG TTCAACCTGC CGGATCTGGG CCAGAACCCC
541 TCGGCCCGCA GCCAGAAGGT GGTCGAGGCG GCCAGCCATG TCTCCGCCTA CCACAACCAG
601 CTGCTGCTGA ACCTGGCACG CCAGCTGGCT CCCACCGGCA TGGTGAAGCT GTTCGAGATC
661 GACAAGCAGT TTGCCGAGAT GCTGCGTGAT CCGCAGAACT TCGGCCTGAG CGACCAGAGG
721 AACGCCTGCT ACGGTGGCAG CTATGTATGG AAGCCGTTTG CCTCCCGCAG CGCCAGCACC
781 GACAGCCAGC TCTCCGCCTT CAACCCGCAG GAGCGCCTCG CCATCGCCGG CAACCCGCTG
841 CTGGCCCAGG CCGTCGCCAG CCCCATGGCT GCCCGCAGCG CCAGCACCCT CAACTGTGAG
901 GGCAAGATGT TCTGGGATCA GGTCCACCCC ACCACTGTCG TGCACGCCGC CCTGAGCGAG
961 CCCGCCGCCA CCTTCATCGA GAGCCAGTAC GAGTTCCTCG CCCAC
```

Figure 10

```
  1  ATGAAAAAAT GGTTTGTTTG TTTATTGGGG TTGATCGCGC TGACAGTTCA GGCAGCCGAC
 61  ACTCGCCCCG CCTTCTCCCG GATCGTGATG TTCGGCGACA GCCTCTCCGA TACCGGCAAA
121  ATGTACAGCA AGATGCGCGG TTACCTCCCC TCCAGCCCGC CCTACTATGA GGGCCGTTTC
181  TCCAACGGAC CCGTCTGGCT GGAGCAGCTG ACCAAGCAGT TCCCGGGTCT GACCATCGCC
241  AACGAAGCGG AAGGCGGTGC CACTGCCGTG GCTTACAACA AGATCTCCTG GAATCCCAAG
301  TATCAGGTCT ACAACAACCT GGACTACGAG GTCACCCAGT TCTTGCAGAA AGACAGCTTC
361  AAGCCGGACG ATCTGGTGAT CCTCTGGGTC GGTGCCAATG ACTATCTGGC ATATGGCTGG
421  AATACGGAGC AGGATGCCAA GCGAGTTCGC GATGCCATCA GCGATGCGGC CAACCGCATG
481  GTACTGAACG GTGCCAAGCA GATACTGCTG TTCAACCTGC CGGATCTGGG CCAGAACCCG
541  TCAGCCCGCA GTCAGAAGGT GGTCGAGGCG GTCAGCCATG TCTCCGCCTA TCACAACAAG
601  CTGCTGCTGA ACCTGGCACG CCAGCTGGCC CCCACCGGCA TGGTAAAGCT GTTCGAGATC
661  GACAAGCAAT TTGCCGAGAT GCTGCGTGAT CCGCAGAACT TCGGCCTGAG CGACGTCGAG
721  AACCCCTGCT ACGACGGCGG CTATGTGTGG AAGCCGTTTG CCACCCGCAG CGTCAGCACC
781  GACCGCCAGC TCTCCGCCTT CAGTCCGCAG GAACGCCTCG CCATCGCCGG CAACCCGCTG
841  CTGGCACAGG CCGTTGCCAG TCCTATGGCC CGCCGCAGCG CCAGCCCCCT CAACTGTGAG
901  GGCAAGATGT TCTGGGATCA GGTACACCCG ACCACTGTCG TGCACGCAGC CCTGAGCGAG
961  CGCGCCGCCA CCTTCATCGA GACCCAGTAC GAGTTCCTCG CCCACGGATG A
```

Figure 11

```
  1 ATGCCGAAGC CTGCCCTTCG CCGTGTCATG ACCGCGACAG TCGCCGCCGT CGGCACGCTC
 61 GCCCTCGGCC TCACCGACGC CACCGCCCAC GCCGCGCCCG CCCAGGCCAC TCCGACCCTG
121 GACTACGTCG CCCTCGGCGA CAGCTACAGC GCCGGCTCCG GCGTCCTGCC CGTCGACCCC
181 GCCAACCTGC TCTGTCTGCG CTCGACGGCC AACTACCCCC ACGTCATCGC GGACACGACG
241 GGCGCCCGCC TCACGGACGT CACCTGCGGC GCCGCGCAGA CCGCCGACTT CACGCGGGCC
301 CAGTACCCGG GCGTCGCACC CCAGTTGGAC GCGCTCGGCA CCGGCACGGA CCTGGTCACG
361 CTCACCATCG GCGGCAACGA CAACAGCACC TTCATCAACG CCATCACGGC CTGCGGCACG
421 GCGGGTGTCC TCAGCGGCGG CAAGGGCAGC CCCTGCAAGG ACAGGCACGG CACCTCCTTC
481 GACGACGAGA TCGAGGCCAA CACGTACCCC GCGCTCAAGG AGGCGCTGCT CGGCGTCCGC
541 GCCAGGGCTC CCCACGCCAG GGTGGCGGCT CTCGGCTACC CGTGGATCAC CCCGGCCACC
601 GCCGACCCGT CCTGCTTCCT GAAGCTCCCC CTCGCCGCCG GTGACGTGCC CTACCTGCGG
661 GCCATCCAGG CACACCTCAA CGACGCGGTC CGGCGGGCCG CCGAGGAGAC CGGAGCCACC
721 TACGTGGACT TCTCCGGGGT GTCCGACGGC CACGACGCCT GCGAGGCCCC CGGCACCCGC
781 TGGATCGAAC CGCTGCTCTT CGGGCACAGC CTCGTTCCCG TCCACCCCAA CGCCCTGGGC
841 GAGCGGCGCA TGGCCGAGCA CACGATGGAC GTCCTCGGCC TGGACTGA
```

Figure 12

```
  1 TCAGTCCAGG CCGAGGACGT CCATCGTGTG CTCGGCCATG CGCCGCTCGC CCAGGGCGTT
 61 GGGGTGGACG GGAACGAGGC TGTGCCCGAA GAGCAGCGGT TCGATCCAGC GGGTGCCGGG
121 GGCCTCGCAG GCGTCGTGGC CGTCGGACAC CCCGGAGAAG TCCACGTAGG TGGCTCCGGT
181 CTCCTCGGCG GCCCGCCGGA CCGCGTCGTT GAGGTGTGCC TGGATGGCCC GCAGGTAGGG
241 CACGTCACCG GCGGCGAGGG GGAGCTTCAG GAAGCAGGAC GGGTCGGCGG TGGCCGGGGT
301 GATCCACGGG TAGCCGAGAG CCGCCACCCT GGCGTGGGGA GCCCTGGCGC GGACGCCGAG
361 CAGCGCCTCC TTGAGCGCGG GGTACGTGTT GGCCTCGATC TCGTCGTCGA AGGAGGTGCC
421 GTGCCTGTCC TTGCAGGGGC TGCCCTTGCC GCCGCTGAGG ACACCCGCCG TGCCGCAGGC
481 CGTGATGGCG TTGATGAAGG TGCTGTTGTC GTTGCCGCCG ATGGTGAGCG TGACCAGGTC
541 CGTGCCGGTG CCGAGCGCGT CCAACTGGGG TGCGACGCCC GGGTACTGGG CCCGCGTGAA
601 GTCGGCGGTC TGCGCGGCGC CGCAGGTGAC GTCCGTGAGG CGGGCGCCCG TCGTGTCCGC
661 GATGACGTGG GGGTAGTTGG CCGTCGAGCG CAGACAGAGC AGGTTGGCGG GGTCGACGGG
721 CAGGACGCCG GAGCCGGCGC TGTAGCTGTC GCCGAGGGCG ACGTAGTCCA GGGTCGGAGT
781 GGCCTGGGCG GGCGCGGCGT GGGCGGTGGC GTCGGTGAGG CCGAGGGCGA GCGTGCCGAC
841 GGCGGCGACT GTCGCGGTCA TGACACGGCG AAGGGCAGGC TTCGGCAT
```

Figure 13

```
  1 ATGGATTACG AGAAGTTTCT GTTATTTGGG GATTCCATTA CTGAATTTGC TTTTAATACT
 61 AGGCCCATTG AAGATGGCAA AGATCAGTAT GCTCTTGGAG CCGCATTAGT CAACGAATAT
121 ACGAGAAAAA TGGATATTCT TCAAAGAGGG TTCAAAGGGT ACACTTCTAG ATGGGCGTTG
181 AAAATACTTC CTGAGATTTT AAAGCATGAA TCCAATATTG TCATGGCCAC AATATTTTTG
241 GGTGCCAACG ATGCATGCTC AGCAGGTCCC CAAAGTGTCC CCCTCCCCGA ATTTATCGAT
301 AATATTCGTC AAATGGTATC TTTGATGAAG TCTTACCATA TCCGTCCTAT TATAATAGGA
361 CCGGGGCTAG TAGATAGAGA GAAGTGGGAA AAAGAAAAAT CTGAAGAAAT AGCTCTCGGA
421 TACTTCCGTA CCAACGAGAA CTTTGCCATT TATTCCGATG CCTTAGCAAA ACTAGCCAAT
481 GAGGAAAAAG TTCCCTTCGT GGCTTTGAAT AAGGCGTTTC AACAGGAAGG TGGTGATGCT
541 TGGCAACAAC TGCTAACAGA TGGACTGCAC TTTTCCGGAA AAGGGTACAA AATTTTTCAT
601 GACGAATTAT TGAAGGTCAT TGAGACATTC TACCCCCAAT ATCATCCCAA AAACATGCAG
661 TACAAACTGA AAGATTGGAG AGATGTGCTA GATGATGGAT CTAACATAAT GTCTTGA
```

Figure 14

(SEQ ID No. 12)

```
        10         20         30         40         50         60
         |          |          |          |          |          |
MNLRQWMGAA TAALALGLAA CGGGGTDQSG NPNVAKVQRM VVFGDSLSDI GTYTPVAQAV

        70         80         90        100        110        120
         |          |          |          |          |          |
GGGKFTTNPG PIWAETVAAQ LGVTLTPAVM GYATSVQNCP KAGCFDYAQG GSRVTDPNGI

       130        140        150        160        170        180
         |          |          |          |          |          |
GHNGGAGALT YPVQQQLANF YAASNNTFNG NNDVVFVLAG SNDIFFWTTA AATSGSGVTP

       190        200        210        220        230        240
         |          |          |          |          |          |
AIATAQVQQA ATDLVGYVKD MIAKGATQVY VFNLPDSSLT PDGVASGTTG QALLHALVGT

       250        260        270        280        290        300
         |          |          |          |          |          |
FNTTLQSGLA GTSARIIDFN AQLTAAIQNG ASFGFANTSA RACDATKINA LVPSAGGSSL

       310        320        330        340
         |          |          |          |
FCSANTLVAS GADQSYLFAD GVHPTTAGHR LIASNVLARL LADNVAH
```

Figure 15

(SEQ ID No. 13)

```
atgaacctgc gtcaatggat gggcgccgcc acggctgccc ttgccttggg cttggccgcg     60
tgcgggggcg gtgggaccga ccagagcggc aatcccaatg tcgccaaggt gcagcgcatg    120
gtggtgttcg gcgacagcct gagcgatatc ggcacctaca cccccgtcgc gcaggcggtg    180
ggcggcggca agttcaccac caacccgggc ccgatctggg ccgagaccgt ggccgcgcaa    240
ctgggcgtga cgctcacgcc ggcggtgatg ggctacgcca cctccgtgca gaattgcccc    300
aaggccggct gcttcgacta tgcgcagggc ggctcgcgcg tgaccgatcc gaacggcatc    360
ggccacaacg gcggcgcggg ggcgctgacc tacccggttc agcagcagct cgccaacttc    420
tacgcggcca gcaacaacac attcaacggc aataacgatg tcgtcttcgt gctggccggc    480
agcaacgaca ttttcttctg gaccactgcg gcggccacca gcggctccgg cgtgacgccc    540
gccattgcca cggcccaggt gcagcaggcc gcgacggacc tggtcggcta tgtcaaggac    600
atgatcgcca agggtgcgac gcaggtctac gtgttcaacc tgcccgacag cagcctgacg    660
ccggacggcg tggcaagcgg cacgaccggc caggcgctgc tgcacgcgct ggtgggcacg    720
ttcaacacga cgctgcaaag cgggctggcc ggcacctcgg cgcgcatcat cgacttcaac    780
gcacaactga ccgcggcgat ccagaatggc gcctcgttcg gcttcgccaa caccagcgcc    840
cgggcctgcg acgccaccaa gatcaatgcc ctggtgccga gcgccggcgg cagctcgctg    900
ttctgctcgg ccaacacgct ggtggcttcc ggtgcggacc agagctacct gttcgccgac    960
ggcgtgcacc cgaccacggc cggccatcgc ctgatcgcca gcaacgtgct ggcgcgcctg   1020
ctggcggata acgtcgcgca ctga                                          1044
```

Figure 16 (SEQ ID No. 20)

```
  1 migsyvavgd sftegvgdpg pdgafvgwad rlavlladrr pegdftytnl avrgrlldqi
 61 vaeqvprvvg lapdlvsfaa ggndiirpgt dpdevaerfe lavaaltaaa gtvlvttgfd
121 trgvpvlkhl rgkiatyngh vraiadrygc pvldlwslrs vqdrrawdad rlhlspeght
181 rvalraqqal glrvpadpdq pwpplpprgt ldvrrddvhw areylvpwig rrlrgessgd
241 hvtakgtlsp daiktriaav a
```

Figure 17 (SEQ ID No. 21)

```
  1 gtgatcgggt cgtacgtggc ggtgggggac agcttcaccg agggcgtcgg cgaccccggc
 61 cccgacgggg cgttcgtcgg ctgggccgac cggctcgccg tactgctcgc ggaccggcgc
121 cccgagggcg acttcacgta cacgaacctc gccgtgcgcg gcaggctcct cgaccagatc
181 gtggcggaac aggtcccgcg ggtcgtcgga ctcgcgcccg acctcgtctc gttcgcggcg
241 ggcggcaacg acatcatccg gcccggcacc gatcccgacg aggtcgccga gcggttcgag
301 ctggcggtgg ccgcgctgac cgccgcggcc ggaaccgtcc tggtgaccac cgggttcgac
361 acccgggggg tgcccgtcct caagcacctg cgcggcaaga tcgccacgta caacgggcac
421 gtccgcgcca tcgccgaccg ctacggctgc ccggtgctcg acctgtggtc gctgcggagc
481 gtccaggacc gcagggcgtg ggacgccgac cggctgcacc tgtcgccgga ggggcacacc
541 cgggtggcgc tgcgcgcggg gcaggccctg ggcctgcgcg tcccggccga ccctgaccag
601 ccctggccgc ccctgccgcc gcgcggcacg ctcgacgtcc ggcgcgacga cgtgcactgg
661 gcgcgcgagt acctggtgcc gtggatcggg cgccggctgc ggggcgagtc gtcgggcgac
721 cacgtgacgg ccaaggggac gctgtcgccg gacgccatca agacgcggat cgccgcggtg
781 gcctga
```

Figure 18
(SEQ ID No. 22)

```
  1 mqtnpaytsl vavgdsfteg msdllpdgsy rgwadllatr maarspgfry anlavrgkli
 61 gqivdeqvdv aaamgadvit lvgglndtlr pkcdmarvrd lltqaverla phceqlvlmr
121 spgrqgpvle rfrprmealf aviddlagrh gavvvdlyga qsladprmwd vdrlhltaeg
181 hrrvaeavwq slghepedpe whapipatpp pgwvtrrtad vrfarqhllp wigrrltgrs
241 sgdglpakrp dllpyedpar
```

Figure 19 (SEQ ID No. 23)

```
  1 atgcagacga accccgcgta caccagtctc gtcgccgtcg gcgactcctt caccgagggc
 61 atgtcggacc tgctgcccga cggctcctac cgtggctggg ccgacctcct cgccacccgg
121 atggcggccc gctcccccgg cttccggtac gccaacctgg cggtgcgcgg gaagctgatc
181 ggacagatcg tcgacgagca ggtggacgtg gccgccgcca tgggagccga cgtgatcacg
241 ctggtcggcg ggctcaacga cacgctgcgg cccaagtgcg acatggcccg ggtgcgggac
301 ctgctgaccc aggccgtgga acggctcgcc ccgcactgcg agcagctggt gctgatgcgc
361 agtcccggtc gccagggtcc ggtgctggag cgcttccggc cccgcatgga ggccctgttc
421 gccgtgatcg acgacctggc cgggcggcac ggcgccgtgg tcgtcgacct gtacggggcc
481 cagtcgctgg ccgaccctcg gatgtgggac gtggaccggc tgcacctgac cgccgagggc
541 caccgccggg tcgcggaggc ggtgtggcag tcgctcggcc acgagcccga ggaccccgag
601 tggcacgcgc cgatcccggc gacgccgccg ccggggtggg tgacgcgcag gaccgcggac
661 gtccggttcg cccggcagca cctgctgccc tggataggcc gcaggctgac cgggcgctcg
721 tccggggacg gcctgccggc caagcgcccg gacctgctgc cctacgagga ccccgcacgg
781 tga
```

## Figure 20 (SEQ ID No. 24)

```
  1 mtrgrdggag apptkhrall aaivtlivai saaiyagasa ddgsrdhalq aggrlprgda
 61 apastgawvg awatapaaae pgtettglag rsvrnvvhts vggtgaritl snlygqsplt
121 vthasialaa gpdtaaaiad tmrrltfggs arviipaggq vmsdtarlai pyganvlvtt
181 yspipsgpvt yhpqarqtsy ladgdrtadv tavayttptp ywryltaldv lsheadgtvv
241 afgdsitdga rsqsdanhrw tdvlaarlhe aagdgrdtpr ysvvnegisg nrlltsrpgr
301 padnpsglsr fqrdvlertn vkavvvvlgv ndvlnspela drdailtglr tlvdraharg
361 lrvvgatitp fggyggytea retmrqevne eirsgrvfdt vvdfdkalrd pydprrmrsd
421 ydsgdhlhpg dkgyarmgav idlaalkgaa pvka
```

## Figure 21 (SEQ ID No. 25)

```
   1 atgacccggg gtcgtgacgg gggtgcgggg gcgcccccca ccaagcaccg tgccctgctc
  61 gcggcgatcg tcaccctgat agtggcgatc tccgcggcca tatacgccgg agcgtccgcg
 121 gacgacggca gcagggacca cgcgctgcag gccggaggcc gtctcccacg aggagacgcc
 181 gcccccgcgt ccaccggtgc ctgggtgggc gcctgggcca ccgcaccggc cgcggccgag
 241 ccgggcaccg agacgaccgg cctggcgggc cgctccgtgc gcaacgtcgt gcacacctcg
 301 gtcggcggca ccggcgcgcg gatcaccctc tcgaacctgt acgggcagtc gccgctgacc
 361 gtcacacacg cctcgatcgc cctggccgcc gggcccgaca ccgccgccgc gatcgccgac
 421 accatgcgcc ggctcacctt cggcggcagc gcccgggtga tcatcccggc gggcggccag
 481 gtgatgagcg acaccgcccg cctcgccatc ccctacgggg cgaacgtcct ggtcaccacg
 541 tactccccca tcccgtccgg gccggtgacc taccatccgc aggcccggca gaccagctac
 601 ctggccgacg gcgaccgcac ggcggacgtc accgccgtcg cgtacaccac ccccacgccc
 661 tactggcgct acctgaccgc cctcgacgtg ctgagccacg aggccgacgg cacggtcgtg
 721 gcgttcggcg actccatcac cgacggcgcc cgctcgcaga gcgacgccaa ccaccgctgg
 781 accgacgtcc tcgccgcacg cctgcacgag gcggcgggcg acggccggga cacgccccgc
 841 tacagcgtcg tcaacgaggg catcagcggc aaccggctcc tgaccagcag gccgggggcgg
 901 ccggccgaca acccgagcgg actgagccgg ttccagcggg acgtgctgga acgcaccaac
 961 gtcaaggccg tcgtcgtcgt cctcggcgtc aacgacgtcc tgaacagccc ggaactcgcc
1021 gaccgcgacg ccatcctgac cggcctgcgc accctcgtcg accgggcgca cgcccgggga
1081 ctgcgggtcg tcggcgccac gatcacgccg ttcggcggct acggcggcta caccgaggcc
1141 cgcgagacga tgcggcagga ggtcaacgag gagatccgct ccggccgggt cttcgacacg
1201 gtcgtcgact tcgacaaggc cctgcgcgac ccgtacgacc cgcgccggat gcgctccgac
1261 tacgacagcg gcgaccacct gcaccccggc gacaaggggt acgcgcgcat gggcgcggtc
1321 atcgacctgg ccgcgctgaa gggcgcggcg ccggtcaagg cgtag
```

Figure 22 (SEQ ID No. 26)

1 mtsmsrarva rriaagaayg gggiglagaa avglvvaevq larrrvgvgt ptrvpnaqgl
61 yggtlptagd pplrlmmlgd staagqgvhr agqtpgalla sglaavaerp vrlgsvaqpg
121 acsddldrqv alvlaepdrv pdicvimvga ndvthrmpat fsvrhlssav rrlrtagaev
181 vvgtcpdlgt iervrqplrw larrasrqla aaqtigaveq ggrtvslgdl lgpefaqnpr
241 elfgpdnyhp saegyataam avlpsvcaal glwpadeehp dalrregflp varaaaeaas
301 eagtevaaam ptgprgpwal lkrrrrrrvs eaepsspsgv

Figure 23 (SEQ ID No. 27)

1 atgacgagca tgtcgagggc gagggtggcg cggcggatcg cggccggcgc ggcgtacggc
61 ggcggcggca tcggcctggc gggagcggcg gcggtcggtc tggtggtggc cgaggtgcag
121 ctggccagac gcagggtggg ggtgggcacg ccgacccggg tgccgaacgc gcagggactg
181 tacggcggca ccctgcccac ggccggcgac ccgccgctgc ggctgatgat gctgggcgac
241 tccacggccg ccgggcaggg cgtgcaccgg gccgggcaga cgccgggcgc gctgctggcg
301 tccgggctcg cggcggtggc ggagcggccg gtgcggctgg ggtcggtcgc ccagccgggg
361 gcgtgctcgg acgacctgga ccggcaggtg gcgctggtgc tcgccgagcc ggaccgggtg
421 cccgacatct gcgtgatcat ggtcggcgcc aacgacgtca cccaccggat gccggcgacc
481 cgctcggtgc ggcacctgtc ctcggcggta cggcggctgc gcacggccgg tgcggaggtg
541 gtggtcggca cctgtccgga cctgggcacg atcgagcggg tgcggcagcc gctgcgctgg
601 ctggcccggc gggcctcacg gcagctcgcg gcggcacaga ccatcggcgc cgtcgagcag
661 ggcgggcgca cggtgtcgct gggcgacctg ctgggtccgg agttcgcgca gaacccgcgg
721 gagctcttcg gccccgacaa ctaccacccc tccgccgagg ggtacgccac ggccgcgatg
781 gcggtactgc cctcggtgtg cgccgcgctc ggcctgtggc cggccgacga ggagcacccg
841 gacgcgctgc gccgcgaggg cttcctgccg gtggcgcgcg cggcggcgga ggcggcgtcc
901 gaggcgggta cggaggtcgc cgccgccatg cctacggggc ctcgggggcc ctgggcgctg
961 ctgaagcgcc ggagacggcg tcgggtgtcg gaggcggaac cgtccagccc gtccggcgtt
1021 tga

## Figure 24 (SEQ ID No. 28)

```
  1 mgrgtdqrtr ygrrrarval aaltaavlgv gvagcdsvgg dspapsgsps krtrtapawd
 61 tspasvaavg dsitrgfdac avlsdcpevs watgssakvd slavrllgka daaehswnya
121 vtgarmadlt aqvtraaqre pelvavmaga ndacrsttsa mtpvadfraq feeamatlrk
181 klpkaqvyvs sipdlkrlws qgrtnplgkq vwklglcpsm lgdadsldsa atlrrntvrd
241 rvadynevlr evcakdrrcr sddgavhefr fgtdqlshwd wfhpsvdgqa rlaeiayrav
301 taknp
```

## Figure 25 (SEQ ID No. 29)

```
  1 atgggtcgag ggacggacca gcggacgcgg tacggccgtc gccgggcgcg tgtcgcgctc
 61 gccgccctga ccgccgccgt cctgggcgtg ggcgtggcgg gctgcgactc cgtgggcggc
121 gactcacccg ctccttccgg cagcccgtcg aagcggacga ggacggcgcc cgcctgggac
181 accagcccgg cgtccgtcgc cgccgtgggc gactccatca cgcgcggctt cgacgcctgt
241 gcggtgctgt cggactgccc ggaggtgtcg tgggcgaccg gcagcagcgc gaaggtcgac
301 tcgctggccg tacggctgct ggggaaggcg gacgcggccg agcacagctg gaactacgcg
361 gtcaccgggg cccggatggc ggacctgacc gctcaggtga cgcgggcggc gcagcgcgag
421 ccggagctgg tggcggtgat ggccggggcg aacgacgcgt gccggtccac gacctcggcg
481 atgacgccgg tggcggactt ccgggcgcag ttcgaggagg cgatggccac cctgcgcaag
541 aagctcccca aggcgcaggt gtacgtgtcg agcatcccgg acctcaagcg gctctggtcc
601 cagggccgca ccaacccgct gggcaagcag gtgtggaagc tcggcctgtg cccgtcgatg
661 ctgggcgacg cggactccct ggactcggcg gcgaccctgc ggcgcaacac ggtgcgcgac
721 cgggtggcgg actacaacga ggtgctgcgg gaggtctgcg cgaaggaccg gcggtgccgc
781 agcgacgacg gcgcggtgca cgagttccgg ttcggcacgg accagttgag ccactgggac
841 tggttccacc cgagtgtgga cggccaggcc cggctggcgg agatcgccta ccgcgcggtc
901 accgcgaaga atccctga
```

## Figure 26 (SEQ ID No. 30)

```
  1 mrlsrraata sallltpala lfgasaavsa priqatdyva lgdsyssgvg agsydsssgs
 61 ckrstksypa lwaashtgtr fnftacsgar tgdvlakqlt pvnsgtdlvs itiggndagf
121 adtmttcnlq gesaclaria karayiqqtl paqldqvyda idsrapaaqv vvlgyprfyk
181 lggscavgls eksraainaa addinavtak raadhgfafg dvnttfaghe lcsgapwlhs
241 vtlpvensyh ptangqskgy lpvlnsat
```

## Figure 27 (SEQ ID No. 31)

```
   1 ttcatcacaa cgatgtcaca acaccggcca tccgggtcat ccctgatcgt gggaatgggt
  61 gacaagcctt cccgtgacga aagggtcctg ctacatcaga aatgacagaa atcctgctca
 121 gggaggttcc atgagactgt cccgacgcgc ggccacggcg tccgcgctcc tcctcacccc
 181 ggcgctcgcg ctcttcggcg cgagcgccgc cgtgtccgcg ccgcgaatcc aggccaccga
 241 ctacgtggcc ctcggcgact cctactcctc gggggtcggc gcgggcagct acgacagcag
 301 cagtggctcc tgtaagcgca gcaccaagtc ctacccggcc ctgtgggccg cctcgcacac
 361 cggtacgcgg ttcaacttca ccgcctgttc gggcgcccgc acaggagacg tgctggccaa
 421 gcagctgacc ccggtcaact ccggcaccga cctggtcagc attaccatcg gcggcaacga
 481 cgcgggcttc gccgacacca tgaccacctg caacctccag ggcgagagcg cgtgcctggc
 541 gcggatcgcc aaggcgcgcg cctacatcca gcagacgctg cccgcccagc tggaccaggt
 601 ctacgacgcc atcgacagcc gggcccccgc agcccaggtc gtcgtcctgg gctacccgcg
 661 cttctacaag ctgggcggca gctgcgccgt cggtctctcg gagaagtccc gcgcggccat
 721 caacgccgcc gccgacgaca tcaacgccgt caccgccaag cgcgccgccg accacggctt
 781 cgccttcggg gacgtcaaca cgaccttcgc cgggcacgag ctgtgctccg gcgcccccctg
 841 gctgcacagc gtcacccttc ccgtggagaa ctcctaccac cccacggcca acggacagtc
 901 caagggctac ctgcccgtcc tgaactccgc cacctgatct cgcggctact ccgcccctga
 961 cgaagtcccg cccccgggcg gggcttcgcc gtaggtgcgc gtaccgccgt cgcccgtcgc
1021 gccggtggcc ccgccgtacg tgccgccgcc cccggacgcg gtcggttc
```

Figure 28 (SEQ ID No. 32)

```
1   MKKWFVCLLG LVALTVQAAD SRPAFSRIVM FGDSLSDTGK MYSKMRGYLP
51  SSPPYYEGRF SNGPVWLEQL TKQFPGLTIA NEAEGGATAV AYNKISWNPK
101 YQVINNLDYE VTQFLQKDSF KPDDLVILWV GANDYLAYGW NTEQDAKRVR
151 DAISDAANRM VLNGAKQILL FNLPDLGQNP SARSQKVVEA VSHVSAYHNQ
201 LLLNLARQLA PTGMVKLFEI DKQFAEMLRD PQNFGLSDVE NPCYDGGYVW
251 KPFATRSVST DRQLSAFSPQ ERLAIAGNPL LAQAVASPMA RRSASPLNCE
301 GKMFWDQVHP TTVVHAALSE RAATFIANQY EFLAH*
```

# Figure 29 (SEQ ID No. 33)

```
   1  ATGAAAAAAT GGTTTGTGTG TTTATTGGGA TTGGTCGCGC TGACAGTTCA
      TACTTTTTTA CCAAACACAC AAATAACCCT AACCAGCGCG ACTGTCAAGT

  51  GGCAGCCGAC AGTCGCCCCG CCTTTTCCCG GATCGTGATG TTCGGCGACA
      CCGTCGGCTG TCAGCGGGGC GGAAAAGGGC CTAGCACTAC AAGCCGCTGT

 101  GCCTCTCCGA TACCGGCAAA ATGTACAGCA AGATGCGCGG TTACCTCCCC
      CGGAGAGGCT ATGGCCGTTT TACATGTCGT TCTACGCGCC AATGGAGGGG

 151  TCCAGCCCGC CCTACTATGA GGGCCGTTTC TCCAACGGAC CCGTCTGGCT
      AGGTCGGGCG GGATGATACT CCCGGCAAAG AGGTTGCCTG GGCAGACCGA

 201  GGAGCAGCTG ACCAAACAGT TCCCGGGTCT GACCATCGCC AACGAAGCGG
      CCTCGTCGAC TGGTTTGTCA AGGGCCCAGA CTGGTAGCGG TTGCTTCGCC

 251  AAGGCGGTGC CACTGCCGTG GCTTACAACA AGATCTCCTG GAATCCCAAG
      TTCCGCCACG GTGACGGCAC CGAATGTTGT TCTAGAGGAC CTTAGGGTTC

 301  TATCAGGTCA TCAACAACCT GGACTACGAG GTCACCCAGT TCTTGCAGAA
      ATAGTCCAGT AGTTGTTGGA CCTGATGCTC CAGTGGGTCA AGAACGTCTT

 351  AGACAGCTTC AAGCCGGACG ATCTGGTGAT CCTCTGGGTC GGTGCCAATG
      TCTGTCGAAG TTCGGCCTGC TAGACCACTA GGAGACCCAG CCACGGTTAC

 401  ACTATCTGGC CTATGGCTGG AACACGGAGC AGGATGCCAA GCGGGTTCGC
      TGATAGACCG GATACCGACC TTGTGCCTCG TCCTACGGTT CGCCCAAGCG

 451  GATGCCATCA GCGATGCGGC CAACCGCATG GTACTGAACG GTGCCAAGCA
      CTACGGTAGT CGCTACGCCG GTTGGCGTAC CATGACTTGC CACGGTTCGT

 501  GATACTGCTG TTCAACCTGC CGGATCTGGG CCAGAACCCG TCAGCTCGCA
      CTATGACGAC AAGTTGGACG GCCTAGACCC GGTCTTGGGC AGTCGAGCGT

 551  GTCAGAAGGT GGTCGAGGCG GTCAGCCATG TCTCCGCCTA TCACAACCAG
      CAGTCTTCCA CCAGCTCCGC CAGTCGGTAC AGAGGCGGAT AGTGTTGGTC

 601  CTGCTGCTGA ACCTGGCACG CCAGCTGGCC CCCACCGGCA TGGTAAAGCT
      GACGACGACT TGGACCGTGC GGTCGACCGG GGGTGGCCGT ACCATTTCGA

 651  GTTCGAGATC GACAAGCAAT TTGCCGAGAT GCTGCGTGAT CCGCAGAACT
      CAAGCTCTAG CTGTTCGTTA AACGGCTCTA CGACGCACTA GGCGTCTTGA

 701  TCGGCCTGAG CGACGTCGAG AACCCCTGCT ACGACGGCGG CTATGTGTGG
      AGCCGGACTC GCTGCAGCTC TTGGGGACGA TGCTGCCGCC GATACACACC

 751  AAGCCGTTTG CCACCCGCAG CGTCAGCACC GACCGCCAGC TCTCCGCCTT
      TTCGGCAAAC GGTGGGCGTC GCAGTCGTGG CTGGCGGTCG AGAGGCGGAA

 801  CAGTCCGCAG GAACGCCTCG CCATCGCCGG CAACCCGCTG CTGGCACAGG
      GTCAGGCGTC CTTGCGGAGC GGTAGCGGCC GTTGGGCGAC GACCGTGTCC

 851  CCGTTGCCAG TCCTATGGCC CGCCGCAGCG CCAGCCCCCT CAACTGTGAG
      GGCAACGGTC AGGATACCGG GCGGCGTCGC GGTCGGGGGA GTTGACACTC

 901  GGCAAGATGT TCTGGGATCA GGTACACCCG ACCACTGTCG TGCACGCAGC
      CCGTTCTACA AGACCCTAGT CCATGTGGGC TGGTGACAGC ACGTGCGTCG

 951  CCTGAGCGAG CGCGCCGCCA CCTTCATCGC GAACCAGTAC GAGTTCCTCG
      GGACTCGCTC GCGCGGCGGT GGAAGTAGCG CTTGGTCATG CTCAAGGAGC

1001  CCCAC TGA
      GGGTG ACT
```

Figure 30 (SEQ ID No. 34)

```
  1  MKKWFVCLLG LIALTVQAAD TRPAFSRIVM FGDSLSDTGK MYSKMRGYLP
 51  SSPPYYEGRF SNGPVWLEQL TKQFPGLTIA NEAEGGATAV AYNKISWNPK
101  YQVINNLDYE VTQFLQKDSF KPDDLVILWV GANDYLAYGW NTEQDAKRVR
151  DAISDAANRM VLNGAKQILL FNLPDLGQNP SARSQKVVEA VSHVSAYHNK
201  LLLNLARQLA PTGMVKLFEI DKQFAEMLRD PQNFGLSDVE NPCYDGGYVW
251  KPFATRSVST DRQLSAFSPQ ERLAIAGNPL LAQAVASPMA RRSASPLNCE
301  GKMFWDQVHP TTVVHAALSE RAATFIETQY EFLAHG*
```

# Figure 31 (SEQ ID No. 35)

```
   1  ATGAAAAAAT GGTTTGTTTG TTTATTGGGG TTGATCGCGC TGACAGTTCA
      TACTTTTTTA CCAAACAAAC AAATAACCCC AACTAGCGCG ACTGTCAAGT

  51  GGCAGCCGAC ACTCGCCCCG CCTTCTCCCG GATCGTGATG TTCGGCGACA
      CCGTCGGCTG TGAGCGGGGC GGAAGAGGGC CTAGCACTAC AAGCCGCTGT

 101  GCCTCTCCGA TACCGGCAAA ATGTACAGCA AGATGCGCGG TTACCTCCCC
      CGGAGAGGCT ATGGCCGTTT TACATGTCGT TCTACGCGCC AATGGAGGGG

 151  TCCAGCCCGC CCTACTATGA GGGCCGTTTC TCCAACGGAC CCGTCTGGCT
      AGGTCGGGCG GGATGATACT CCCGGCAAAG AGGTTGCCTG GGCAGACCGA

 201  GGAGCAGCTG ACCAAGCAGT TCCCGGGTCT GACCATCGCC AACGAAGCGG
      CCTCGTCGAC TGGTTCGTCA AGGGCCCAGA CTGGTAGCGG TTGCTTCGCC

 251  AAGGCGGTGC CACTGCCGTG GCTTACAACA AGATCTCCTG GAATCCCAAG
      TTCCGCCACG GTGACGGCAC CGAATGTTGT TCTAGAGGAC CTTAGGGTTC

 301  TATCAGGTCA TCAACAACCT GGACTACGAG GTCACCCAGT TCTTGCAGAA
      ATAGTCCAGT AGTTGTTGGA CCTGATGCTC CAGTGGGTCA AGAACGTCTT

 351  AGACAGCTTC AAGCCGGACG ATCTGGTGAT CCTCTGGGTC GGTGCCAATG
      TCTGTCGAAG TTCGGCCTGC TAGACCACTA GGAGACCCAG CCACGGTTAC

 401  ACTATCTGGC ATATGGCTGG AATACGGAGC AGGATGCCAA GCGAGTTCGC
      TGATAGACCG TATACCGACC TTATGCCTCG TCCTACGGTT CGCTCAAGCG

 451  GATGCCATCA GCGATGCGGC CAACCGCATG GTACTGAACG GTGCCAAGCA
      CTACGGTAGT CGCTACGCCG GTTGGCGTAC CATGACTTGC CACGGTTCGT

 501  GATACTGCTG TTCAACCTGC CGGATCTGGG CCAGAACCCG TCAGCCCGCA
      CTATGACGAC AAGTTGGACG GCCTAGACCC GGTCTTGGGC AGTCGGGCGT

 551  GTCAGAAGGT GGTCGAGGCG GTCAGCCATG TCTCCGCCTA TCACAACAAG
      CAGTCTTCCA CCAGCTCCGC CAGTCGGTAC AGAGGCGGAT AGTGTTGTTC

 601  CTGCTGCTGA ACCTGGCACG CCAGCTGGCC CCCACCGGCA TGGTAAAGCT
      GACGACGACT TGGACCGTGC GGTCGACCGG GGGTGGCCGT ACCATTTCGA

 651  GTTCGAGATC GACAAGCAAT TTGCCGAGAT GCTGCGTGAT CCGCAGAACT
      CAAGCTCTAG CTGTTCGTTA AACGGCTCTA CGACGCACTA GGCGTCTTGA

 701  TCGGCCTGAG CGACGTCGAG AACCCCTGCT ACGACGGCGG CTATGTGTGG
      AGCCGGACTC GCTGCAGCTC TTGGGGACGA TGCTGCCGCC GATACACACC

 751  AAGCCGTTTG CCACCCGCAG CGTCAGCACC GACCGCCAGC TCTCCGCCTT
      TTCGGCAAAC GGTGGGCGTC GCAGTCGTGG CTGGCGGTCG AGAGGCGGAA

 801  CAGTCCGCAG GAACGCCTCG CCATCGCCGG CAACCCGCTG CTGGCACAGG
      GTCAGGCGTC CTTGCGGAGC GGTAGCGGCC GTTGGGCGAC GACCGTGTCC

 851  CCGTTGCCAG TCCTATGGCC CGCCGCAGCG CCAGCCCCCT CAACTGTGAG
      GGCAACGGTC AGGATACCGG GCGGCGTCGC GGTCGGGGGA GTTGACACTC

 901  GGCAAGATGT TCTGGGATCA GGTACACCCG ACCACTGTCG TGCACGCAGC
      CCGTTCTACA AGACCCTAGT CCATGTGGGC TGGTGACAGC ACGTGCGTCG

 951  CCTGAGCGAG CGCGCCGCCA CCTTCATCGA GACCCAGTAC GAGTTCCTCG
      GGACTCGCTC GCGCGGCGGT GGAAGTAGCT CTGGGTCATG CTCAAGGAGC

1001  CCCACGGATG A
      GGGTGCCTAC T
```

Figure 32

```
           1       10        20        30        40        50
           |--------+---------+---------+---------+---------|
     satA     ADTRPAFSRIVMFGDSLSDTGKMYSKMRGYLPSSPPYYEGRFSN--G
    R.sol  QSGNPNVAKVQRMVVFGDSLSDIGT-------YTPVAQAVGGGKFTTNPG
Consensus  ...adnraafqRiVmFGDSLSDiGk.......YlPsaqaygeGrFsn..G

           51      60        70        80        90        100
           |--------+---------+---------+---------+---------|
     satA  PVWLEQLTKQFPGLTIANEAEGGATAVAYNKISWNPKYQVINNLDYEVTQ
    R.sol  PIWAETVAAQL-GVTLTPAVMGYATSVQNCPKAGCFDYAQGGSRVTDPNG
Consensus  P!WaEqlaaQl.GlTianaaeGgATaVannkiagnfdYaqgnnrdt#pnq

           101     110       120       130       140       150
           |--------+---------+---------+---------+---------|
     satA  FLQKDSFKPDDLVILWVGANDYLAYG--WNTEQDAKRVRDAISDAANRMV
    R.sol  IGHNGGAGALTYPVQQQLANFYAASNNTFNGNNDVVFVLAGSNDIFFWTT
Consensus  igqndgagaddlp!qqqgANdYaAsn..fNg##DakrVraainDaanrmt

           151     160       170       180       190       200
           |--------+---------+---------+---------+---------|
     satA  LNGAKQILLFNLPDLGQNPSARSQKVVEAVSHVSAYHNKL-LLNLARQLA
    R.sol  AAATSGSGVTPAIATAQVQQAATDLVGYVKDMIAKGATQVYVFNLPDSSL
Consensus  aaaakqiglfnaialaQnqqAas#lVgeakdh!aaganql.llNLarqla

           201     210       220       230       240       250
           |--------+---------+---------+---------+---------|
     satA  PTGMVKLFEIDKQFAEMLRDPQNFGLSDVENPCYDGGYVWKPFATRSVST
    R.sol  TPDGVASGTTGQALLHALVGTFNTTLQSGLAGTSARIIDFNAQLTAAIQN
Consensus  ppdgValgeidqalaeaLrdpqNfgLqdgeagcsargidfnaqaTaa!qn

           251     260       270       280       290       300
           |--------+---------+---------+---------+---------|
     satA  DRQLSAFSPQERLAIAG--NPLLAQAVASPM---ARRSASPLNCEGKMFW
    R.sol  GASFGFANTSARACDATKINALVPSAGGSSLFCSANTLVASGADQSYLFA
Consensus  daqlgaanpqaRaadAg..NaLlaqAgaSp$...Arrlaapgad#gk$Fa

           301     310       320       330
           |--------+---------+---------|
     satA  DQVHPTTVVHAALSERAATFIETQYEFLAH
    R.sol  DGVHPTTAGHRLIASNVLARLLA--DNVAH
Consensus  DqVHPTTagHaaiaeraaariea..#nlAH
```

Figure 33

```
                        ▼
Pfam       *->ivafGDSltdggg..............ayygdsdgggwgagladrltsla..rlrargrgvdv
Sriml  38     YVALGDSYSSGVG............agSYDSSSGSCKRSTKSYPALWAAS..-----HTGTRF 81
Scoe1   5     YVAVGDSFTEG--..............--VGDPGPDGAFVGWADRLAVLL..ADRRPEGDFTY 47
Scoe2  10     LVAVGDSFTEG--..............--MSDLLPDGSYRGWADLLATRM..--AARSPGFRY 50
Scoe3 239     VVAFGDSITDG--..............ARSQSDANHRWTDVLAARLHEAA..GDGRDTPRYSV 283
Scoe4  75     LMMLGDSTAAG--..............------QGVHRAGQTPGALLASG..LAAVAERPVRL 113
Scoe5  66     VAAVGDSITRGFD............acAVLSDCPEVSWATGSSAKVDSLAvrLLGKADAAEHS 116
Ahyd1  28     IVMFGDSLSDTGKmyskmrgylpssppyYEGRFSNGPVWLEQLTNEFPGLTiaNEAEGGPTAVA 91
Asal1  28     IVMFGDSLSDTGKmyskmrgylpssppyYEGRFSNGPVWLEQLTKQF----.------PGLTI 79
Ahyd2  40     IVMFGDSLSDTGKmyskmrgylpssppyYEGRFSNGPVWLEQLTKQFPGLTiaNEAEGGATAVA 103

Pfam       fnrgisGrtsdGrlvvDarlvatllFlaqflGlnlpPYLsgdflrGANFAsagAtIlgtslipflni
Sriml  82 NFTACSGAR---------------------------------------------------------- 90
Scoe1  48 TNLAVRGRL---------------------------------------------------------- 56
Scoe2  51 ANLAVRGKL---------------------------------------------------------- 59
Scoe3 284 VNEGISGNR---------------------------------------------------------- 292
Scoe4 114 GSVAQPGAC---------------------------------------------------------- 122
Scoe5 117 WNYAVTGAR---------------------------------------------------------- 125
Ahyd1  92 YNKISWNPK----------------------------------------------------------- 100
Asal1  80 ANEAEGGAT---------------------------------------------------------- 88
Ahyd2 104 YNKISWNPK---------------------------------------------------------- 112

                                                               ▼
Pfam       QvqFkdfkskvlelrqa.....lgllqellrlvpvldakspdlvtimiGtNDl...itvakfgpks
Sriml  91 -----------------.....---TGDVLAKQLTPVNSGTDLVSITIGGNDAgfaDTMTTCNLQG 131
Scoe1  57 -----------------.....--LDQIVAEQVPRVVGLAPDLVSFAAGGNDI...------I---- 86
Scoe2  60 -----------------.....--IGQIVDEQVDVAAAMGADVITLVGGLNDT...----------- 88
Scoe3 293 -------LLTSRPGRPA.....DNPSGLSRFQRDVLERTNVKAVVVVLGVNDV...----------- 333
Scoe4 123 -----------------.....SDDLDRQVALVLAEPDRVPDICVIMVGANDV...----------- 153
Scoe5 126 -----------------.....---MADLTAQVTRAAQREPELVAVMAGANDA...---------CR 155
Ahyd1 101 -------------YQVI.....NNLDYEVTQFLQKDSFKPDDLVILWVGANDY...---------LA 137
Asal1   89 -------AVAYNKISWNpkyqvyNNLDYEVTQFLQKDSFKPDDLVILWVGANDY...---------LA 137
Ahyd2 113 -------------YQVI.....NNLDYEVTQFLQKDSFKPDDLVILWVGANDY...---------LA 149

Pfam       ......tksdrnvsvpefrdnlrklikrLrsangariiilitlvllnlpl..........plGCl
Sriml 132 esaclarIAKARAYIQQTLPAQLDQVYDAIDSRAPAA-----QVVVLGYP-..........----- 176
Scoe1  87 ......---RPGTDPDEVAERFELAVAALT-AAAGTVLVTTGFDTRGVP-..........----- 125
Scoe2  89 ......---------LRPKCDMARVRDLLTQAVERLAPHCEQLVLMRSP-..........----- 122
Scoe3 334 ......LNSPELADRDAILTGLRTLVDRAHARGLRVVGATITPFGGYGG-..........----- 376
Scoe4 154 ......---THRMPATRSVRHLSSAVRRLR-TAGAEVVVGTCPDLGTIE-..........----- 192
Scoe5 156 ......STTSAMTPVADFRAQFEEAMATLR-KKLPKAQVYVSSIPDLKRLwsqgrtnplgkQVWKL 214
Ahyd1 138 ......YGWNTEQDAKRVRDAISDAANRMV-LNGAK-----EILLFNLP-..........----- 174
Asal1 138 ......YGWNTEQDAKRVRDAISDAANRMV-LNGAK-----QILLFNLP-..........----- 174
Ahyd2 150 ......YGWNTEQDAKRVRDAISDAANRMV-LNGAK-----QILLFNLP-..........----- 186

Pfam       pq.klalalassknvdatgclerlneavadynealrelaei.ek.l.q.aqlrkdglpdlkeanvpy
Sriml 177 --.RFYKLGGSCAVGLSEKSRAAINAAADDINAVTAKRA--.--.-.-.----------ADHGFAF 219
Scoe1 126 --.---------------VLKHLRGKIATYNGHVRAIA--.--.-.-.----------DRYGCPV 152
Scoe2 123 --.-----------GRQGPVLERFRPRMEALFAVIDDLA--.--.-.-.----------GRHGAVV 154
Scoe3 377 --.YTEARETMRQEVNEEIRSGRVFDTVVDFDKALRDPY--.--.-.-.----------------- 412
Scoe4 193 --.-----------------------------RVRQPLRWLaRRaSrQlAAAQTIGAVEQGGRTVSL 227
Scoe5 215 GLcPSMLGDADSLDSAATLRRNTVRDRVADYNEVLREVC--.--.-.AkDRRCRSDDGAVHEFRFGT 273
Ahyd1 175 --.----DLGQNPSARSQKVVEAASHVSAYHNQLLLNLA--.--.-.-.RQLAPTGMVKLFEIDKQF 224
Asal1 175 --.----DLGQNPSARSQKVVEAVSHVSAYHNKLLLNLA--.--.-.-.RQLAPTGMVKLFEIDKQF 224
Ahyd2 187 --.----DLGQNPSARSQKVVEAVSHVSAYHNQLLLNLA--.--.-.-.RQLAPTGMVKLFEIDKQF 236

Pfam       VDlysifqdldgiqnpsayv.y....GFeet.kaCCGyGgr.yNyn.rv.CGnag.l.ck.vtakaC
Sriml 220 GDVNT---------------.-....-----.----------.-TFAgHElCSGAPwL.HS.VT---- 242
Scoe1 153 LDLWSLRSVQDRRA------.-....-----.----------.----.--.-----.-.--.------ 166
Scoe2 155 VDLYGAQSLADPRM------.-....-----.----------.----.--.-----.-.--.------ 168
Scoe3 413 --------------------.-....-----.----------.----.--.-----.-.--.------ 413
Scoe4 228 GDLLGPEFAQNPREL-----.-....-----.----------.----.--.-----.-.--.------ 242
```

```
Scoe5   274 DQL-----------------.-....-----.---------.----.--.-----.-.--.------ 276
Ahyd1 225 AEMLRDPQNFGLSDQRNACYgGsyvwKPFASrSASTDSQLSaFNPQeRLaIAGNPlLaQAvASPMAA 291
Asal1 225 AEMLRDPQNFGLSDVENPCYdGgyvwKPFATrSVSTDRQLSaFSPQeRLaIAGNPlLaQAvASPMAR 291
Ahyd2 237 AEMLRDPQNFGLSDVENPCYdGgyvwKPFATrSVSTDRQLSaFSPQeRLaIAGNPlLaQAvASPMAR 303

                      ▼
Pfam      .dassyll.atlfwDgf.HpsekGykavAeal<-*
Sriml 243 .-------.--LPVENSyHPTANGQSKGYLPV     263
Scoe1 167 .-------.--WDADRL.HLSPEGHTRVALRA     186
Scoe2 169 .-------.--WDVDRL.HLTAEGHRRVAEAV     188
Scoe3 413 .-DPRRMRsDYDSGDHL.HPGDKGYARMGAVI     441
Scoe4 243 .-------.--FGPDNY.HPSAEGYATAAMAV     262
Scoe5 277 .-------.--SHWDWF.HPSVDGQARLAEIA     296
Ahyd1 292 rSASTLNCeGKMFWDQV.HPTTVVHAALSEPA     322
Asal1 292 rSASPLNCeGKMFWDQV.HPTTVVHAALSERA     322
Ahyd2 304 rSASPLNCeGKMFWDQV.HPTTVVHAALSERA     334
```

Figure 34

```
                         ▼
Pfam        *->ivafGDSltdggg...............ayygdsdgggwgagladrltsla..rlrargrgvdv
Sriml  38      YVALGDSYSSGVG.............agSYDSSSGSCKRSTKSYPALWAAS..-----HTGTRF 81
Scoel   5      YVAVGDSFTEG--...............--VGDPGPDGAFVGWADRLAVLL..ADRRPEGDFTY 47
Scoe2  10      LVAVGDSFTEG--...............--MSDLLPDGSYRGWADLLATRM..--AARSPGFRY 50
Ahyd1  28      IVMFGDSLSDTGKmyskmrgylpssppyYEGRFSNGPVWLEQLTNEFPGLTiaNEAEGGPTAVA 91
Asal1  28      IVMFGDSLSDTGKmyskmrgylpssppyYEGRFSNGPVWLEQLTKQF----..------PGLTI 79
Ahyd2  40      IVMFGDSLSDTGKmyskmrgylpssppyYEGRFSNGPVWLEQLTKQFPGLTiaNEAEGGATAVA 103

Pfam        fnrgisGrtsdGrlvvDarlvatllFlaqflGlnlpPYLsgdflrGANFAsagAtIlgtslipflni
Sriml  82 NFTACSGAR----------------------------------------------------------- 90
Scoel  48 TNLAVRGRL----------------------------------------------------------- 56
Scoe2  51 ANLAVRGKL----------------------------------------------------------- 59
Ahyd1   92 YNKISWNPK----------------------------------------------------------- 100
Asal1   80 ANEAEGGAT----------------------------------------------------------- 88
Ahyd2 104 YNKISWNPK----------------------------------------------------------- 112

                                                                    ▼
Pfam        QvqFkdfkskvlelrqa......lgllqellrlvpvldakspdlvtimiGtNDl...itvakfgpks
Sriml  91 -----------------......---TGDVLAKQLTPVNSGTDLVSITIGGNDAgfaDTMTTCNLQG.131
Scoel  57 -----------------......--LDQIVAEQVPRVVGLAPDLVSFAAGGNDI...-----I---- 86
Scoe2  60 -----------------......--IGQIVDEQVDVAAAMGADVITLVGGLNDT...---------- 88
Ahyd1 101 -------------YQVI.....NNLDYEVTQFLQKDSFKPDDLVILWVGANDY...---------LA 137
Asal1   89 -------AVAYNKISWNpkyqvyNNLDYEVTQFLQKDSFKPDDLVILWVGANDY...---------LA 137
Ahyd2 113 -------------YQVI.....NNLDYEVTQFLQKDSFKPDDLVILWVGANDY...---------LA 149

Pfam        .......tksdrnvsvpefrdnlrklikrLrsangariiilitlvllnlplplGCl
Sriml 132 esaclarIAKARAYIQQTLPAQLDQVYDAIDSRAPAA-----QVVVLGYP------ 176
Scoel  87 .......---RPGTDPDEVAERFELAVAALT-AAAGTVLVTTGFDTRGVP------ 125
Scoe2  89 .......---------LRPKCDMARVRDLLTQAVERLAPHCEQLVLMRSP------ 122
Ahyd1 138 .......YGWNTEQDAKRVRDAISDAANRMV-LNGAK-----EILLFNLP------ 174
Asal1 138 .......YGWNTEQDAKRVRDAISDAANRMV-LNGAK-----QILLFNLP------ 174
Ahyd2 150 .......YGWNTEQDAKRVRDAISDAANRMV-LNGAK-----QILLFNLP------ 186

Pfam        pqklalalassknvdatgclerlneavadynealrelaeieklqaqlrkdglpdlkeanvpy
Sriml 177 --RFYKLGGSCAVGLSEKSRAAINAAADDINAVTAKRA-----------------ADHGFAF 219
Scoel 126 -----------------VLKHLRGKIATYNGHVRAIA-----------------DRYGCPV 152
Scoe2 123 -------------GRQGPVLERFRPRMEALFAVIDDLA-----------------GRHGAVV 154
Ahyd1 175 ------DLGQNPSARSQKVVEAASHVSAYHNQLLLNLA------RQLAPTGMVKLFEIDKQF 224
Asal1 175 ------DLGQNPSARSQKVVEAVSHVSAYHNKLLLNLA------RQLAPTGMVKLFEIDKQF 224
Ahyd2 187 ------DLGQNPSARSQKVVEAVSHVSAYHNQLLLNLA------RQLAPTGMVKLFEIDKQF 236

Pfam        VDlysifqdldgiqnpsayv.y....GFeet.kaCCGyGgr.yNyn.rv.CGnag.l.ck.vtakaC
Sriml 220 GDVNT---------------.-...-----.----------.-TFAgHElCSGAPwL.HS.VT---- 242
Scoel 153 LDLWSLRSVQDRRA------.-...-----.----------.----.--.-----.-.--.------ 166
Scoe2 155 VDLYGAQSLADPRM------.-...-----.----------.----.--.-----.-.--.------ 168
Ahyd1 225 AEMLRDPQNFGLSDQRNACYgGsyvwKPFASrSASTDSQLSaFNPQeRLaIAGNPlLaQAvASPMAA 291
Asal1 225 AEMLRDPQNFGLSDVENPCYdGgyvwKPFATrSVSTDRQLSaFSPQeRLaIAGNPlLaQAvASPMAR 291
Ahyd2 237 AEMLRDPQNFGLSDVENPCYdGgyvwKPFATrSVSTDRQLSaFSPQeRLaIAGNPlLaQAvASPMAR 303

                         ▼
Pfam        .dassyll.atlfwDgf.HpsekGykavAeal<-*
Sriml 243 .-------.--LPVENSyHPTANGQSKGYLPV    263
Scoel 167 .-------.--WDADRL.HLSPEGHTRVALRA    186
Scoe2 169 .-------.--WDVDRL.HLTAEGHRRVAEAV    188
Ahyd1 292 rSASTLNCeGKMFWDQV.HPTTVVHAALSEPA    322
Asal1 292 rSASPLNCeGKMFWDQV.HPTTVVHAALSERA    322
Ahyd2 304 rSASPLNCeGKMFWDQV.HPTTVVHAALSERA    334
```

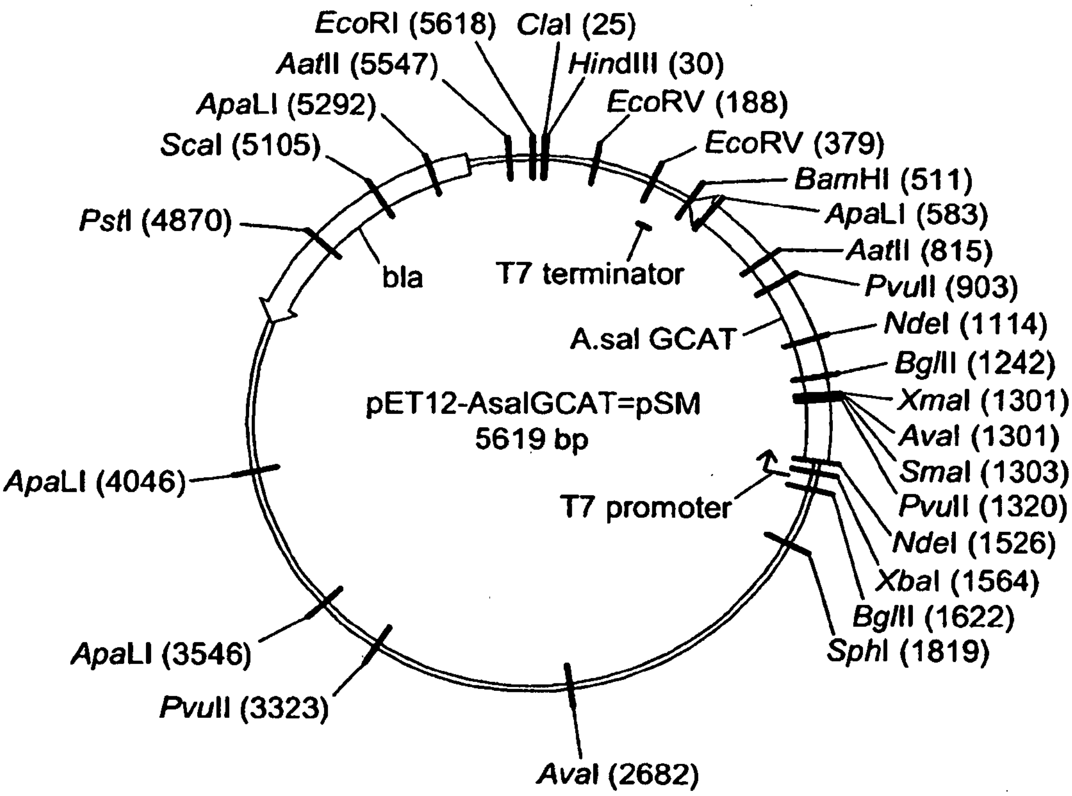
EcoRI (5618)
ClaI (25)
HindIII (30)
AatII (5547)
EcoRV (188)
ApaLI (5292)
EcoRV (379)
ScaI (5105)
BamHI (511)
ApaLI (583)
PstI (4870)
AatII (815)
bla
T7 terminator
PvuII (903)
A.sal GCAT
NdeI (1114)
BglII (1242)
pET12-AsalGCAT=pSM
5619 bp
XmaI (1301)
AvaI (1301)
SmaI (1303)
ApaLI (4046)
PvuII (1320)
T7 promoter
NdeI (1526)
XbaI (1564)
BglII (1622)
ApaLI (3546)
SphI (1819)
PvuII (3323)
AvaI (2682)

Figure 35

Figure 36

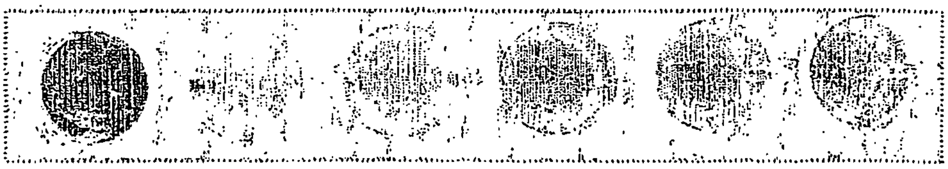

Figure 37

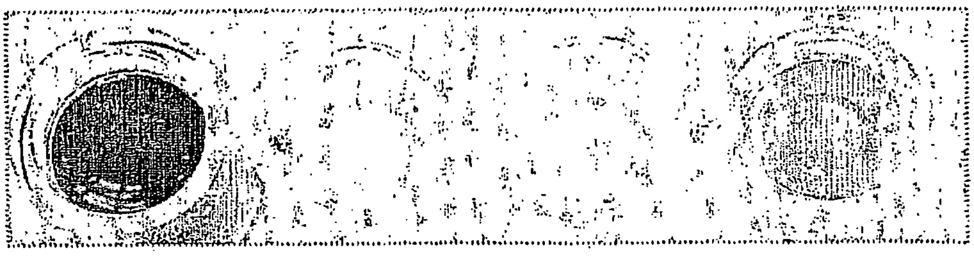

Figure 38

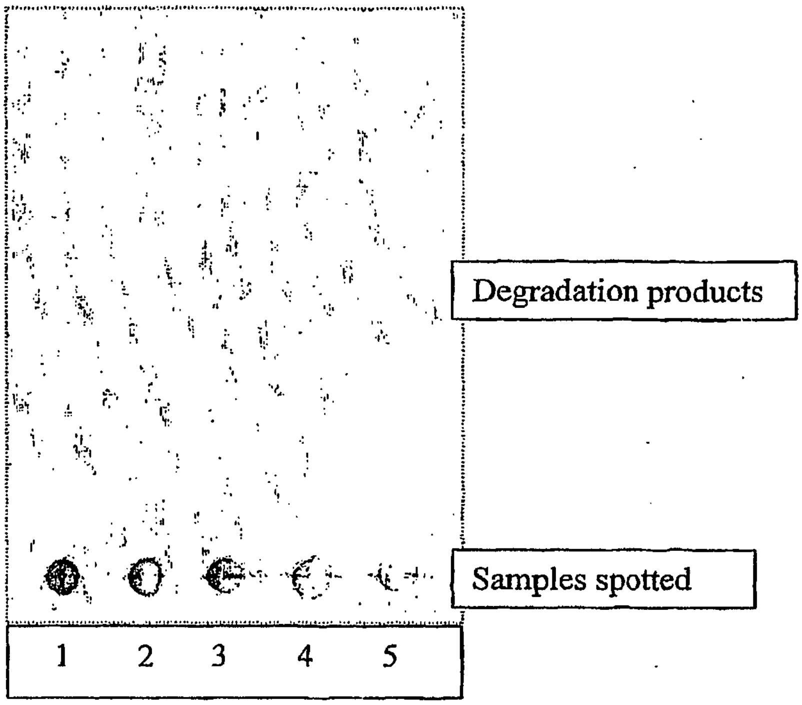

Figure 39

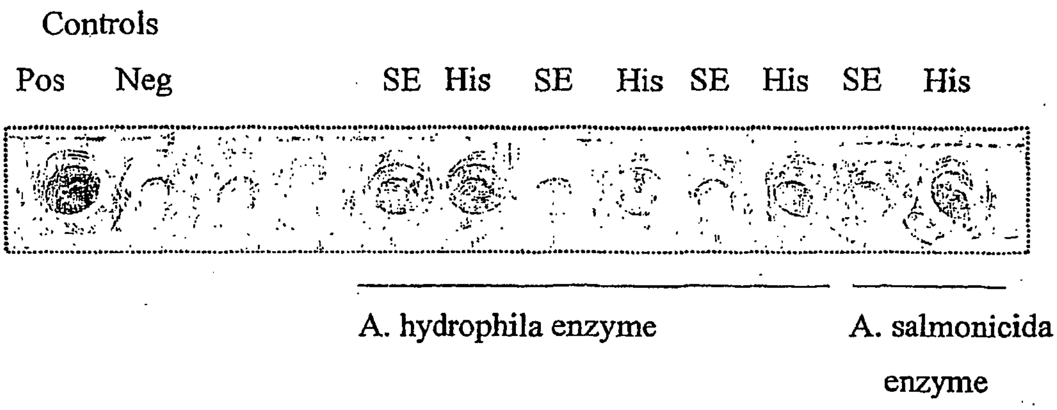
Controls
Pos
Neg
SE
His
SE
His
SE
His
SE
His
A. hydrophila enzyme
A. salmonicida enzyme

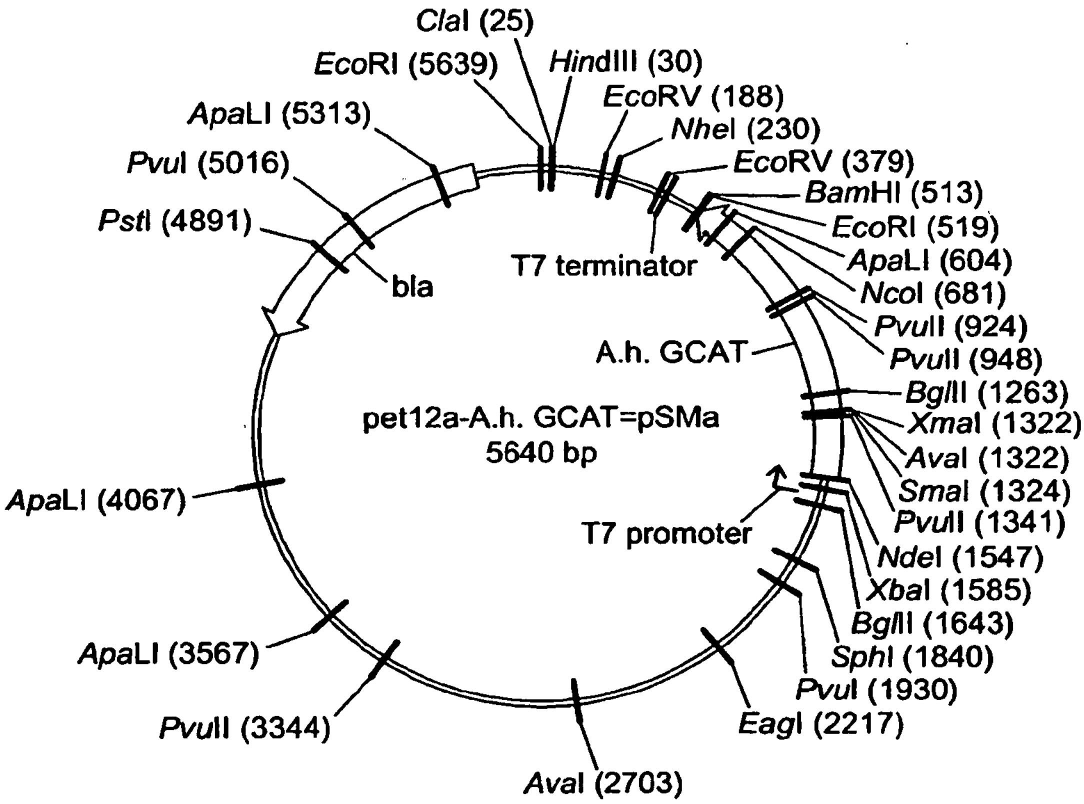
ClaI (25)
EcoRI (5639)
HindIII (30)
EcoRV (188)
NheI (230)
EcoRV (379)
BamHI (513)
EcoRI (519)
ApaLI (604)
NcoI (681)
PvuII (924)
PvuII (948)
BglII (1263)
XmaI (1322)
AvaI (1322)
SmaI (1324)
PvuII (1341)
NdeI (1547)
XbaI (1585)
BglII (1643)
SphI (1840)
PvuI (1930)
EagI (2217)
AvaI (2703)
PvuII (3344)
ApaLI (3567)
ApaLI (4067)
PstI (4891)
PvuI (5016)
ApaLI (5313)
bla
T7 terminator
A.h. GCAT
pet12a-A.h. GCAT=pSMa
5640 bp
T7 promoter

Figure 40

Figure 41:

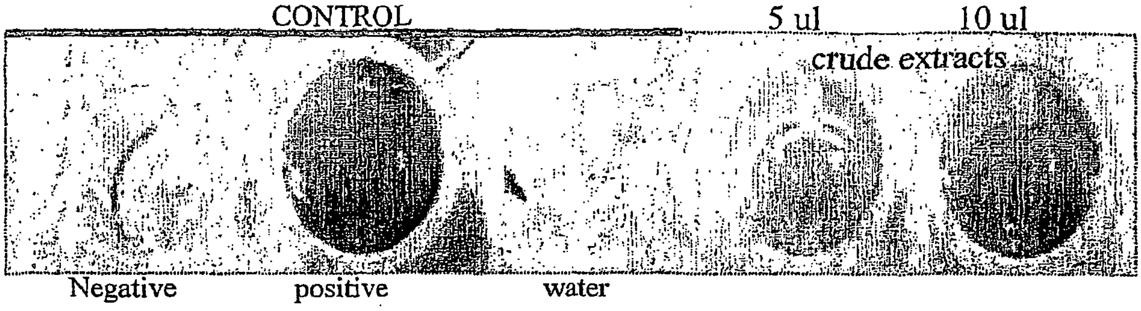

Figure 42

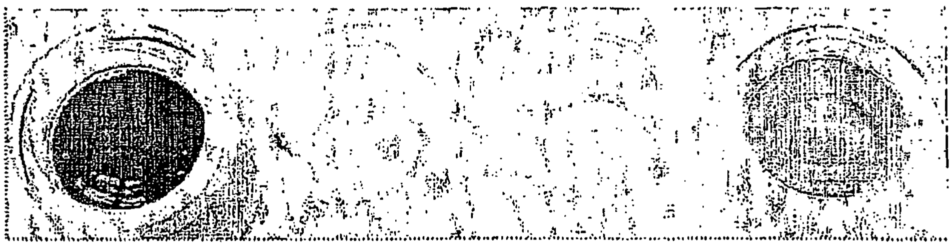

Figure 43

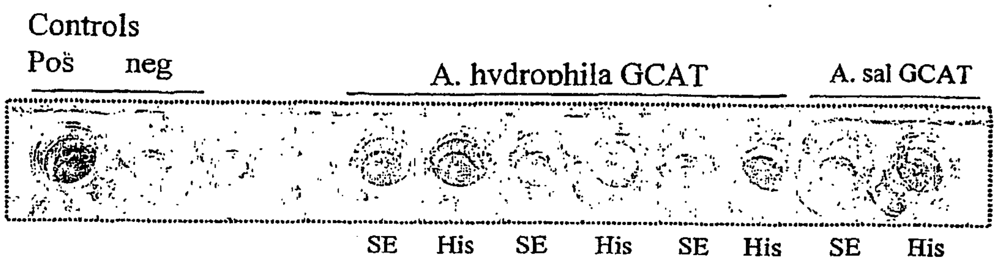
Controls
Pos neg
A. hydrophila GCAT
A. sal GCAT
SE His SE His SE His SE His

Figure 44

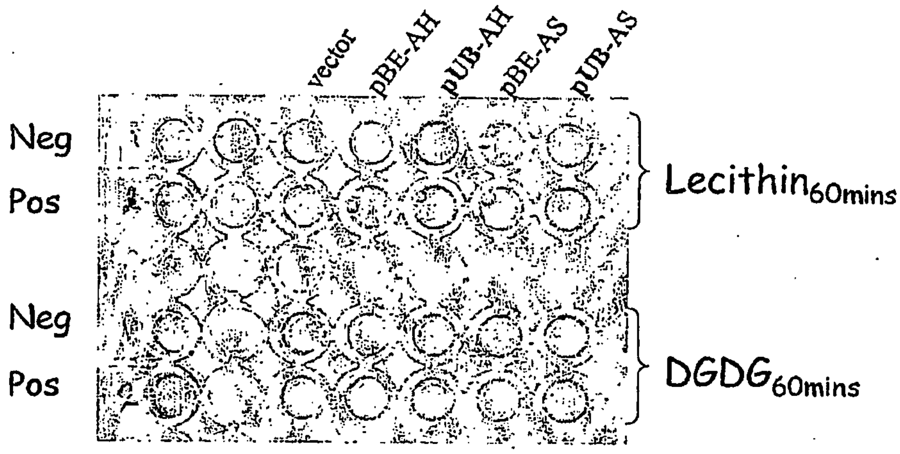
vector
pBE-AH
pUB-AH
pBE-AS
pUB-AS
Neg
Pos
Neg
Pos
Lecithin60mins
DGDG60mins

Figure 45

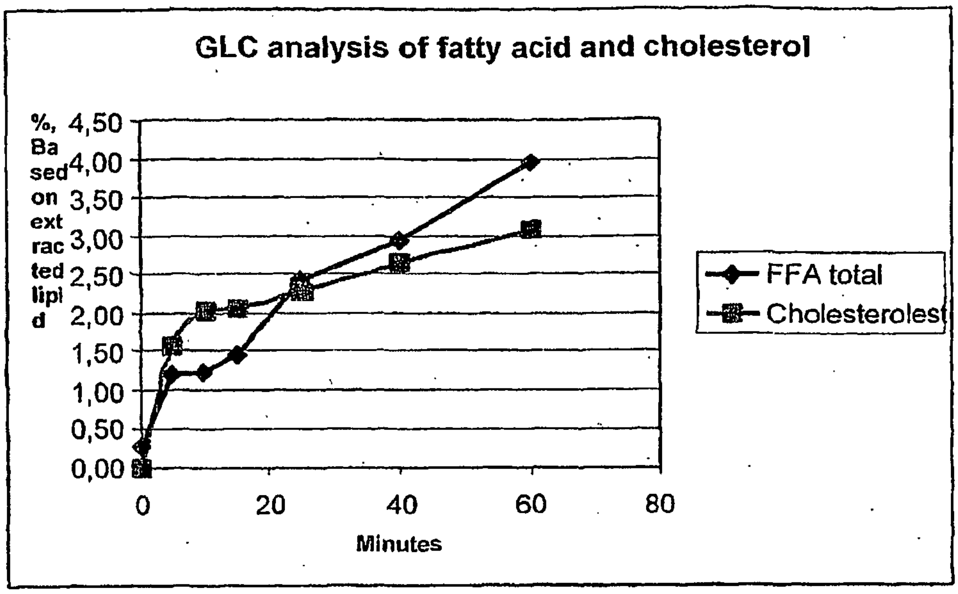
GLC analysis of fatty acid and cholesterol
%, Based on extracted lipid
4,50
4,00
3,50
3,00
2,50
2,00
1,50
1,00
0,50
0,00
0
20
40
60
80
Minutes
FFA total
Cholesterolest

Figure 46

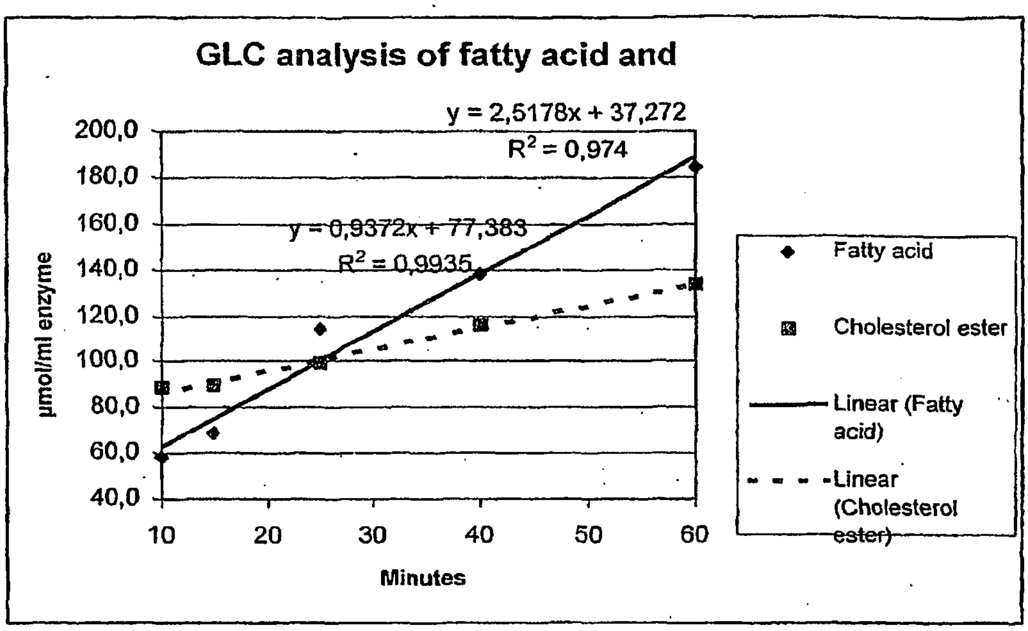
GLC analysis of fatty acid and
y = 2,5178x + 37,272
$R^2$ = 0,974
y = 0,9372x + 77,383
$R^2$ = 0,9935
200,0
180,0
160,0
140,0
120,0
100,0
80,0
60,0
40,0
µmol/ml enzyme
10
20
30
40
50
60
Minutes
Fatty acid
Cholesterol ester
Linear (Fatty acid)
Linear (Cholesterol ester)

Figure 47

(SEQ ID No. 36)

```
  1  MFKFKKNFLV GLSAALMSIS LFSATASAAS ADSRPAFSRI VMFGDSLSDT
 51  GKMYSKMRGY LPSSPPYYEG RFSNGPVWLE QLTKQFPGLT IANEAEGGAT
101  AVAYNKISWN PKYQVINNLD YEVTQFLQKD SFKPDDLVIL WVGANDYLAY
151  GWNTEQDAKR VRDAISDAAN RMVLNGAKQI LLFNLPDLGQ NPSARSQKVV
201  EAVSHVSAYH NQLLLNLARQ LAPTGMVKLF EIDKQFAEML RDPQNFGLSD
251  VENPCYDGGY VWKPFATRSV STDRQLSAFS PQERLAIAGN PLLAQAVASP
301  MARRSASPLN CEGKMFWDQV HPTTVVHAAL SERAATFIAN QYEFLAH**
```

Figure 48 (SEQ ID No. 54)

```
1    ATGTTTAAGT TTAAAAAGAA TTTCTTAGTT GGATTATCGG CAGCTTTAAT
     TACAAATTCA AATTTTTCTT AAAGAATCAA CCTAATAGCC GTCGAAATTA

51   GAGTATTAGC TTGTTTTCGG CAACCGCCTC TGCAGCTAGC GCCGACAGCC
     CTCATAATCG AACAAAAGCC GTTGGCGGAG ACGTCGATCG CGGCTGTCGG

101  GTCCCGCCTT TTCCCGGATC GTGATGTTCG GCGACAGCCT CTCCGATACC
     CAGGGCGGAA AAGGGCCTAG CACTACAAGC CGCTGTCGGA GAGGCTATGG

151  GGCAAAATGT ACAGCAAGAT GCGCGGTTAC CTCCCCTCCA GCCCGCCCTA
     CCGTTTTACA TGTCGTTCTA CGCGCCAATG GAGGGGAGGT CGGGCGGGAT

201  CTATGAGGGC CGTTTCTCCA ACGGACCCGT CTGGCTGGAG CAGCTGACCA
     GATACTCCCG GCAAAGAGGT TGCCTGGGCA GACCGACCTC GTCGACTGGT

251  AACAGTTCCC GGGTCTGACC ATCGCCAACG AAGCGGAAGG CGGTGCCACT
     TTGTCAAGGG CCCAGACTGG TAGCGGTTGC TTCGCCTTCC GCCACGGTGA

301  GCCGTGGCTT ACAACAAGAT CTCCTGGAAT CCCAAGTATC AGGTCATCAA
     CGGCACCGAA TGTTGTTCTA GAGGACCTTA GGGTTCATAG TCCAGTAGTT

351  CAACCTGGAC TACGAGGTCA CCCAGTTCTT GCAGAAAGAC AGCTTCAAGC
     GTTGGACCTG ATGCTCCAGT GGGTCAAGAA CGTCTTTCTG TCGAAGTTCG

401  CGGACGATCT GGTGATCCTC TGGGTCGGTG CCAATGACTA TCTGGCCTAT
     GCCTGCTAGA CCACTAGGAG ACCCAGCCAC GGTTACTGAT AGACCGGATA

451  GGCTGGAACA CGGAGCAGGA TGCCAAGCGG GTTCGCGATG CCATCAGCGA
     CCGACCTTGT GCCTCGTCCT ACGGTTCGCC CAAGCGCTAC GGTAGTCGCT

501  TGCGGCCAAC CGCATGGTAC TGAACGGTGC CAAGCAGATA CTGCTGTTCA
     ACGCCGGTTG GCGTACCATG ACTTGCCACG GTTCGTCTAT GACGACAAGT

551  ACCTGCCGGA TCTGGGCCAG AACCCGTCAG CTCGCAGTCA GAAGGTGGTC
     TGGACGGCCT AGACCCGGTC TTGGGCAGTC GAGCGTCAGT CTTCCACCAG

601  GAGGCGGTCA GCCATGTCTC CGCCTATCAC AACCAGCTGC TGCTGAACCT
     CTCCGCCAGT CGGTACAGAG GCGGATAGTG TTGGTCGACG ACGACTTGGA

651  GGCACGCCAG CTGGCCCCCA CCGGCATGGT AAAGCTGTTC GAGATCGACA
     CCGTGCGGTC GACCGGGGGT GGCCGTACCA TTTCGACAAG CTCTAGCTGT

701  AGCAATTTGC CGAGATGCTG CGTGATCCGC AGAACTTCGG CCTGAGCGAC
     TCGTTAAACG GCTCTACGAC GCACTAGGCG TCTTGAAGCC GGACTCGCTG

751  GTCGAGAACC CCTGCTACGA CGGCGGCTAT GTGTGGAAGC CGTTTGCCAC
     CAGCTCTTGG GGACGATGCT GCCGCCGATA CACACCTTCG GCAAACGGTG

801  CCGCAGCGTC AGCACCGACC GCCAGCTCTC CGCCTTCAGT CCGCAGGAAC
     GGCGTCGCAG TCGTGGCTGG CGGTCGAGAG GCGGAAGTCA GGCGTCCTTG

851  GCCTCGCCAT CGCCGGCAAC CCGCTGCTGG CACAGGCCGT TGCCAGTCCT
     CGGAGCGGTA GCGGCCGTTG GGCGACGACC GTGTCCGGCA ACGGTCAGGA

901  ATGGCCCGCC GCAGCGCCAG CCCCCTCAAC TGTGAGGGCA AGATGTTCTG
     TACCGGGCGG CGTCGCGGTC GGGGGAGTTG ACACTCCCGT TCTACAAGAC

951  GGATCAGGTA CACCCGACCA CTGTCGTGCA CGCAGCCCTG AGCGAGCGCG
     CCTAGTCCAT GTGGGCTGGT GACAGCACGT GCGTCGGGAC TCGCTCGCGC

1001 CCGCCACCTT CATCGCGAAC CAGTACGAGT TCCTCGCCCA CTGATGA
     GGCGGTGGAA GTAGCGCTTG GTCATGCTCA AGGAGCGGGT GACTACT
```

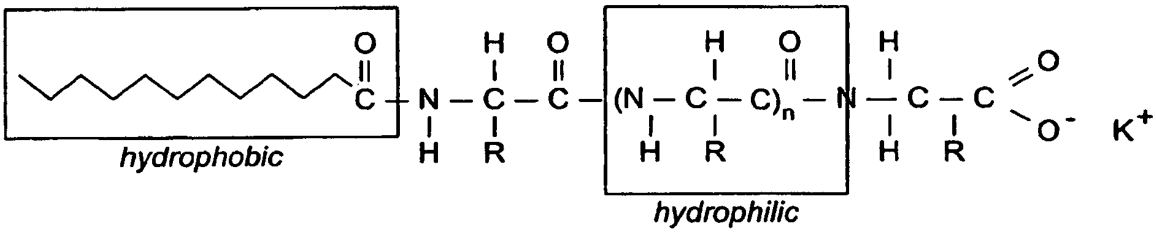


Figure 49

Figure 50

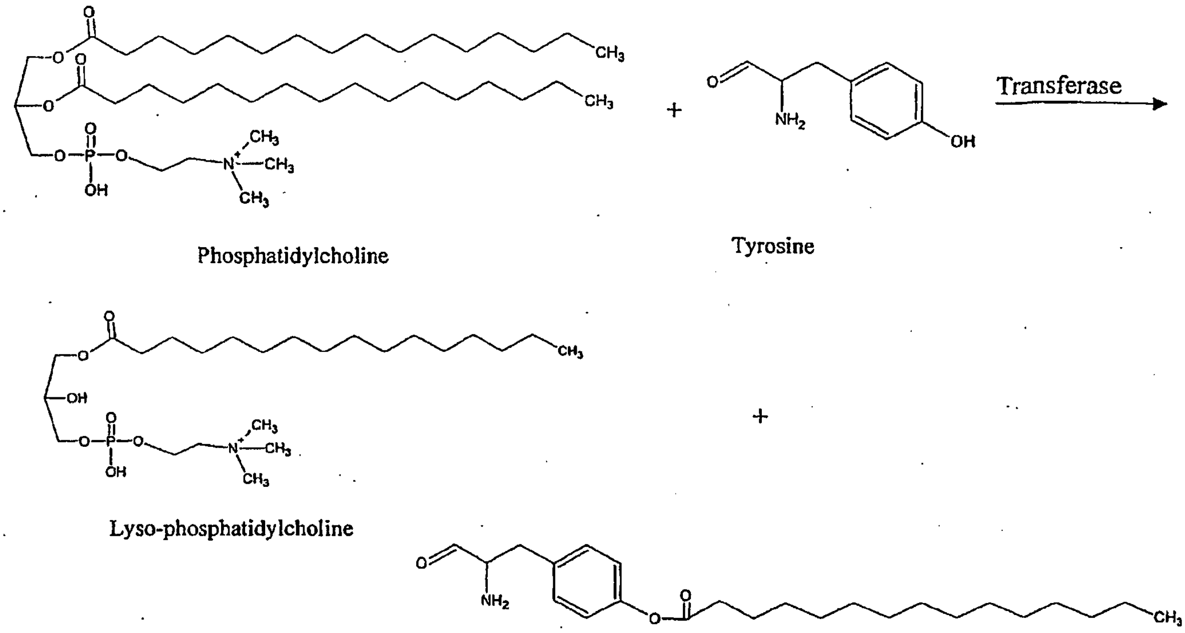


Phosphatidylcholine

Tyrosine

Lyso-phosphatidylcholine

Tyrosine fatty acid condensate

Figure 51

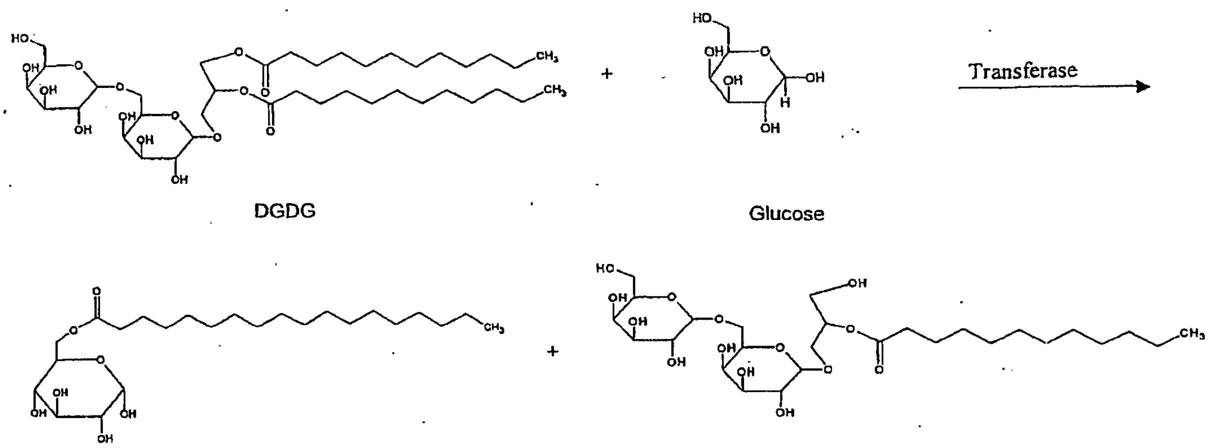


DGDG

Glucose

DGMG

Glucoseester

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0005396 A
- EP 0746608 A
- WO 0397835 A
- EP 0977869 A
- EP 1193314 A
- US 4683202 A
- EP 0583265 A
- EP 0866796 A
- WO 0206457 A
- EP 0752008 A
- EP 1138763 A
- EP 1103606 A
- US 6180406 B
- WO 0134835 A
- WO 0058517 A
- US 6344328 B
- US 6361974 B
- WO 9117243 A
- EP 0238023 A
- EP 0449375 A
- US 5741665 A
- US 5674707 A
- WO 0138544 A
- WO 0116308 A
- US 3817837 A
- US 3850752 A
- US 3939350 A
- US 3996345 A
- US 4277437 A
- US 4275149 A
- US 4366241 A
- US 4816567 A
- GB 0301121 A
- GB 0301122 A
- GB 0301117 A
- GB 0301120 A
- GB 0301119 A
- GB 0301118 A
- GB 0330016 A
- US 60489441 B


### Non-patent literature cited in the description


- **LECOINTE et al.** *Biotechnology Letters,* vol. 18 (8), 869-874
- **VAYSSE et al.** *J. of Biotechnology,* 1997, vol. 53, 41-46
- **BUCKLEY.** *Biochemistry,* 1983, vol. 22, 5490-5493
- **UPTON ; BUCKLEY.** *TIBS,* 20 May 1995, 178-179
- **BRUMLIK ; BUCKLEY.** *J. of Bacteriology,* April 1996, 2060-2064
- **BRUMLIK ; BUCKLEY.** *Journal of Bacteriology,* April 1996, vol. 178 (7), 2060-2064
- **BATEMAN A et al.** *Nucleic Acids Res.,* 2002, vol. 30, 276-280
- **BATEMAN A ; HAFT DH.** *Brief Bioinform,* 2002, vol. 3, 236-245
- **DURBIN R ; EDDY S ; KROGH A.** Biological sequence analysis; probabilistic models of proteins and nucleic acids. Cambridge University Press, 1998
- **NEEDLEMAN ; WUNSCH.** *J. of Molecular Biology,* 1970, vol. 48, 443-45
- **BALCAO V.M. ; PAIVA A.L. ; MALCATA F.X.** *Enzyme Microb Technol.,* 01 May 1996, vol. 18 (6), 392-416
- **RETZ M.T. ; JAEGER K.E.** *Chem Phys Lipids,* June 1998, vol. 93 (1-2), 3-14
- **BOMSCHEUER U.T. ; BESSLER C ; SRINIVAS R ; KRISHNA S.H.** *Trends Biotechnol.,* October 2002, vol. 20 (10), 433-7
- **PLOU et al.** *J. Biotechnology,* 2002, vol. 92, 55-66
- **WARMUTH et al.** *Bio Forum,* 1992, vol. 9, 282-283
- **FERRER et al.** *J. Chem. Technol. Biotechnol.,* 2000, vol. 75, 1-8
- **CHRISTENSEN et al.** *Nachwachsende Rohstoff,* 1998, vol. 10, 98-105
- **PETERSEN ; CHRISTENEN.** Applied Biocatalysis. Harwood Academic Publishers, 2000
- Ulmann's Encyclopedia of Industrial Chemistry. Wiley-VCH Verlag GmbH & Co. KgaA, 2002
- **BEUCAGE S.L. et al.** *Tetrahedron Letters,* 1981, vol. 22, 1859-1869
- **MATTHES et al.** *EMBO J.,* 1984, vol. 3, 801-805
- **SAIKI R K et al.** *Science,* 1988, vol. 239, 487-491
- **CARUTHERS MH et al.** *Nuc Acids Res Symp Ser,* 1980, 215-23

- **HORN T et al.** *Nuc Acids Res Symp Ser,* 1990, 225-232
- **MORINAGA et al.** *Biotechnology,* 1984, vol. 2, 646-649
- **NELSON ; LONG.** *Analytical Biochemistry,* 1989, vol. 180, 147-151
- **HILTON S ; BUCKLEY JT.** Studies on the reaction mechanism of a microbial lipase/acyltransferase using chemical modification and site-directed mutagenesis. *J Biol Chem.,* 15 January 1991, vol. 266 (2), 997-1000
- **ROBERTSON DL ; HILTON S ; WONG KR ; KOEPKE A ; BUCKLEY JT.** Influence of active site and tyrosine modification on the secretion and activity of the Aeromonas hydrophila lipase/acyltransferase. *J Biol Chem.,* 21 January 1994, vol. 269 (3), 2145-50
- **BRUMLIK MJ ; BUCKLEY JT.** Identification of the catalytic triad of the lipase/acyltransferase from Aeromonas hydrophila. *J Bacteriol.,* April 1996, vol. 178 (7), 2060-4
- **PEELMAN F ; VINAIMONT N ; VERHEE A ; VANLOO B ; VERSCHELDE JL ; LABEUR C ; SEGURET-MACE S ; DUVERGER N ; HUTCHINSON G ; VANDEKERCKHOVE J.** A proposed architecture for lecithin cholesterol acyl transferase (LCAT): identification of the catalytic triad and molecular modeling. *Protein Sci.,* March 1998, vol. 7 (3), 587-99
- **DEVEREUX et al.** *Nuc. Acids Research,* 1984, vol. 12, 387
- **AUSUBEL et al.** Short Protocols in Molecular Biology. 1999
- **ALTSCHUL et al.** *J. Mol. Biol.,* 1990, 403-410
- *FEMS Microbiol Lett,* 1999, vol. 174 (2), 247-50
- *FEMS Microbiol Lett,* 1999, vol. 177 (1), 187-8, tatiana@ncbi.nlm.nih.gov
- **HIGGINS DG ; SHARP PM.** *Gene,* 1988, vol. 73 (1), 237-244
- **SIMON RJ et al.** *PNAS,* 1992, vol. 89 (20), 9367-9371
- **HORWELL DC.** *Trends Biotechnol.,* 1995, vol. 13 (4), 132-134
- Guide to Molecular Cloning Techniques. **BERGER ; KIMMEL.** Methods in Enzymology. Academic Press, 1987, vol. 152
- **SAMBROOK et al.** Molecular Cloning: A Laboratory Manual. Cold Spring Harbor Laboratory Press, 1989
- **POTRYKUS.** *Annu Rev Plant Physiol Plant Mol Biol,* 1991, vol. 42, 205-225
- **CHRISTOU.** *Agro-Food-Industry Hi-Tech.,* March 1994, 17-27
- **DAVIS ; DE SERRES.** *Methods Enzymol,* 1971, vol. 17A, 79-143
- Vectors for genetic manipulation. **TURNER G.** Aspergillus: 50 years on. Progress in industrial microbiology. Elsevier, 1994, vol. 29, 641-666
- **PUNT et al.** *Trends Biotechnol,* May 2002, vol. 20 (5), 200-6
- *Archer & Peberdy Crit Rev Biotechnol,* 1997, vol. 17 (4), 273-306
- *Methods Mol Biol,* 1995, vol. 49, 341-54
- *Curr Opin Biotechnol,* October 1997, vol. 8 (5), 554-60
- *FEMS Microbiol Rev,* 2000, vol. 24 (1), 45-66
- Yeast as a vehicle for the expression of heterologous genes. **E HINCHCLIFFE ; E KENNY.** Yeasts. Academic Press Ltd, 1993, vol. 5
- **HINNEN et al.** *Proceedings of the National Academy of Sciences of the USA,* 1978, 75
- **BEGGS, J D.** *Nature,* 1978, vol. 275, 104
- **ITO, H et al.** *J Bacteriology,* 1983, vol. 153, 163-168
- **CHRISTOU.** *Agro-Food-Industry Hi-Tech,* March 1994, 17-27
- *Curr. Opin. Biotechnol.,* 1995, vol. 6 (5), 501-6
- **UPTON C ; BUCKLEY JT.** *Trends Biochem Sci,* 1995, vol. 20, 179-179
- *Journal of the Americal Oil Chemists' Society,* 1978, vol. 55 (4), 398-401
- Centrifiges, Filtering. Ullmann's Encyclopedia of Industrial Chemistry. Wiley-VCH Verlag GmbH & Co. KgaA, 2002
- Basic Principles of Chromatography. Ullmann's Encyclopedia of Industrial Chemistry. Wiley-VCH Verlag GmbH & Co. KGaA, 2002
- Bailey's Industrial Oil and Fat Products. Edible Oil and Fat Products: Products and Application Technology. vol. 3, 502-511

SEQUENCE LISTING

<110> DANISCO A/S

<120> METHOD

<130> P015575EPB

<150> GB 0301121.0
<151> 2003-01-17

<150> GB 0301122.8
<151> 2003-01-17

<150> GB 0301117.8
<151> 2003-01-17

<150> GB 0301120.2
<151> 2003-01-17

<150> GB 0301119.4
<151> 2003-01-17

<150> GB 0301118.6
<151> 2003-01-17

<150> GB 0330016.7

<151> 2003-12-24

<150> US 60/489441
<151> 2003-07-23

<160> 68

<170> PatentIn version 3.5

<210> 1
<211> 361
<212> PRT
<213> Artificial Sequence

<220>
<223> Consensus sequence

<400> 1

```
Ile Val Ala Phe Gly Asp Ser Leu Thr Asp Gly Glu Ala Tyr Tyr Gly
1               5                   10                  15

Asp Ser Asp Gly Gly Gly Trp Gly Ala Gly Leu Ala Asp Arg Leu Thr
            20                  25                  30

Ala Leu Leu Arg Leu Arg Ala Arg Pro Arg Gly Val Asp Val Phe Asn
        35                  40                  45

Arg Gly Ile Ser Gly Arg Thr Ser Asp Gly Arg Leu Ile Val Asp Ala
    50                  55                  60

Leu Val Ala Leu Leu Phe Leu Ala Gln Ser Leu Gly Leu Pro Asn Leu
65                  70                  75                  80
```

Pro Pro Tyr Leu Ser Gly Asp Phe Leu Arg Gly Ala Asn Phe Ala Ser
85 90 95

Ala Gly Ala Thr Ile Leu Pro Thr Ser Gly Pro Phe Leu Ile Gln Val
100 105 110

Gln Phe Lys Asp Phe Lys Ser Gln Val Leu Glu Leu Arg Gln Ala Leu
115 120 125

Gly Leu Leu Gln Glu Leu Leu Arg Leu Leu Pro Val Leu Asp Ala Lys
130 135 140

Ser Pro Asp Leu Val Thr Ile Met Ile Gly Thr Asn Asp Leu Ile Thr
145 150 155 160

Ser Ala Phe Phe Gly Pro Lys Ser Thr Glu Ser Asp Arg Asn Val Ser
165 170 175

Val Pro Glu Phe Lys Asp Asn Leu Arg Gln Leu Ile Lys Arg Leu Arg
180 185 190

Ser Asn Asn Gly Ala Arg Ile Ile Val Leu Ile Thr Leu Val Ile Leu
195 200 205

Asn Leu Gly Pro Leu Gly Cys Leu Pro Leu Lys Leu Ala Leu Ala Leu
210 215 220

Ala Ser Ser Lys Asn Val Asp Ala Ser Gly Cys Leu Glu Arg Leu Asn
225 230 235 240

Glu Ala Val Ala Asp Phe Asn Glu Ala Leu Arg Glu Leu Ala Ile Ser
245 250 255

Lys Leu Glu Asp Gln Leu Arg Lys Asp Gly Leu Pro Asp Val Lys Gly
260 265 270

Ala Asp Val Pro Tyr Val Asp Leu Tyr Ser Ile Phe Gln Asp Leu Asp
275 280 285

Gly Ile Gln Asn Pro Ser Ala Tyr Val Tyr Gly Phe Glu Thr Thr Lys
290 295 300

Ala Cys Cys Gly Tyr Gly Gly Arg Tyr Asn Tyr Asn Arg Val Cys Gly
305 310 315 320

Asn Ala Gly Leu Cys Asn Val Thr Ala Lys Ala Cys Asn Pro Ser Ser
325 330 335

```
Tyr Leu Leu Ser Phe Leu Phe Trp Asp Gly Phe His Pro Ser Glu Lys
            340                 345                 350

Gly Tyr Lys Ala Val Ala Glu Ala Leu
        355                 360
```

<210> 2
<211> 335
<212> PRT
<213> Aeromonas hydrophila

<400> 2

```
Met Lys Lys Trp Phe Val Cys Leu Leu Gly Leu Val Ala Leu Thr Val
1               5                   10                  15

Gln Ala Ala Asp Ser Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Asn Glu Phe Pro Gly Leu Thr Ile Ala
65                  70                  75                  80

Asn Glu Ala Glu Gly Gly Pro Thr Ala Val Ala Tyr Asn Lys Ile Ser
                85                  90                  95

Trp Asn Pro Lys Tyr Gln Val Ile Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160

Val Leu Asn Gly Ala Lys Glu Ile Leu Leu Phe Asn Leu Pro Asp Leu
                165                 170                 175

Gly Gln Asn Pro Ser Ala Arg Ser Gln Lys Val Val Glu Ala Ala Ser
            180                 185                 190
```

His Val Ser Ala Tyr His Asn Gln Leu Leu Leu Asn Leu Ala Arg Gln
195 200 205

Leu Ala Pro Thr Gly Met Val Lys Leu Phe Glu Ile Asp Lys Gln Phe
210 215 220

Ala Glu Met Leu Arg Asp Pro Gln Asn Phe Gly Leu Ser Asp Gln Arg
225 230 235 240

Asn Ala Cys Tyr Gly Gly Ser Tyr Val Trp Lys Pro Phe Ala Ser Arg
245 250 255

Ser Ala Ser Thr Asp Ser Gln Leu Ser Ala Phe Asn Pro Gln Glu Arg
260 265 270

Leu Ala Ile Ala Gly Asn Pro Leu Leu Ala Gln Ala Val Ala Ser Pro
275 280 285

Met Ala Ala Arg Ser Ala Ser Thr Leu Asn Cys Glu Gly Lys Met Phe
290 295 300

Trp Asp Gln Val His Pro Thr Thr Val Val His Ala Ala Leu Ser Glu
305 310 315 320

Pro Ala Ala Thr Phe Ile Glu Ser Gln Tyr Glu Phe Leu Ala His
325 330 335

<210> 3
<211> 336
<212> PRT
<213> Aeromonas salmonicida

<400> 3

Met Lys Lys Trp Phe Val Cys Leu Leu Gly Leu Ile Ala Leu Thr Val
1 5 10 15

Gln Ala Ala Asp Thr Arg Pro Ala Phe Ser Arg Ile Val Met Phe Gly
20 25 30

Asp Ser Leu Ser Asp Thr Gly Lys Met Tyr Ser Lys Met Arg Gly Tyr
35 40 45

Leu Pro Ser Ser Pro Pro Tyr Tyr Glu Gly Arg Phe Ser Asn Gly Pro
50 55 60

Val Trp Leu Glu Gln Leu Thr Lys Gln Phe Pro Gly Leu Thr Ile Ala
65 70 75 80

Asn Glu Ala Glu Gly Gly Ala Thr Ala Val Ala Tyr Asn Lys Ile Ser
85 90 95

Trp Asn Pro Lys Tyr Gln Val Tyr Asn Asn Leu Asp Tyr Glu Val Thr
100 105 110

Gln Phe Leu Gln Lys Asp Ser Phe Lys Pro Asp Asp Leu Val Ile Leu
115 120 125

Trp Val Gly Ala Asn Asp Tyr Leu Ala Tyr Gly Trp Asn Thr Glu Gln
130 135 140

Asp Ala Lys Arg Val Arg Asp Ala Ile Ser Asp Ala Ala Asn Arg Met
145 150 155 160

Val Leu Asn Gly Ala Lys Gln Ile Leu Leu Phe Asn Leu Pro Asp Leu
165 170 175

Gly Gln Asn Pro Ser Ala Arg Ser Gln Lys Val Val Glu Ala Val Ser
180 185 190

His Val Ser Ala Tyr His Asn Lys Leu Leu Leu Asn Leu Ala Arg Gln
195 200 205

Leu Ala Pro Thr Gly Met Val Lys Leu Phe Glu Ile Asp Lys Gln Phe
210 215 220

Ala Glu Met Leu Arg Asp Pro Gln Asn Phe Gly Leu Ser Asp Val Glu
225 230 235 240

Asn Pro Cys Tyr Asp Gly Gly Tyr Val Trp Lys Pro Phe Ala Thr Arg
245 250 255

Ser Val Ser Thr Asp Arg Gln Leu Ser Ala Phe Ser Pro Gln Glu Arg
260 265 270

Leu Ala Ile Ala Gly Asn Pro Leu Leu Ala Gln Ala Val Ala Ser Pro
275 280 285

Met Ala Arg Arg Ser Ala Ser Pro Leu Asn Cys Glu Gly Lys Met Phe
290 295 300

Trp Asp Gln Val His Pro Thr Thr Val Val His Ala Ala Leu Ser Glu
305 310 315 320

Arg Ala Ala Thr Phe Ile Glu Thr Gln Tyr Glu Phe Leu Ala His Gly
325 330 335

<210> 4
<211> 295

<212> PRT
<213> Streptomyces coelicolor

<400> 4

```
Met Pro Lys Pro Ala Leu Arg Arg Val Met Thr Ala Thr Val Ala Ala
1               5                   10                  15

Val Gly Thr Leu Ala Leu Gly Leu Thr Asp Ala Thr Ala His Ala Ala
            20                  25                  30

Pro Ala Gln Ala Thr Pro Thr Leu Asp Tyr Val Ala Leu Gly Asp Ser
        35                  40                  45

Tyr Ser Ala Gly Ser Gly Val Leu Pro Val Asp Pro Ala Asn Leu Leu
    50                  55                  60

Cys Leu Arg Ser Thr Ala Asn Tyr Pro His Val Ile Ala Asp Thr Thr
65                  70                  75                  80

Gly Ala Arg Leu Thr Asp Val Thr Cys Gly Ala Ala Gln Thr Ala Asp
                85                  90                  95

Phe Thr Arg Ala Gln Tyr Pro Gly Val Ala Pro Gln Leu Asp Ala Leu
            100                 105                 110

Gly Thr Gly Thr Asp Leu Val Thr Leu Thr Ile Gly Gly Asn Asp Asn
        115                 120                 125

Ser Thr Phe Ile Asn Ala Ile Thr Ala Cys Gly Thr Ala Gly Val Leu
    130                 135                 140

Ser Gly Gly Lys Gly Ser Pro Cys Lys Asp Arg His Gly Thr Ser Phe
145                 150                 155                 160

Asp Asp Glu Ile Glu Ala Asn Thr Tyr Pro Ala Leu Lys Glu Ala Leu
                165                 170                 175

Leu Gly Val Arg Ala Arg Ala Pro His Ala Arg Val Ala Ala Leu Gly
            180                 185                 190

Tyr Pro Trp Ile Thr Pro Ala Thr Ala Asp Pro Ser Cys Phe Leu Lys
        195                 200                 205

Leu Pro Leu Ala Ala Gly Asp Val Pro Tyr Leu Arg Ala Ile Gln Ala
    210                 215                 220

His Leu Asn Asp Ala Val Arg Arg Ala Ala Glu Glu Thr Gly Ala Thr
```

```
225                 230                 235                 240

Tyr Val Asp Phe Ser Gly Val Ser Asp Gly His Asp Ala Cys Glu Ala
                245                 250                 255

Pro Gly Thr Arg Trp Ile Glu Pro Leu Leu Phe Gly His Ser Leu Val
            260                 265                 270

Pro Val His Pro Asn Ala Leu Gly Glu Arg Arg Met Ala Glu His Thr
        275                 280                 285

Met Asp Val Leu Gly Leu Asp
    290                 295
```

<210> 5
<211> 295
<212> PRT
<213> Streptomyces coelicolor

<400> 5

```
Met Pro Lys Pro Ala Leu Arg Arg Val Met Thr Ala Thr Val Ala Ala
1               5                   10                  15

Val Gly Thr Leu Ala Leu Gly Leu Thr Asp Ala Thr Ala His Ala Ala
            20                  25                  30

Pro Ala Gln Ala Thr Pro Thr Leu Asp Tyr Val Ala Leu Gly Asp Ser
        35                  40                  45

Tyr Ser Ala Gly Ser Gly Val Leu Pro Val Asp Pro Ala Asn Leu Leu
    50                  55                  60

Cys Leu Arg Ser Thr Ala Asn Tyr Pro His Val Ile Ala Asp Thr Thr
65                  70                  75                  80

Gly Ala Arg Leu Thr Asp Val Thr Cys Gly Ala Ala Gln Thr Ala Asp
                85                  90                  95

Phe Thr Arg Ala Gln Tyr Pro Gly Val Ala Pro Gln Leu Asp Ala Leu
            100                 105                 110

Gly Thr Gly Thr Asp Leu Val Thr Leu Thr Ile Gly Gly Asn Asp Asn
        115                 120                 125

Ser Thr Phe Ile Asn Ala Ile Thr Ala Cys Gly Thr Ala Gly Val Leu
    130                 135                 140

Ser Gly Gly Lys Gly Ser Pro Cys Lys Asp Arg His Gly Thr Ser Phe
145                 150                 155                 160
```

Asp Asp Glu Ile Glu Ala Asn Thr Tyr Pro Ala Leu Lys Glu Ala Leu
165 170 175

Leu Gly Val Arg Ala Arg Ala Pro His Ala Arg Val Ala Ala Leu Gly
180 185 190

Tyr Pro Trp Ile Thr Pro Ala Thr Ala Asp Pro Ser Cys Phe Leu Lys
195 200 205

Leu Pro Leu Ala Ala Gly Asp Val Pro Tyr Leu Arg Ala Ile Gln Ala
210 215 220

His Leu Asn Asp Ala Val Arg Arg Ala Ala Glu Glu Thr Gly Ala Thr
225 230 235 240

Tyr Val Asp Phe Ser Gly Val Ser Asp Gly His Asp Ala Cys Glu Ala
245 250 255

Pro Gly Thr Arg Trp Ile Glu Pro Leu Leu Phe Gly His Ser Leu Val
260 265 270

Pro Val His Pro Asn Ala Leu Gly Glu Arg Arg Met Ala Glu His Thr
275 280 285

Met Asp Val Leu Gly Leu Asp
290 295

<210> 6
<211> 238
<212> PRT
<213> Saccharomyces cerevisiae

<400> 6

Met Asp Tyr Glu Lys Phe Leu Leu Phe Gly Asp Ser Ile Thr Glu Phe
1 5 10 15

Ala Phe Asn Thr Arg Pro Ile Glu Asp Gly Lys Asp Gln Tyr Ala Leu
20 25 30

Gly Ala Ala Leu Val Asn Glu Tyr Thr Arg Lys Met Asp Ile Leu Gln
35 40 45

Arg Gly Phe Lys Gly Tyr Thr Ser Arg Trp Ala Leu Lys Ile Leu Pro
50 55 60

Glu Ile Leu Lys His Glu Ser Asn Ile Val Met Ala Thr Ile Phe Leu
65 70 75 80

Gly Ala Asn Asp Ala Cys Ser Ala Gly Pro Gln Ser Val Pro Leu Pro
85 90 95

Glu Phe Ile Asp Asn Ile Arg Gln Met Val Ser Leu Met Lys Ser Tyr
100 105 110

His Ile Arg Pro Ile Ile Ile Gly Pro Gly Leu Val Asp Arg Glu Lys
115 120 125

Trp Glu Lys Glu Lys Ser Glu Glu Ile Ala Leu Gly Tyr Phe Arg Thr
130 135 140

Asn Glu Asn Phe Ala Ile Tyr Ser Asp Ala Leu Ala Lys Leu Ala Asn
145 150 155 160

Glu Glu Lys Val Pro Phe Val Ala Leu Asn Lys Ala Phe Gln Gln Glu
165 170 175

Gly Gly Asp Ala Trp Gln Gln Leu Leu Thr Asp Gly Leu His Phe Ser
180 185 190

Gly Lys Gly Tyr Lys Ile Phe His Asp Glu Leu Leu Lys Val Ile Glu
195 200 205

Thr Phe Tyr Pro Gln Tyr His Pro Lys Asn Met Gln Tyr Lys Leu Lys
210 215 220

Asp Trp Arg Asp Val Leu Asp Asp Gly Ser Asn Ile Met Ser
225 230 235

<210> 7
<211> 1005
<212> DNA
<213> Aeromonas hydrophila

<400> 7

atgaaaaaat ggtttgtgtg tttattggga ttggtcgcgc tgacagttca ggcagccgac 60
agccgtcccg ccttctcccg gatcgtgatg tttggcgaca gcctctccga taccggcaag 120
atgtacagca agatgcgcgg ttacctcccc tccagccccc cctactatga gggccgcttc 180
tccaacgggc ccgtctggct ggagcagctg accaacgagt tcccgggcct gaccatagcc 240
aacgaggcgg aaggcggacc gaccgccgtg gcttacaaca agatctcctg gaatcccaag 300
tatcaggtca tcaacaacct ggactacgag gtcacccagt tcctgcaaaa agacagcttc 360
aagccggacg atctggtgat cctctgggtc ggcgccaacg actatctggc ctatggctgg 420
aacacagagc aggatgccaa gcgggtgcgc gacgccatca gcgatgcggc caaccgcatg 480
gtgctgaacg gcgccaagga gatactgctg ttcaacctgc cggatctggg ccagaacccc 540

```
tcggcccgca gccagaaggt ggtcgaggcg gccagccatg tctccgccta ccacaaccag      600
ctgctgctga acctggcacg ccagctggct cccaccggca tggtgaagct gttcgagatc      660
gacaagcagt ttgccgagat gctgcgtgat ccgcagaact tcggcctgag cgaccagagg      720
aacgcctgct acggtggcag ctatgtatgg aagccgtttg cctcccgcag cgccagcacc      780
gacagccagc tctccgcctt caacccgcag gagcgcctcg ccatcgccgg caacccgctg      840
ctggcccagg ccgtcgccag ccccatggct gcccgcagcg ccagcaccct caactgtgag      900
ggcaagatgt tctgggatca ggtccacccc accactgtcg tgcacgccgc cctgagcgag      960
cccgccgcca ccttcatcga gagccagtac gagttcctcg cccac                     1005
```

<210> 8
<211> 1011
<212> DNA
<213> Aeromonas salmonicida

<400> 8

```
atgaaaaaat ggtttgtttg tttattgggg ttgatcgcgc tgacagttca ggcagccgac       60
actcgccccg ccttctcccg gatcgtgatg ttcggcgaca gcctctccga taccggcaaa      120
atgtacagca agatgcgcgg ttacctcccc tccagcccgc cctactatga gggccgtttc      180
tccaacggac ccgtctggct ggagcagctg accaagcagt tcccgggtct gaccatcgcc      240
aacgaagcgg aaggcggtgc cactgccgtg gcttacaaca agatctcctg gaatcccaag      300
tatcaggtct acaacaacct ggactacgag gtcacccagt tcttgcagaa agacagcttc      360
aagccggacg atctggtgat cctctgggtc ggtgccaatg actatctggc atatggctgg      420
aatacggagc aggatgccaa gcgagttcgc gatgccatca gcgatgcggc caaccgcatg      480
gtactgaacg gtgccaagca gatactgctg ttcaacctgc cggatctggg ccagaacccg      540
tcagcccgca gtcagaaggt ggtcgaggcg gtcagccatg tctccgccta tcacaacaag      600
ctgctgctga acctggcacg ccagctggcc cccaccggca tggtaaagct gttcgagatc      660
gacaagcaat ttgccgagat gctgcgtgat ccgcagaact tcggcctgag cgacgtcgag      720
aacccctgct acgacggcgg ctatgtgtgg aagccgtttg ccacccgcag cgtcagcacc      780
gaccgccagc tctccgcctt cagtccgcag gaacgcctcg ccatcgccgg caacccgctg      840
ctggcacagg ccgttgccag tcctatggcc cgccgcagcg ccagccccct caactgtgag      900
ggcaagatgt tctgggatca ggtacacccg accactgtcg tgcacgcagc cctgagcgag      960
cgcgccgcca ccttcatcga gacccagtac gagttcctcg cccacggatg a              1011
```

<210> 9
<211> 888
<212> DNA
<213> Streptomyces coelicolor

<400> 9

```
atgccgaagc ctgcccttcg ccgtgtcatg accgcgacag tcgccgccgt cggcacgctc      60
gccctcggcc tcaccgacgc caccgcccac gccgcgcccg cccaggccac tccgaccctg     120
gactacgtcg ccctcggcga cagctacagc gccggctccg gcgtcctgcc cgtcgacccc     180
gccaacctgc tctgtctgcg ctcgacggcc aactaccccc acgtcatcgc ggacacgacg     240
ggcgcccgcc tcacggacgt cacctgcggc gccgcgcaga ccgccgactt cacgcgggcc     300
cagtacccgg gcgtcgcacc ccagttggac gcgctcggca ccggcacgga cctggtcacg     360
ctcaccatcg gcggcaacga caacagcacc ttcatcaacg ccatcacggc ctgcggcacg     420
gcgggtgtcc tcagcggcgg caagggcagc cctgcaagg acaggcacgg cacctccttc      480
gacgacgaga tcgaggccaa cacgtacccc gcgctcaagg aggcgctgct cggcgtccgc     540
gccagggctc cccacgccag ggtggcggct ctcggctacc cgtggatcac cccggccacc     600
gccgacccgt cctgcttcct gaagctcccc ctcgccgccg gtgacgtgcc ctacctgcgg     660
gccatccagg cacacctcaa cgacgcggtc cggcgggccg ccgaggagac cggagccacc     720
tacgtggact tctccggggt gtccgacggc cacgacgcct gcgaggcccc cggcacccgc     780
tggatcgaac cgctgctctt cgggcacagc ctcgttcccg tccaccccaa cgccctgggc     840
gagcggcgca tggccgagca cacgatggac gtcctcggcc tggactga                  888
```

<210> 10
<211> 888
<212> DNA
<213> Streptomyces coelicolor

<400> 10

tcagtccagg ccgaggacgt ccatcgtgtg ctcggccatg cgccgctcgc ccagggcgtt 60
ggggtggacg ggaacgaggc tgtgcccgaa gagcagcggt tcgatccagc gggtgccggg 120
ggcctcgcag gcgtcgtggc cgtcggacac cccggagaag tccacgtagg tggctccggt 180
ctcctcggcg gcccgccgga ccgcgtcgtt gaggtgtgcc tggatggccc gcaggtaggg 240
cacgtcaccg gcggcgaggg ggagcttcag gaagcaggac gggtcggcgg tggccggggt 300
gatccacggg tagccgagag ccgccacccct ggcgtgggga gccctggcgc ggacgccgag 360
cagcgcctcc ttgagcgcgg ggtacgtgtt ggcctcgatc tcgtcgtcga aggaggtgcc 420
gtgcctgtcc ttgcaggggc tgcccttgcc gccgctgagg acacccgccg tgccgcaggc 480
cgtgatggcg ttgatgaagg tgctgttgtc gttgccgccg atggtgagcg tgaccaggtc 540
cgtgccggtg ccgagcgcgt ccaactgggg tgcgacgccc gggtactggg cccgcgtgaa 600
gtcggcggtc tgcgcggcgc cgcaggtgac gtccgtgagg cgggcgcccg tcgtgtccgc 660
gatgacgtgg gggtagttgg ccgtcgagcg cagacagagc aggttggcgg ggtcgacggg 720
caggacgccg gagccggcgc tgtagctgtc gccgagggcg acgtagtcca gggtcggagt 780

ggcctgggcg ggcgcggcgt gggcggtggc gtcggtgagg ccgagggcga gcgtgccgac 840
ggcggcgact gtcgcggtca tgacacggcg aagggcaggc ttcggcat 888

<210> 11
<211> 717
<212> DNA
<213> Saccharomyces cerevisiae

<400> 11

atggattacg agaagtttct gttatttggg gattccatta ctgaatttgc ttttaatact 60
aggcccattg aagatggcaa agatcagtat gctcttggag ccgcattagt caacgaatat 120
acgagaaaaa tggatattct tcaaagaggg ttcaaagggt acacttctag atgggcgttg 180
aaaatacttc ctgagatttt aaagcatgaa tccaatattg tcatggccac aatatttttg 240
ggtgccaacg atgcatgctc agcaggtccc caaagtgtcc ccctccccga atttatcgat 300
aatattcgtc aaatggtatc tttgatgaag tcttaccata tccgtcctat tataatagga 360
ccggggctag tagatagaga gaagtgggaa aaagaaaaat ctgaagaaat agctctcgga 420
tacttccgta ccaacgagaa ctttgccatt tattccgatg ccttagcaaa actagccaat 480
gaggaaaaag ttcccttcgt ggctttgaat aaggcgtttc aacaggaagg tggtgatgct 540
tggcaacaac tgctaacaga tggactgcac ttttccggaa aagggtacaa aatttttcat 600
gacgaattat tgaaggtcat tgagacattc taccccccaat atcatcccaa aaacatgcag 660
tacaaactga aagattggag agatgtgcta gatgatggat ctaacataat gtcttga 717

<210> 12

<211> 347
<212> PRT
<213> Ralstonia sp.

<400> 12

```
Met Asn Leu Arg Gln Trp Met Gly Ala Ala Thr Ala Ala Leu Ala Leu
1               5                   10                  15

Gly Leu Ala Ala Cys Gly Gly Gly Gly Thr Asp Gln Ser Gly Asn Pro
            20                  25                  30

Asn Val Ala Lys Val Gln Arg Met Val Val Phe Gly Asp Ser Leu Ser
        35                  40                  45

Asp Ile Gly Thr Tyr Thr Pro Val Ala Gln Ala Val Gly Gly Gly Lys
    50                  55                  60

Phe Thr Thr Asn Pro Gly Pro Ile Trp Ala Glu Thr Val Ala Ala Gln
65                  70                  75                  80

Leu Gly Val Thr Leu Thr Pro Ala Val Met Gly Tyr Ala Thr Ser Val
```

85 90 95

Gln Asn Cys Pro Lys Ala Gly Cys Phe Asp Tyr Ala Gln Gly Gly Ser
100 105 110

Arg Val Thr Asp Pro Asn Gly Ile Gly His Asn Gly Gly Ala Gly Ala
115 120 125

Leu Thr Tyr Pro Val Gln Gln Gln Leu Ala Asn Phe Tyr Ala Ala Ser
130 135 140

Asn Asn Thr Phe Asn Gly Asn Asn Asp Val Val Phe Val Leu Ala Gly
145 150 155 160

Ser Asn Asp Ile Phe Phe Trp Thr Thr Ala Ala Ala Thr Ser Gly Ser
165 170 175

Gly Val Thr Pro Ala Ile Ala Thr Ala Gln Val Gln Gln Ala Ala Thr
180 185 190

Asp Leu Val Gly Tyr Val Lys Asp Met Ile Ala Lys Gly Ala Thr Gln
195 200 205

Val Tyr Val Phe Asn Leu Pro Asp Ser Ser Leu Thr Pro Asp Gly Val
210 215 220

Ala Ser Gly Thr Thr Gly Gln Ala Leu Leu His Ala Leu Val Gly Thr
225 230 235 240

Phe Asn Thr Thr Leu Gln Ser Gly Leu Ala Gly Thr Ser Ala Arg Ile
245 250 255

Ile Asp Phe Asn Ala Gln Leu Thr Ala Ala Ile Gln Asn Gly Ala Ser
260 265 270

Phe Gly Phe Ala Asn Thr Ser Ala Arg Ala Cys Asp Ala Thr Lys Ile
275 280 285

Asn Ala Leu Val Pro Ser Ala Gly Gly Ser Ser Leu Phe Cys Ser Ala
290 295 300

Asn Thr Leu Val Ala Ser Gly Ala Asp Gln Ser Tyr Leu Phe Ala Asp
305 310 315 320

Gly Val His Pro Thr Thr Ala Gly His Arg Leu Ile Ala Ser Asn Val
325 330 335

Leu Ala Arg Leu Leu Ala Asp Asn Val Ala His

340 345

<210> 13
<211> 1044
<212> DNA
<213> Ralstonia sp.

<400> 13

```
atgaacctgc gtcaatggat gggcgccgcc acggctgccc ttgccttggg cttggccgcg      60
tgcgggggcg gtgggaccga ccagagcggc aatcccaatg tcgccaaggt gcagcgcatg     120
gtggtgttcg gcgacagcct gagcgatatc ggcacctaca cccccgtcgc gcaggcggtg     180
ggcggcggca agttcaccac caacccgggc ccgatctggg ccgagaccgt ggccgcgcaa     240
ctgggcgtga cgctcacgcc ggcggtgatg ggctacgcca cctccgtgca gaattgcccc     300
aaggccggct gcttcgacta tgcgcagggc ggctcgcgcg tgaccgatcc gaacggcatc     360
ggccacaacg gcggcgcggg ggcgctgacc tacccggttc agcagcagct cgccaacttc     420
tacgcggcca gcaacaacac attcaacggc aataacgatg tcgtcttcgt gctggccggc     480
agcaacgaca ttttcttctg gaccactgcg gcggccacca gcggctccgg cgtgacgccc     540
gccattgcca cggcccaggt gcagcaggcc gcgacggacc tggtcggcta tgtcaaggac     600
atgatcgcca agggtgcgac gcaggtctac gtgttcaacc tgcccgacag cagcctgacg     660
ccggacggcg tggcaagcgg cacgaccggc caggcgctgc tgcacgcgct ggtgggcacg     720
ttcaacacga cgctgcaaag cgggctggcc ggcacctcgg cgcgcatcat cgacttcaac     780
gcacaactga ccgcggcgat ccagaatggc gcctcgttcg gcttcgccaa caccagcgcc     840
cgggcctgcg acgccaccaa gatcaatgcc ctggtgccga gcgccggcgg cagctcgctg     900
ttctgctcgg ccaacacgct ggtggcttcc ggtgcggacc agagctacct gttcgccgac     960
ggcgtgcacc cgaccacggc cggccatcgc ctgatcgcca gcaacgtgct ggcgcgcctg    1020
ctggcggata acgtcgcgca ctga                                           1044
```

<210> 14
<211> 295
<212> PRT
<213> Aeromonas hydrophila

<400> 14

```
Ile Val Met Phe Gly Asp Ser Leu Ser Asp Thr Gly Lys Met Tyr Ser
1               5                   10                  15

Lys Met Arg Gly Tyr Leu Pro Ser Ser Pro Pro Tyr Tyr Glu Gly Arg
            20                  25                  30

Phe Ser Asn Gly Pro Val Trp Leu Glu Gln Leu Thr Asn Glu Phe Pro
        35                  40                  45
```

```
Gly Leu Thr Ile Ala Asn Glu Ala Glu Gly Gly Pro Thr Ala Val Ala
    50                  55                  60

Tyr Asn Lys Ile Ser Trp Asn Pro Lys Tyr Gln Val Ile Asn Asn Leu
65                  70                  75                  80

Asp Tyr Glu Val Thr Gln Phe Leu Gln Lys Asp Ser Phe Lys Pro Asp
                85                  90                  95

Asp Leu Val Ile Leu Trp Val Gly Ala Asn Asp Tyr Leu Ala Tyr Gly
            100                 105                 110

Trp Asn Thr Glu Gln Asp Ala Lys Arg Val Arg Asp Ala Ile Ser Asp
        115                 120                 125

Ala Ala Asn Arg Met Val Leu Asn Gly Ala Lys Glu Ile Leu Leu Phe
    130                 135                 140

Asn Leu Pro Asp Leu Gly Gln Asn Pro Ser Ala Arg Ser Gln Lys Val
145                 150                 155                 160

Val Glu Ala Ala Ser His Val Ser Ala Tyr His Asn Gln Leu Leu Leu
                165                 170                 175

Asn Leu Ala Arg Gln Leu Ala Pro Thr Gly Met Val Lys Leu Phe Glu
            180                 185                 190

Ile Asp Lys Gln Phe Ala Glu Met Leu Arg Asp Pro Gln Asn Phe Gly
        195                 200                 205

Leu Ser Asp Gln Arg Asn Ala Cys Tyr Gly Gly Ser Tyr Val Trp Lys
    210                 215                 220

Pro Phe Ala Ser Arg Ser Ala Ser Thr Asp Ser Gln Leu Ser Ala Phe
225                 230                 235                 240

Asn Pro Gln Glu Arg Leu Ala Ile Ala Gly Asn Pro Leu Leu Ala Gln
                245                 250                 255

Ala Val Ala Ser Pro Met Ala Ala Arg Ser Ala Ser Thr Leu Asn Cys
            260                 265                 270

Glu Gly Lys Met Phe Trp Asp Gln Val His Pro Thr Thr Val Val His
        275                 280                 285

Ala Ala Leu Ser Glu Pro Ala
    290                 295
```

<210> 15
<211> 295

<212> PRT
<213> Aeromonas salmonicida

<400> 15

```
Ile Val Met Phe Gly Asp Ser Leu Ser Asp Thr Gly Lys Met Tyr Ser
1               5                   10                  15

Lys Met Arg Gly Tyr Leu Pro Ser Ser Pro Pro Tyr Tyr Glu Gly Arg
            20                  25                  30

Phe Ser Asn Gly Pro Val Trp Leu Glu Gln Leu Thr Lys Gln Phe Pro
        35                  40                  45

Gly Leu Thr Ile Ala Asn Glu Ala Glu Gly Gly Ala Thr Ala Val Ala
    50                  55                  60

Tyr Asn Lys Ile Ser Trp Asn Pro Lys Tyr Gln Val Tyr Asn Asn Leu
65                  70                  75                  80

Asp Tyr Glu Val Thr Gln Phe Leu Gln Lys Asp Ser Phe Lys Pro Asp
                85                  90                  95

Asp Leu Val Ile Leu Trp Val Gly Ala Asn Asp Tyr Leu Ala Tyr Gly
            100                 105                 110

Trp Asn Thr Glu Gln Asp Ala Lys Arg Val Arg Asp Ala Ile Ser Asp
        115                 120                 125

Ala Ala Asn Arg Met Val Leu Asn Gly Ala Lys Gln Ile Leu Leu Phe
    130                 135                 140

Asn Leu Pro Asp Leu Gly Gln Asn Pro Ser Ala Arg Ser Gln Lys Val
145                 150                 155                 160

Val Glu Ala Val Ser His Val Ser Ala Tyr His Asn Lys Leu Leu Leu
                165                 170                 175

Asn Leu Ala Arg Gln Leu Ala Pro Thr Gly Met Val Lys Leu Phe Glu
            180                 185                 190

Ile Asp Lys Gln Phe Ala Glu Met Leu Arg Asp Pro Gln Asn Phe Gly
        195                 200                 205

Leu Ser Asp Val Glu Asn Pro Cys Tyr Asp Gly Gly Tyr Val Trp Lys
    210                 215                 220
```

Pro Phe Ala Thr Arg Ser Val Ser Thr Asp Arg Gln Leu Ser Ala Phe
225 230 235 240

Ser Pro Gln Glu Arg Leu Ala Ile Ala Gly Asn Pro Leu Leu Ala Gln
245 250 255

Ala Val Ala Ser Pro Met Ala Arg Arg Ser Ala Ser Pro Leu Asn Cys
260 265 270

Glu Gly Lys Met Phe Trp Asp Gln Val His Pro Thr Thr Val Val His
275 280 285

Ala Ala Leu Ser Glu Arg Ala
290 295

<210> 16
<211> 247
<212> PRT
<213> Streptomyces coelicolor

<400> 16

Tyr Val Ala Leu Gly Asp Ser Tyr Ser Ala Gly Ser Gly Val Leu Pro
1 5 10 15

Val Asp Pro Ala Asn Leu Leu Cys Leu Arg Ser Thr Ala Asn Tyr Pro
20 25 30

His Val Ile Ala Asp Thr Thr Gly Ala Arg Leu Thr Asp Val Thr Cys
35 40 45

Gly Ala Ala Gln Thr Ala Asp Phe Thr Arg Ala Gln Tyr Pro Gly Val
50 55 60

Ala Pro Gln Leu Asp Ala Leu Gly Thr Gly Thr Asp Leu Val Thr Leu
65 70 75 80

Thr Ile Gly Gly Asn Asp Asn Ser Thr Phe Ile Asn Ala Ile Thr Ala
85 90 95

Cys Gly Thr Ala Gly Val Leu Ser Gly Gly Lys Gly Ser Pro Cys Lys
100 105 110

Asp Arg His Gly Thr Ser Phe Asp Asp Glu Ile Glu Ala Asn Thr Tyr
115 120 125

Pro Ala Leu Lys Glu Ala Leu Leu Gly Val Arg Ala Arg Ala Pro His
130 135 140

Ala Arg Val Ala Ala Leu Gly Tyr Pro Trp Ile Thr Pro Ala Thr Ala

145 150 155 160

Asp Pro Ser Cys Phe Leu Lys Leu Pro Leu Ala Ala Gly Asp Val Pro
165 170 175

Tyr Leu Arg Ala Ile Gln Ala His Leu Asn Asp Ala Val Arg Arg Ala
180 185 190

Ala Glu Glu Thr Gly Ala Thr Tyr Val Asp Phe Ser Gly Val Ser Asp
195 200 205

Gly His Asp Ala Cys Glu Ala Pro Gly Thr Arg Trp Ile Glu Pro Leu
210 215 220

Leu Phe Gly His Ser Leu Val Pro Val His Pro Asn Ala Leu Gly Glu
225 230 235 240

Arg Arg Met Ala Glu His Thr
245

<210> 17
<211> 247
<212> PRT
<213> Streptomyces coelicolor

<400> 17

Tyr Val Ala Leu Gly Asp Ser Tyr Ser Ala Gly Ser Gly Val Leu Pro
1 5 10 15

Val Asp Pro Ala Asn Leu Leu Cys Leu Arg Ser Thr Ala Asn Tyr Pro
20 25 30

His Val Ile Ala Asp Thr Thr Gly Ala Arg Leu Thr Asp Val Thr Cys
35 40 45

Gly Ala Ala Gln Thr Ala Asp Phe Thr Arg Ala Gln Tyr Pro Gly Val
50 55 60

Ala Pro Gln Leu Asp Ala Leu Gly Thr Gly Thr Asp Leu Val Thr Leu
65 70 75 80

Thr Ile Gly Gly Asn Asp Asn Ser Thr Phe Ile Asn Ala Ile Thr Ala
85 90 95

Cys Gly Thr Ala Gly Val Leu Ser Gly Gly Lys Gly Ser Pro Cys Lys
100 105 110

Asp Arg His Gly Thr Ser Phe Asp Asp Glu Ile Glu Ala Asn Thr Tyr
115 120 125

Pro Ala Leu Lys Glu Ala Leu Leu Gly Val Arg Ala Arg Ala Pro His
130 135 140

Ala Arg Val Ala Ala Leu Gly Tyr Pro Trp Ile Thr Pro Ala Thr Ala
145 150 155 160

Asp Pro Ser Cys Phe Leu Lys Leu Pro Leu Ala Ala Gly Asp Val Pro
165 170 175

Tyr Leu Arg Ala Ile Gln Ala His Leu Asn Asp Ala Val Arg Arg Ala
180 185 190

Ala Glu Glu Thr Gly Ala Thr Tyr Val Asp Phe Ser Gly Val Ser Asp
195 200 205

Gly His Asp Ala Cys Glu Ala Pro Gly Thr Arg Trp Ile Glu Pro Leu
210 215 220

Leu Phe Gly His Ser Leu Val Pro Val His Pro Asn Ala Leu Gly Glu
225 230 235 240

Arg Arg Met Ala Glu His Thr
245

<210> 18
<211> 198
<212> PRT
<213> Saccharomyces cerevisiae

<400> 18

Phe Leu Leu Phe Gly Asp Ser Ile Thr Glu Phe Ala Phe Asn Thr Arg
1 5 10 15

Pro Ile Glu Asp Gly Lys Asp Gln Tyr Ala Leu Gly Ala Ala Leu Val
20 25 30

Asn Glu Tyr Thr Arg Lys Met Asp Ile Leu Gln Arg Gly Phe Lys Gly
35 40 45

Tyr Thr Ser Arg Trp Ala Leu Lys Ile Leu Pro Glu Ile Leu Lys His
50 55 60

Glu Ser Asn Ile Val Met Ala Thr Ile Phe Leu Gly Ala Asn Asp Ala
65 70 75 80

Cys Ser Ala Gly Pro Gln Ser Val Pro Leu Pro Glu Phe Ile Asp Asn
85 90 95

```
Ile Arg Gln Met Val Ser Leu Met Lys Ser Tyr His Ile Arg Pro Ile
            100                 105                 110

Ile Ile Gly Pro Gly Leu Val Asp Arg Glu Lys Trp Glu Lys Glu Lys
        115                 120                 125

Ser Glu Glu Ile Ala Leu Gly Tyr Phe Arg Thr Asn Glu Asn Phe Ala
    130                 135                 140

Ile Tyr Ser Asp Ala Leu Ala Lys Leu Ala Asn Glu Glu Lys Val Pro
145                 150                 155                 160

Phe Val Ala Leu Asn Lys Ala Phe Gln Gln Glu Gly Gly Asp Ala Trp
                165                 170                 175

Gln Gln Leu Leu Thr Asp Gly Leu His Phe Ser Gly Lys Gly Tyr Lys
            180                 185                 190

Ile Phe His Asp Glu Leu
        195
```

<210> 19
<211> 317
<212> PRT
<213> Aeromonas salmonicida

<400> 19

```
Ala Asp Thr Arg Pro Ala Phe Ser Arg Ile Val Met Phe Gly Asp Ser
1               5                   10                  15

Leu Ser Asp Thr Gly Lys Met Tyr Ser Lys Met Arg Gly Tyr Leu Pro
            20                  25                  30

Ser Ser Pro Pro Tyr Tyr Glu Gly Arg Phe Ser Asn Gly Pro Val Trp
        35                  40                  45

Leu Glu Gln Leu Thr Lys Gln Phe Pro Gly Leu Thr Ile Ala Asn Glu
    50                  55                  60

Ala Glu Gly Gly Ala Thr Ala Val Ala Tyr Asn Lys Ile Ser Trp Asn
65                  70                  75                  80

Pro Lys Tyr Gln Val Tyr Asn Asn Leu Asp Tyr Glu Val Thr Gln Phe
                85                  90                  95

Leu Gln Lys Asp Ser Phe Lys Pro Asp Asp Leu Val Ile Leu Trp Val
            100                 105                 110
```

```
Gly Ala Asn Asp Tyr Leu Ala Tyr Gly Trp Asn Thr Glu Gln Asp Ala
        115                 120                 125

Lys Arg Val Arg Asp Ala Ile Ser Asp Ala Ala Asn Arg Met Val Leu
    130                 135                 140

Asn Gly Ala Lys Gln Ile Leu Leu Phe Asn Leu Pro Asp Leu Gly Gln
145                 150                 155                 160

Asn Pro Ser Ala Arg Ser Gln Lys Val Val Glu Ala Val Ser His Val
                165                 170                 175

Ser Ala Tyr His Asn Lys Leu Leu Leu Asn Leu Ala Arg Gln Leu Ala
            180                 185                 190

Pro Thr Gly Met Val Lys Leu Phe Glu Ile Asp Lys Gln Phe Ala Glu
        195                 200                 205

Met Leu Arg Asp Pro Gln Asn Phe Gly Leu Ser Asp Val Glu Asn Pro
    210                 215                 220

Cys Tyr Asp Gly Gly Tyr Val Trp Lys Pro Phe Ala Thr Arg Ser Val
225                 230                 235                 240

Ser Thr Asp Arg Gln Leu Ser Ala Phe Ser Pro Gln Glu Arg Leu Ala
                245                 250                 255

Ile Ala Gly Asn Pro Leu Leu Ala Gln Ala Val Ala Ser Pro Met Ala
            260                 265                 270

Arg Arg Ser Ala Ser Pro Leu Asn Cys Glu Gly Lys Met Phe Trp Asp
        275                 280                 285

Gln Val His Pro Thr Thr Val Val His Ala Ala Leu Ser Glu Arg Ala
    290                 295                 300

Ala Thr Phe Ile Glu Thr Gln Tyr Glu Phe Leu Ala His
305                 310                 315
```

<210> 20
<211> 261
<212> PRT
<213> Streptomyces coelicolor

<400> 20

```
Met Ile Gly Ser Tyr Val Ala Val Gly Asp Ser Phe Thr Glu Gly Val
1               5                   10                  15

Gly Asp Pro Gly Pro Asp Gly Ala Phe Val Gly Trp Ala Asp Arg Leu
```

```
                20                  25                  30

Ala Val Leu Leu Ala Asp Arg Arg Pro Glu Gly Asp Phe Thr Tyr Thr
        35                  40                  45

Asn Leu Ala Val Arg Gly Arg Leu Leu Asp Gln Ile Val Ala Glu Gln
    50                  55                  60

Val Pro Arg Val Val Gly Leu Ala Pro Asp Leu Val Ser Phe Ala Ala
65                  70                  75                  80

Gly Gly Asn Asp Ile Ile Arg Pro Gly Thr Asp Pro Asp Glu Val Ala
                85                  90                  95

Glu Arg Phe Glu Leu Ala Val Ala Ala Leu Thr Ala Ala Ala Gly Thr
            100                 105                 110

Val Leu Val Thr Thr Gly Phe Asp Thr Arg Gly Val Pro Val Leu Lys
        115                 120                 125

His Leu Arg Gly Lys Ile Ala Thr Tyr Asn Gly His Val Arg Ala Ile
    130                 135                 140

Ala Asp Arg Tyr Gly Cys Pro Val Leu Asp Leu Trp Ser Leu Arg Ser
145                 150                 155                 160

Val Gln Asp Arg Arg Ala Trp Asp Ala Asp Arg Leu His Leu Ser Pro
                165                 170                 175

Glu Gly His Thr Arg Val Ala Leu Arg Ala Gly Gln Ala Leu Gly Leu
            180                 185                 190

Arg Val Pro Ala Asp Pro Asp Gln Pro Trp Pro Pro Leu Pro Pro Arg
        195                 200                 205

Gly Thr Leu Asp Val Arg Arg Asp Asp Val His Trp Ala Arg Glu Tyr
    210                 215                 220

Leu Val Pro Trp Ile Gly Arg Arg Leu Arg Gly Glu Ser Ser Gly Asp
225                 230                 235                 240

His Val Thr Ala Lys Gly Thr Leu Ser Pro Asp Ala Ile Lys Thr Arg
                245                 250                 255

Ile Ala Ala Val Ala
            260
```

<210> 21
<211> 786
<212> DNA

<213> Streptomyces coelicolor

<400> 21

```
gtgatcgggt cgtacgtggc ggtgggggac agcttcaccg agggcgtcgg cgaccccggc      60
cccgacgggg cgttcgtcgg ctgggccgac cggctcgccg tactgctcgc ggaccggcgc     120
cccgagggcg acttcacgta cacgaacctc gccgtgcgcg gcaggctcct cgaccagatc     180
gtggcggaac aggtcccgcg ggtcgtcgga ctcgcgcccg acctcgtctc gttcgcggcg     240
ggcggcaacg acatcatccg gcccggcacc gatcccgacg aggtcgccga gcggttcgag     300
ctggcggtgg ccgcgctgac cgccgcggcc ggaaccgtcc tggtgaccac cgggttcgac     360
acccgggggg tgcccgtcct caagcacctg cgcggcaaga tcgccacgta caacgggcac     420
gtccgcgcca tcgccgaccg ctacggctgc ccggtgctcg acctgtggtc gctgcggagc     480
gtccaggacc gcagggcgtg ggacgccgac cggctgcacc tgtcgccgga ggggcacacc     540
cgggtggcgc tgcgcgcggg gcaggccctg ggcctgcgcg tcccggccga ccctgaccag     600
ccctggccgc ccctgccgcc gcgcggcacg ctcgacgtcc ggcgcgacga cgtgcactgg     660
gcgcgcgagt acctggtgcc gtggatcggg cgccggctgc ggggcgagtc gtcgggcgac     720
cacgtgacgg ccaaggggac gctgtcgccg gacgccatca agacgcggat cgccgcggtg     780
gcctga                                                                786
```

<210> 22
<211> 260
<212> PRT
<213> Streptomyces coelicolor

<400> 22

```
Met Gln Thr Asn Pro Ala Tyr Thr Ser Leu Val Ala Val Gly Asp Ser
1               5                   10                  15

Phe Thr Glu Gly Met Ser Asp Leu Leu Pro Asp Gly Ser Tyr Arg Gly
            20                  25                  30

Trp Ala Asp Leu Leu Ala Thr Arg Met Ala Ala Arg Ser Pro Gly Phe
        35                  40                  45

Arg Tyr Ala Asn Leu Ala Val Arg Gly Lys Leu Ile Gly Gln Ile Val
    50                  55                  60

Asp Glu Gln Val Asp Val Ala Ala Ala Met Gly Ala Asp Val Ile Thr
65                  70                  75                  80

Leu Val Gly Gly Leu Asn Asp Thr Leu Arg Pro Lys Cys Asp Met Ala
                85                  90                  95
```

Arg Val Arg Asp Leu Leu Thr Gln Ala Val Glu Arg Leu Ala Pro His
100 105 110

Cys Glu Gln Leu Val Leu Met Arg Ser Pro Gly Arg Gln Gly Pro Val
115 120 125

Leu Glu Arg Phe Arg Pro Arg Met Glu Ala Leu Phe Ala Val Ile Asp
130 135 140

Asp Leu Ala Gly Arg His Gly Ala Val Val Val Asp Leu Tyr Gly Ala
145 150 155 160

Gln Ser Leu Ala Asp Pro Arg Met Trp Asp Val Asp Arg Leu His Leu
165 170 175

Thr Ala Glu Gly His Arg Arg Val Ala Glu Ala Val Trp Gln Ser Leu
180 185 190

Gly His Glu Pro Glu Asp Pro Glu Trp His Ala Pro Ile Pro Ala Thr
195 200 205

Pro Pro Pro Gly Trp Val Thr Arg Arg Thr Ala Asp Val Arg Phe Ala
210 215 220

Arg Gln His Leu Leu Pro Trp Ile Gly Arg Arg Leu Thr Gly Arg Ser
225 230 235 240

Ser Gly Asp Gly Leu Pro Ala Lys Arg Pro Asp Leu Leu Pro Tyr Glu
245 250 255

Asp Pro Ala Arg
260

<210> 23
<211> 783
<212> DNA
<213> Streptomyces coelicolor

<400> 23

atgcagacga accccgcgta caccagtctc gtcgccgtcg gcgactcctt caccgagggc 60

atgtcggacc tgctgcccga cggctcctac cgtggctggg ccgacctcct cgccacccgg 120

atggcggccc gctcccccgg cttccggtac gccaacctgg cggtgcgcgg gaagctgatc 180

ggacagatcg tcgacgagca ggtggacgtg gccgccgcca tgggagccga cgtgatcacg 240

ctggtcggcg ggctcaacga cacgctgcgg cccaagtgcg acatggcccg ggtgcgggac 300

ctgctgaccc aggccgtgga acggctcgcc ccgcactgcg agcagctggt gctgatgcgc 360

agtcccggtc gccagggtcc ggtgctggag cgcttccggc cccgcatgga ggccctgttc 420

```
gccgtgatcg acgacctggc cgggcggcac ggcgccgtgg tcgtcgacct gtacggggcc      480
cagtcgctgg ccgaccctcg gatgtgggac gtggaccggc tgcacctgac cgccgagggc      540
caccgccggg tcgcggaggc ggtgtggcag tcgctcggcc acgagcccga ggaccccgag      600
tggcacgcgc cgatcccggc gacgccgccg ccggggtggg tgacgcgcag gaccgcggac      660
gtccggttcg cccggcagca cctgctgccc tggataggcc gcaggctgac cgggcgctcg      720
tccggggacg gcctgccggc caagcgcccg gacctgctgc cctacgagga ccccgcacgg      780
tga                                                                    783
```

<210> 24
<211> 454
<212> PRT
<213> Streptomyces coelicolor

<400> 24

```
Met Thr Arg Gly Arg Asp Gly Gly Ala Gly Ala Pro Pro Thr Lys His
1               5                   10                  15

Arg Ala Leu Leu Ala Ala Ile Val Thr Leu Ile Val Ala Ile Ser Ala
            20                  25                  30

Ala Ile Tyr Ala Gly Ala Ser Ala Asp Asp Gly Ser Arg Asp His Ala
        35                  40                  45

Leu Gln Ala Gly Gly Arg Leu Pro Arg Gly Asp Ala Ala Pro Ala Ser
    50                  55                  60

Thr Gly Ala Trp Val Gly Ala Trp Ala Thr Ala Pro Ala Ala Ala Glu
65                  70                  75                  80

Pro Gly Thr Glu Thr Thr Gly Leu Ala Gly Arg Ser Val Arg Asn Val
                85                  90                  95

Val His Thr Ser Val Gly Gly Thr Gly Ala Arg Ile Thr Leu Ser Asn
            100                 105                 110

Leu Tyr Gly Gln Ser Pro Leu Thr Val Thr His Ala Ser Ile Ala Leu
        115                 120                 125

Ala Ala Gly Pro Asp Thr Ala Ala Ala Ile Ala Asp Thr Met Arg Arg
    130                 135                 140

Leu Thr Phe Gly Gly Ser Ala Arg Val Ile Ile Pro Ala Gly Gly Gln
145                 150                 155                 160

Val Met Ser Asp Thr Ala Arg Leu Ala Ile Pro Tyr Gly Ala Asn Val
```

```
                165                 170                 175

Leu Val Thr Thr Tyr Ser Pro Ile Pro Ser Gly Pro Val Thr Tyr His
            180                 185                 190

Pro Gln Ala Arg Gln Thr Ser Tyr Leu Ala Asp Gly Asp Arg Thr Ala
        195                 200                 205

Asp Val Thr Ala Val Ala Tyr Thr Thr Pro Thr Pro Tyr Trp Arg Tyr
    210                 215                 220

Leu Thr Ala Leu Asp Val Leu Ser His Glu Ala Asp Gly Thr Val Val
225                 230                 235                 240

Ala Phe Gly Asp Ser Ile Thr Asp Gly Ala Arg Ser Gln Ser Asp Ala
                245                 250                 255

Asn His Arg Trp Thr Asp Val Leu Ala Ala Arg Leu His Glu Ala Ala
            260                 265                 270

Gly Asp Gly Arg Asp Thr Pro Arg Tyr Ser Val Val Asn Glu Gly Ile
        275                 280                 285

Ser Gly Asn Arg Leu Leu Thr Ser Arg Pro Gly Arg Pro Ala Asp Asn
    290                 295                 300

Pro Ser Gly Leu Ser Arg Phe Gln Arg Asp Val Leu Glu Arg Thr Asn
305                 310                 315                 320

Val Lys Ala Val Val Val Val Leu Gly Val Asn Asp Val Leu Asn Ser
                325                 330                 335

Pro Glu Leu Ala Asp Arg Asp Ala Ile Leu Thr Gly Leu Arg Thr Leu
            340                 345                 350

Val Asp Arg Ala His Ala Arg Gly Leu Arg Val Val Gly Ala Thr Ile
        355                 360                 365

Thr Pro Phe Gly Gly Tyr Gly Gly Tyr Thr Glu Ala Arg Glu Thr Met
    370                 375                 380

Arg Gln Glu Val Asn Glu Glu Ile Arg Ser Gly Arg Val Phe Asp Thr
385                 390                 395                 400

Val Val Asp Phe Asp Lys Ala Leu Arg Asp Pro Tyr Asp Pro Arg Arg
                405                 410                 415

Met Arg Ser Asp Tyr Asp Ser Gly Asp His Leu His Pro Gly Asp Lys
```

420 425 430

Gly Tyr Ala Arg Met Gly Ala Val Ile Asp Leu Ala Ala Leu Lys Gly
435 440 445

Ala Ala Pro Val Lys Ala
450

450

<210> 25
<211> 1365
<212> DNA
<213> Streptomyces coelicolor

<400> 25

```
atgacccggg gtcgtgacgg gggtgcgggg gcgcccccca ccaagcaccg tgccctgctc      60
gcggcgatcg tcaccctgat agtggcgatc tccgcggcca tatacgccgg agcgtccgcg     120
gacgacggca gcagggacca cgcgctgcag gccggaggcc gtctcccacg aggagacgcc     180
gcccccgcgt ccaccggtgc ctgggtgggc gcctgggcca ccgcaccggc cgcggccgag     240
ccgggcaccg agacgaccgg cctggcgggc cgctccgtgc gcaacgtcgt gcacacctcg     300
gtcggcggca ccggcgcgcg gatcaccctc tcgaacctgt acgggcagtc gccgctgacc     360
gtcacacacg cctcgatcgc cctggccgcc gggcccgaca ccgccgccgc gatcgccgac     420
accatgcgcc ggctcacctt cggcggcagc gcccgggtga tcatcccggc gggcggccag     480
gtgatgagcg acaccgcccg cctcgccatc ccctacgggg cgaacgtcct ggtcaccacg     540
tactccccca tcccgtccgg gccggtgacc taccatccgc aggcccggca gaccagctac     600
ctggccgacg gcgaccgcac ggcggacgtc accgccgtcg cgtacaccac ccccacgccc     660
tactggcgct acctgaccgc cctcgacgtg ctgagccacg aggccgacgg cacggtcgtg     720
gcgttcggcg actccatcac cgacggcgcc cgctcgcaga gcgacgccaa ccaccgctgg     780
accgacgtcc tcgccgcacg cctgcacgag gcggcgggcg acggccggga cacgccccgc     840
tacagcgtcg tcaacgaggg catcagcggc aaccggctcc tgaccagcag gccggggcgg     900
ccggccgaca acccgagcgg actgagccgg ttccagcggg acgtgctgga acgcaccaac     960
gtcaaggccg tcgtcgtcgt cctcggcgtc aacgacgtcc tgaacagccc ggaactcgcc    1020
gaccgcgacg ccatcctgac cggcctgcgc accctcgtcg accgggcgca cgcccgggga    1080
ctgcgggtcg tcggcgccac gatcacgccg ttcggcggct acggcggcta caccgaggcc    1140
cgcgagacga tgcggcagga ggtcaacgag gagatccgct ccggccgggt cttcgacacg    1200
gtcgtcgact tcgacaaggc cctgcgcgac ccgtacgacc cgcgccggat gcgctccgac    1260
tacgacagcg gcgaccacct gcaccccggc gacaaggggt acgcgcgcat gggcgcggtc    1320
atcgacctgg ccgcgctgaa gggcgcggcg ccggtcaagg cgtag                     1365
```

<210> 26
<211> 340
<212> PRT
<213> Streptomyces coelicolor

<400> 26

Met Thr Ser Met Ser Arg Ala Arg Val Ala Arg Arg Ile Ala Ala Gly
1               5                   10                  15

Ala Ala Tyr Gly Gly Gly Gly Ile Gly Leu Ala Gly Ala Ala Ala Val
            20                  25                  30

Gly Leu Val Val Ala Glu Val Gln Leu Ala Arg Arg Arg Val Gly Val
        35                  40                  45

Gly Thr Pro Thr Arg Val Pro Asn Ala Gln Gly Leu Tyr Gly Gly Thr
    50                  55                  60

Leu Pro Thr Ala Gly Asp Pro Pro Leu Arg Leu Met Met Leu Gly Asp
65                  70                  75                  80

Ser Thr Ala Ala Gly Gln Gly Val His Arg Ala Gly Gln Thr Pro Gly
                85                  90                  95

Ala Leu Leu Ala Ser Gly Leu Ala Ala Val Ala Glu Arg Pro Val Arg
            100                 105                 110

Leu Gly Ser Val Ala Gln Pro Gly Ala Cys Ser Asp Asp Leu Asp Arg
        115                 120                 125

Gln Val Ala Leu Val Leu Ala Glu Pro Asp Arg Val Pro Asp Ile Cys
    130                 135                 140

Val Ile Met Val Gly Ala Asn Asp Val Thr His Arg Met Pro Ala Thr
145                 150                 155                 160

Arg Ser Val Arg His Leu Ser Ser Ala Val Arg Arg Leu Arg Thr Ala
                165                 170                 175

Gly Ala Glu Val Val Val Gly Thr Cys Pro Asp Leu Gly Thr Ile Glu
            180                 185                 190

Arg Val Arg Gln Pro Leu Arg Trp Leu Ala Arg Arg Ala Ser Arg Gln
        195                 200                 205

Leu Ala Ala Ala Gln Thr Ile Gly Ala Val Glu Gln Gly Gly Arg Thr
    210                 215                 220

Val Ser Leu Gly Asp Leu Leu Gly Pro Glu Phe Ala Gln Asn Pro Arg

225 230 235 240

Glu Leu Phe Gly Pro Asp Asn Tyr His Pro Ser Ala Glu Gly Tyr Ala
245 250 255

Thr Ala Ala Met Ala Val Leu Pro Ser Val Cys Ala Ala Leu Gly Leu
260 265 270

Trp Pro Ala Asp Glu Glu His Pro Asp Ala Leu Arg Arg Glu Gly Phe
275 280 285

Leu Pro Val Ala Arg Ala Ala Ala Glu Ala Ala Ser Glu Ala Gly Thr
290 295 300

Glu Val Ala Ala Ala Met Pro Thr Gly Pro Arg Gly Pro Trp Ala Leu
305 310 315 320

Leu Lys Arg Arg Arg Arg Arg Arg Val Ser Glu Ala Glu Pro Ser Ser
325 330 335

Pro Ser Gly Val
340

<210> 27
<211> 1023
<212> DNA
<213> Streptomyces coelicolor

<400> 27

atgacgagca tgtcgagggc gagggtggcg cggcggatcg cggccggcgc ggcgtacggc 60
ggcggcggca tcggcctggc gggagcggcg gcggtcggtc tggtggtggc cgaggtgcag 120
ctggccagac gcagggtggg ggtgggcacg ccgacccggg tgccgaacgc gcagggactg 180
tacggcggca ccctgcccac ggccggcgac ccgccgctgc ggctgatgat gctgggcgac 240
tccacggccg ccgggcaggg cgtgcaccgg gccgggcaga cgccgggcgc gctgctggcg 300
tccgggctcg cggcggtggc ggagcggccg gtgcggctgg ggtcggtcgc ccagccgggg 360
gcgtgctcgg acgacctgga ccggcaggtg gcgctggtgc tcgccgagcc ggaccgggtg 420
cccgacatct gcgtgatcat ggtcggcgcc aacgacgtca cccaccggat gccggcgacc 480
cgctcggtgc ggcacctgtc ctcggcggta cggcggctgc gcacggccgg tgcggaggtg 540
gtggtcggca cctgtccgga cctgggcacg atcgagcggg tgcggcagcc gctgcgctgg 600
ctggcccggc gggcctcacg gcagctcgcg gcggcacaga ccatcggcgc cgtcgagcag 660
ggcgggcgca cggtgtcgct gggcgacctg ctgggtccgg agttcgcgca gaacccgcgg 720
gagctcttcg gccccgacaa ctaccacccc tccgccgagg ggtacgccac ggccgcgatg 780
gcggtactgc cctcggtgtg cgccgcgctc ggcctgtggc cggccgacga ggagcacccg 840

gacgcgctgc gccgcgaggg cttcctgccg gtggcgcgcg cggcggcgga ggcggcgtcc 900

gaggcgggta cggaggtcgc cgccgccatg cctacggggc ctcgggggcc ctgggcgctg 960

ctgaagcgcc ggagacggcg tcgggtgtcg gaggcggaac cgtccagccc gtccggcgtt 1020

tga 1023

<210> 28
<211> 305
<212> PRT
<213> Streptomyces coelicolor

<400> 28

Met Gly Arg Gly Thr Asp Gln Arg Thr Arg Tyr Gly Arg Arg Arg Ala
1               5                   10                  15

Arg Val Ala Leu Ala Ala Leu Thr Ala Ala Val Leu Gly Val Gly Val
            20                  25                  30

Ala Gly Cys Asp Ser Val Gly Gly Asp Ser Pro Ala Pro Ser Gly Ser
        35                  40                  45

Pro Ser Lys Arg Thr Arg Thr Ala Pro Ala Trp Asp Thr Ser Pro Ala
    50                  55                  60

Ser Val Ala Ala Val Gly Asp Ser Ile Thr Arg Gly Phe Asp Ala Cys
65                  70                  75                  80

Ala Val Leu Ser Asp Cys Pro Glu Val Ser Trp Ala Thr Gly Ser Ser
                85                  90                  95

Ala Lys Val Asp Ser Leu Ala Val Arg Leu Leu Gly Lys Ala Asp Ala
            100                 105                 110

Ala Glu His Ser Trp Asn Tyr Ala Val Thr Gly Ala Arg Met Ala Asp
        115                 120                 125

Leu Thr Ala Gln Val Thr Arg Ala Ala Gln Arg Glu Pro Glu Leu Val
    130                 135                 140

Ala Val Met Ala Gly Ala Asn Asp Ala Cys Arg Ser Thr Thr Ser Ala
145                 150                 155                 160

Met Thr Pro Val Ala Asp Phe Arg Ala Gln Phe Glu Glu Ala Met Ala
                165                 170                 175

Thr Leu Arg Lys Lys Leu Pro Lys Ala Gln Val Tyr Val Ser Ser Ile
            180                 185                 190

Pro Asp Leu Lys Arg Leu Trp Ser Gln Gly Arg Thr Asn Pro Leu Gly
195 200 205

Lys Gln Val Trp Lys Leu Gly Leu Cys Pro Ser Met Leu Gly Asp Ala
210 215 220

Asp Ser Leu Asp Ser Ala Ala Thr Leu Arg Arg Asn Thr Val Arg Asp
225 230 235 240

Arg Val Ala Asp Tyr Asn Glu Val Leu Arg Glu Val Cys Ala Lys Asp
245 250 255

Arg Arg Cys Arg Ser Asp Asp Gly Ala Val His Glu Phe Arg Phe Gly
260 265 270

Thr Asp Gln Leu Ser His Trp Asp Trp Phe His Pro Ser Val Asp Gly
275 280 285

Gln Ala Arg Leu Ala Glu Ile Ala Tyr Arg Ala Val Thr Ala Lys Asn
290 295 300

Pro
305

<210> 29
<211> 918
<212> DNA
<213> Streptomyces coelicolor

<400> 29

atgggtcgag ggacggacca gcggacgcgg tacggccgtc gccgggcgcg tgtcgcgctc 60
gccgccctga ccgccgccgt cctgggcgtg ggcgtggcgg gctgcgactc cgtgggcggc 120
gactcacccg ctccttccgg cagcccgtcg aagcggacga ggacggcgcc cgcctgggac 180
accagcccgg cgtccgtcgc cgccgtgggc gactccatca cgcgcggctt cgacgcctgt 240
gcggtgctgt cggactgccc ggaggtgtcg tgggcgaccg gcagcagcgc gaaggtcgac 300
tcgctggccg tacggctgct ggggaaggcg gacgcggccg agcacagctg gaactacgcg 360
gtcaccgggg cccggatggc ggacctgacc gctcaggtga cgcgggcggc gcagcgcgag 420
ccggagctgg tggcggtgat ggccggggcg aacgacgcgt gccggtccac gacctcggcg 480
atgacgccgg tggcggactt ccgggcgcag ttcgaggagg cgatggccac cctgcgcaag 540
aagctcccca aggcgcaggt gtacgtgtcg agcatcccgg acctcaagcg gctctggtcc 600
cagggccgca ccaacccgct gggcaagcag gtgtggaagc tcggcctgtg cccgtcgatg 660
ctgggcgacg cggactccct ggactcggcg gcgaccctgc ggcgcaacac ggtgcgcgac 720
cgggtggcgg actacaacga ggtgctgcgg gaggtctgcg cgaaggaccg gcggtgccgc 780

agcgacgacg gcgcggtgca cgagttccgg ttcggcacgg accagttgag ccactgggac 840

tggttccacc cgagtgtgga cggccaggcc cggctggcgg agatcgccta ccgcgcggtc 900

accgcgaaga atccctga 918

<210> 30
<211> 268
<212> PRT
<213> Streptomyces rimosus

<400> 30

```
Met Arg Leu Ser Arg Arg Ala Ala Thr Ala Ser Ala Leu Leu Leu Thr
1               5                   10                  15

Pro Ala Leu Ala Leu Phe Gly Ala Ser Ala Ala Val Ser Ala Pro Arg
            20                  25                  30

Ile Gln Ala Thr Asp Tyr Val Ala Leu Gly Asp Ser Tyr Ser Ser Gly
        35                  40                  45

Val Gly Ala Gly Ser Tyr Asp Ser Ser Ser Gly Ser Cys Lys Arg Ser
    50                  55                  60

Thr Lys Ser Tyr Pro Ala Leu Trp Ala Ala Ser His Thr Gly Thr Arg
65                  70                  75                  80

Phe Asn Phe Thr Ala Cys Ser Gly Ala Arg Thr Gly Asp Val Leu Ala
                85                  90                  95

Lys Gln Leu Thr Pro Val Asn Ser Gly Thr Asp Leu Val Ser Ile Thr
            100                 105                 110

Ile Gly Gly Asn Asp Ala Gly Phe Ala Asp Thr Met Thr Thr Cys Asn
        115                 120                 125

Leu Gln Gly Glu Ser Ala Cys Leu Ala Arg Ile Ala Lys Ala Arg Ala
    130                 135                 140

Tyr Ile Gln Gln Thr Leu Pro Ala Gln Leu Asp Gln Val Tyr Asp Ala
145                 150                 155                 160

Ile Asp Ser Arg Ala Pro Ala Ala Gln Val Val Val Leu Gly Tyr Pro
                165                 170                 175

Arg Phe Tyr Lys Leu Gly Gly Ser Cys Ala Val Gly Leu Ser Glu Lys
            180                 185                 190

Ser Arg Ala Ala Ile Asn Ala Ala Ala Asp Asp Ile Asn Ala Val Thr
```

```
            195                 200                 205

Ala Lys Arg Ala Ala Asp His Gly Phe Ala Phe Gly Asp Val Asn Thr
    210                 215                 220

Thr Phe Ala Gly His Glu Leu Cys Ser Gly Ala Pro Trp Leu His Ser
225                 230                 235                 240

Val Thr Leu Pro Val Glu Asn Ser Tyr His Pro Thr Ala Asn Gly Gln
                245                 250                 255

Ser Lys Gly Tyr Leu Pro Val Leu Asn Ser Ala Thr
            260                 265
```

<210> 31
<211> 1068
<212> DNA
<213> Streptomyces rimosus

<400> 31

```
ttcatcacaa cgatgtcaca acaccggcca tccgggtcat ccctgatcgt gggaatgggt      60
gacaagcctt cccgtgacga aagggtcctg ctacatcaga aatgacagaa atcctgctca     120
gggaggttcc atgagactgt cccgacgcgc ggccacggcg tccgcgctcc tcctcacccc     180
ggcgctcgcg ctcttcggcg cgagcgccgc cgtgtccgcg ccgcgaatcc aggccaccga     240
ctacgtggcc ctcggcgact cctactcctc ggggtcggc gcgggcagct acgacagcag     300
cagtggctcc tgtaagcgca gcaccaagtc ctacccggcc ctgtgggccg cctcgcacac     360
cggtacgcgg ttcaacttca ccgcctgttc gggcgcccgc acaggagacg tgctggccaa     420
gcagctgacc ccggtcaact ccggcaccga cctggtcagc attaccatcg gcggcaacga     480
cgcgggcttc gccgacacca tgaccacctg caacctccag ggcgagagcg cgtgcctggc     540
gcggatcgcc aaggcgcgcg cctacatcca gcagacgctg cccgcccagc tggaccaggt     600
ctacgacgcc atcgacagcc gggcccccgc agcccaggtc gtcgtcctgg gctacccgcg     660
cttctacaag ctgggcggca gctgcgccgt cggtctctcg gagaagtccc gcgcggccat     720
caacgccgcc gccgacgaca tcaacgccgt caccgccaag cgcgccgccg accacggctt     780
cgccttcggg gacgtcaaca cgaccttcgc cgggcacgag ctgtgctccg gcgccccctg     840
gctgcacagc gtcacccttc ccgtggagaa ctcctaccac cccacggcca acggacagtc     900
caagggctac ctgcccgtcc tgaactccgc cacctgatct cgcggctact ccgcccctga     960
cgaagtcccg cccccgggcg gggcttcgcc gtaggtgcgc gtaccgccgt cgcccgtcgc    1020
gccggtggcc ccgccgtacg tgccgccgcc cccggacgcg gtcggttc                 1068
```

<210> 32
<211> 335
<212> PRT

<213> Aeromonas hydrophila

<400> 32

```
Met Lys Lys Trp Phe Val Cys Leu Leu Gly Leu Val Ala Leu Thr Val
1               5                   10                  15

Gln Ala Ala Asp Ser Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Lys Gln Phe Pro Gly Leu Thr Ile Ala
65                  70                  75                  80

Asn Glu Ala Glu Gly Gly Ala Thr Ala Val Ala Tyr Asn Lys Ile Ser
                85                  90                  95

Trp Asn Pro Lys Tyr Gln Val Ile Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160

Val Leu Asn Gly Ala Lys Gln Ile Leu Leu Phe Asn Leu Pro Asp Leu
                165                 170                 175

Gly Gln Asn Pro Ser Ala Arg Ser Gln Lys Val Val Glu Ala Val Ser
            180                 185                 190

His Val Ser Ala Tyr His Asn Gln Leu Leu Leu Asn Leu Ala Arg Gln
        195                 200                 205

Leu Ala Pro Thr Gly Met Val Lys Leu Phe Glu Ile Asp Lys Gln Phe
    210                 215                 220

Ala Glu Met Leu Arg Asp Pro Gln Asn Phe Gly Leu Ser Asp Val Glu
225                 230                 235                 240
```

```
Asn Pro Cys Tyr Asp Gly Gly Tyr Val Trp Lys Pro Phe Ala Thr Arg
                245                 250                 255

Ser Val Ser Thr Asp Arg Gln Leu Ser Ala Phe Ser Pro Gln Glu Arg
            260                 265                 270

Leu Ala Ile Ala Gly Asn Pro Leu Leu Ala Gln Ala Val Ala Ser Pro
        275                 280                 285

Met Ala Arg Arg Ser Ala Ser Pro Leu Asn Cys Glu Gly Lys Met Phe
    290                 295                 300

Trp Asp Gln Val His Pro Thr Thr Val Val His Ala Ala Leu Ser Glu
305                 310                 315                 320

Arg Ala Ala Thr Phe Ile Ala Asn Gln Tyr Glu Phe Leu Ala His
                325                 330                 335
```

<210> 33
<211> 1008
<212> DNA
<213> Aeromonas hydrophila

<400> 33

```
atgaaaaaat ggtttgtgtg tttattggga ttggtcgcgc tgacagttca ggcagccgac      60
agtcgccccg ccttttcccg gatcgtgatg ttcggcgaca gcctctccga taccggcaaa     120
atgtacagca agatgcgcgg ttacctcccc tccagcccgc cctactatga gggccgtttc     180
tccaacggac ccgtctggct ggagcagctg accaaacagt tcccgggtct gaccatcgcc     240
aacgaagcgg aaggcggtgc cactgccgtg gcttacaaca agatctcctg gaatcccaag     300
tatcaggtca tcaacaacct ggactacgag gtcacccagt tcttgcagaa agacagcttc     360
aagccggacg atctggtgat cctctgggtc ggtgccaatg actatctggc ctatggctgg     420
aacacggagc aggatgccaa gcgggttcgc gatgccatca gcgatgcggc caaccgcatg     480
gtactgaacg gtgccaagca gatactgctg ttcaacctgc cggatctggg ccagaacccg     540
tcagctcgca gtcagaaggt ggtcgaggcg gtcagccatg tctccgccta tcacaaccag     600
ctgctgctga acctggcacg ccagctggcc cccaccggca tggtaaagct gttcgagatc     660
gacaagcaat ttgccgagat gctgcgtgat ccgcagaact tcggcctgag cgacgtcgag     720
aacccctgct acgacggcgg ctatgtgtgg aagccgtttg ccacccgcag cgtcagcacc     780
gaccgccagc tctccgcctt cagtccgcag gaacgcctcg ccatcgccgg caacccgctg     840
ctggcacagg ccgttgccag tcctatggcc cgccgcagcg ccagcccccct caactgtgag     900
ggcaagatgt tctgggatca ggtacacccg accactgtcg tgcacgcagc cctgagcgag     960
cgcgccgcca ccttcatcgc gaaccagtac gagttcctcg cccactga                 1008
```

<210> 34
<211> 336
<212> PRT
<213> Aeromonas salmonicida subsp. Salmonicida

<400> 34

```
Met Lys Lys Trp Phe Val Cys Leu Leu Gly Leu Ile Ala Leu Thr Val
1               5                   10                  15

Gln Ala Ala Asp Thr Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Lys Gln Phe Pro Gly Leu Thr Ile Ala
65                  70                  75                  80

Asn Glu Ala Glu Gly Gly Ala Thr Ala Val Ala Tyr Asn Lys Ile Ser
                85                  90                  95

Trp Asn Pro Lys Tyr Gln Val Ile Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160

Val Leu Asn Gly Ala Lys Gln Ile Leu Leu Phe Asn Leu Pro Asp Leu
                165                 170                 175

Gly Gln Asn Pro Ser Ala Arg Ser Gln Lys Val Val Glu Ala Val Ser
            180                 185                 190

His Val Ser Ala Tyr His Asn Lys Leu Leu Leu Asn Leu Ala Arg Gln
        195                 200                 205

Leu Ala Pro Thr Gly Met Val Lys Leu Phe Glu Ile Asp Lys Gln Phe
    210                 215                 220
```

```
Ala Glu Met Leu Arg Asp Pro Gln Asn Phe Gly Leu Ser Asp Val Glu
225                 230                 235                 240

Asn Pro Cys Tyr Asp Gly Gly Tyr Val Trp Lys Pro Phe Ala Thr Arg
                245                 250                 255

Ser Val Ser Thr Asp Arg Gln Leu Ser Ala Phe Ser Pro Gln Glu Arg
            260                 265                 270

Leu Ala Ile Ala Gly Asn Pro Leu Leu Ala Gln Ala Val Ala Ser Pro
        275                 280                 285

Met Ala Arg Arg Ser Ala Ser Pro Leu Asn Cys Glu Gly Lys Met Phe
    290                 295                 300

Trp Asp Gln Val His Pro Thr Thr Val Val His Ala Ala Leu Ser Glu
305                 310                 315                 320

Arg Ala Ala Thr Phe Ile Glu Thr Gln Tyr Glu Phe Leu Ala His Gly
                325                 330                 335
```

<210> 35
<211> 1011
<212> DNA
<213> Aeromonas salmonicida subsp. Salmonicida

<400> 35

```
atgaaaaaat ggtttgtttg tttattgggg ttgatcgcgc tgacagttca ggcagccgac      60
actcgccccg ccttctcccg gatcgtgatg ttcggcgaca gcctctccga taccggcaaa     120
atgtacagca agatgcgcgg ttacctcccc tccagcccgc cctactatga gggccgtttc     180
tccaacggac ccgtctggct ggagcagctg accaagcagt tcccgggtct gaccatcgcc     240
aacgaagcgg aaggcggtgc cactgccgtg gcttacaaca agatctcctg gaatcccaag     300
tatcaggtca tcaacaacct ggactacgag gtcacccagt tcttgcagaa agacagcttc     360
aagccggacg atctggtgat cctctgggtc ggtgccaatg actatctggc atatggctgg     420
aatacggagc aggatgccaa gcgagttcgc gatgccatca gcgatgcggc caaccgcatg     480
gtactgaacg gtgccaagca gatactgctg ttcaacctgc cggatctggg ccagaacccg     540
tcagcccgca gtcagaaggt ggtcgaggcg gtcagccatg tctccgccta tcacaacaag     600
ctgctgctga acctggcacg ccagctggcc cccaccggca tggtaaagct gttcgagatc     660
gacaagcaat ttgccgagat gctgcgtgat ccgcagaact tcggcctgag cgacgtcgag     720
aacccctgct acgacggcgg ctatgtgtgg aagccgtttg ccacccgcag cgtcagcacc     780
gaccgccagc tctccgcctt cagtccgcag gaacgcctcg ccatcgccgg caacccgctg     840
ctggcacagg ccgttgccag tcctatggcc cgccgcagcg ccagccccct caactgtgag     900
```

ggcaagatgt tctgggatca ggtacacccg accactgtcg tgcacgcagc cctgagcgag 960

cgcgccgcca ccttcatcga gacccagtac gagttcctcg cccacggatg a 1011

<210> 36
<211> 347
<212> PRT
<213> Artificial Sequence

<220>
<223> Fusion construct

<400> 36

```
Met Phe Lys Phe Lys Lys Asn Phe Leu Val Gly Leu Ser Ala Ala Leu
1               5                   10                  15

Met Ser Ile Ser Leu Phe Ser Ala Thr Ala Ser Ala Ala Ser Ala Asp
            20                  25                  30

Ser Arg Pro Ala Phe Ser Arg Ile Val Met Phe Gly Asp Ser Leu Ser
        35                  40                  45

Asp Thr Gly Lys Met Tyr Ser Lys Met Arg Gly Tyr Leu Pro Ser Ser
    50                  55                  60

Pro Pro Tyr Tyr Glu Gly Arg Phe Ser Asn Gly Pro Val Trp Leu Glu
65                  70                  75                  80

Gln Leu Thr Lys Gln Phe Pro Gly Leu Thr Ile Ala Asn Glu Ala Glu
                85                  90                  95

Gly Gly Ala Thr Ala Val Ala Tyr Asn Lys Ile Ser Trp Asn Pro Lys
            100                 105                 110

Tyr Gln Val Ile Asn Asn Leu Asp Tyr Glu Val Thr Gln Phe Leu Gln
        115                 120                 125

Lys Asp Ser Phe Lys Pro Asp Asp Leu Val Ile Leu Trp Val Gly Ala
    130                 135                 140

Asn Asp Tyr Leu Ala Tyr Gly Trp Asn Thr Glu Gln Asp Ala Lys Arg
145                 150                 155                 160

Val Arg Asp Ala Ile Ser Asp Ala Ala Asn Arg Met Val Leu Asn Gly
                165                 170                 175

Ala Lys Gln Ile Leu Leu Phe Asn Leu Pro Asp Leu Gly Gln Asn Pro
            180                 185                 190
```

```
Ser Ala Arg Ser Gln Lys Val Val Glu Ala Val Ser His Val Ser Ala
        195                 200                 205

Tyr His Asn Gln Leu Leu Leu Asn Leu Ala Arg Gln Leu Ala Pro Thr
    210                 215                 220

Gly Met Val Lys Leu Phe Glu Ile Asp Lys Gln Phe Ala Glu Met Leu
225                 230                 235                 240

Arg Asp Pro Gln Asn Phe Gly Leu Ser Asp Val Glu Asn Pro Cys Tyr
                245                 250                 255

Asp Gly Gly Tyr Val Trp Lys Pro Phe Ala Thr Arg Ser Val Ser Thr
            260                 265                 270

Asp Arg Gln Leu Ser Ala Phe Ser Pro Gln Glu Arg Leu Ala Ile Ala
        275                 280                 285

Gly Asn Pro Leu Leu Ala Gln Ala Val Ala Ser Pro Met Ala Arg Arg
    290                 295                 300

Ser Ala Ser Pro Leu Asn Cys Glu Gly Lys Met Phe Trp Asp Gln Val
305                 310                 315                 320

His Pro Thr Thr Val Val His Ala Ala Leu Ser Glu Arg Ala Ala Thr
                325                 330                 335

Phe Ile Ala Asn Gln Tyr Glu Phe Leu Ala His
            340                 345
```

<210> 37
<211> 27
<212> DNA
<213> Artificial Sequence

<220>
<223> Primer

<400> 37
gtgatggtgg gcgaggaact cgtactg 27

<210> 38
<211> 35
<212> DNA
<213> Artificial Sequence

<220>
<223> Primer

<400> 38
agcatatgaa aaaatggttt gtttgtttat tgggg 35

<210> 39

<211> 39
<212> DNA
<213> Artificial Sequence

<220>
<223> Primer

<400> 39
ttggatccga attcatcaat ggtgatggtg atggtgggc 39

<210> 40
<211> 18
<212> DNA
<213> Artificial Sequence

<220>
<223> Primer

<400> 40
taatacgact cactatag 18

<210> 41
<211> 18
<212> DNA
<213> Artificial Sequence

<220>
<223> Primer

<400> 41
ctagttattg ctcagcgg 18

<210> 42
<211> 41
<212> DNA
<213> Artificial Sequence

<220>
<223> Primer

<400> 42
gtcatatgaa aaaatggttt gtgtgtttat tgggattggt c 41

<210> 43
<211> 30
<212> DNA
<213> Artificial Sequence

<220>
<223> Primer

<400> 43
atggtgatgg tgggcgagga actcgtactg 30

<210> 44
<211> 41
<212> DNA
<213> Artificial Sequence

<220>
<223> Primer

<400> 44
gtcatatgaa aaaatggttt gtgtgtttat tgggattggt c 41

<210> 45
<211> 39
<212> DNA
<213> Artificial Sequence

<220>
<223> Primer

<400> 45
ttggatccga attcatcaat ggtgatggtg atggtgggc 39

<210> 46
<211> 26
<212> DNA
<213> Artificial Sequence

<220>
<223> Primer

<400> 46
atgccatggc cgacagccgt cccgcc 26

<210> 47
<211> 27
<212> DNA
<213> Artificial Sequence

<220>
<223> Primer

<400> 47
ttggatccga attcatcaat ggtgatg 27

<210> 48
<211> 26
<212> DNA
<213> Artificial Sequence

<220>
<223> Primer

<400> 48
ttgctagcgc cgacagccgt cccgcc 26

<210> 49
<211> 27
<212> DNA
<213> Artificial Sequence

<220>
<223> Primer

<400> 49
ttggatccga attcatcaat ggtgatg 27

<210> 50
<211> 26
<212> DNA
<213> Artificial Sequence

<220>
<223> Primer

<400> 50
ttgccatggc cgacactcgc cccgcc 26

<210> 51
<211> 27
<212> DNA
<213> Artificial Sequence

<220>
<223> Primer

<400> 51
ttggatccga attcatcaat ggtgatg 27

<210> 52
<211> 26
<212> DNA
<213> Artificial Sequence

<220>
<223> Primer

<400> 52
ttgctagcgc cgacactcgc cccgcc 26

<210> 53
<211> 27
<212> DNA
<213> Artificial Sequence

<220>
<223> Primer

<400> 53
ttggatccga attcatcaat ggtgatg 27

<210> 54
<211> 1047
<212> DNA
<213> Aeromonas hydrophila

<400> 54

```
atgtttaagt ttaaaaagaa tttcttagtt ggattatcgg cagctttaat gagtattagc      60
ttgttttcgg caaccgcctc tgcagctagc gccgacagcc gtcccgcctt ttcccggatc     120
gtgatgttcg gcgacagcct ctccgatacc ggcaaaatgt acagcaagat gcgcggttac     180
ctcccctcca gcccgcccta ctatgagggc cgtttctcca acggacccgt ctggctggag     240
cagctgacca aacagttccc gggtctgacc atcgccaacg aagcggaagg cggtgccact     300
gccgtggctt acaacaagat ctcctggaat cccaagtatc aggtcatcaa caacctggac     360
tacgaggtca cccagttctt gcagaaagac agcttcaagc cggacgatct ggtgatcctc     420
tgggtcggtg ccaatgacta tctggcctat ggctggaaca cggagcagga tgccaagcgg     480
gttcgcgatg ccatcagcga tgcggccaac cgcatggtac tgaacggtgc caagcagata     540
ctgctgttca acctgccgga tctgggccag aacccgtcag ctcgcagtca gaaggtggtc     600
gaggcggtca gccatgtctc cgcctatcac aaccagctgc tgctgaacct ggcacgccag     660
ctggccccca ccggcatggt aaagctgttc gagatcgaca agcaatttgc cgagatgctg     720
cgtgatccgc agaacttcgg cctgagcgac gtcgagaacc cctgctacga cggcggctat     780
gtgtggaagc cgtttgccac ccgcagcgtc agcaccgacc gccagctctc cgccttcagt     840
ccgcaggaac gcctcgccat cgccggcaac ccgctgctgg cacaggccgt tgccagtcct     900
atggcccgcc gcagcgccag ccccctcaac tgtgagggca agatgttctg ggatcaggta     960
cacccgacca ctgtcgtgca cgcagccctg agcgagcgcg ccgccacctt catcgcgaac    1020
cagtacgagt tcctcgccca ctgatga                                        1047
```

<210> 55
<211> 320
<212> PRT
<213> Ralstonia solanacearum

<400> 55

```
Gln Ser Gly Asn Pro Asn Val Ala Lys Val Gln Arg Met Val Val Phe
1               5                   10                  15

Gly Asp Ser Leu Ser Asp Ile Gly Thr Tyr Thr Pro Val Ala Gln Ala
            20                  25                  30

Val Gly Gly Gly Lys Phe Thr Thr Asn Pro Gly Pro Ile Trp Ala Glu
        35                  40                  45

Thr Val Ala Ala Gln Leu Gly Val Thr Leu Thr Pro Ala Val Met Gly
    50                  55                  60

Tyr Ala Thr Ser Val Gln Asn Cys Pro Lys Ala Gly Cys Phe Asp Tyr
65                  70                  75                  80

Ala Gln Gly Gly Ser Arg Val Thr Asp Pro Asn Gly Ile Gly His Asn
                85                  90                  95

Gly Gly Ala Gly Ala Leu Thr Tyr Pro Val Gln Gln Gln Leu Ala Asn
            100                 105                 110
```

Phe Tyr Ala Ala Ser Asn Asn Thr Phe Asn Gly Asn Asn Asp Val Val
115 120 125

Phe Val Leu Ala Gly Ser Asn Asp Ile Phe Phe Trp Thr Thr Ala Ala
130 135 140

Ala Thr Ser Gly Ser Gly Val Thr Pro Ala Ile Ala Thr Ala Gln Val
145 150 155 160

Gln Gln Ala Ala Thr Asp Leu Val Gly Tyr Val Lys Asp Met Ile Ala
165 170 175

Lys Gly Ala Thr Gln Val Tyr Val Phe Asn Leu Pro Asp Ser Ser Leu
180 185 190

Thr Pro Asp Gly Val Ala Ser Gly Thr Thr Gly Gln Ala Leu Leu His
195 200 205

Ala Leu Val Gly Thr Phe Asn Thr Thr Leu Gln Ser Gly Leu Ala Gly
210 215 220

Thr Ser Ala Arg Ile Ile Asp Phe Asn Ala Gln Leu Thr Ala Ala Ile
225 230 235 240

Gln Asn Gly Ala Ser Phe Gly Phe Ala Asn Thr Ser Ala Arg Ala Cys
245 250 255

Asp Ala Thr Lys Ile Asn Ala Leu Val Pro Ser Ala Gly Gly Ser Ser
260 265 270

Leu Phe Cys Ser Ala Asn Thr Leu Val Ala Ser Gly Ala Asp Gln Ser
275 280 285

Tyr Leu Phe Ala Asp Gly Val His Pro Thr Thr Ala Gly His Arg Leu
290 295 300

Ile Ala Ser Asn Val Leu Ala Arg Leu Leu Ala Asp Asn Val Ala His
305 310 315 320

<210> 56
<211> 226
<212> PRT
<213> Streptomyces rimosus

<400> 56

Tyr Val Ala Leu Gly Asp Ser Tyr Ser Ser Gly Val Gly Ala Gly Ser
1 5 10 15

Tyr Asp Ser Ser Ser Gly Ser Cys Lys Arg Ser Thr Lys Ser Tyr Pro
20 25 30

Ala Leu Trp Ala Ala Ser His Thr Gly Thr Arg Phe Asn Phe Thr Ala
35 40 45

Cys Ser Gly Ala Arg Thr Gly Asp Val Leu Ala Lys Gln Leu Thr Pro
50 55 60

Val Asn Ser Gly Thr Asp Leu Val Ser Ile Thr Ile Gly Gly Asn Asp
65 70 75 80

Ala Gly Phe Ala Asp Thr Met Thr Thr Cys Asn Leu Gln Gly Glu Ser
85 90 95

Ala Cys Leu Ala Arg Ile Ala Lys Ala Arg Ala Tyr Ile Gln Gln Thr
100 105 110

Leu Pro Ala Gln Leu Asp Gln Val Tyr Asp Ala Ile Asp Ser Arg Ala
115 120 125

Pro Ala Ala Gln Val Val Val Leu Gly Tyr Pro Arg Phe Tyr Lys Leu
130 135 140

Gly Gly Ser Cys Ala Val Gly Leu Ser Glu Lys Ser Arg Ala Ala Ile
145 150 155 160

Asn Ala Ala Ala Asp Asp Ile Asn Ala Val Thr Ala Lys Arg Ala Ala
165 170 175

Asp His Gly Phe Ala Phe Gly Asp Val Asn Thr Thr Phe Ala Gly His
180 185 190

Glu Leu Cys Ser Gly Ala Pro Trp Leu His Ser Val Thr Leu Pro Val
195 200 205

Glu Asn Ser Tyr His Pro Thr Ala Asn Gly Gln Ser Lys Gly Tyr Leu
210 215 220

Pro Val
225

<210> 57
<211> 182
<212> PRT
<213> Streptomyces coelicolor

<400> 57

Tyr Val Ala Val Gly Asp Ser Phe Thr Glu Gly Val Gly Asp Pro Gly
1 5 10 15

Pro Asp Gly Ala Phe Val Gly Trp Ala Asp Arg Leu Ala Val Leu Leu
20 25 30

Ala Asp Arg Arg Pro Glu Gly Asp Phe Thr Tyr Thr Asn Leu Ala Val
35 40 45

Arg Gly Arg Leu Leu Asp Gln Ile Val Ala Glu Gln Val Pro Arg Val
50 55 60

Val Gly Leu Ala Pro Asp Leu Val Ser Phe Ala Ala Gly Gly Asn Asp
65 70 75 80

Ile Ile Arg Pro Gly Thr Asp Pro Asp Glu Val Ala Glu Arg Phe Glu
85 90 95

Leu Ala Val Ala Ala Leu Thr Ala Ala Ala Gly Thr Val Leu Val Thr
100 105 110

Thr Gly Phe Asp Thr Arg Gly Val Pro Val Leu Lys His Leu Arg Gly
115 120 125

Lys Ile Ala Thr Tyr Asn Gly His Val Arg Ala Ile Ala Asp Arg Tyr
130 135 140

Gly Cys Pro Val Leu Asp Leu Trp Ser Leu Arg Ser Val Gln Asp Arg
145 150 155 160

Arg Ala Trp Asp Ala Asp Arg Leu His Leu Ser Pro Glu Gly His Thr
165 170 175

Arg Val Ala Leu Arg Ala
180

<210> 58
<211> 179
<212> PRT
<213> Streptomyces coelicolor

<400> 58

Leu Val Ala Val Gly Asp Ser Phe Thr Glu Gly Met Ser Asp Leu Leu
1 5 10 15

Pro Asp Gly Ser Tyr Arg Gly Trp Ala Asp Leu Leu Ala Thr Arg Met
20 25 30

Ala Ala Arg Ser Pro Gly Phe Arg Tyr Ala Asn Leu Ala Val Arg Gly

35 40 45

Lys Leu Ile Gly Gln Ile Val Asp Glu Gln Val Asp Val Ala Ala Ala
50 55 60

Met Gly Ala Asp Val Ile Thr Leu Val Gly Gly Leu Asn Asp Thr Leu
65 70 75 80

Arg Pro Lys Cys Asp Met Ala Arg Val Arg Asp Leu Leu Thr Gln Ala
85 90 95

Val Glu Arg Leu Ala Pro His Cys Glu Gln Leu Val Leu Met Arg Ser
100 105 110

Pro Gly Arg Gln Gly Pro Val Leu Glu Arg Phe Arg Pro Arg Met Glu
115 120 125

Ala Leu Phe Ala Val Ile Asp Asp Leu Ala Gly Arg His Gly Ala Val
130 135 140

Val Val Asp Leu Tyr Gly Ala Gln Ser Leu Ala Asp Pro Arg Met Trp
145 150 155 160

Asp Val Asp Arg Leu His Leu Thr Ala Glu Gly His Arg Arg Val Ala
165 170 175

Glu Ala Val

<210> 59
<211> 203
<212> PRT
<213> Streptomyces coelicolor

<400> 59

Val Val Ala Phe Gly Asp Ser Ile Thr Asp Gly Ala Arg Ser Gln Ser
1 5 10 15

Asp Ala Asn His Arg Trp Thr Asp Val Leu Ala Ala Arg Leu His Glu
20 25 30

Ala Ala Gly Asp Gly Arg Asp Thr Pro Arg Tyr Ser Val Val Asn Glu
35 40 45

Gly Ile Ser Gly Asn Arg Leu Leu Thr Ser Arg Pro Gly Arg Pro Ala
50 55 60

Asp Asn Pro Ser Gly Leu Ser Arg Phe Gln Arg Asp Val Leu Glu Arg
65 70 75 80

Thr Asn Val Lys Ala Val Val Val Val Leu Gly Val Asn Asp Val Leu
85 90 95

Asn Ser Pro Glu Leu Ala Asp Arg Asp Ala Ile Leu Thr Gly Leu Arg
100 105 110

Thr Leu Val Asp Arg Ala His Ala Arg Gly Leu Arg Val Val Gly Ala
115 120 125

Thr Ile Thr Pro Phe Gly Gly Tyr Gly Gly Tyr Thr Glu Ala Arg Glu
130 135 140

Thr Met Arg Gln Glu Val Asn Glu Glu Ile Arg Ser Gly Arg Val Phe
145 150 155 160

Asp Thr Val Val Asp Phe Asp Lys Ala Leu Arg Asp Pro Tyr Asp Pro
165 170 175

Arg Arg Met Arg Ser Asp Tyr Asp Ser Gly Asp His Leu His Pro Gly
180 185 190

Asp Lys Gly Tyr Ala Arg Met Gly Ala Val Ile
195 200

<210> 60
<211> 188
<212> PRT
<213> Streptomyces coelicolor

<400> 60

Leu Met Met Leu Gly Asp Ser Thr Ala Ala Gly Gln Gly Val His Arg
1 5 10 15

Ala Gly Gln Thr Pro Gly Ala Leu Leu Ala Ser Gly Leu Ala Ala Val
20 25 30

Ala Glu Arg Pro Val Arg Leu Gly Ser Val Ala Gln Pro Gly Ala Cys
35 40 45

Ser Asp Asp Leu Asp Arg Gln Val Ala Leu Val Leu Ala Glu Pro Asp
50 55 60

Arg Val Pro Asp Ile Cys Val Ile Met Val Gly Ala Asn Asp Val Thr
65 70 75 80

His Arg Met Pro Ala Thr Arg Ser Val Arg His Leu Ser Ser Ala Val
85 90 95

Arg Arg Leu Arg Thr Ala Gly Ala Glu Val Val Val Gly Thr Cys Pro
100 105 110

Asp Leu Gly Thr Ile Glu Arg Val Arg Gln Pro Leu Arg Trp Leu Ala
115 120 125

Arg Arg Ala Ser Arg Gln Leu Ala Ala Ala Gln Thr Ile Gly Ala Val
130 135 140

Glu Gln Gly Gly Arg Thr Val Ser Leu Gly Asp Leu Leu Gly Pro Glu
145 150 155 160

Phe Ala Gln Asn Pro Arg Glu Leu Phe Gly Pro Asp Asn Tyr His Pro
165 170 175

Ser Ala Glu Gly Tyr Ala Thr Ala Ala Met Ala Val
180 185

<210> 61
<211> 231
<212> PRT
<213> Streptomyces coelicolor

<400> 61

Val Ala Ala Val Gly Asp Ser Ile Thr Arg Gly Phe Asp Ala Cys Ala
1 5 10 15

Val Leu Ser Asp Cys Pro Glu Val Ser Trp Ala Thr Gly Ser Ser Ala
20 25 30

Lys Val Asp Ser Leu Ala Val Arg Leu Leu Gly Lys Ala Asp Ala Ala
35 40 45

Glu His Ser Trp Asn Tyr Ala Val Thr Gly Ala Arg Met Ala Asp Leu
50 55 60

Thr Ala Gln Val Thr Arg Ala Ala Gln Arg Glu Pro Glu Leu Val Ala
65 70 75 80

Val Met Ala Gly Ala Asn Asp Ala Cys Arg Ser Thr Thr Ser Ala Met
85 90 95

Thr Pro Val Ala Asp Phe Arg Ala Gln Phe Glu Glu Ala Met Ala Thr
100 105 110

Leu Arg Lys Lys Leu Pro Lys Ala Gln Val Tyr Val Ser Ser Ile Pro
115 120 125

Asp Leu Lys Arg Leu Trp Ser Gln Gly Arg Thr Asn Pro Leu Gly Lys
130 135 140

Gln Val Trp Lys Leu Gly Leu Cys Pro Ser Met Leu Gly Asp Ala Asp
145 150 155 160

Ser Leu Asp Ser Ala Ala Thr Leu Arg Arg Asn Thr Val Arg Asp Arg
165 170 175

Val Ala Asp Tyr Asn Glu Val Leu Arg Glu Val Cys Ala Lys Asp Arg
180 185 190

Arg Cys Arg Ser Asp Asp Gly Ala Val His Glu Phe Arg Phe Gly Thr
195 200 205

Asp Gln Leu Ser His Trp Asp Trp Phe His Pro Ser Val Asp Gly Gln
210 215 220

Ala Arg Leu Ala Glu Ile Ala
225 230

<210> 62
<211> 295
<212> PRT
<213> Aeromonas salmonicida subsp. Salmonicida

<400> 62

Ile Val Met Phe Gly Asp Ser Leu Ser Asp Thr Gly Lys Met Tyr Ser
1 5 10 15

Lys Met Arg Gly Tyr Leu Pro Ser Ser Pro Pro Tyr Tyr Glu Gly Arg
20 25 30

Phe Ser Asn Gly Pro Val Trp Leu Glu Gln Leu Thr Lys Gln Phe Pro
35 40 45

Gly Leu Thr Ile Ala Asn Glu Ala Glu Gly Gly Ala Thr Ala Val Ala
50 55 60

Tyr Asn Lys Ile Ser Trp Asn Pro Lys Tyr Gln Val Tyr Asn Asn Leu
65 70 75 80

Asp Tyr Glu Val Thr Gln Phe Leu Gln Lys Asp Ser Phe Lys Pro Asp
85 90 95

Asp Leu Val Ile Leu Trp Val Gly Ala Asn Asp Tyr Leu Ala Tyr Gly
100 105 110

Trp Asn Thr Glu Gln Asp Ala Lys Arg Val Arg Asp Ala Ile Ser Asp

115 120 125

Ala Ala Asn Arg Met Val Leu Asn Gly Ala Lys Gln Ile Leu Leu Phe
130 135 140

Asn Leu Pro Asp Leu Gly Gln Asn Pro Ser Ala Arg Ser Gln Lys Val
145 150 155 160

Val Glu Ala Val Ser His Val Ser Ala Tyr His Asn Lys Leu Leu Leu
165 170 175

Asn Leu Ala Arg Gln Leu Ala Pro Thr Gly Met Val Lys Leu Phe Glu
180 185 190

Ile Asp Lys Gln Phe Ala Glu Met Leu Arg Asp Pro Gln Asn Phe Gly
195 200 205

Leu Ser Asp Val Glu Asn Pro Cys Tyr Asp Gly Gly Tyr Val Trp Lys
210 215 220

Pro Phe Ala Thr Arg Ser Val Ser Thr Asp Arg Gln Leu Ser Ala Phe
225 230 235 240

Ser Pro Gln Glu Arg Leu Ala Ile Ala Gly Asn Pro Leu Leu Ala Gln
245 250 255

Ala Val Ala Ser Pro Met Ala Arg Arg Ser Ala Ser Pro Leu Asn Cys
260 265 270

Glu Gly Lys Met Phe Trp Asp Gln Val His Pro Thr Thr Val Val His
275 280 285

Ala Ala Leu Ser Glu Arg Ala
290 295

<210> 63
<211> 295
<212> PRT
<213> Aeromonas hydrophila

<400> 63

Ile Val Met Phe Gly Asp Ser Leu Ser Asp Thr Gly Lys Met Tyr Ser
1 5 10 15

Lys Met Arg Gly Tyr Leu Pro Ser Ser Pro Pro Tyr Tyr Glu Gly Arg
20 25 30

Phe Ser Asn Gly Pro Val Trp Leu Glu Gln Leu Thr Lys Gln Phe Pro
35 40 45

Gly Leu Thr Ile Ala Asn Glu Ala Glu Gly Gly Ala Thr Ala Val Ala
50 55 60

Tyr Asn Lys Ile Ser Trp Asn Pro Lys Tyr Gln Val Ile Asn Asn Leu
65 70 75 80

Asp Tyr Glu Val Thr Gln Phe Leu Gln Lys Asp Ser Phe Lys Pro Asp
85 90 95

Asp Leu Val Ile Leu Trp Val Gly Ala Asn Asp Tyr Leu Ala Tyr Gly
100 105 110

Trp Asn Thr Glu Gln Asp Ala Lys Arg Val Arg Asp Ala Ile Ser Asp
115 120 125

Ala Ala Asn Arg Met Val Leu Asn Gly Ala Lys Gln Ile Leu Leu Phe
130 135 140

Asn Leu Pro Asp Leu Gly Gln Asn Pro Ser Ala Arg Ser Gln Lys Val
145 150 155 160

Val Glu Ala Val Ser His Val Ser Ala Tyr His Asn Gln Leu Leu Leu
165 170 175

Asn Leu Ala Arg Gln Leu Ala Pro Thr Gly Met Val Lys Leu Phe Glu
180 185 190

Ile Asp Lys Gln Phe Ala Glu Met Leu Arg Asp Pro Gln Asn Phe Gly
195 200 205

Leu Ser Asp Val Glu Asn Pro Cys Tyr Asp Gly Gly Tyr Val Trp Lys
210 215 220

Pro Phe Ala Thr Arg Ser Val Ser Thr Asp Arg Gln Leu Ser Ala Phe
225 230 235 240

Ser Pro Gln Glu Arg Leu Ala Ile Ala Gly Asn Pro Leu Leu Ala Gln
245 250 255

Ala Val Ala Ser Pro Met Ala Arg Arg Ser Ala Ser Pro Leu Asn Cys
260 265 270

Glu Gly Lys Met Phe Trp Asp Gln Val His Pro Thr Thr Val Val His
275 280 285

Ala Ala Leu Ser Glu Arg Ala
290 295

<210> 64
<211> 4

<212> PRT
<213> Artificial Sequence

<220>
<223> Sequence motif

<220>
<221> MISC_FEATURE
<222> (4)..(4)
<223> Xaa may be one of the following amino acids residues Leu, Ala, Val, Ile, Phe, Tyr, His, Gln, Thr, Asn, Met or Ser.

<400> 64

```
Gly Asp Ser Xaa
1
```

<210> 65
<211> 8
<212> PRT
<213> Artificial Sequence

<220>
<223> Block 1 GDSX block

<220>
<221> MISC_FEATURE
<222> (1)..(4)
<223> Xaa is a hydrophobic residue selected from Met, Ile, Leu, Val, Ala, Gly, Cys, His, Lys, Trp, Tyr or Phe.

<220>
<221> MISC_FEATURE
<222> (8)..(8)
<223> Xaa is a hydrophobic residue selected from Met, Ile, Leu, Val, Ala, Gly, Cys, His, Lys, Trp, Tyr or Phe.

<400> 65

```
Xaa Xaa Xaa Xaa Gly Asp Ser Xaa
1               5
```

<210> 66
<211> 6
<212> PRT
<213> Artificial Sequence

<220>
<223> Block 2 GANDY block

<220>
<221> MISC_FEATURE
<222> (1)..(1)
<223> Xaa is a hydrophobic residue selected from Met, Ile, Leu, Val, Ala, Gly, Cys, His, Lys, Trp, Tyr or Phe.

<220>
<221> MISC_FEATURE
<222> (3)..(3)
<223> Xaa is a hydrophobic residue selected from Met, Ile, Leu, Val, Ala, Gly, Cys, His, Lys, Trp, Tyr or Phe.

<220>

<221> MISC_FEATURE
<222> (6)..(6)
<223> Xaa is a hydrophobic residue selected from Met, Ile, Leu, Val, Ala, Gly, Cys, His, Lys, Trp, Tyr or Phe.

<400> 66

```
Xaa Gly Xaa Asn Asp Xaa
1               5
```

<210> 67
<211> 5
<212> PRT
<213> Artificial Sequence

<220>
<223> Sequence motif

<400> 67

```
Gly Ala Asn Asp Tyr
1               5
```

<210> 68
<211> 35
<212> DNA
<213> Artificial Sequence

<220>
<223> Primer

<400> 68
agcatatgaa aaaatggttt gtttgtttat tgggg 35